# FASCICULE DE BREVET EUROPEEN

(11) **EP 1 858 918 B1**
(45) Date de publication et mention de la délivrance du brevet: **16.10.2013**
(21) Numéro de dépôt: 06743586.7
(22) Date de dépôt: 16.03.2006
(51) Int. Cl.: C12N 15/11, C07H 21/00, C07K 14/22, C12N 9/12, C07K 14/195, C12Q 1/68

(54) **FRAGMENTS D'ACIDES NUCLEIQUES ET METHODE DE DETECTION SPECIFIQUE PAR IDENTIFICATION MOLECULAIRE DE DIFFERENTES ESPECES DE BACTERIES DU GENRE ACINETOBACTER**
NUKLEINSÄUREFRAGMENTE UND SPEZIFISCHES DETEKTIONSVERFAHREN DURCH MOLEKULARIDENTIFIZIERUNG VERSCHIEDENER BAKTERIENSPEZIES DER ART ACINETOBACTER
NUCLEIC ACID FRAGMENTS AND SPECIFIC DETECTION METHOD BY MOLECULAR IDENTIFICATION OF DIFFERENT BACTERIA SPECIES OF THE GENUS ACINETOBACTER

(30) Priorité: 17.03.2005 FR 0502630
(43) Date de publication de la demande: 28.11.2007
(73) Titulaire: Université de la Méditerranée, Aix-Marseille II, 13284 Marseille Cedex 07 (FR); CENTRE NATIONAL DE LA RECHERCHE SCIENTIFIQUE (CNRS), 75794 Paris Cedex 16 (FR)
(72) Inventeur: LA SCOLA, Bernard, 13790 Rousset (FR); RAOULT, Didier, F-13008 Marseille (FR)
(74) Mandataire: Domange, Maxime
(86) Numéro de dépôt international: PCT/FR2006/000588
(87) Numéro de publication internationale: WO 2006/097636

(56) Documents cités:
- MOLLET C ET AL: "RPOB SEQUENCE ANALYSIS AS A NOVEL BASIS FOR BACTERIAL IDENTIFICATION" MOLECULAR MICROBIOLOGY, BLACKWELL SCIENTIFIC, OXFORD, GB, vol. 26, no. 5, 1997, pages 1005-1011, XP000913977 ISSN: 0950-382X

## Description

La présente invention concerne le domaine du diagnostic. Plus précisément, l'invention concerne une méthode pour l'identification moléculaire des bactéries du genre *Acinetobacter* par les techniques d'amplification et séquençage d'acides nucléiques à l'aide d'amorces oligonucléotidiques.

Les bactéries du genre *Acinetobacter* sont des bactéries apparaissant sous forme de cocco-bacilles Gram négatif, de croissance aérobie. On reconnaît actuellement presque 60 espèces et 2 sous espèces.

Ces bactéries sont essentiellement, mais pas uniquement, des agents d'infections nosocomiales. Leur spectre va de la simple colonisation à des infections mettant en jeu le pronostic vital, notamment des pneumonies, des infections urinaires, des bactériémies et des méningites (van Dessel et al., 2004). Leur excellente capacité de survie dans l'environnement extérieur de l'hôpital et leur capacité à développer rapidement des résistances à la majorité des antibiotiques sont des facteurs majeurs de leur émergence comme agent d'infections nosocomiales, notamment dans les unités de soin intensif (Bergogne-Berezin and Towner, 1996; Towner, 1997).

Bien que la plupart des espèces soient responsables d'infections chez l'homme, certaines espèces n'ont été isolées que dans l'environnement (Nemec et al., 2001, 2003; Carr et al., 2003).

Alors que, dans le genre *Acinetobacter,* 32 espèces ont été proposées (van Dessel et al., 2004), 24 espèces génomiques (" genomic species » selon la nomenclature en vigueur et abrégées ci-après « geno species » ou « g.sp. ») sont reconnues et seulement 17 espèces ont un nom validé (Nemec et al., 2003; Carr et al., 2003). Malheureusement, les membres de ce genre ont une très grande variabilité phénotypique intra-spécifique.et ne sont donc pas différentiables sur un plan phénotypique L'accroissement du nombre d'infections liées à ces agents a stimulé la recherche de méthodes d'analyse pour l'identification et la taxonomie de ces bactéries. Il a aussi été démontré que les méthodes d'identification basées sur les caractères phénotypiques et le séquençage du gène de l'ARN 16S ribosomique par comparaison à l'hybridation DNA-DNA n'étaient pas valables. Ainsi, la recherche de méthodes d'identification rapide de ces bactéries est toujours d'actualité (Gerner-Smidt et al., 1991; Ibrahim et al., 1997; Rainey et al., 1994). Notamment, les techniques moléculaires basées sur le séquençage du gène de l'ARN 16S ribosomique ne permettent pas de distinguer les espèces les plus proches, notamment en raison d'un manque de polymorphisme de ce gène dans ce genre. (Yamamoto and Harayama, 1998; Ochman and Wilson, 1987; Stackebrandt and Goebel, 1994). De plus, en raison de ce manque de polymorphisme, il y a nécessité de déterminer la séquence complète du gène 16S ARNr si l'on désire pouvoir identifier une espèce. Cela impose de séquencer la totalité du gène qui fait environ 1600 paires de bases. La conséquence pratique est que le séquençage doit s'appuyer sur un minimum de 6 réactions de séquençage en plus de la réaction d'amplification pour avoir un résultat évaluable. Des tentatives de phylogénies ont été faites en utilisant la comparaison des gènes *gyr*B (Yamamoto and Harayama, 1996; Yamamoto et al., 1999) et *rec*A (Krawczyk et al., 2002) en alternative au gène de l'ARN 16S ribosomique (Ibrahim et al., 1997). Malheureusement, les séquences de ces gènes n'ont pas été déterminées sur les 10 espèces les plus récentes (Nemec et al., 2001, 2003; Carr et al., 2003).

Il existe donc toujours une demande d'un outil d'identification moléculaire des bactéries des espèces du genre *Acinetobacter*, utilisable en routine au laboratoire de bactériologie, avec notamment un gène suffisamment polymorphique, tel que la détermination d'une séquence courte (moins de 500 paires de bases) avec seulement 1 réaction d'amplification et deux réactions de séquences soit identifiante, c'est-à-dire amplifiable et séquençable par l'utilisation d'un seul jeu d'amorces.

Les inventeurs ont découvert et démontré selon la présente invention, que
- le gène *rpo*B et les séquences non codantes qui le bornent, à savoir
- les séquences non codantes en 5' entre les séquences des gènes rp/L et rpoB (ci- après dénommées "spacer rp1L-rpoB " ou « fragment intergénique rp/L- rpoB »), et
- les séquences non codantes en 3' entre les séquences des gènes rpoC et rpoB (ci-après dénommées " spacer rpoB-rpoC " ou « fragment intergénique rpoB-rpoC »),
constituent un marqueur génétique permettant la détection et l'identification spécifique de la bactérie de chaque espèce du genre *Acinetobacter* et, en particulier, les 24 espèces suivantes : *A. calcoaceticus* (espèce génomique 1), *A. baumannii* (espèce génomique2), espèce génomique *3, A. haemolyticus* (espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, *A. radioresistens* (espèce génomique 12), espèce génomique 13, espèce génomique *16, A. schindleri, A. ursingii*, *A. baylyi, A. bouvetii*, *A. gerneri, A. grimontii, A. tandoii, A. tjernbergiae, A. towneri, A. parvus.*

Les espèces génomiques 1 à 16 ("genomic species ») correspondent à des souches référencées dans GENEBANK, pour certaines (n°3, 6, 9, 10, 13 et 16) aucun nom ne leur ayant encore été attribuées.

Les inventeurs ont découverts certaines zones hypervariables entre les différentes espèces et donc spécifiques de chaque espèce, encadrées par des séquences conservées entre les différentes espèces, permettant de mettre en oeuvre une méthode d'identification moléculaire des différentes espèces de bactéries Acinetobacter par amplification à l'aide d'amorces choisies dans les séquences conservées et hybridation et/ou séquençage des séquences spécifiques ainsi amplifiées.

Plus particulièrement, la présente invention concerne des séquences d'acides nucléiques spécifiques de chaque espèce du genre *Acinetobacter* citée ci-dessus dont la séquence nucléotidique est tirée du gène *rpo*B des dites bactéries.

Selon Lazcano et al. [J. Mol. Evol. (1988) 27:365-376], les ARN polymérases sont divisées en deux groupes selon leur origine, l'un constitué par les ARN polymérases virales ARN- ou ADN-dépendantes, et l'autre constitué par les ARN polymérases ADN-dépendantes d'origine eucaryote ou procaryotes (archaébactéries et eubactéries). Les ARN polymérases ADN-dépendantes eubactériennes sont caractérisées par une constitution multimérique simple et conservée notée " core enzyme ", représentée par αββ', ou " holoenzyme " représentée par αββ'σ [Yura and Ishihama, Ann. Rev. Genet. (1979) 13 :59-97]. De nombreux travaux ont mis en évidence le rôle fonctionnel, au sein du complexe enzymatique multimérique, de la sous-unité β de l'ARN polymérase eubactérienne. Les ARN polymérases archaébactérienne et eucaryote présentent, pour leur part, une structure plus complexe pouvant atteindre une dizaine, voire une trentaine de sous-unités [Pühlet et al. Proc . Natl. Acad. Sci. USA (1989) 86 :4569-4573].

Les gènes qui codent les différentes sous-unités αββ'σ de l'ARN polymérase ADN-dépendante chez les eubactéries, respectivement les gènes *rpoA, rpoB*, *rpoC* et *rpoD,* sont classés en différents groupes comprenant les gènes codant pour des protéines constitutives des sous-unités ribosomiques ou pour des enzymes impliqués dans la réplication et la réparation du génome [Yura and Yshihma, Ann. Rev. Genet. (1979) 13 :59-97]. Certains auteurs ont montré que les séquences des gènes *rpoB* et *rpoC* pouvaient être utilisées afin de construire des arbres phylogénétiques [Rowland et al. Biochem. Soc. Trans. (1992) 21 :40S] permettant de séparer les différents embranchements et sous-embranchements parmi les règnes du vivant.

Avant d'exposer plus en détail l'invention, différents termes, utilisés dans la description et les revendications, sont définis ci-après:
- pair " acide nucléique extrait de bactéries " on entend soit l'acide nucléique total, soit l'ADN génomique, soit les ARN messagers, soit encore l'ADN obtenu à partir de la transcription inverse des ARN messagers ;
- un " fragment nucléotidique " ou un " oligonucléotide " sont deux termes synonymes désignant un enchaînement de motifs nucléotidiques caractérisé par une séquence informationnelle des acides nucléiques naturels (ou éventuellement modifiés) et susceptibles de s'hybrider, comme les acides nucléiques naturels, avec un fragment nucléotidique complémentaire ou sensiblement complémentaire, dans des conditions prédéterminées de stringence stricte. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 10, de préférence de 18 à 35, motifs nucléotidiques et peut être obtenu à partir d'une molécule d'acide nucléique naturelle et/ou par recombinaison génétique et/ou par synthèse chimique,
- un motif nucléotidique est dérivé d'un monomère qui peut être un nucléotide naturel d'acide nucléique dont les éléments constitutifs sont un sucre, un groupement phosphate et une base azotée choisie parmi l'adénine (A), la guanine (G), l'uracile (U), la cytosine (C), la thymine (T) ; ou bien le monomère est un nucléotide modifié dans l'un au moins des trois éléments constitutifs précédents ; à titre d'exemple, la modification peut intervenir soit au niveau des bases, avec des bases modifiées telles que l'inosine, qui peut s'hydrider avec toute base A, T, U, C ou G, la méthyl-5-désoxycytidine, la désoxyuridine, la diméthylamino-5-désoxyuridine ou toute autre base modifiée capable d'hybridation, soit au niveau du sucre, par exemple le remplacement d'au moins un désoxyribose par un polyamide [Nielsen PE et al., Science (1991) 254 :1497-1500], soit encore au niveau du groupement phosphate, par exemple par remplacement par des esters choisis notamment parmi les diphosphates, les alkylphosphonates et les phosphorothioates,
- par " hybridation ", on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la " stringence ", c'est à dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépend notamment des sondes utilisées. Toutes ces données sont bien connues et les conditions appropriées peuvent éventuellement être déterminées dans chaque cas par des expériences de routine. En général, selon la longueur des sondes utilisées, la température pour la réaction d'hybridation est comprise entre environ 20 et 65°C, en particulier entre 35 et 65°C dans une solution saline à une concentration d'environ 0,8 à 1 M.
- une " sonde " est un fragment nucléotidique possédant une spécificité d'hybridation dans des conditions déterminées pour former un complexe d'hybridation avec un acide nucléique ayant, dans le cas présent, une séquence nucléotidique comprise soit dans un ARN messager, soit dans un ADN obtenu par transcription inverse dudit ARN messager, produit de transcription ; une sonde peut être utilisée à des fins de diagnostic (notamment sondes de capture ou de détection) ou à des fins de thérapie,
- une sonde peut être immobilisée ou immobilisable sur un support solide par tout moyen approprié, par exemple par covalence, par adsorption, ou par synthèse directe sur un solide. Des exemples de supports comprennent les plaques de microtitration et les puces à ADN,
- une "sonde" est en général marquée au moyen d'un agent marqueur choisi par exemple parmi les isotopes radioactifs, les enzymes, en particulier les enzymes susceptibles d'agir sur un substrat chromogéne, fluorigène ou luminescent (notamment une peroxydase ou une phosphatase alcaline), les composés chimiques chromophores, les composés chromogènes, fluorigènes ou luminescents, les analogues des bases nucléotidiques .Ce marquage peut être directe entre l'ADN et le dit marqueur ou indirecte c'est à dire par l'intermédiaire de ligands tels que la biotine ou autre molécule apte à se lier à des agents de marquage,
- une " sonde d'espèce " est une sonde permettant l'identification spécifique de l'espèce d'une bactérie d'un genre donné, en l'espèce ici acinetobacter,
- une "sonde de genre" est une sonde permettant l'identification spécifique du genre de la bactérie quelque soit l'espèce de la bactérie dudit genre dans certaines conditions d'hybridation,
- une " amorce " est une sonde comprenant par exemple 10 à 100 motifs nucléotidiques et possédant une spécificité d'hybridation dans des conditions déterminées pour les réactions d'amplification enzymatique,
- par "amorce de genre", on entend un jeu d'amorces permettant l'amplification spécifique de toute bactérie d'un même genre donné, sans distinction de l'espèce, dans des certaines conditions d'hybridation et d'amplification (les "amorces de genre" sont aussi appelées "amorces consensus" ou "amorces universelles" dans la présente demande)
- par "réaction d'amplification" on entend une réaction de polymérisation enzymatique, par exemple dans une technique d'amplification telle que la PCR, initiée par des oligonucléotides amorces et utilisant une ADN polymérase.
- par "réaction de séquençage", on entend une réaction conduisant à la détermination de la séquence d'un fragment d'acide nucléique ou d'un gène complet par un procédé de polymérisation abortive à partir d'amorces oligonucléotidiques et utilisant lesdits didésoxynucléotides (Sanger F, Coulson AR (1975), J.Mol.Biol. 94 : 441) ou par hybridations multiples avec des sondes multiples fixées sur support solide telles qu'utilisées dans les puces ADN par exemple,ou d'autres techniques connues de l'homme de l'Art.

Les inventeurs ont déterminé les séquences complètes des gènes *rpo*B et des séquences non codantes flanquantes qui le bornent et le séparent des gènes rp/L et rpoC (" fragment intergénique *rpl*L*-rpo*B " et " fragment intergénique *rpo*B*-rpo*C ") de 24 espèces du genre *Acinetobacter*: *A. calcoaceticus* (espèce génomique 1), *A. baumannii* (espèce génomique 2), espèce génomique *3, A. haemolyticus*(espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, *A. radioresistens* (espèce génomique 12), espèce génomique 13, espèce génomique *16, A. schindleri, A. ursingii, A. baylyi, A. bouvetii, A. gerneri, A. grimontii, A. tandoii, A. tjernbergiae, A. towneri, A. parvus.*

Pour arriver à déterminer lesdites séquences complètes de rpoB et ses séquences flanquantes de la totalités des espèces de bactéries *Acinetobacter,* les inventeurs ont du, après un grand nombre d'essais infructueux, déterminer tout d'abord 51 amorces (Tableau 2), à partir de la seule séquence rpoB et des zones non codantes qui la bornent de bactéries du genre *Acinetobacter* des séquences correspondantes de bactéries qu'ils ont identifiées comme proches disponibles sur GENBANK, à savoir *Acinetobacter* sp. ADP1 (GeneBank accession number NC_005966), *Pseudomonas syringae* pv.tomato str.DC3000 (GeneBank accession number NC 004578) et *P. putida* KT2440 (GeneBank accession number NC 006347).

La souche Acinetobacter sp.ADP1 est une souche qui n'avait pas été caractérisée avant l'invention et qui ne correspond à aucune des souches pathogènes décrites dans la présente demande de brevet et listées ci-après.

La présente invention a donc pour objet un gène complet *rpo*B d'une bactérie du genre *Acinetobacter* choisie parmi les 23 espèces suivantes : *A. calcoaceticus* (espèce génomique 1), espèce génomique 3, *A. haemolyticus*(espèce génomique 4), *A. junii* (espèce génomique 5), espèce génomique 6, *A. johnsonii* (espèce génomique 7), *A. lwoffïi* (espèce génomique 8), espèce génomique 9, espèce génomique 10, espèce génomique 11, *A. radioresistens* (espèce génomique 12), espèce génomique 13, espèce génomique 16, *A. schindleri, A. ursingii, A. baylyi, A. bouvetii, A. gerneri, A. grimontii, A. tandoii, A. tjernbergiae, A. towneri, A. parvus,* caractérisé en ce que sa séquence comprend ,et plus particulièrement consiste en une séquence choisie parmi les séquences telle que décrite dans les séquences SEQ.ID. n°9 et 11 à 32 respectivement, et les séquences présentant au moins 98% d'identité, ainsi que leurs séquences complémentaires.

La présente invention a également pour objet les fragment d'acide nucléique comprenant, et plus particulièrement consistant dans un fragment non codant encadrant le gène rpoB d'une bactérie du genre *Acinetobacter* choisie parmi les 24 espèces suivantes : *A. calcoaceticus* (espèce génomique 1), *A. baumannii* (espèce génomique 2), espèce génomique 3, *A. haemolyticus*(espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, *A. radioresistens* (espèce génomique 12), espèce génomique 13, espèce génomique 16, *A. schindleri, A. ursingii, A. baylyi, A. bouvetii, A. gerneri*, *A. grimontii, A. tandoii, A. tjernbergiae, A. towneri, A. parvus,* caractérisé en ce que sa séquence comprend, et plus particulièrement consiste en une séquence choisie parmi les séquences telles que décrites dans les séquences SEQ. ID. n°121 à 144 respectivement, et les séquences SEQ.ID. n°165 à 188 respectivement, et les séquences présentant au moins 98% d'identité et leurs séquences complémentaires.

Les dites séquences présentant des taux d'identité (encore appelés taux de similitude) d'au moins 98% avec les dites séquences SEQ. ID. 9 à 32, 121 à 144 et 165 à 188 correspondent aux variations possibles entre les différentes souches d'une même espèce correspondant éventuellement à des sous-espèces. Ainsi, les séquences SEQ. ID. n°145 à 164 et 189 à 208 correspondent à diverses souches d'A. baumannii présentant au moins 98% d'homologie avec, respectivement, les séquences SEQ. ID. n°122 et 166.

On entend ici par "taux d'identité (ou taux de similitude)" ou "pourcentage d'homologie", un pourcentage d'identité de séquences, c'est-à-dire un pourcentage de nucléotides de la séquence qui sont identiques à la même position par rapport à une autre séquence.

Les séquences SEQ. ID. n° 121 à 144 correspondent aux séquences flanquantes en 5' représentant le fragment intergénique *rpl*L*-rpo*B complet qui borne le gène *rpo*B entre les gènes rp/L et rpoB, d'une longueur comprise entre 301 et 310 pb, pour chaque espèce de la bactérie du genre *Acinetobacter,* pour les 24 espèces mentionnées ci-dessus.

Les séquences SEQ. ID. n° 165 à 188 correspondent aux séquences flanquantes en 3' représentant le fragment intergénique *rpo*B*-rpo*C complet qui borne le gène *rpo*B entre les gènes rpoB et rpoC, d'une longueur comprise entre 86 et 177 pb, pour chaque espèce de la bactérie du genre *Acinetobacter,* pour les 24 espèces mentionnées ci-dessus.

Les fragments intergéniques *rpo*B*-rpo*C et rp/L-rpoB qui bornent le gène *rpo*B et la séquence complète du gène *rpo*B peuvent être utilisées pour identifier la bactérie pas seulement à titre de sonde et/ou par l'étude de sa séquence primaire, mais aussi, par l'étude des structures secondaire et tertiaire de l'ARN messager provenant de la transcription de la séquence complète d'ADN.

Dans ces gènes *rpo*B et les séquences non codantes qui les bornent (fragment intergénique *rpl*L*-rpo*B et fragment intergénique *rpoB-rpoC)* d' *Acinetobacter,* les inventeurs ont mis en évidence des séquences consensus SEQ. ID. n°1, 2, 3, 4, 5, 6, 7 et 8 (Tableau 3) qui sont des séquences conservées entre toutes les bactéries du genre *Acinetobacter,* c'est-à-dire pouvant être utilisées comme amorces permettant d'amplifier la même portion du gène *rpo*B et les zones non codantes qui le bornent (fragment intergénique *rpl*L*-rpo*B et fragment intergénique *rpo*B*-rpo*C) de toutes lesdites bactéries *Acinetobacter,* à savoir :
- SEQ. ID. n°1, et 2 pour une première zone du gène rpoB,
- SEQ. ID. n°3, et 4 pour une seconde zone du gène rpoB,
- SEQ. ID. n°5, et 6 pour le fragment intergénique *rpl*L*-rpo*B*,* et
- SEQ. ID. n°7, et 8 pour le fragment intergénique *rpo*B*-rpo*C
- SEQ. ID. n° 1 à 4 et 6-7 sont à l'intérieur du gène rpoB, avec :
- SEQ. ID. n° 8 et 6 sont à proximité des extrémités 5' des gènes rpoB, et respectivement rpoC.
- SEQ. ID. n° 7 et 5 sont à proximité des extrémités 3' des gènes rp/L et respectivement rpoB.

La présente invention fournit donc des oligonucléotides qui présentent des séquences conservées d'une bactérie Acitenobacter choisie parmi les dites 24 espèces comprenant une séquence d'au moins 12, de préférence d'au moins 18 motifs nucléotidiques consécutifs inclus dans l'une des séquences choisie parmi les séquences telles que décrites dans les séquences SEQ. ID. n°1 à 8 suivantes, leurs séquences complémentaires, de préférence consistant dans les dites séquences:
- SEQ. ID. n° 1 : 5'-TAYCGYAAAGAYTTGAAAGAAG-3',
- SEQ. ID. n° 2 : 5'-CMACACCYTTGTTMCCRTGA-3',.
- SEQ. ID. n° 3 : 5'-GTGATAARATGGCBGGTCGT-3',
- SEQ. ID. n° 4 : 5'-CGBGCRTGCATYTTGTCRT-3',
- SEQ. ID. n° 5 :5'-GAAGARCTTAAGAMDAARCTTG-3'
- SEQ. ID. n° 6 : 5'-CGTTTCTTTTCGGTATATGAGT-3',
- SEQ. ID. n° 7 : 5'- GTTCTTTAGGTATCAACATTGAA -3',
- SEQ. ID. n° 8 : 5'- GACGCAAGACCAATACGRAT -3',
dans lesquelles :
- D représente A, G ou T,
- Y représente C ou T,
- B représente C, G ou T,
- R représente A ou G, et
- M représente A ou C.

A la position correspondant à un nucléotide D, Y, B, M ou R dans les séquences SEQ. ID. n°1 2, 3, 4, 5 et 8 on trouve dans les séquences cibles complémentaires des nucléotides variables en fonction de l'espèce de la bactérie considérée, mais tous les autres nucléotides sont conservés dans toutes les espèces des bactéries du genre *Acinetobacter.*

Les séquences SEQ. ID. n° 1, 3, 5 et 7 sont utilisées à titre d'amorces sur le brin direct et les séquences SEQ. ID. n°2, 4, 6 et 8 sont utilisées à titre d'amorce sur le brin indirect. Les séquences SEQ. ID. n°2, 4, 6 et 8 correspondent donc à des séquences complémentaires de celles du brin direct.

Pour être utilisés à titre d'amorces consensuelles, ces oligonucléotides de séquences SEQ. ID. n°1, 2, 3, 4, 5 et 8 sont donc mis en oeuvre, en fait, sous forme de mélanges équimolaires d'oligonucléotides de séquences différentes, lesdits oligonucléotides de séquences différentes répondant pour chaque séquence SEQ. ID. n°1 à 8 aux diverses définitions possibles des séquences respectivement n°1, 2, 3, 4, 5 et 8.

Ces mélanges équimolaires d'oligonucléotides sont obtenus en mettant en oeuvre, lors de la synthèse oligonucléotidique, des mélanges équimolaires des différents nucléotides concernés respectivement :
- A, G et T pour D,
- C et T pour Y,
- C, G et T pour B,
- A et G pour R,
- A et C pour M.

Les mélanges d'oligonucléotides, répondant aux nucléotides de définition des séquences SEQ. ID. n°1 2, 3, 4, 5 et 8, peuvent donc s'hybrider avec les différentes séquences complémentaires cibles incluses dans les gènes *rpo*B et aux extrémités des gènes rp/L et rpoC encadrant les zones non codantes qui les bornent (fragment intergénique *rpl*L*-rpo*B et fragment intergénique *rpo*B*-rpo*C) de toutes les espèces de bactéries du genre *Acinetobacter* et, plus particulièrement, les 24 espèces citées ci-dessus.

La capacité de ces amorces à amplifier des fragments de gènes rpoB et des zones non codantes qui les bornent (fragment intergénique *rpl*L*-rpo*B et fragment intergénique *rpo*B*-rpo*C), de toutes les espèces d'*Acinetobacter* permet de considérer que ces amorces seront efficaces pour l'identification d'espèces d'*Acinetobacter* additionnelles qui seraient décrites dans le futur.

La présente invention a donc également pour objet un mélange d'oligonucléotides caractérisé en ce qu'il comprend un mélange équimolaire d'oligonucléotides de séquences différentes comprenant au moins 12, de préférence au moins 18 motifs nucléotidiques consécutifs inclus dans l'une des séquences SEQ. ID. n° 1 à 5 et 8 ou les oligonucléotides de séquences complémentaires.

Plus particulièrement, la présente invention a pour objet les mélanges d'oligonucléotides suivants :
- un mélange équimolaire de 8 oligonucléotides de séquences différentes consistant dans la séquence SEQ. ID. n°1, ou des oligonucléotides de séquences complémentaires.
- un mélange équimolaire de 16 oligonucléotides de séquences différentes consistant dans la séquence SEQ. ID. n°2 ou des oligonucléotides de séquences complémentaires.
- un mélange équimolaire de 6 oligonucléotides de séquences différentes consistant dans la séquence SEQ. ID. n°3 ou des oligonucléotides de séquences complémentaires.
- un mélange équimolaire de 24 oligonucléotides de séquences différentes consistant dans la séquence SEQ. ID. n° 4 ou des oligonucléotides de séquences complémentaires.
- un mélange équimolaire de 24 oligonucléotides de séquences différentes consistant dans la séquence SEQ. ID. n° 5 ou des oligonucléotides de séquences complémentaires.
- oligonucléotide de séquence consistant dans la séquence SEQ. ID. n°6 ou séquence complémentaire.
- oligonucléotide de séquence consistant dans la séquence SEQ. ID. n°7 ou séquence complémentaire.
- un mélange équimolaire de 2 oligonucléotides de séquences différentes consistant dans la séquence SEQ.ID. n°8 ou des oligonucléotides de séquences complémentaires.

Les oligonucléotides ou mélanges d'oligonucléotides comprenant une séquence incluse dans l'une des séquences SEQ. ID. n° 1 à 8 selon l'invention, peuvent être utilisés à titre d'amorces de genre des bactéries du genre acinetobacter.

Comme mentionné précédemment, en outre, les séquences consensus SEQ. ID. n°1, 2, 3, 4, 5 et 8, ainsi définies, encadrent des séquences hyper variables dont la séquence est spécifique pour chaque espèce des bactéries du genre *Acinetobacter.*

Les inventeurs ont ainsi pu mettre en évidence des séquences spécifiques d'espèces pour chacune des 24 espèces de bactéries citées ci-dessus, correspondant aux séquences :
- SEQ. ID. n°33 à 56, encadrées par les séquences consensus SEQ. ID. n°1 et 2 (ci-après "zone 1 de rpoB");
- SEQ. ID. n°77 à 100, encadrées par les séquences consensus SEQ. ID. n°3 et 4 (ci-après "zone 2 de rpoB");
- SEQ. ID. n°121 à 144, encadrées par les séquences consensus SEQ. ID. n°5 et 6 (ci-après "fragment intergénique rpo/L-rpoB"); et
- SEQ. ID. n°165 à 188, encadrées par les séquences consensus SEQ. ID. n°7 et 8 (ci-après "fragment intergénique rpoB-rpoC");

Les oligonucléotides de séquences encadrées par les SEQ. ID. n°1 2, 3, 4, 5, 6, 7 et 8, peuvent donc être utilisés à titre de sonde d'espèce des bactéries du genre *Acinetobacter.*

Lesdites séquences hyper variables spécifiques SEQ. ID. n°33 à 56 encadrées par les séquences SEQ. ID. n°1 et 2, représentent un fragment du gène *rpo*B d'une longueur de 350 pb avec moins de 96% d'identité entre les différentes espèces (tableau 7) à l'exception des couples *A. baylii*/geno. species 11 et *A. grimontii*/*A. junii,* de sorte qu'elles constituent une courte séquence spécifique cible, pour identifier spécifiquement chaque espèce de la bactérie du genre *Acinetobacter,* plus précisément pour les 24 espèces mentionnées ci-dessus, à l'exception des couples *A. baylii*/geno. species 11 et *A. grimontii*/*A. junii,.*

Lesdites séquences hyper variables spécifiques SEQ. ID. n°77 à 100 encadrées par les séquences SEQ. ID. n°3 et 4, représentent un fragment du gène *rpo*B d'une longueur de 450 pb avec moins de 96% d'identité entre les différentes espèces (tableau 8) à l'exception des couples *A. baylii*/geno. species 11 et *A. grimontii*/*A. junii,* de sorte qu'elles constituent une deuxième courte séquence spécifique cible, pour identifier spécifiquement chaque espèce de la bactérie du genre *Acinetobacter,* plus précisément pour les 24 espèces mentionnées ci-dessus, à l'exception des couples *A. baylii*/geno.species 11 et *A. grimontii*/*A. juni.*

Lesdites séquences hyper variables spécifiques SEQ. ID. n° 121 à 164 encadrées par les séquences SEQ. ID. n°5 et 6, représentent le fragment intergénique *rpl*L*-rpo*B qui borne le gène *rpo*B d'une longueur comprise entre 301 et 310 pb avec moins de 97% d'identité entre les différentes espèces (voir tableau 5 ci-après) à l'exception des couples *A. baylii*/geno.species 11, *A. grimontii*/*A. junii,* et *A. lwoffi*/geno.species 9, de sorte qu'elles constituent une troisième courte séquence spécifique cible pour identifier spécifiquement chaque espèce de la bactérie du genre *Acinetobacter,* plus précisément pour les 24 espèces mentionnées ci-dessus, à l'exception des couples *A. baylii*/geno.species 11, *A. grimontii*/*A. junii,* et *A. lwoffi*/geno.species 9.

Enfin, lesdites séquences hyper variables spécifiques SEQ. ID. n° 165 à 188 encadrées par les séquences SEQ. ID. n°7 et 8, représentent le fragment intergénique *rpo*B*-rpo*C qui borne le gène *rpo*B d'une longueur comprise entre 86 et 177 pb avec moins de 97% d'identité entre les différentes espèces (voir tableau 6 ci-après) à l'exception des couples *A. grimontii*/*A. junii* et des espèces du groupe Acb (*A. baumannii, A ; calcoaceticus* et genospecies 3, de sorte qu'elles constituent une quatrième courte séquence spécifique cible, pour identifier spécifiquement chaque espèce de la bactérie du genre *Acinetobacter,* plus précisément pour les 24 espèces mentionnées ci-dessus , à l'exception des couples *A. grimontii*/*A. junii* et des espèces du groupe Acb *(A. baumannii, A ; calcoaceticus* et genospecies 3.

Un autre objet de la présente invention est donc un fragment de gène *rpo*B d'une bactérie du genre *Acinetobacter* choisie parmi les 24 espèces: *A. calcoaceticus* (espèce génomique 1), *A. baumannii* (espèce génomique 2), espèce génomique 3, *A. haemolyticus*(espèce génomique 4), *A. junii* (espèce génomique 5), espèce génomique 6, *A. johnsonii* (espèce génomique 7), *A. lwoffïi* (espèce génomique 8), espèce génomique 9, espèce génomique 10, espèce génomique 11, *A. radioresistens* (espèce génomique 12), espèce génomique 13, espèce génomique 16, *A. schindleri, A. ursingii, A. baylyi, A. bouvetii, A. gerneri, A. grimontii, A. tandoii, A. tjernbergiae, A. towneri, A. parvus,* caractérisé en ce que sa séquence consiste en une séquence choisie parmi les séquences telles que décrites dans les séquences SEQ. ID. n° 33 à 56 respectivement, et les séquences SEQ. ID. n°77 à 100 respectivement, et les séquences présentant au moins 98% d'identité et leurs séquences complémentaires.

La présente invention a également pour objet un fragment de gène *rpo*B d'une bactérie du genre *Acinetobacter* choisie parmi les 23 espèces: *A. calcoaceticus* (espèce génomique 1), espèce génomique 3, A. *haemolyticus*(espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, *A. radioresistens* (espèce génomique 12), espèce génomique 13, espèce génomique 16, *A. schindleri, A. ursingii, A. baylyi, A. bouvetii, A. gerneri, A. grimontii, A. tandoii, A. tjernbergiae, A. towneri, A. parvus,* caractérisé en ce que sa séquence comprend une séquence choisie parmi les séquences telles que décrites dans les séquences SEQ. ID. n° 33 et 35 à 56 respectivement, et les séquences SEQ. ID. n°77 et 79 à 100 respectivement, et les séquences présentant au moins 98% d'identité et leurs séquences complémentaires.

Les dites séquences présentant des taux d'identité d'au moins 98% avec les dites séquences SEQ. ID. n°33 à 56 et 77 à 100 correspondent aux variations possibles entre les différentes souches d'une même espèce correspondant éventuellement et plus particulièrement à des sous-espèces. Ainsi les séquences SEQ. ID. n°57 à 76 et 101 à 120 correspondent à diverses souches de *A. baumannii* présentant au moins 98% d'homologie avec, respectivement, SEQ. ID. n° 34 et 78.

La présente invention a donc également pour objet l'utilisation à titre de sonde d'espèce d'un fragment de gène rpoB, rp/l ou rpoC selon l'invention ou d'un oligonucléotide de séquences spécifiques d'une dite espèce de bactérie *Acinetobacter* selon l'invention.

Plus précisément, la présente invention fournit un procédé de détection par identification moléculaire d'une bactérie de l'une des espèces du genre *Acinetobacter* caractérisé en ce qu'on utilise :
- le gène *rpo*B complet de ladite bactérie selon l'invention, comprenant une dite séquence SEQ. ID. n°9 à 32, ou de préférence consistant en une dite séquence SEQ. ID. n°9 à 32, ou les séquences complémentaires, ou séquences présentant au moins 98% d'identité,
- un fragment de gène *rpo*B d'une dite bactérie selon l'invention, comprenant une dite séquence SEQ. ID. n°33 à 56 ou 77 à 100, ou consistant en une dite séquence SEQ. ID. n°33 à 56 ou 77 à 100 les séquences complémentaires, ou séquences présentant au moins 98% d'identité,
- un fragment de gène *rpo*B d'une dite bactérie selon l'invention, comprenant une dite séquence SEQ. ID. n°77 à 100, ou, de préférence, consistant en une dite séquence SEQ. ID. n°77 à 100, ou séquences complémentaires, ou séquences présentant au moins 98% d'identité,
- un fragment de gène comprenant un fragment intergénique *rpl*L*-rpo*B ou *rpo*B*-rpo*C complet de ladite bactérie selon l'invention, comprenant une dite séquence SEQ. ID. n°121 à 144 ou 165 à 188, ou de préférence consistant en une dite séquence SEQ. ID. n°121 à 144 ou 165 à 188, les séquences complémentaires ou séquences présentant au moins 98% d'identité.
- un oligonucléotide présentant une séquence spécifique d'une bactérie Acitenobacter choisie parmi les 24 espèces ci-dessus d'au moins 18 , de préférence de 18 à 35, motifs nucléotidiques consécutifs inclus dans l'une des séquences choisie parmi les séquences telles que décrites dans les séquences :
- SEQ. ID. n°33 à 56 respectivement,
- SEQ. ID. n°77 à 100 respectivement,
- SEQ. ID. n°121 à 144 respectivement,
- SEQ. ID. n°165 à 188 respectivement, et
- les séquences présentant au moins 98% d'identité et leurs séquences complémentaires; ou
- un oligonucléotide ou mélange équimolaire d'oligonucléotides selon l'invention, comprenant une séquence d'au moins 12, de préférence 18 nucléotides consécutifs inclus dans l'une des séquences SEQ. ID. n°1 2, 3, 4, 5, 6, 7 et 8 ou les séquences complémentaires, ou de préférence consistant dans l'une desdites séquences SEQ. ID. n°1 2, 3, 4, 5, 6, 7 et 8.

Dans un premier mode de réalisation d'un procédé de détection d'une bactérie selon l'invention, on cherche à détecter spécifiquement une espèce donnée d'une bactérie Acitenobacter choisie parmi les 24 espèces suivantes : *A. calcoaceticus* (espèce génomique 1), *A. baumannii* (espèce génomique 2), espèce génomique 3, *A. haemolyticus*(espèce génomique 4), *A. junii* (espèce génomique 5), espèce génomique 6, *A. johnsonii* (espèce génomique 7), *A. lwoffïi* (espèce génomique 8), espèce génomique 9, espèce génomique 10, espèce génomique 11, *A. radioresistens* (espèce génomique 12), espèce génomique 13, espèce génomique 16, *A. schindleri, A. ursingii, A. baylyi, A. bouvetii, A. gerneri, A. grimontii, A. tandoii, A. tjernbergiae, A. towneri, A. parvus,* procédé dans lequel :
1- on met en contact un échantillon contenant ou susceptible de contenir des acides nucléiques d'au moins une telle bactérie, avec au moins une sonde d'espèce consistant dans un oligonucléotide ou un fragment de gène selon l'invention, de préférence un fragment de gène consistant respectivement dans l'une desdites séquences choisie parmi :
   - SEQ. ID. n°33 à 56 respectivement,
   - SEQ. ID. n°77 à 100 respectivement,
   - SEQ. ID. n°121 à 144 respectivement,
   - SEQ. ID. n°165 à 188 respectivement, et
   - les séquences présentant au moins 98% d'identité et leurs séquences complémentaires, et
2- on détermine la formation ou l'absence d'un complexe d'hybridation entre ladite sonde et les acides nucléiques de l'échantillon, et on détermine ainsi la présence de ladite espèce de *Acinetobacter* dans l'échantillon s'il y a formation d'un complexe d'hybridation.

Dans un deuxième mode de réalisation d'un procédé de détection d'une bactérie du genre *Acinetobacter* d'une espèce spécifique, on réalise les étapes dans lesquelles :
1- on met en contact des amorces d'amplification comprenant desdits mélanges d'oligonucléotides selon l'invention, avec un échantillon contenant ou susceptible de contenir des acides nucléiques d'au moins une telle bactérie du genre *Acinetobacter,* et on réalise une amplification d'acides nucléiques par réaction de polymérisation enzymatique comprenant :
   - comme amorce 5', au moins un oligonucléotide ou mélange d'oligonucléotides selon l'invention comprenant une séquence incluse dans l'une des séquences SEQ. ID. n° 1, 3, 5 et 7 de préférence consistant dans ladite séquence SEQ. ID. n°1, 3, 5 et 7 complète ou les séquences complémentaires, et
   - comme amorce 3', au moins un oligonucléotide ou mélange d'oligonucléotides selon l'invention comprenant des séquences incluses dans l'une des séquences SEQ. ID. n° 2, 4 6, et 8 respectivement, de préférence consistant dans ladite séquence SEQ. ID. n°2, 4, 6, et 8 complète ou respectivement une séquence complémentaire.
2- et on détermine l'apparition ou l'absence d'un produit d'amplification, et on détermine ainsi la présence ou l'absence de ladite bactérie dans l'échantillon si un produit d'amplification est ou n'est pas apparu respectivement.

Plus particulièrement, on cherche à détecter une espèce donnée d'une bactérie Acitenobacter choisie parmi les 24 espèces suivantes : *A. calcoaceticus (espèce génomique 1), A. baumannii (espèce génomique* 2), *espèce génomique 3, A. haemolytius (espèce génomique 4), A. junii (espèce génomique 5), espèce génomique* 6, *A. johnsonii (espèce génomique* 7), *A. lwoffii (espèce génomique 8), espèce génomique 9, espèce génomique 10, espèce génomique 11, A. radioresistens (espèce génomique 12), espèce génomique 13, espèce génomique 16, A. schindleri, A. ursingii, A. baylyi, A. bouvetii, A. gerueri, A. grimontii, A. tandoii, A. tjernbergiae, A. towneri, A. parvus.,* et, à l'étape 2 ci-dessus, on détermine la présence ou l'absence de l'espèce donnée d'une dite bactérie en effectuant les étapes dans lesquelles :
a) on réalise une réaction de séquençage d'un fragment de gène amplifié avec des dites amorces, et
b) on compare la séquence dudit fragment amplifié obtenu avec la séquence d'un fragment de gène de ladite bactérie comprenant respectivement :
   - lesdites séquences SEQ. ID. n° 33 à 56, lorsque les dites amorces 5' et 3' sont des oligonucléotides de séquences incluses dans les séquences SEQ. ID. n° 1 et 2 respectivement
   - les dites séquences SEQ. ID. n° 77 à 100, lorsque les dites amorces 5' et 3' sont des oligonucléotides de séquences incluses dans les séquences SEQ. ID. n° 3 et 4 respectivement, et
   - les dites séquences SEQ. ID. n° 121 à 144, lorsque les dites amorces 5' et 3' sont des oligonucléotides de séquences incluses dans les séquences SEQ. ID. n° 5 et 6 respectivement, et
   - les dites séquences SEQ. ID. n° 165 à 188, lorsque les dites amorces 5' et 3' sont des oligonucléotides de séquences incluses dans les séquences SEQ. ID. n° 7 et 8 respectivement.

Dans une autre variante dudit deuxième mode de réalisation d'un procédé de détection d'une bactérie du genre *Acinetobacter* d'une espèce spécifique, dans lequel, on cherche à détecter une espèce donnée d'une bactérie Acitenobacter choisie parmi les 24 espèces suivantes : *A. calcoaceticus (espèce génomique 1), A. baumannii (espèce génomique* 2), *espèce génomique 3, A. haemolyticus*(espèce *génomique 4), A. junii (espèce génomique 5), espèce génomique 6, A. johnsonii (espèce génomique* 7), *A. lwoffii (espèce génomique 8), espèce génomique* 9, *espèce génomique 10, espèce génomique 11, A. radioresistens (espèce génomique 12), espèce génomique 13, espèce génomique 16, A. schindleri, A. ursingii, A. baylyi, A. bouvetii, A. gerneri, A. grimontii, A. tandoii, A. tjernbergiae, A. towneri, A. parvus,* à l'étape 2 ci-dessus on détermine la présence ou l'absence de l'espèce donnée d'une dite bactérie par en effectuant les étapes dans lesquelles :
a- on met en contact un échantillon contenant ou susceptible de contenir des acides nucléiques amplifié d'au moins une telle bactérie, avec au moins une sonde d'espèce consistant dans un fragment de gène *rpo*B ou un oligonucléotide spécifique selon l'invention, de préférence un fragment consistant respectivement dans l'une desdites séquences choisie parmi :
   - SEQ. ID. n°33 à 56 respectivement, lorsque les dites amorces 5' et 3' sont des oligonucléotides de séquences incluses dans les séquences SEQ. ID. n° 1 et 2 respectivement
   - SEQ. ID. n°77 à 100 respectivement, lorsque les dites amorces 5' et 3' sont des oligonucléotides de séquences incluses dans les séquences SEQ. ID. n° 3 et 4 respectivement, et
   - SEQ. ID. n°121 à 144 respectivement, lorsque les dites amorces 5' et 3' sont des oligonucléotides de séquences incluses dans les séquences SEQ. ID. n° 5 et 6 respectivement, et
   - SEQ. ID. n°165 à 188 respectivement, lorsque les dites amorces 5' et 3' sont des oligonucléotides de séquences incluses dans les séquences SEQ. ID. n° 7 et 8 respectivement, et.
b- on détermine la formation ou l'absence d'un complexe d'hybridation entre ladite sonde et les acides nucléiques amplifié de l'échantillon, et on détermine ainsi la présence ou l'absence de ladite espèce de *Acinetobacter* dans l'échantillon s'il y a formation ou non d'un complexe d'hybridation.

Dans un mode préféré de réalisation d'un procédé selon l'invention, on réalise les étapes comprenant :
1- une première amplification de l'acide nucléique dudit échantillon avec un couple d'amorces 5' et 3' choisi parmi desdits mélanges d'oligonucléotides selon l'invention, comprenant des séquences incluses respectivement dans les séquences SEQ. ID. n°1 et SEQ. ID. n°2, de préférence consistant dans lesdites séquences SEQ. ID. n°1 et 2, ou les séquences complémentaires, et
2- une première détermination de l'apparition ou l'absence d'un produit d'amplification comprenant des acides nucléiques d'au moins une dite bactérie, par hybridation ou le cas échéant séquençage et comparaison des amplifiats obtenus à l'étape 1 avec les fragments consistant respectivement dans l'une desdites séquences choisie parmi SEQ. ID. n°33 à 56 et 34 à 76 respectivement, et
   - si à cette étape 2 on détermine la présence des espèces *A. grimontii* ou *A. junii,* on réalise en outre:
      3a - une seconde réaction d'amplification avec des amorces 5' et 3' choisi parmi desdits mélanges d'oligonucléotides selon l'invention, comprenant des séquences incluses respectivement dans les séquences SEQ. ID. n°3 et SEQ. ID. n°4, de préférence consistant dans lesdites séquences SEQ. ID. n°3 et 4, ou les séquences complémentaires, et
      4a- une détermination de l'apparition ou l'absence d'un produit d'amplification comprenant des acides nucléiques d'au moins une dite bactérie, par hybridation ou le cas échéant séquençage et comparaison des amplifiats obtenus à l'étape 3a avec les fragments consistant respectivement dans l'une desdites séquences choisie parmi SEQ. ID. n°77 à 100 respectivement, ou
   - si à la première étape 2 on détermine la présence des espèces *A. baylii* ou A. *espèce génomique 11,* on réalise en outre :
      3b- une seconde réaction d'amplification avec des amorces 5' et 3' choisis parmi desdits mélanges d'oligonucléotides selon l'invention comprenant des séquences incluses respectivement dans les séquences SEQ. ID. n°7 et SEQ. ID. n°8, de préférence consistant dans lesdites séquences SEQ. ID. n° 7 et 8, ou les séquences complémentaires, et
      4b- une détermination de l'apparition ou l'absence d'un produit d'amplification comprenant des acides nucléiques d'au moins une dite bactérie, par hybridation ou le cas échéant séquençage et comparaison des amplifiats obtenus à l'étape 3b avec les fragments consistant respectivement dans l'une desdites séquences choisie parmi SEQ. ID. n°165 à 188 respectivement.

Les séquences SEQ. ID. n°1 à 208 peuvent être préparées par génie génétique et/ou par synthèse automatique ou synthèse chimique en utilisant les techniques bien connues de l'homme du métier.

Les sondes selon l'invention peuvent être utilisées, à des fins de diagnostic, comme mentionné précédemment, par la détermination de la formation ou de l'absence de formation d'un complexe d'hybridation entre la sonde et un acide nucléique cible dans un échantillon, selon toutes les techniques d'hybridation connues et notamment les techniques de dépôt ponctuel sur filtre, dites " DOT-BLOT " [Maniatis et al. (1982) Molecular Cloning, Cold Spring Harbor], les techniques de transfert d'ADN dites " SOUTHERN BLOT " [Southern E.M., J. Mol. Biol. (1975) 98 :503], les techniques de transfert d'ARN dites " NORTHERN BLOT ", ou les techniques dites " sandwich ", en particulier avec une sonde de capture et/ou une sonde de détection, lesdites sondes étant capables de s'hybrider avec deux régions différentes de l'acide nucléique cible, et l'une au moins desdites sondes (généralement la sonde de détection) étant capable de s'hybrider avec une région de la cible qui est spécifique de l'espèce, étant entendu que la sonde de capture et al sonde de détection doivent avoir des séquences nucléotidiques au moins partiellement différentes.

L'acide nucléique à détecter (cible) peut être de l'ADN ou de l'ARN (le premier obtenu après amplification par PCR). Dans le cas de la détection d'une cible de type acide nucléique double brin, il convient de procéder à la dénaturation de ce dernier avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80°C.

Pour mettre en oeuvre les techniques d'hybridation précitées, et en particulier les techniques " sandwich ", une sonde de l'invention, appelée sonde de capture est immobilisée sur un support solide, et une autre sonde de l'invention, appelée sonde de détection, est marquée avec un agent marqueur. Les exemples de support et d'agent marqueur sont tels que définis précédemment.

De manière avantageuse, une sonde d'espèce est immobilisée sur un support solide, et une autre sonde d'espèce est marquée par un agent marqueur.

Une autre application d'un dit mélange d'oligonucléotides de l'invention est son utilisation comme amorce nucléotidique comprenant un oligonucléotide monocaténaire choisi parmi les oligonucléotides ayant une séquence d'au moins 12 motifs nucléotidiques incluses dans l'une des séquences SEQ. ID. n°1 à 8, qui est utilisable dans la synthèse d'un acide nucléique en présence d'une polymérase par un procédé connu en soi, notamment dans des méthodes d'amplification utilisant une telle synthèse en présence d'une polymérase (PCR, RT-PCR, etc.). En particulier, une amorce de l'invention peut être utilisée pour la transcription inverse spécifique d'une séquence d'ARN messager de bactérie d'une espèce du genre *Acinetobacter* pour obtenir une séquence d'ADN complémentaire correspondante. Une telle transcription inverse peut constituer le premier stade de la technique RT-PCR, le stade suivant étant l'amplification par PCR de l'ADN complémentaire obtenu.

Selon un cas particulier, ladite amorce comprenant un oligonucléotide de l'invention comprend en outre la séquence sens ou anti-sens d'un promoteur reconnu par une ARN polymérase (promoteurs T7, T3, SP6 par exemple [Studier FW, BA Moffatt (1986) J. Mol. Biol. 189 :113] : de telles amorces sont utilisables dans des procédés d'amplification d'acide nucléique faisant intervenir une étape de transcription, tels que, par exemple, les techniques NASBA ou 3SR [Van Gemen B. et al. Abstract MA 1091, 7th International Conference on AIDS (1991) Florence, Italy].

Un autre objet de l'invention est une amorce nucléotidique comprenant un mélange d'oligonucléotides monocaténaires choisis parmi les oligonucléotides ayant des séquences comprenant l'une des séquences SEQ. ID. n°1 à 8 ou de préférence, consistant dans l'une des séquences SEQ. ID. n°1 à 8 qui est utilisable pour le séquençage total ou partiel du gène *rpo*B ou du fragment intergénique *rpl*L*-rpo*B ou du fragment intergénique *rpo*B*-rpo*C d'une quelconque espèce du genre *Acinetobacter*.

Le séquençage du gène *rpo*B partiel ou complet ou du fragment intergénique *rpl*L-*rpo*B ou du fragment intergénique *rpo*B*-rpo*C chez toute bactérie du genre *Acinetobacter* permet l'identification de toute bactérie *Acinetobacter* par analyse bioinformatique de cette séquence et la reconnaissance de nouvelles espèces de bactéries *Acinetobacter* inconnues.

De préférence, dans une utilisation comme amorce ou pour le séquençage des gènes *rpo*B, ou du fragment intergénique *rpl*L*-rpo*B ou du fragment intergénique *rpo*B*-rpo*C on utilise des dits mélanges d'oligonucléotides de séquence SEQ. ID. n°1 et 2.

La présente invention a également pour objet une trousse de diagnostic utile dans un procédé selon l'invention comprenant au moins un dit fragment de gène *rpo*B ou du fragment intergénique *rpl*L*-rpo*B ou du fragment intergénique *rpo*B*-rpo*C selon l'invention, comprenant ou consistant dans l'une des séquences SEQ. ID. n°9 à 188 ou un oligonucléotide ou dit mélange d'oligonucléotides équimolaires selon l'invention, comprenant des séquences incluses dans les séquences SEQ. ID. n°1 à 8, et les oligonucléotides et fragments de gènes *rpo*B ou du fragment intergénique *rpl*L*-rpo*B ou du fragment intergénique *rpo*B*-rpo*C de séquences complémentaires, tels que définis ci-dessus, ainsi que, de préférence, des réactifs utiles dans les réactions d'hybridations ou réactions d'amplification ou séquençage le cas échéant.

Comme mentionné dans les définitions, un oligonucléotide ou fragment d'acide nucléique selon l'invention peut être sous forme d'un acide désoxyribonucléique (ADN) ou d'un acide ribonucléique (ARN) pour lesquels dans ce cas T est remplacé par U.

Enfin, un dernier objet de l'invention est une sonde de thérapie génique pour traiter les infections provoquées par une souche appartenant à une espèce du genre *Acinetobacter,* ladite sonde comprenant un oligonucléotide tel que défini précédemment. Cette sonde de thérapie génique, capable de s'hybrider sur l'ARN messager et/ou sur l'ADN génomique desdites bactéries, peut bloquer les phénomènes de traduction et/ou transcription et/ou de réplication.

Le principe des méthodes de thérapie génique est connu et repose notamment sur l'utilisation d'une sonde correspondant à un brin anti-sens : la formation d'un hybride entre la sonde et le brin sens est capable de perturber au moins l'une des étapes du décryptage de l'information génétique. Les sondes de thérapie génique sont donc utilisables comme médicaments antibactériens, permettant de lutter contre les infections causées par les bactéries des espèces du genre *Acinetobacter.*

D'autres caractéristiques et avantages de la présente invention apparaîtront et l'invention sera mieux comprise à l'aide de l'exposé ci-après qui concernent les expériences effectuées et résultats obtenus dans le but de réaliser l'invention et qui sont donnés à titre purement illustratif.

Le tableau 1, ci-après, reprend la liste des espèces d'*Acinetobacter* pour lesquelles des séquences *rpo*B et le fragment intergénique *rpl*L*-rpo*B et le fragment intergénique *rpo*B*-rpo*C ont été déterminées, les souches mentionnées proviennent de la Collection de l'Institut Pasteur (CIP), les séquences SEQ. ID. n° 1 à 208 sont décrites dans le listage de séquences annexé à la description.

Dans le tableau 2, sont listées les différentes amorces utilisées pour l'amplification et le séquençage des gènes *rpo*B.

Dans le tableau 2, lorsque l'on présente des séquences comprenant des nucléotides W, H, Y, V, R, B, M, K, S ou D, ceux-ci ont les significations connues de l'homme de l'art et, de manière également conventionnelle, ces amorces sont en fait utilisées sous forme de mélange équimolaire d'oligonucléotides de séquences différentes à l'emplacement des dits nucléotides comme expliqué ci-dessus.

Le tableau 3 présente des comparaisons de similitudes des séquences des gènes 16S ARNr et *rpo*B entre les deux sous-espèces *C. affermentans* et entre les 11 couples d'espèces considérées comme proches pour lesquelles les similitudes entre séquences de gènes 16S ARNr sont supérieures ou égales à 98,5%, avec comparaison statistique des moyennes de similitude obtenues.

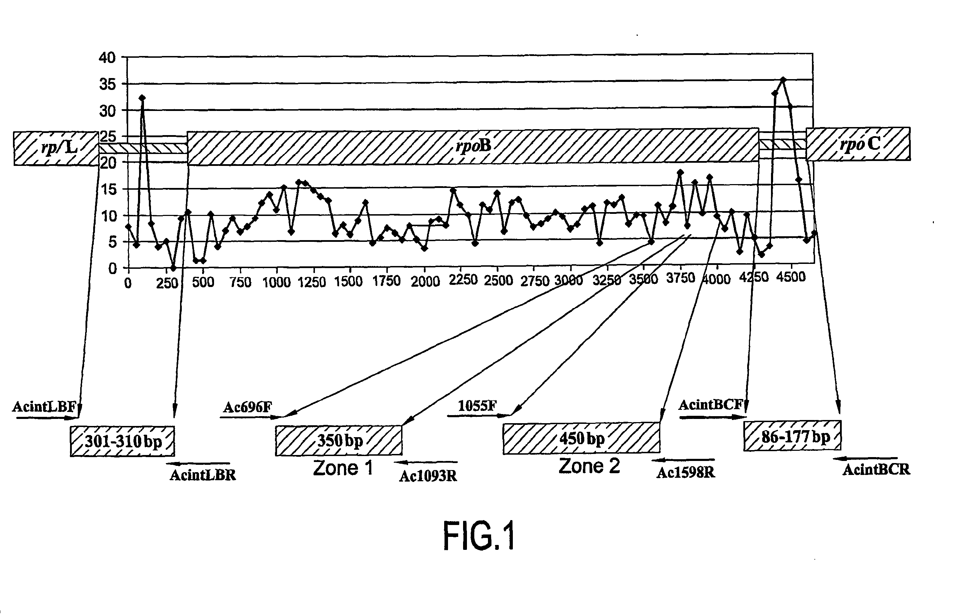

La figure 1 est une représentation graphiques du taux de variabilité (range site variability : RSV (axe des Y)) des séquences des gènes *rpo*B et séquences flanquantes des différentes espèces du genre Acinetobacter étudiées par fenêtres de 50 nucléotides (axe des X). Les régions hyper variables, bordées par les régions conservées, utilisée pour l'identification d'espèce à l'aide des amorces consensus sont encadrées.

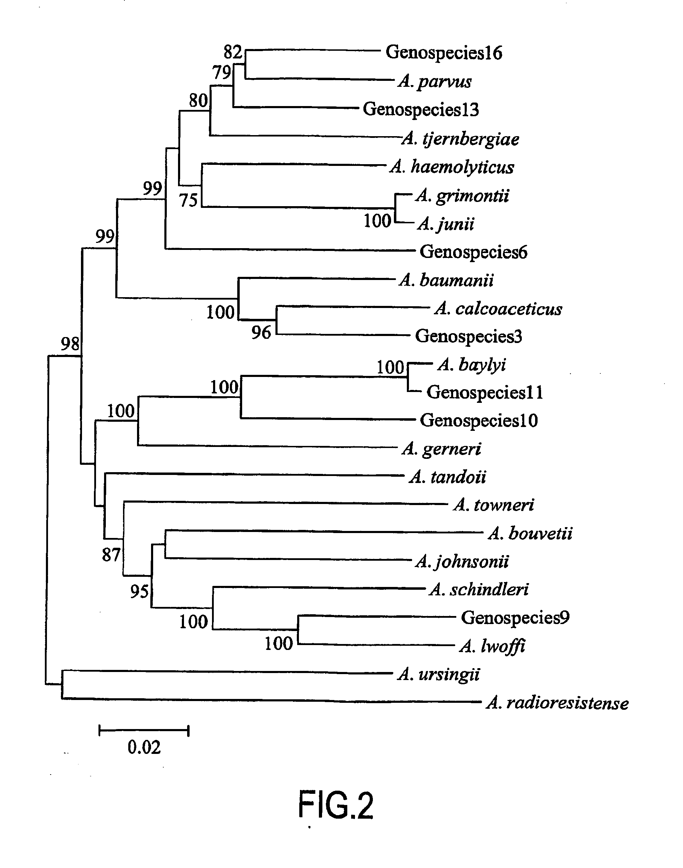

La figure 2 est un dendogramme représentant les relations phylogéniques des différentes espèces de *A. cinetobacter* par la méthode du "neighbour-joining". L'arbre a été construit par l'alignement des séquences du gène *rpo*B. Les valeurs d'échantillonnage de "bootstrap" (probabilité d'exactitude des noeuds en pourcentage) calculées sur une base d'un échantillon de 1000 arbres, sont indiquées à chaque noeud, seules les valeurs supérieures ou égales à 75% sont indiquées

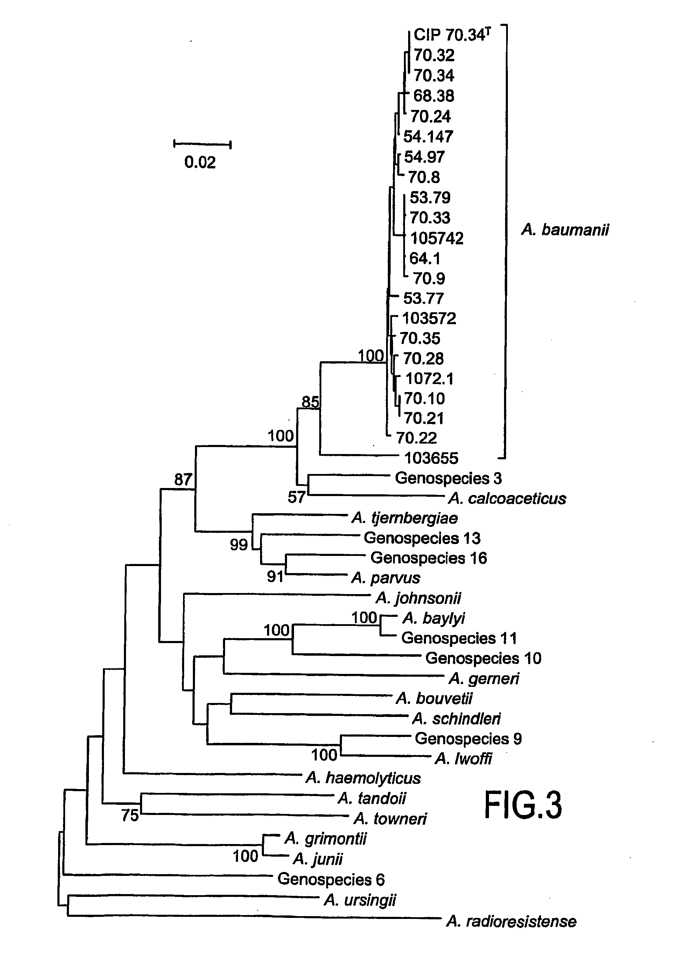

La figure 3 est un dendogramme représentant les relations phylogéniques des différentes espèces de *A. cinetobacter* par la méthode du "neighbour-joining". L'arbre a été construit par l'alignement des séquences hypervariable (zone 1 et zone 2) du gène rpoB. Les valeurs de "bootstrap" (probabilité d'exactitude des noeuds en pourcentage) calculées sur une base d'un échantillon de 1000 arbres sont indiquées à chaque noeud.

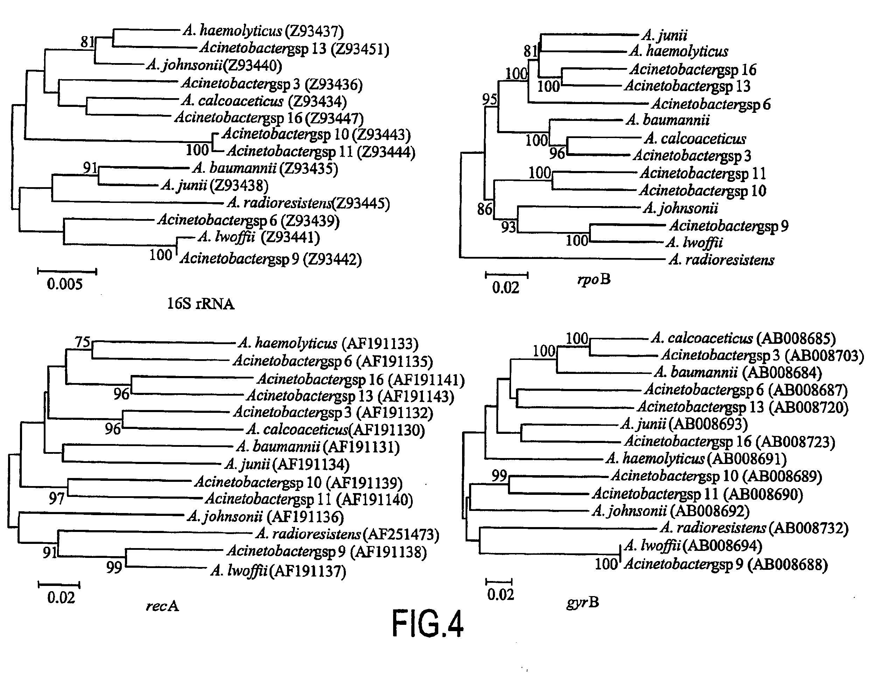

La figure 4 est un dendogramme représentant les relations phylogéniques des différentes espèces de *A. cinetobacter* par la méthode du "neighbour-joining". Les 4 arbres ont été construits par l'alignement des séquences des gène *rpo*B*,* 16SRNA, *rpo*B*, gyrB, recA* Les valeurs d'échantillonnage de "bootstrap" (probabilité d'exactitude des noeuds en pourcentage) calculées sur une base d'un échantillon de 1000 arbres, sont indiquées à chaque noeud, seules les valeurs supérieures ou égales à 75% sont indiquées

### 1- Matériels et méthodes.

### 1.1- Souches bactériennes.

Les souches bactériennes utilisées sont listées dans le tableau 1. Toutes les souches ont été cultivées sur géloses Columbia 5% de sang de mouton et ont été incubée 48 h à 37 °C en condition d'aérobie.

### 1.2- amplification et séquençage du gène rpoB et des fragment intergéniques rplL-rpoB et rpoB-rpoC.

La séquence du gène *rpo*B et des fragments intergéniques qui le bordent des espèces les plus proches, ont été alignées afin de produire une séquence consensus. Les séquences choisies étaient celles d'*Acinetobacter* sp. ADP1 (GeneBank accession number NC_005966), *Pseudomonas syringae* pv.tomato str.DC3000 (GeneBank accession number NC 004578) and *P. putida* KT2440 (GeneBank accession number NC 006347). La séquence consensus a permis de déterminer les amorces utilisées ensuite pour les PCR, la technique de marche sur le génome ("genome walking") et pour le séquençage. Certaines amorces ont été déterminées ultérieurement à l'analyse des résultats obtenus. Les amorces sont présentées au tableau 2 ci-après.

L'ADN bactérien a été extrait de suspensions des souches par QIAamp blood kit (Qiagen, Hilden, Germany) selon les recommandations du fabricant. Tous les mélanges réactionnels de PCR comportaient 2.5 X 10⁻² U de polymerase *Taq* par µl, 1X tampon *Taq,* 1.8 mM MgCl₂ (Gibco BRL, Life Technologies, Cergy Pontoise, France), 200 µM de dATP, dCTP, dTTP et dGTP (Boehringer Manheim GmbH, Hilden, Germany), et 0.2 µM de chaque amorce (Eurogentec, Seraing, Belgium). Les mélanges réactionnels de PCR ont été soumis à 35 cycles de dénaturation à 94°C pendant 30 s, une hybridation des amorces pendant 30 s, et une extension à 72°C pendant 2 min. Chaque programme d'amplification débutait par une étape de dénaturation à 95°C pendant 2 min. et terminait par une étape d'élongation à 72°C pendant 10 min. La détermination de la séquence des extrémités des gènes a été réalisée par l'utilisation du Universal GenomeWalker Kit (Clontech Laboratories, Palo Alto, CA). Brièvement, l'ADN génomique était digéré par *Eco* RV, *Dra* I, *Pvu* II, *Stu* I et *Sca* I. Les fragments d'ADN été liés avec le GenomeWalker adaptor, La PCR été réalisée en incorporant l'amorce "adaptor primer" fournie par le fabricant et les amorces spécifiques. Pour l'amplification, 1.5 U d'enzyme ELONGASE (Boehringer Manheim) été utilisée avec 10 pmol de chaque amorce, 20 mM de chaque dNTP, 10 mM Tris-HCl, 50 mM KCl, 1.6 mM MgCl₂ et 5 µl d'ADN digéré pour un volume final de 50 µl. Les amplicons ont été purifiés à l'aide du "QIAquick spin PCR purification kit" (Qiagen). Les réactions de séquence ont été réalisées à l'aide des réactifs du séquenceur ABI Prism 3100 ADN séquencer (dRhod.Terminator RR Mix, Perkin Elmer Applied Biosystems).

Ces conditions d'extraction de l'ADN et amplification PCR et séquençage ont été décrites dans Khamis et al 2003.

### 1.3- Détermination des séquences partielles discriminantes dans le gène rpoB.

Afin de détecter les portions de séquence avec une haute variabilité entourées de régions conservées, on a utilisé le programme SVARAP (for Sequence VARiability Analysis Program, Hypertext link "Téléchargement" at the URL: http://ifr48.free.fr/recherche/jeu_cadre/jeu_rickettsie.html). Une fois cette analyse faite, les zones les plus polymorphiques du gène *rpo*B ont été déterminées et des amorces universelles, choisies dans les zones bordantes conservées, ont été désignées après différents essais infructueux. Les conditions de PCR qui incorporaient les amorces universelles étaient les mêmes que précédemment mentionnées. Ces amorces ont été utilisées pour l'amplification et le séquençage des 4 zones hyper variables pour toutes les souches étudiées.

Ces amorces sont présentées au tableau 3 ci-après et figure 1.

### 1.4- Analyse des séquences rpoB et des fragments intergéniques qui le bordent.

Les fragments de séquences des gènes *rpo*B et des fragment intergéniques qui le bordent obtenus dans cette étude, ont été analysées à l'aide de "Sequence Analysis Software" (Applied Biosystems), et les séquences partielles ont été combinées en une seule séquence consensus à l'aide du "Sequence Assembler Software" (Applied Biosystems). Toutes les références de dépôt des souches à la Collection de l'Institut Pasteur (« CIP ») (France, Paris) sont listées dans le tableau 1. Les alignements multiples et les pourcentages de similitude entres les gènes des différentes espèces ont été réalisés par CLUSTAL W sur le serveur EMBL-EBI (http://www.ebi.ac.uk/clustalw/) (Thompson et al.1994). Des arbres phylogéniques ont été réalisés à partir des séquences par la méthode du "neighbor-joining"(Felsenstein et al.1989). Les "bootstraps" ont été réalisées pour évaluer la solidité des noeuds en utilisant SEQBOOT dans le logiciel PHYLIP.

### 2- Résultats

### a. Séquences complètes des gènes rpoB des différentes espèces d'Acinetobacter

Les amorces désignées ont permis d'amplifier les régions tests de toutes les souches du travail; la taille du gène complet est de 4089 pb pour toutes les espèces. Les pourcentages de similitude entre les souches varient de 83 à 94 %, excepté 2 paires d'espèces (tableau 4). En effet, 2 paires d'espèces, *A. junii*/*A. grimontii et A. baylyi*/*espèce génomique 11 ont* des similitudes de 99%. Les autres espèces ont moins de 95% de similitude entre elles.

### b. Identification des différentes espèces d'Acinetobacter basées sur les séquences partielles du gène rpoB.

Le programme SVARAP a permis l'identification de 2 zones variables bordées par des zones conservées qui ont permis de générer des amorces universelles:
- zone 1 : entre les positions 2900 et 3250, et
- zone 2 : entre les positions 3250 et 3700 bp (Figure 1).

Ces zones sont amplifiées à l'aide des amorces suscitées chez toutes les espèces d'*Acinetobacter* et toutes les souches d'A. *baumannii.* La taille de la zone 1 est de 350 pb et la zone 2 est de 450 pb. Le pourcentage de similitude entre les différentes espèces de la zone 1 varie de 78,6 à 95,4% pour toutes les espèces excepté 2 paires. En effet, comme pour la séquence complète, *A*. *baylyi*/*espèce génomique 11* et *A. junii*/*A. grimontii* ont les valeurs de similitude plus élevées, respectivement 98 et 99,1%, alors que les autres espèces ont moins de 96%. Le pourcentage de similitude la zone 2 est entre 75,8 et 95,3% pour toutes les espèces excepté, là encore, les espèces *A. junii*/*A. grimontii* et A. *baylyi*/*espèce génomique 11* qui ont de plus hautes similitude, respectivement 98,8 et 99,6%, alors que les autres espèces ont moins de 96% entre elles.

La similitude intra-spécifique des différentes souches *d'A. baumannii* pour la zone 1 varie de 98,3 à 100% à l'exception de la souche CIP 103655. En effet, cette souche ne possède qu'entre 94,9 et 95,7% avec les autres souches. De la même façon, la zone 2 varie de 98,7 à 100% pour toutes les souches de *A. Baumanii* à l'exception de la souche CIP 103655. Cette souche a des similitudes comprises entre 93,6 et 94,4% avec les autres souches de l'espèce. Les espèces les plus proches *d'A. baumannii* sont espèce génomique 3 pour la zone 1 et *A. calcoaceticus* pour la zone 2 avec des similitudes respectives de 95,1% et 93,6%. Ce sont des valeurs nettement inférieures à la variabilité intraspécifique à l'exception de la souche CIP 103655 pour la quelle la souche *d'A. baumannii* la plus éloignée possède 94,9% et 93,6% de similitude, respectivement dans les zones 1 et 2.

Au total, la variabilité de séquence est de 0,4 à 24,2% pour les séquences partielles contre 0,8 à 16,9% pour les séquences complètes de rpoB. Les 2 séquences partielles permettent donc une identification non ambiguë des 24 espèces.

### c. Analyse des zones flanquantes du gène rpoB (fragment intergénique rplL-rpoB et fragment intergénique rpoB-rpoC).

La taille des 2 fragments intergéniques est variable en fonction des espèces. La taille du fragment intergénique entre *rpl*L et *rpo*B varie de 301 à 310 pb (Tableau 1). Entre les espèces, le taux de similitude de ce fragment intergénique *rpl*L-*rpo*B varie de 80,8 à 96,9%, excepté que le fragment intergénique est identique entre *A. junnii* et *A*. *grimontii.,* et les couples d'espèces comme *A. baylyi*-espèce génomique 11 et *A. lwoffii*-espèce génomique 9 ont des taux de similitude comprises entre 98,4 et 99,7%.

La taille du fragment intergénique entre *rpo*B et *rpo*C varie de 86 à 177 pb (Table 1) avec des taux similitude entre les espèces comprises entre 70,2 et 96,5%, excepté pour *A. junnii*/*A. grimontii qui* ont une similitude élevée de 99,5%, et au sein du complexe *Acb* (*A. calcoaceticus, A*. *baumannii* and espèce génomique 3) dont les taux de similitude sont compris entre 98,5 et 99,0% pour le fragment intergénique *rpo*B-*rpo*C. En revanche, par rapport au fragment intergénique *rpl*L-*rpo*B, *A. baylyi*-espèce génomique 11 et *A*. *lwoffii-*espèce génomique 9 n'ont que 83,8 et 87,9% respectivement de taux de similitude pour le fragment intergénique *rpo*B-*rpo*C.

Au sein de l'espèce *A. baumannii,* la taille du fragment intergénique *rpl*L-*rpo*B est de 305 pb pour toutes les souches à l'exception de la souche CIP 103655 pour laquelle la taille est de 304 pb. Le fragment intergénique *rpo*B-*rpo*C a une taille de 86 pb pour toutes les souches. Toujours dans cette espèce, le pourcentage de similitude dans le fragment intergénique *rpol*L-*rpo*B varie de 99 à 100% pour toutes les souches à l'exception de la souche CIP 103655. Cette souche possède entre 96,1 et 96,4% de similitude avec les autres souches. L'autre fragment intergénique *rpo*B-*rpo*C est 100% similaire pour toutes les souches à l'exception de la souche CIP 103655 qui est 97,7 à 98,8% similaire aux autres souches. L'espèce la plus proche d'*A. baumannii* est espèce génomique 3 pour le fragment intergénique *rpl*L-*rpo*B et *A. calcoaceticus* pour le fragment intergénique *rpo*B-*rpo*C avec des similitudes respectives de 95,9% et 98,5%. Ce sont des valeurs nettement supérieures à la similitude intra-spécifique à l'exception de la souche CIP 103655 pour laquelles les espèces *d'A. baumannii* sont des similarités de 96,1% et 97,7% respectivement pour les fragment intergéniques *rpl*L-*rpo*B et *rpo*B-*rpo*C.

En fait, comme il ressort des positions des amorces des séquences SEQ. ID. n°5, 6, 7 et 8, les fragments correspondant aux amplifiats obtenus à l'aide de ces amorces ont des séquences qui dépassent celles des fragments intergéniques proprement dits aux extrémités 5' et 3', mais les séquences SEQ. ID. n°121 à 144 et 165 à 188 données dans le listage de séquences annexé à la présente description, correspondent aux fragments intergéniques complets et ne débordent pas dans les gènes rp/L, rpoB et rpoC respectivement.

### d. Analyse phylogénique des espèces d'Acinetobacter.

L'arbre phylogénique construit avec les séquences complètes du gène *rpo*B construit par la technique du neighbour-joining est supporté par de très hautes valeurs de bootstrap (Figure 2). Le nombre de valeurs de bootstrap > 75% est de 17/22 en utilisant le gène *rpo*B complet alors qu'il est de seulement 7/22 par l'utilisation du gène 16S rRNA (p< 0.01). Toutes les espèces sont bien séparées en différents groupes. L'arbre basé sur le gène *rpo*B partiel (zone 1 et zone 2 concaténées) montre un groupement homogène des souches d'*A*. *baumannii.* La souche CIP 103655 apparaît dans le même groupe mais est clairement séparée des autres isolats d'*A. baumannii.* Ce regroupement est supporté par une valeur de bootstrap de 85%.

### 2.4- Discussion.

A l'exception du gène 16S rRNA, il n'existe actuellement aucune séquence de gène de ménage réalisée sur toutes les espèces d'*Acinetobacter.* Les séquences des gènes gyrB et recA ne sont pas disponibles pour les 10 espèces les plus récemment décrites. En effet, Yamamoto et al (1996, 1999) et Krawczyk et al (2002) ont séquencé les gènes *gyr*B et *rec*A de 14 espèces et ont comparé cette technique à l'hybridation DNA-DNA (Bouvet and Jeanjean, 1989; Bouvet and Grimont, 1986; Tjernberg and Ursing, 1989). Par construction d'un arbre basé sur le gène *rpo*B qui incorpore 14 espèces, aucune congruence n'est observée entre les différentes espèces. Toutefois, l'arbre basé sur le gène *rpo*B a de façon significative plus de valeurs de bootstrap > 75% (11/12) que celui basé sur le gène 16S rRNA (4/12), *gyr*B (5/12) et *rec*A (6/12) (p < 0,01, p = 0,01 et p = 0,02 respectivement) (Figure 4). Cela démontre la robustesse de l'arbre basé sur le gène *rpo*B. *A. lwoffi* et *Acinetobacter* espèce génomique 9 sont 100% identiques sur le gène *gyr*B, mais sont séparés sur la séquence de *gyr*D et *rec*A (Yamamoto *et al.*, 1999; Krawczyk et al (2002). Les espèces mal délimitées par le gene *rpo*B sont les couples *A*. *grimontii*/*A. junii* et *A. baylii*/espèce génomique 9. Malheureusement il est impossible de les comparer en *gyr*B et *rec*A, les séquences de *A*. *grimontii* et *A. baylii* n'étant pas disponibles.

Pour l'identification moléculaire en routine des *Acinetobacter,* chacune des séquences partielles du gène rpoB et des 2 fragments intergéniques qui le bordent, peut être utilisée en raison de son pouvoir discriminant et de sa longueur. L'inconvénient de l'utilisation d'une seule de ces séquences est l'absence de bonne discrimination entre les paires *A. grimontii*/*A. junii* et *A*. *baylii*/espèce génomique 9 (Table 4 à 11). Cependant, cet inconvénient peut être réduit en combinant la séquence d'au moins 2 de ces séquences hypervariables. En raison de sa taille nous pensons qu'il est préférable de débuter par la séquence de la zone 1 car elle permet d'identifier parfaitement 20 espèces sur 24. Si les séquences obtenues sont celles de *A. grimontii*/*A. junii,* il vaut mieux par la suite réaliser la séquence de la zone 2 qui différencie mieux ces 2 espèces. Si la séquence obtenue est plus proche de *A*. *baylii*/espèce génomique 9, il sera préférable de déterminer la séquence du fragment intergénique *rpo*B-*rpo*C qui discrimine mieux ces 2 espèces.

La variabilité intra-spécifique des courts fragments observée au sein de l'espèce *A*. *baumannii* montre qu'à l'exception de la souche CIP 103655, tous les isolats ont des similitudes nettement inférieures à celles que l'on peut observer entre *A*. *baumannii* et les espèces qui en sont le plus proches. Toutefois, les similitudes faibles observées entre la souche d'A. baumannii CIP 103655 et les autres isolats de l'espèce montrent que l'identification de certains isolats de cette espèce peut rester ambiguë. L'espèce *A*. *baumannii* est l'espèce la plus fréquente en affection chez l'homme. Les résultats montrent que la souche CIP 103655 est une souche différentes des 24 espèces répertoriées et n'est vraisemblablement pas une souche d'espèce *A*. *baumannii.*

En conclusion, les résultats obtenus par l'utilisation des séquences partielles du gène *rpo*B et les fragments intergéniques *rpl*L-*rpo*B et *rpo*B-*rpo*C montrent que ces outils sont efficaces pour l'identification moléculaire de routine des souches d'*Acinetobacter.* Cependant, en raison de la forte similitude entre certaines espèces, des travaux complémentaires étudiant les similitudes intra-spécifiques dans plusieurs espèces seront nécessaires. De même, le statut de certaines souches comme la souche d'*A*. *baumannii* CIP 103655 et de certaines espèces comme *A*. *grimontii et A. baylii,* devra être étudié par hybridation ADN- ADN et séquences d'autres gènes de ménage (*rec*A and *gyr*B).

**Tableau 1. Souches d'Acinetobacter étudiées.**

| espèce | Souche | SEQ. ID. rpoB complete | SEQ. ID rpoB zone 1 | SEQ. ID. rpoB zone 2 | fragments intergéniquescomplets | | | |
|---|---|---|---|---|---|---|---|---|
| | | | | | *rpl*L - *rpo*B | | *rpo*B - *rpo*C | |
| | | | | | SEQ. ID. n° | Taille | SEQ. ID. | Taille |
| espèce génomique 1, *A. calcoaceticus* | CIP 81.8^{T} | 9 | 33 | 77 | 121 | 305 | 165 | 86 |
| espèce génomique 2, *baumannii* | CIP 70.34^{T} | 10 | 34 | 78 | 122 | 305 | 166 | 86 |
| | 1072.1 (ref) | | 57 | 101 | 144 | 305 | 189 | 86 |
| | CIP 53.77 | | 58 | 102 | 145 | 305 | 190 | 86 |
| | CIP 53.79 | | 59 | 103 | 146 | 305 | 191 | 86 |
| | CIP 54.97 | | 60 | 104 | 147 | 305 | 192 | 86 |
| | CIP 54.147 | | 61 | 105 | 148 | 305 | 193 | 86 |
| | CIP 64.1 | | 62 | 106 | 149 | 305 | 194 | 86 |
| | CIP 68.38 | | 63 | 107 | 150 | 305 | 195 | 86 |
| | CIP 70.8 | | 64 | 108 | 151 | 305 | 196 | 86 |
| | CIP 70.9 | | 65 | 109 | 152 | 305 | 197 | 86 |
| | CIP 70.10 | | 66 | 110 | 153 | 305 | 198 | 86 |
| | CIP 70.21 | | 67 | 111 | 154 | 305 | 199 | 86 |
| | CIP 70.22 | | 68 | 112 | 155 | 305 | 200 | 86 |
| | CIP 70.24 | | 69 | 113 | 156 | 305 | 201 | 86 |
| | CIP 70.28 | | 70 | 114 | 157 | 305 | 202 | 86 |
| | CIP 70.32 | | 71 | 115 | 158 | 305 | 203 | 86 |
| | CIP 70.33 | | 72 | 116 | 159 | 305 | 204 | 86 |
| | CIP 70.35 | | 73 | 117 | 160 | 305 | 205 | 86 |
| | CIP 103572 | | 74 | 118 | 161 | 305 | 206 | 86 |
| | CIP 103655 | | 75 | 119 | 162 | 304 | 207 | 86 |
| | CIP 105742 | | 76 | 120 | 163 | 304 | 208 | 86 |
| espèce génomique 3 | CIP 70.15 | 11 | 35 | 79 | 123 | 305 | 167 | 86 |
| espèce génomique 4, *A. haemolyticus* | CIP 64.3^{T} | 12 | 36 | 80 | 124 | 308 | 168 | 172 |
| espèce génomique 5, *A. junii* | CIP 64.5^{T} | 13 | 37 | 81 | 125 | 308 | 169 | 149 |
| espèce génomique 6 | CIP A165 | 14 | 38 | 82 | 126 | 308 | 170 | 170 |
| espèce génomique 7, *A. johnsonii* | CIP 64.6^{T} | 15 | 39 | 83 | 127 | 301 | 171 | 141 |
| espèce génomique 8, *A. lwoffii* | CIP 64.10^{T} | 16 | 40 | 84 | 128 | 308 | 172 | 177 |
| espèce génomique 9 | CIP 64.7 | 17 | 41 | 85 | 129 | 306 | 173 | 150 |
| espèce génomique 10 | CIP 70.12 | 18 | 42 | 86 | 130 | 307 | 174 | 144 |
| espèce génomique 11 | CIP 63.46 | 19 | 43 | 87 | 131 | 304 | 175 | 154 |
| espèce génomique 12, *A. radioresistens* | CIP 103788^{T} | 20 | 44 | 88 | 132 | 304 | 176 | 89 |
| espèce génomique 13 | CIP 70.18 | 21 | 45 | 89 | 133 | 309 | 177 | 154 |
| espèce génomique 16 | CIP 64.2 | 22 | 46 | 90 | 134 | 309 | 178 | 153 |
| *A. schindleri* | CIP 107287^{T} | 23 | 47 | 91 | 135 | 310 | 179 | 159 |
| *A. ursingii* | CIP 107286^{T} | 24 | 48 | 92 | 136 | 308 | 180 | 136 |
| *A. baylyi* | CIP 107474^{T} | 25 | 49 | 93 | 137 | 304 | 181 | 88 |
| *A. bouvetii* | CIP 107468^{T} | 26 | 50 | 94 | 138 | 305 | 182 | 156 |
| *A. gerneri* | CIP 107464^{T} | 27 | 51 | 95 | 139 | 309 | 183 | 170 |
| *A. grimontii* | CIP 107470^{T} | 28 | 52 | 96 | 140 | 308 | 184 | 150 |
| *A. tandoii* | CIP 107469^{T} | 29 | 53 | 97 | 141 | 306 | 185 | 156 |
| *A. tjernbergiae* | CIP 107465^{T} | 30 | 54 | 98 | 142 | 307 | 186 | 143 |
| *A. towneri* | CIP 107472^{T} | 31 | 55 | 99 | 143 | 307 | 187 | 157 |
| *A. parvus* | CIP 108168^{T} | 32 | 56 | 100 | 144 | 308 | 188 | 143 |

**Tableau 2. : Amorces utilisées pour amplifier le gène rpoB et ses zones flanquantes**

| No | Amorce | séquence (5'-3') | Position | *Tm* (°C) |
|---|---|---|---|---|
| 1. | AcintF^{a} | GGTAAAGTDACRCCTAAAGGT | | 60°C |
| 2. | AcintR^{a} | GTATGAACGTGGGDCAGATT | | 58°C |
| 3. | Ac28F^{a} | GTDGGTACVGGYATGGAA | | 52°C |
| 4. | Ac1754R^{a} | GAACGYGCRTGCATYTTGTCA | | 60°C |
| 5. | Ac822F | CGYAAAGAYTTGAAAGAAGA | | 54°C |
| 6. | Ac840R | CTTCTTTCAARTCTTTRCGRT | | 60°C |
| 7. | Ac660F | GAYGTDAAAGAYTCATCTTTA | | 54°C |
| 8. | Ac1720R | GAACGYGCRTGCATYTTGT | | 60°C |
| 9. | Ac660R | CGTAAAGATGARTCTTTHAC | | 54°C |
| 10. | Ac1700F | GACAARATGCAYGCRCGTT | | 60°C |
| 11. | Ac696F* | TAYCGYAAAGAYTTGAAAGAAG | | 60°C |
| 12. | Ac1093R* | CMACACCYTTGTTMCCRTGA | | 60°C |
| 13. | Ac1055F* | GTGATAARATGGCBGGTCGT | | 60°C |
| 14. | Ac1598R* | CGBGCRTGCATYTTGTCRT | | 58°C |
| 15. | Acint1F^{b} | AAGAAGCWGGYGCTAMAG | - | 55°C |
| 16. | Acint2F^{b} | CTKGGYCTKAAAGAAGCYAA | - | 58°C |
| 17. | Acint3F^{b} | CTGCTGCYGYTGTTGAAGA | - | 58°C |
| 18. | Acint1R^{b} | GGTAGTTRATGGTTTCMGG | + | 56°C |
| 19. | Acint2R^{b} | GTTRATGGTTTCMGGCTTYTT | + | 59°C |
| 20. | Acint3R^{b} | GGTTTCMGGCTTYTTAACTT | + | 56°C |
| 21. | Ac1F^{a} | ATGGCWTACTCAYATACYGA | 1 | 57°C |
| 22. | Ac4F^{a} | GCWTACTCATAYACYGARAA | 4 | 56°C |
| 23. | Ac8F^{a} | ACTCATAYACYGARAARAAAC | 8 | 56°C |
| 24. | Ac361F | GARCAAGAAGTMTACATGGG | 361 | 58°C |
| 25. | Ac1215F | GTTCAACCGYCGTWTSGGT | 1215 | 59°C |
| 26. | Ac1503F | GATCAACGCCAAGCCDGT | 1503 | 57°C |
| 27. | Ac2071F | GGYTCRAACATGCAGCGT | 2071 | 56°C |
| 28. | Ac2267F | GYGTVGAYATCTACAACCT | 2267 | 55°C |
| 29. | Ac3684F | TGAYGGHCGTACDGGYG | 3684 | 56°C |
| 30. | Ac3753F | CCAYTTRGTDGAYGACAAAAT | 3753 | 56°C |
| 31. | Ac3850F | TTCGGTGGTCAGCGYTTC | 3850 | 57°C |
| 32. | Ac28R | GTTTYTTYTCRGTRTATGAGT | 28 | 56°C |
| 33. | Ac55R | GCAAYTTRCYAAARTYCTT | 55 | 59°C |
| 34. | Ac211R | CAGCATTGCCRGARTARCT | 211 | 57°C |
| 35. | Ac380R | CCCATGTAKACTTCTTGYTC | 380 | 58°C |
| 36. | Ac1221R | GTTGAACTTCATVCGDCCWA | 1221 | 55°C |
| 37. | Ac1523R | GCHACHGGCTTGGCGTT | 1523 | 56°C |
| 38. | Ac2093R | GCCTGACGCTGCATGTT | 2093 | 55°C |
| 39. | Ac2314R^{a} | TGTTCTGGTTBGAACGVGT | 2314 | 56°C |
| 40. | Ac2928R^{a} | GHGCHGCTTCTTCRAAGA | 2928 | 55°C |
| 41. | Ac2936R^{a} | CGYTCACGHGCHGCTTCT | 2936 | 55°C |
| 42. | Ac1170F | GCTTCCATYTGGCGHACRT | 1170 | 58°C |
| 43. | Ac1705F | GTACGTCACGBACYTCRAA | 1705 | 58°C |
| 44. | Ac1804F | TCCATRAACTGDGAYAAYTG | 1804 | 56°C |
| 45. | Ac2231F | GTATCACGYGCDACACAHGA | 2231 | 60°C |
| 46. | Ac2348F | GTCATGAAYGCDACRCGCA | 2348 | 58°C |
| 47. | Ac1379R | CGGTTACCYAARTGRTCRAT | 1379 | 58°C |
| 48. | Ac1391R | GAACGNACRCGVCGGTTA | 1391 | 58°C |
| 49. | Ac2325R | GTTRATACADGTRTTYTGGTT | 2325 | 56°C |
| 50. | Ac2439R | GAACGCRACRCGCATGTT | 2439 | 56°C |
| 51. | Ac2442R | CATGAACGCRACRCGCAT | 2442 | 56°C |

**Tableau 3 : Amorces utilisées pour amplifier et séquencer la zone 1 et la zone 2 du gène rpoB ainsi que les fragments intergéniques qui la bordent des espèces d'Acinetobacter de la présente étude.**

| amorces | SEQ. ID. n° | séquence (5'-3') | Position* | *Tm* (°C) | Cible |
|---|---|---|---|---|---|
| Ac696F | 1 | TAYCGYAAAGAYTTGAAAGAAG | +2916 | 60°C | rpoB zone 1 |
| Ac1093R | 2 | CMACACCYTTGTTMCCRTGA | +3267 | 60°C | rpoB zone 1 |
| Ac1055F | 3 | GTGATAARATGGCBGGTCGT | +3263 | 60°C | rpoB zone 2 |
| Ac1598R | 4 | CGBGCRTGCATYTTGTCRT | +3773 | 58°C | rpoB zone 2 |
| AcintLBF | 5 | GAAGARCTTAAGAMDAARCTTG | -361 | 60°C | Spacer rplL-rpoB |
| AcintLBR | 6 | CGTTTCTTTTCGGTATATGAGT | +29 | 60°C | Spacer rplL-rpoB |
| AcintBCF | 7 | GTTCTTTAGGTATCAACATTGAA | +4048 | 60°C | Spacer rpoB-rpoC |
| AcintBCR | 8 | GACGCAAGACCAATACGRAT | +4207 | 59°C | Spacer rpoB-rpoC |

| | | | | | |
|---|---|---|---|---|---|
| * il s'agit de la position du premier nucléotide de la séquence amorce par rapport au gène rpoB | | | | | |

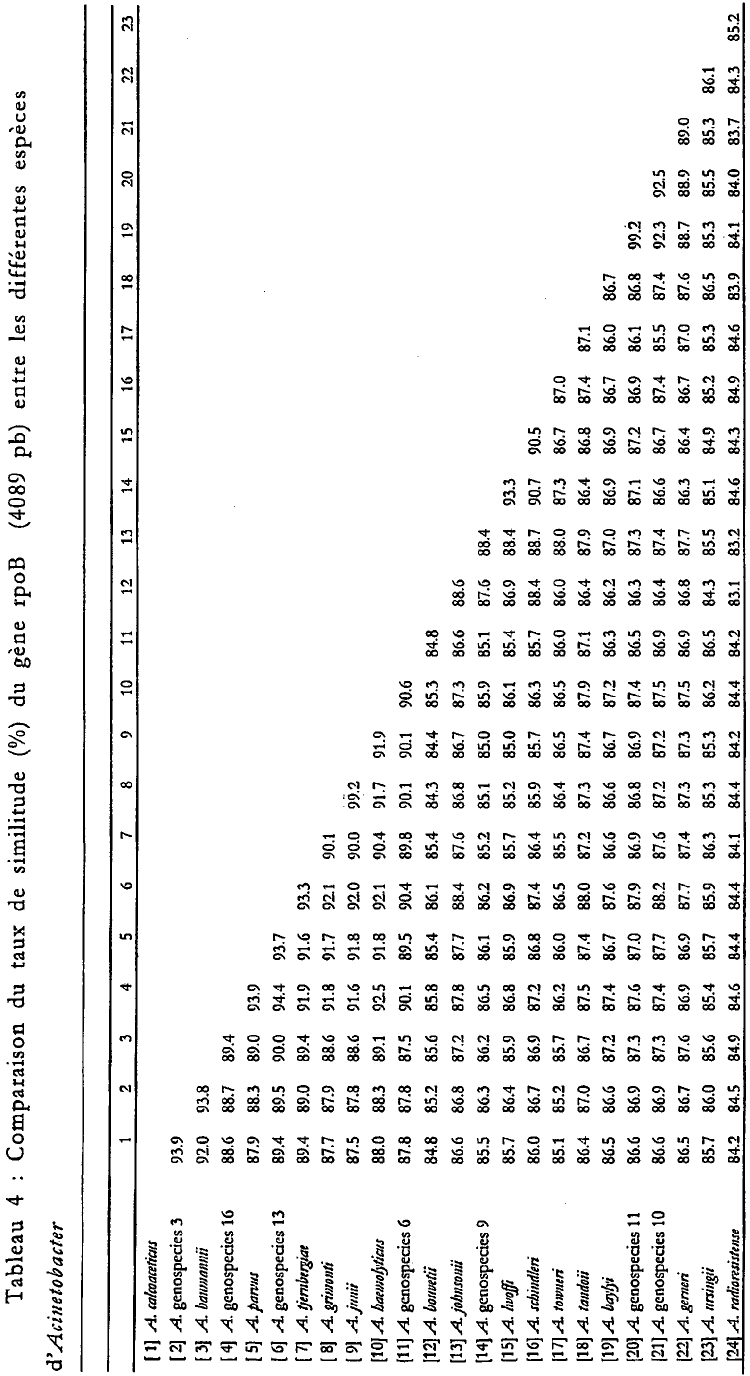

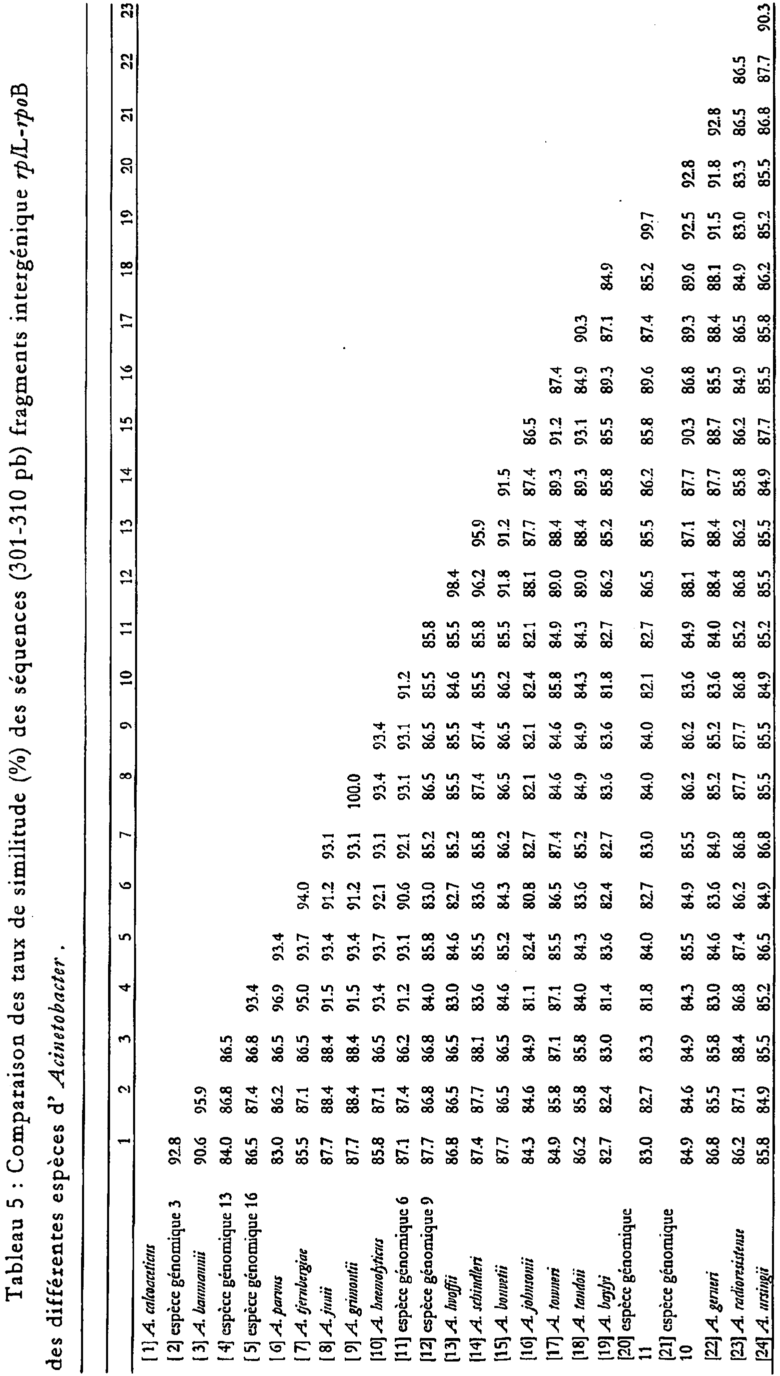

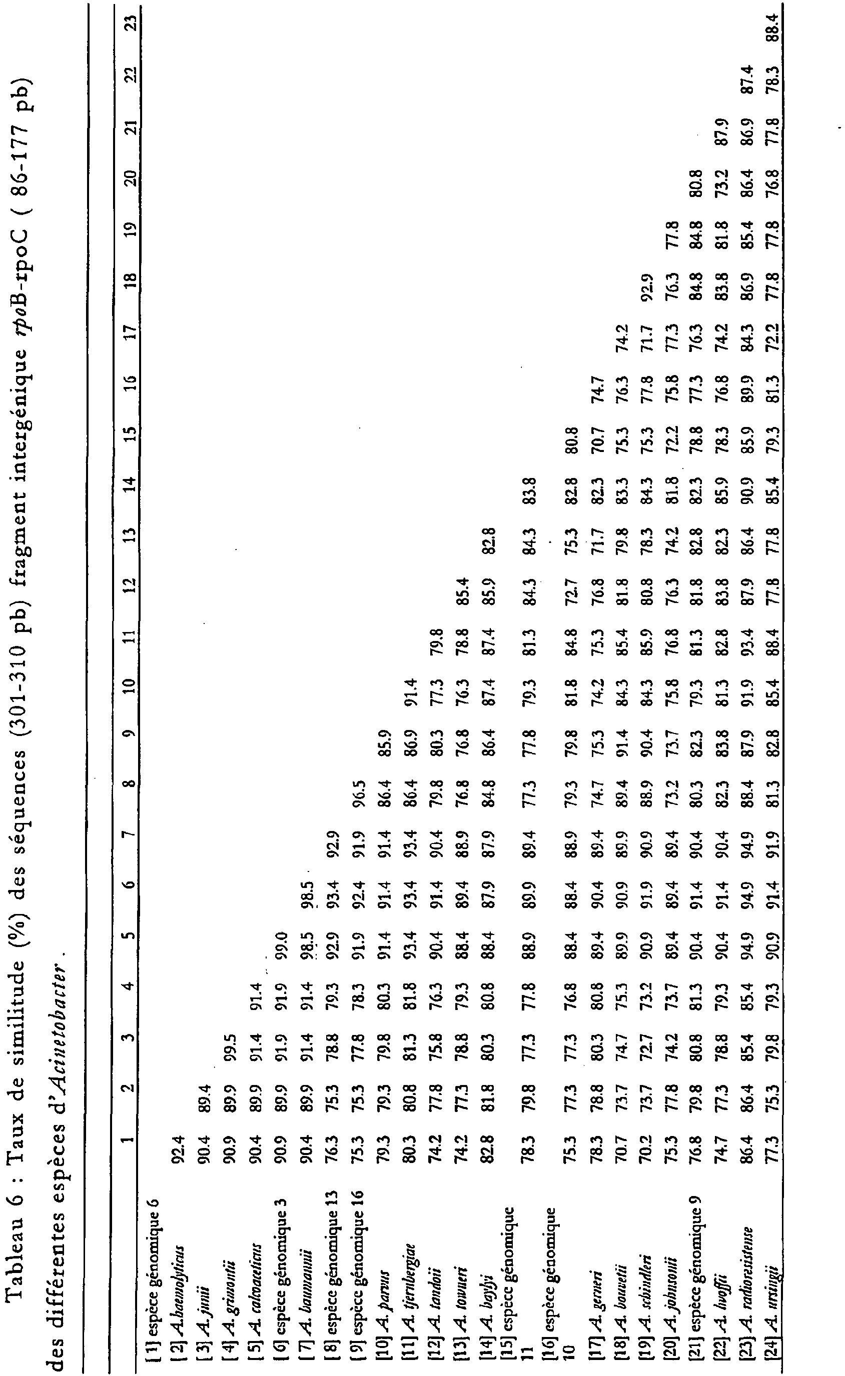

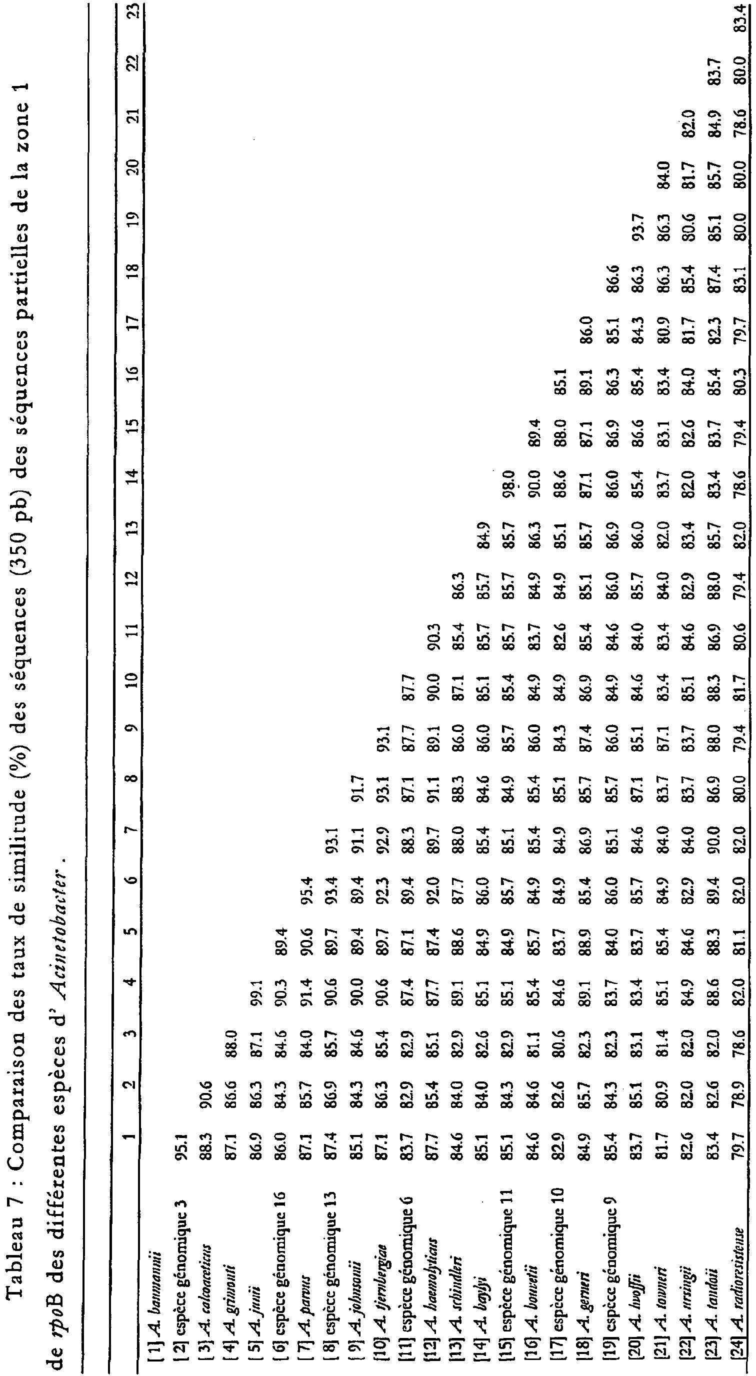

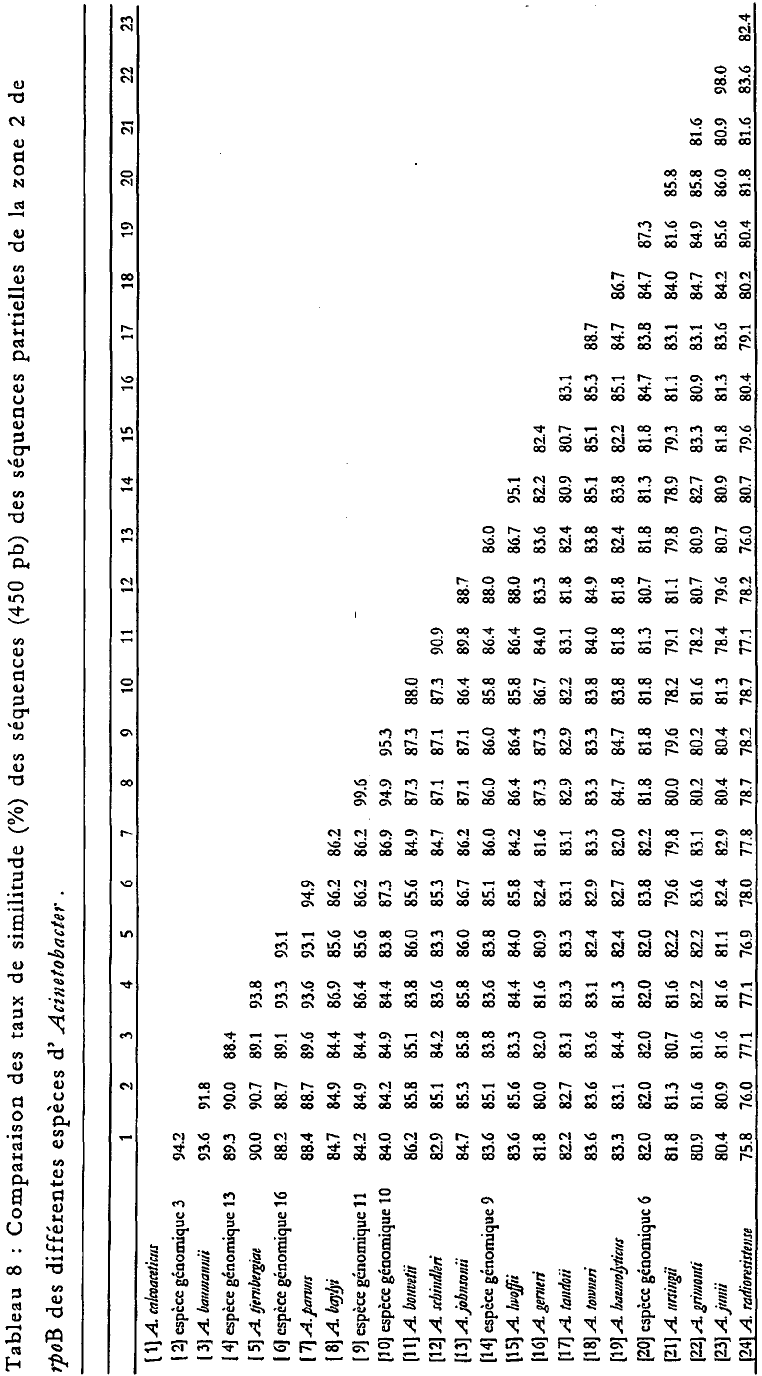

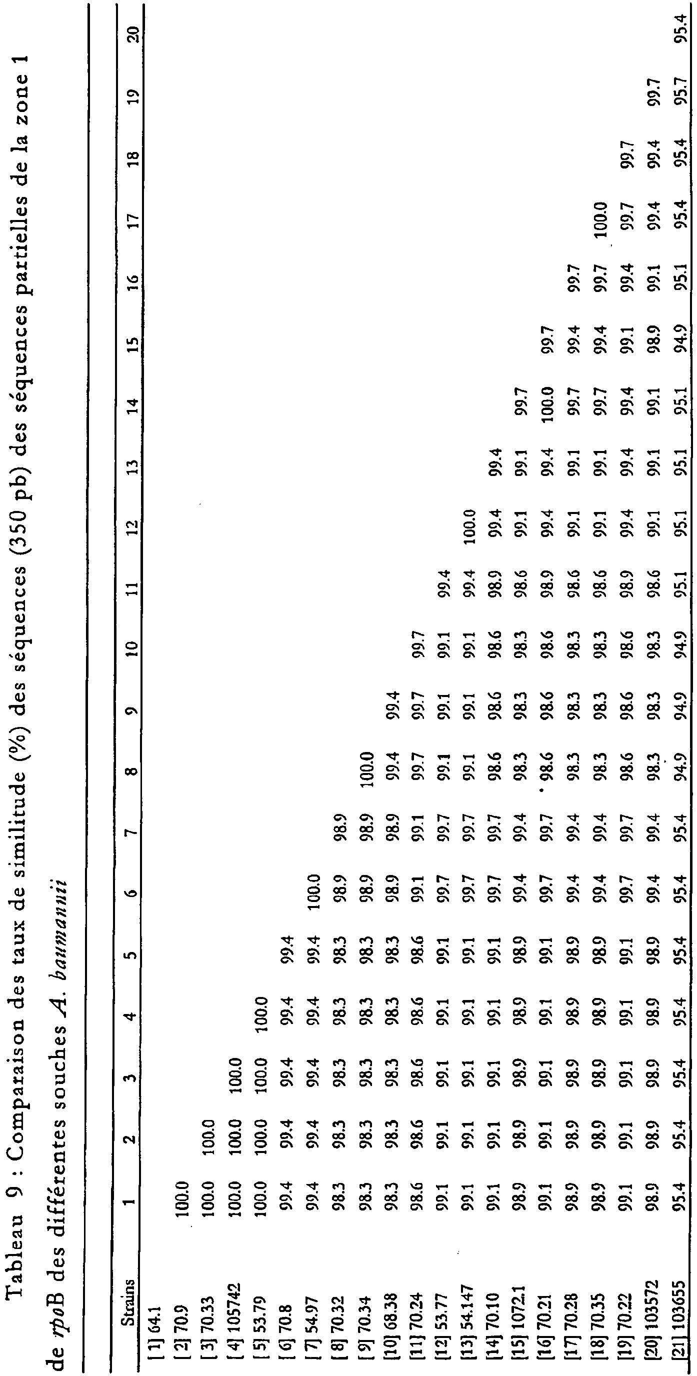

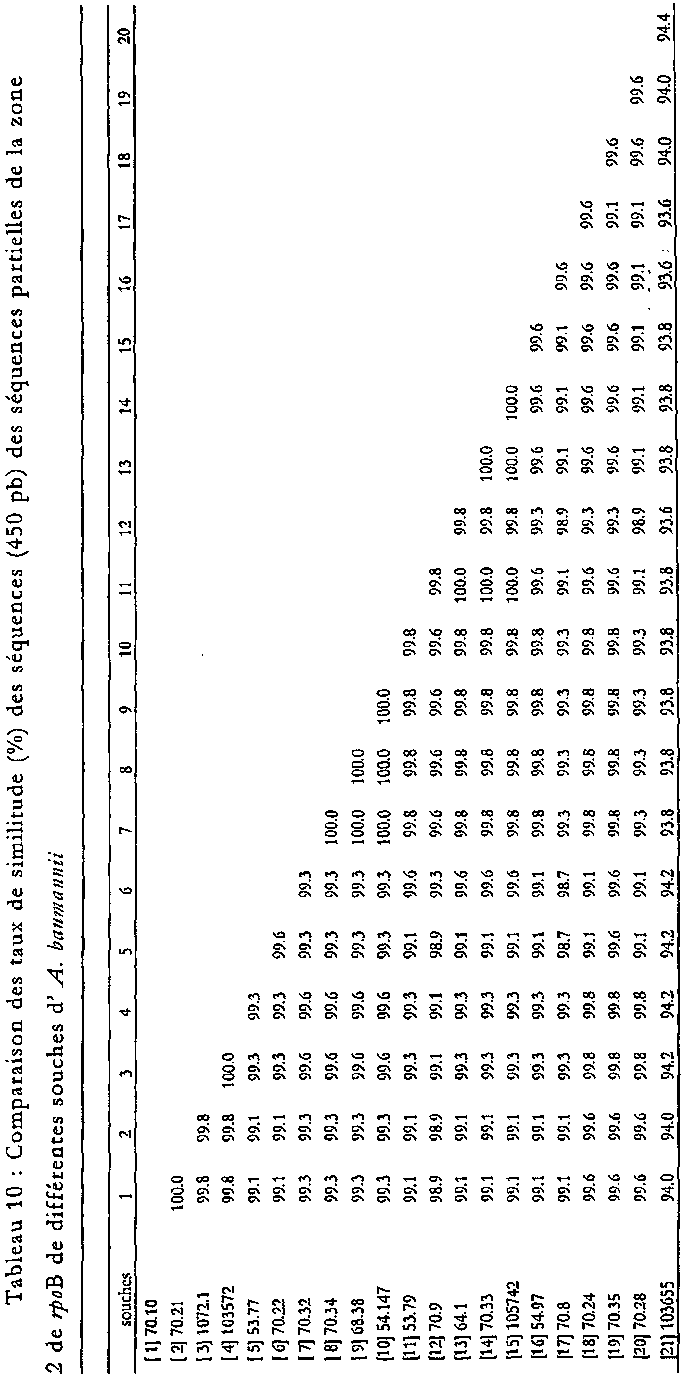

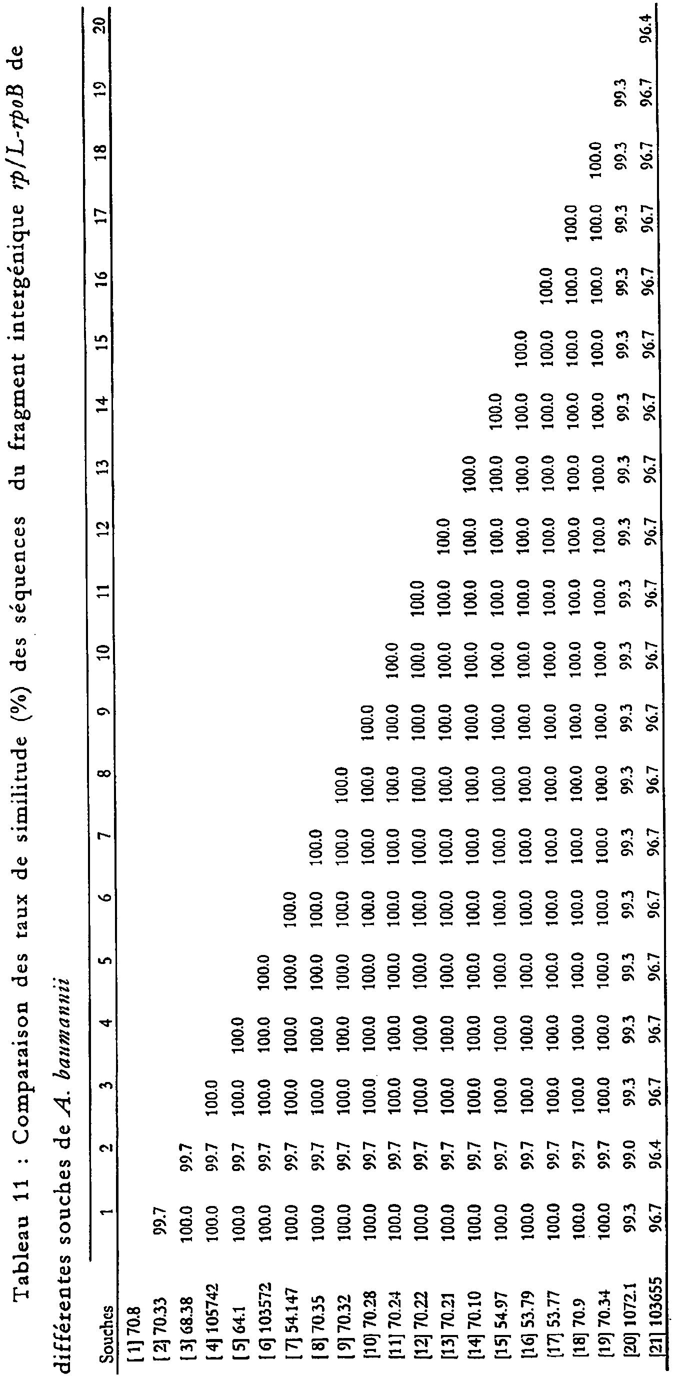

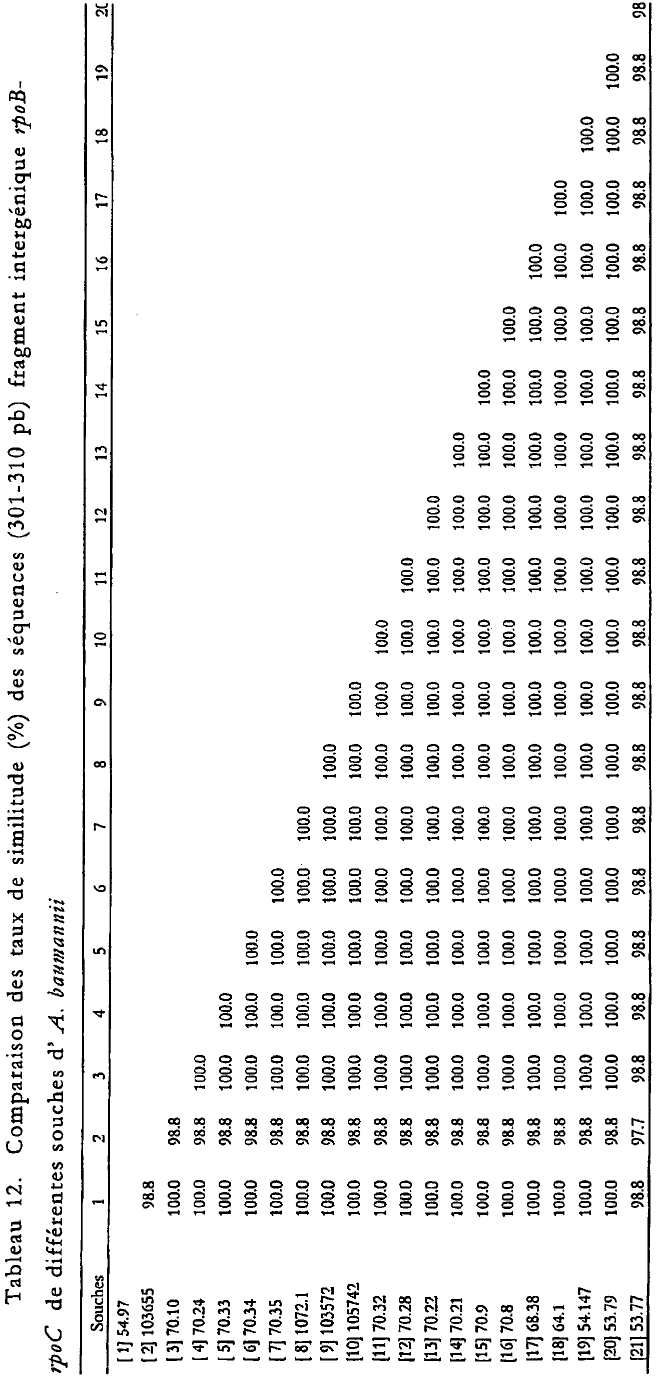

### Références bibliographiques

1. Bouvet PJ, Jeanjean S. Delineation of new proteolytic espèce génomique in the genus Acinetobacter. Res Microbiol. 1989 May-Jun;140(4-5):291-9.
2. Bouvet, P.J.M., and Grimont, P.A.D. Taxonomy of the genus Acinetobacter with the recognition of Acinetobacter baumannii sp. nov., Acinetobacter haemolyticus sp. nov., Acinetobacter johnsonii sp. nov., and Acinetobacter junii sp. nov. and emended descriptions of Acinetobacter calcoaceticus and Acinetobacter lwoffii. Int. J. Syst. Bacteriol., 1986, 36, 228-240.
3. Carr EL, Kampfer P, Patel BK, Gurtler V, Seviour RJ. Seven novel species of Acinetobacter isolated from activated sludge. Int J Syst Evol Microbiol. 2003 Jul;53(Pt 4):953-63.
4. Felseinstein 1989 1989. PHYLIPphylogeny inference package (version 3.2). Cladistics 5:164-166.
5. Gerner-Smidt P, Tjernberg I, Ursing J. Reliability of phenotypic tests for identification of Acinetobacter species. J Clin Microbiol. 1991 Feb;29(2):277-82.
6. Gerner-Smidt P. Ribotyping of the Acinetobacter calcoaceticus-Acinetobacter baumannii complex. J Clin Microbiol. 1992 Oct;30(10):2680-5.
7. Ibrahim A, Gerner-Smidt P, Liesack W. Phylogenetic relationship of the twenty-one DNA groups of the genus Acinetobacter as revealed by 16S ribosomal DNA sequence analysis. Int J Syst Bacteriol. 1997 Jul;47(3):837-41.
8. Khamis A, Colson P, Raoult D, Scola BL. Usefulness of rpoB gene sequencing for identification of Afipia and Bosea species, including a strategy for choosing discriminative partial sequences. Appl Environ Microbiol. 2003 Nov;69(11):6740-9.
9. Krawczyk B, Lewandowski K, Kur J. Comparative studies of the Acinetobacter genus and the species identification method based on the recA sequences. Mol Cell Probes. 2002 Feb;16(1):1-11.
10. Nemec A, De Baere T, Tjernberg I, Vaneechoutte M, van der Reijden TJ, Dijkshoorn L. Acinetobacter ursingii sp. nov. and Acinetobacter schindleri sp. nov., isolated from human clinical specimens. Int J Syst Evol Microbiol. 2001 Sep;51(Pt 5):1891-9
11. Nemec A, Dijkshoorn L, Cleenwerck I, De Baere T, Janssens D, Van Der Reijden TJ, Jezek P, Vaneechoutte M. Acinetobarter parvus sp. nov., a small-colony-forming species isolated from human clinical specimens. Int J Syst Evol Microbiol. 2003 Sep;53(Pt 5):1563-7.
12. Ochman and Wilson AC. R Evolution in bacteria: evidence for a universal substitution rate in cellular genomes. J Mol Evol. 1987;26:74-86.
13. Stackebrandt, E., and B. M. Goebel. 1994. Taxonomic note: a place for DNA-DNA reassociation and 16S rRNA sequence analysis in the present species definition in bacteriology. Int. J. Syst. Bacteriol. 44:846-849.
14. Rainey, F. A., E. Lang, and E. Stackebrandt. 1994. The phylogenetic structure of the genus Acinetobacter. FEMS Microbiol. Lett. 124:349-353.
15. Tjernberg I, Ursing J.Clinical strains of Acinetobacter classified by DNA-DNA hybridization. APMIS. 1989 Jul;97(7):595-605.
16. Towner KJ. Clinical importance and antibiotic resistance of Acinetobacter spp. Proceedings of a symposium held on 4-5 November 1996 at Eilat, Israel. J Med Microbiol. 1997 Sep;46(9):721-46.
17. Van Dessel H, Dijkshoorn L, Van Der Reijden T, Bakker N, Paauw A, Van Den Broek P, Verhoef J, Brisse S. Identification of a new geographically widespread multiresistant Acinetobacter baumannii clone from European hospitals. Res Microbiol. 2004 Mar; 155(2):105-12.
18. Thompson, J. D., D. G. Higgins, and T. J. Gibson. 1994. CLUSTAL W: improving the sensitivity of progressive multiple sequence alignment through sequence weighting, position-specific gap penalties and weight matrix choice. Nucleic Acids Res.22:4673-4680
19. Yamamoto, S., and S. Harayama. 1996. Phylogenetic analysis of Acinetobacter strains based on the nucleotide sequences of gyrB genes and on the amino acid sequences of their products. Int. J. Syst. Bacteriol. 46:506-511.
20. Yamamoto, S., and S. Harayama. 1998. Phylogenetic relationships of Pseudomonas putida strains deduced from the nucleotide sequences of gyrB, rpoD and 16S rRNA genes. Int. J. Syst. Bacteriol. 48:813-819.
21. Yamamoto, S., P. J. Bouvet, and S. Harayama. 1999. Phylogenetic structures of the genus Acinetobacter based on gyrB sequences: comparison with the grouping by DNA-DNA hybridization. Int. J. Syst. Bacteriol. 49:87-95.

## Revendications

1. Gène *rpoB* complet d'une bactérie du genre *Acinetobacter* choisie parmi les 23 espèces suivantes : *A. calcoaceticus* (espèce génomique 1), espèce génomique 3, *A. haemolyticus*(espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique *11, A. radioresistens* (espèce génomique 12), espèce génomique 13, espèce génomique 16, *A. schindleri, A. ursingii*, *A. baylyi*, *A. bouvetii*, *A. gerneri, A. grimontii*, *A. tandoii, A. tjernbergiae, A. towneri, A. parvus,* **caractérisé en ce que** sa séquence comprend une séquence choisie parmi les séquences telle que décrite dans les séquences SEQ. ID. n°9 et 11 à 32 respectivement, et les séquences présentant au moins 98% d'identité, ainsi que leurs séquences complémentaires.

2. Gène *rpoB* complet d'une bactérie du genre *Acinetobacter* choisie parmi lesdites 23 espèces selon la revendication 1, **caractérisé en ce que** sa séquence consiste en une séquence choisie parmi les séquences SEQ. ID. n°9 et 11 à 32, les séquences inverses et séquences complémentaires et les séquences présentant au moins 98% d'identité avec les dites séquences.

3. Fragment de gène *rpoB* d'une bactérie du genre *Acinetobacter* choisie parmi les 23 espèces suivantes : *A. calcoaceticus* (espèce génomique 1), espèce génomique 3, *A. haemolyticus*(espèce génomique 4), *A. junii* (espèce génomique 5), espèce génomique 6, *A. johnsonii* (espèce génomique 7), A. *lwoffii* (espèce génomique 8), espèce génomique 9, espèce génomique 10, espèce génomique 11, *A. radioresistens* (espèce génomique 12), espèce génomique 13, espèce génomique 16, *A. schindleri, A. ursingii, A. baylyi*, *A. bouvetii*, *A. gerneri, A. grimontii*, *A. tandoii, A. tjernbergiae, A. towneri, A. parvus,* **caractérisé en ce que** sa séquence comprend une séquence choisie parmi les séquences telles que décrites dans les séquences SEQ. ID. n°33 et 35 à 56 respectivement, et les séquences SEQ. ID. n°77 et 79 à 100 respectivement, et les séquences présentant au moins 98% d'identité et leurs séquences complémentaires.

4. Fragment de gène *rpoB* d'une bactérie du genre *Acinetobacter* choisie parmi les 24 espèces suivantes : *A. calcoaceticus* (espèce génomique 1), *A. baumannii* (espèce génomique 2), espèce génomique 3, A. *haemolyticus*(espèce génomique 4), *A. junnii* (espèce génomique 5), espèce génomique 6, *A. johnsonii* (espèce génomique 7), *A. lwoffïi* (espèce génomique 8), espèce génomique 9, espèce génomique 10, espèce génomique 11, A. *radioresistens* (espèce génomique 12), espèce génomique 13, espèce génomique 16, *A. schindleri, A. ursingii*, *A. baylyi*, *A. bouvetii*, *A. gerneri*, *A. grimontii*, *A. tandoii*, *A. tjernbergiae*, *A. towneri*, *A. parvus*, **caractérisé en ce que** sa séquence consiste en une séquence choisie parmi les séquences SEQ. ID. n°33 à 56 respectivement et les séquences SEQ. ID. n°77 à 100 respectivement, et les séquences présentant au moins 98% d'identité, et leurs séquences complémentaires.

5. Fragment intergénique comprenant une séquence non codante bordant le gène rpoB d'une bactérie du genre *Acinetobacter* choisie parmi les 24 espèces suivantes : *A. calcoaceticus* (espèce génomique 1), *A. baumannii* (espèce génomique 2), espèce génomique 3, *A. haemolyticus*(espèce génomique 4), *A. junii* (espèce génomique 5), espèce génomique 6, *A. johnsonii* (espèce génomique 7), *A. lwoffïi* (espèce génomique 8), espèce génomique 9, espèce génomique 10, espèce génomique 11, *A. radioresistens* (espèce génomique 12), espèce génomique 13, espèce génomique 16, *A. schindleri*, *A. ursingii*, *A. baylyi*, *A. bouvetii*, *A. gerneri*, *A. grimontii*, *A. tandoii*, *A. tjernbergiae*, *A. towneri*, *A. parvu* **caractérisé en ce que** sa séquence comprend une séquence choisie parmi les séquences telles que décrites dans les séquences SEQ. ID. n°121 à 144 respectivement, et les séquences SEQ. ID. n°165 à 188 respectivement, et les séquences présentant au moins 98% d'identité et leurs séquences complémentaires.

6. Fragment intergénique comprenant une séquence non codante bordant le gène *rpoB* d'une bactérie du genre *Acinetobacter* selon la revendication 5, **caractérisé en ce que** sa séquence consiste en une séquence choisie parmi les séquences SEQ. ID. n°121 à 144 respectivement et les séquences SEQ. ID. n°165 à 188 respectivement, et les séquences présentant au moins 98% d'identité, et leurs séquences complémentaires.

7. Fragment selon l'une des revendications 3 à 6, **caractérisé en ce qu'**il consiste en un oligonucléotide présentant une séquence spécifique d'une bactérie *Acitenobacter* choisie parmi les 24 espèces suivantes : *A. calcoaceticus* (espèce génomique 1), *A. baumannii* (espèce génomique 2), espèce génomique *3, A. haemolyticus* (espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, A. *radioresistens* (espèce génomique 12), espèce génomique 13, espèce génomique 16, *A. schindleri, A. ursingii*, *A. baylyi*, *A. bouvetii, A. gerneri, A. grimontii*, *A. tandoii, A. tjernbergiae, A. towneri, A. parvus.* **caractérisé en ce que** sa séquence consiste en une séquence d'au moins 18, de préférence de 18 à 35, motifs nucléotidiques consécutifs inclus dans l'une des séquences choisie parmi les séquences telles que décrites dans les séquences :
- SEQ. ID. n°33 à 56 respectivement,
- SEQ. ID. n°77 à 100 respectivement,
- SEQ. ID. n°121 à 144 respectivement,
- SEQ. ID. n°165 à 188 respectivement, et
- les séquences présentant au moins 98% d'identité et leurs séquences complémentaires.

8. Utilisation in vitro à titre de sonde d'espèce d'un fragment de gène selon l'une des revendications 3 à 6 ou un oligonucléotide selon la revendication 7.

9. Oligonucléotide **caractérisé en ce qu'**il présente une séquence conservée d'une bactérie *Acitenobacter* choisie parmi les 24 espèces suivantes :
*A. calcoaceticus* (espèce génomique 1), *A. baumannii* (espèce génomique 2), espèce génomique 3, *A. haemolyticus*(espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, *A. radioresistens* (espèce génomique 12), espèce génomique 13, espèce génomique 16, *A. schindleri, A. ursingii*, *A. baylyi*, *A. bouvetii, A. gerneri, A. grimontii*, *A. tandoii, A. tjernbergiae, A. towneri, A. parvu,* comprenant une séquence d'au moins 12, de préférence d'au moins 18 motifs nucléotidiques consécutifs inclus dans l'une des séquences choisie parmi les séquences telles que décrites dans les séquences SEQ. ID. n°1 à 8 suivantes, leurs séquences complémentaires :
- SEQ. ID. n° 1 : 5'-TAYCGYAAAGAYTTGAAAGAAG-3',
- SEQ. ID. n° 2 : 5'-CMACACCYTTGTTMCCRTGA-3',
- SEQ. ID. n° 3 : 5'-GTGATAARATGGCBGGTCGT-3',
- SEQ. ID. n° 4 : 5'-CGBGCRTGCATYTTGTCRT-3',
- SEQ. ID. n° 5 : 5'-GAAGARCTTAAGAMDAARCTTG-3'
- SEQ. ID. n° 6 : 5'-CGTTTCTTTTCGGTATATGAGT-3',
- SEQ. ID. n° 7 : 5'- GTTCTTTAGGTATCAACATTGAA-3',
- SEQ.ID. n° 8 : 5'- GACGCAAGACCAATACGRAT -3',
dans lesquelles :
- D représente A, G ou T,
- Y représente C ou T,
- B représente C, G ou T,
- R représente A ou G,et
- M représente A ou C.

10. Mélange d'oligonucléotides **caractérisé en ce qu'**il comprend un mélange équimolaire d'oligonucléotides tels que définis dans la revendication 9, de séquences différentes comprenant au moins 12, de préférence au moins 18 motifs nucléotidiques consécutifs inclus dans l'une des séquences SEQ. ID. n° 1 à 5 et 8 ou les oligonucléotides de séquences complémentaires.

11. Mélange d'oligonucléotides selon la revendication 10, **caractérisé en ce qu'**il consiste en un mélange équimolaire de 8 oligonucléotides de séquences différentes consistant dans la séquence SEQ. ID. n°1, ou des oligonucléotides de séquences complémentaires.

12. Mélange d'oligonucléotides selon la revendication 10, **caractérisé en ce qu'**il consiste en un mélange équimolaire de 16 oligonucléotides de séquences différentes consistant dans la séquence SEQ. ID. n°2 ou des oligonucléotides de séquences complémentaires.

13. Mélange d'oligonucléotides selon la revendication 10, **caractérisé en ce qu'**il consiste en un mélange équimolaire de 6 oligonucléotides de séquences différentes consistant dans la séquence SEQ. ID. n°3 ou des oligonucléotides de séquences complémentaires.

14. Mélange d'oligonucléotides selon la revendication 10, **caractérisé en ce qu'**il consiste en un mélange équimolaire de 24 oligonucléotides de séquences différentes consistant dans la séquence SEQ. ID. n° 4 ou des oligonucléotides de séquences complémentaires.

15. Mélange d'oligonucléotides selon la revendication 10, **caractérisé en ce qu'**il consiste en un mélange équimolaire de 24 oligonucléotides de séquences différentes consistant dans la séquence SEQ. ID. n° 5 ou des oligonucléotides de séquences complémentaires.

16. Oligonucléotide selon la revendication 9, **caractérisé en ce que** sa séquence consiste dans la séquence SEQ ID. n°6 ou séquence complémentaire.

17. Oligonucléotide selon la revendication 9, **caractérisé en ce que** sa séquence consiste dans la séquence SEQ. ID. n°7 ou une séquence complémentaire.

18. Mélange d'oligonucléotides selon la revendication 10, **caractérisé en ce qu'**il consiste en un mélange équimolaire de 2 oligonucléotides de séquences différentes consistant dans la séquence SEQ. ID. n°8 ou des oligonucléotides de séquences complémentaires.

19. Utilisation à titre d'amorce d'amplification et/ou de réaction de séquençage d'un oligonucléotide ou mélange d'oligonucléotides selon l'une des revendications 9 à 18, pour la détection par identification moléculaire d'une bactérie de l'une desdites espèces du genre *Acitenobacter.*

20. Procédé de détection par identification moléculaire d'une bactérie de l'une des espèces du genre *Acitenobacter,* **caractérisé en ce qu'**on utilise :
- le gène *rpoB* complet de ladite bactérie selon la revendication 1 ou 2, ou
- un fragment de gène selon l'une des revendications 3 à 6, et/ou
- un oligonucléotide selon l'une des revendications 7, 9, 16 ou 17 ou un mélange d'oligonucléotides selon l'une des revendications 10 à 15 ou 18.

21. Procédé selon la revendication 20, **caractérisé en ce qu'**on cherche à détecter spécifiquement une espèce donnée d'une bactérie *Acitenobacter* choisie parmi les 24 espèces suivantes : *A. calcoaceticus* (espèce génomique 1), *A. baumannii* (espèce génomique 2), espèce génomique 3, A. *haemolyticus*(espèce génomique 4), *A. junii* (espèce génomique 5), espèce génomique 6, *A. johnsonii* (espèce génomique 7), *A. lwoffii* (espèce génomique 8), espèce génomique 9, espèce génomique 10, espèce génomique 11, A. *radioresistens* (espèce génomique 12), espèce génomique 13, espèce génomique 16, *A. schindleri, A. ursingii, A. baylyi*, *A. bouvetii*, *A. gerneri, A. grimontii*, *A. tandoii, A. tjernbergiae, A. towneri, A. parvus* , procédé dans lequel :
1- on met en contact un échantillon contenant ou susceptible de contenir des acides nucléiques d'au moins une telle bactérie, avec au moins une sonde d'espèce consistant dans un oligonucléotide selon le revendication 7 ou un fragment de gène selon l'une des revendications 3 à 7, de préférence un fragment de gène consistant respectivement dans l'une desdites séquences choisie parmi :
- SEQ. ID. n°33 à 56 respectivement,
- SEQ. ID. n°77 à 100 respectivement,
- SEQ. ID. n°121 à 144 respectivement,
- SEQ. ID. n°165 à 188 respectivement, et
- les séquences présentant au moins 98% d'identité et leurs séquences inverses et séquences complémentaires, et
2- on détermine la formation ou l'absence d'un complexe d'hybridation entre ladite sonde et les acides nucléiques de l'échantillon, et on détermine ainsi la présence de ladite espèce de *Acinetobacter* dans l'échantillon s'il y a formation d'un complexe d'hybridation.

22. Procédé selon la revendication 20, **caractérisé en ce qu'**il comprend les étapes dans lesquelles :
1- on met en contact des amorces d'amplification comprenant desdits mélanges d'oligonucléotides selon l'une des revendications 10 à 15 ou 18, avec un échantillon contenant ou susceptible de contenir des acides nucléiques d'au moins une telle bactérie du genre *Acinetobacter,* et on réalise une amplification d'acides nucléiques par réaction de polymérisation enzymatique comprenant :
- comme amorce 5', au moins un oligonucléotide ou mélange d'oligonucléotides selon l'une des revendications 10, 11, 13, 15 et 17 comprenant une séquence incluse dans l'une des séquences SEQ.ID. n° 1, 3, 5, et 7 de préférence consistant dans ladite séquence SEQ. ID. n°1, 3, 5, et 7 complète ou les séquences complémentaires, et
- comme amorce 3¹, au moins un oligonucléotide ou mélange d'oligonucléotides selon l'une des revendications 10, 12, 14, 16, et 18 comprenant des séquences incluses dans l'une des séquences SEQ. ID. n° 2, 4 6, et 8 respectivement, de préférence consistant dans ladite séquence SED ID n°2, 4, 6, et 8 complète ou respectivement une séquence complémentaire.
2- et on détermine l'apparition ou l'absence d'un produit d'amplification, et on détermine ainsi la présence ou l'absence de ladite bactérie dans l'échantillon si un produit d'amplification est ou n'est pas apparu respectivement.

23. Procédé selon la revendication 22, **caractérisé en ce qu'**on cherche à détecter une espèce donnée d'une bactérie bactérie *Acitenobacter* choisie parmi les 24 espèces suivantes : *A. calcoaceticus (espèce génomique 1), A. baumannii (espèce génomique* 2), *espèce génomique 3, A. haemolyticus(espèce génomique 4), A. junii (espèce génomique 5), espèce génomique 6, A. johnsonii (espèce génomique 7), A. lwoffii (espèce génomique 8), espèce génomique 9, espèce génomique 10, espèce génomique 11, A. radioresistens (espèce génomique 12), espèce génomique 13, espèce génomique 16, A. schindleri, A. ursingii*, *A. baylyi*, *A. bouvetii*, *A. gerneri, A. grimontii, A. tandoii, A. tjernbergiae, A. towneri, A.parvus*, et, à l'étape 2, on détermine la présence ou l'absence de l'espèce donnée d'une dite bactérie en effectuant les étapes dans lesquelles :
a) on réalise une réaction de séquençage d'un fragment de gène amplifié avec des dites amorces, et
b) on compare la séquence dudit fragment amplifié obtenu avec la séquence d'un fragment de gène de ladite bactérie comprenant respectivement :
- lesdites séquences SEQ. ID. n° 33 à 56, lorsque les dites amorces 5' et 3' sont des oligonucléotides de séquences incluses dans les séquences SEQ.ID. n°1 et 2 respectivement
- les dites séquences SEQ. ID. n° 77 à 100, lorsque les dites amorces 5' et 3' sont des oligonucléotides de séquences incluses dans les séquences SEQ. ID. n° 3 et 4 respectivement, et
- les dites séquences SEQ. ID. n° 121 à 144, lorsque les dites amorces 5' et 3' sont des oligonucléotides de séquences incluses dans les séquences SEQ. ID. n° 5 et 6 respectivement, et
- les dites séquences SEQ. ID. n° 165 à 188, lorsque les dites amorces 5' et 3' sont des oligonucléotides de séquences incluses dans les séquences SEQ. ID. n° 7 et 8 respectivement.

24. Procédé selon la revendication 22, **caractérisé en ce qu'**on cherche à détecter une espèce donnée d'une bactérie Acitenobacter choisie parmi les 24 espèces suivantes : *A. calcoaceticus (espèce génomique 1), A. baumannii (espèce génomique* 2), *espèce génomique 3, A. haemolyticus(espèce génomique 4), A. junii (espèce génomique 5), espèce génomique 6, A. johnsonii (espèce génomique 7), A. lwoffii (espèce génomique 8), espèce génomique 9, espèce génomique 10, espèce génomique 11, A. radioresistens (espèce génomique 12), espèce génomique 13, espèce génomique 16, A. schindleri, A. ursingii*, *A. baylyi*, *A. bouvetii*, *A. gerneri, A. grimontii*, *A. tandoii, A. tjernbergiae, A. towneri, A.parvus*, et, à l'étape 2, on détermine la présence ou l'absence de l'espèce donnée d'une dite bactérie par en effectuant les étapes dans lesquelles :
a- on met en contact un échantillon contenant ou susceptible de contenir des acides nucléiques amplifié d'au moins une telle bactérie, avec au moins une sonde d'espèce consistant dans un fragment de gène *rpoB* selon l'une des revendications 3 à 6, ou un oligonucléotide selon la revendication 7, de préférence un fragment consistant respectivement dans l'une desdites séquences choisie parmi :
- SEQ. ID. n°33 à 56 respectivement, lorsque les dites amorces 5' et 3' sont des oligonucléotides de séquences incluses dans les séquences SEQ.ID. n° 1 et 2 respectivement
- SEQ. ID. n°77 à 100 respectivement, lorsque les dites amorces 5' et 3' sont des oligonucléotides de séquences incluses dans les séquences SEQ. ID. n° 3 et 4 respectivement, et
- SEQ. ID. n°121 à 144 respectivement, lorsque les dites amorces 5' et 3' sont des oligonucléotides de séquences incluses dans les séquences SEQ. ID. n° 5 et 6 respectivement, et
- SEQ. ID. n°165 à 188 respectivement, lorsque les dites amorces 5' et 3' sont des oligonucléotides de séquences incluses dans les séquences SEQ. ID. n° 7 et 8 respectivement, et.
b- on détermine la formation ou l'absence d'un complexe d'hybridation entre ladite sonde et les acides nucléiques amplifié de l'échantillon, et on détermine ainsi la présence ou l'absence de ladite espèce de *Acinetobacter* dans l'échantillon s'il y a formation ou non d'un complexe d'hybridation.

25. Procédé selon l'une des revendications 22 à 24, **caractérisé en ce que** l'on réalise les étapes comprenant :
1- une première amplification de l'acide nucléique dudit échantillon avec un couple d'amorces 5' et 3' choisi parmi desdits mélanges d'oligonucléotides selon les revendications 11 et 12, comprenant des séquences incluses respectivement dans les séquences SEQ. ID. n°1 et SEQ. ID. n°2, de préférence consistant dans lesdites séquences SEQ. ID. n°1 et 2, ou les séquences complémentaires, et
2- une première détermination de l'apparition ou l'absence d'un produit d'amplification comprenant des acides nucléiques d'au moins une dite bactérie, par hybridation ou le cas échéant séquençage et comparaison des amplifiats obtenus à l'étape 1 avec les fragments consistant respectivement dans l'une desdites séquences choisie parmi SEQ. ID. n°33 à 56 respectivement, et
- si à cette étape 2 on détermine la présence des espèces *A. grimontii* ou *A. junii,* on réalise en outre:
3a - une seconde réaction d'amplification avec des amorces 5' et 3' choisi parmi desdits mélanges d'oligonucléotides selon la revendication 10 ou les revendications 13 et 14, comprenant des séquences incluses respectivement dans les séquences SEQ. ID. n° 3 et SEQ. ID. n° 4, de préférence consistant dans lesdites séquences SEQ. ID. n°3 et 4, ou les séquences complémentaires, et
4a- une détermination de l'apparition ou l'absence d'un produit d'amplification comprenant des acides nucléiques d'au moins une dite bactérie, par hybridation ou le cas échéant séquençage et comparaison des amplifiats obtenus à l'étape 3a avec les fragments consistant respectivement dans l'une desdites séquences choisie parmi SEQ. ID. n°77 à 100 respectivement, ou
- si à cette étape 2 on détermine la présence des espèces *A. baylii* ou A. *génomique espèce 11*, on réalise en outre :
3b- une seconde réaction d'amplification avec des amorces 5' et 3' choisi parmi desdits mélanges d'oligonucléotides selon les revendications 17 et 18, comprenant des séquences incluses respectivement dans les séquences SEQ. ID. n° 7 et SEQ. ID. n°8, de préférence consistant dans lesdites séquences SEQ. ID. n° 7 et 8, ou les séquences complémentaires, et
4b- une détermination de l'apparition ou l'absence d'un produit d'amplification comprenant des acides nucléiques d'au moins une dite bactérie, par hybridation ou le cas échéant séquençage et comparaison des amplifiats obtenus à l'étape 3b avec les fragments consistant respectivement dans l'une desdites séquences choisie parmi SEQ. ID. n°165 à 188 respectivement.

26. Trousse de diagnostic utile dans un procédé selon l'une des revendications 20 à 25, **caractérisée en ce qu'**elle comprend au moins un dit oligonucléotide selon l'une des revendications 7, 9, 16 ou 17 ou un mélange d'oligonucléotides selon l'une des revendications 10 à 15 ou 18 ou un fragment de gène selon l'une des revendications 3 à 6, ainsi que, de préférence, des réactifs utiles dans les réactions d'hybridations ou réactions d'amplification ou séquençage le cas échéant.

## Patentansprüche

1. Vollständiges Gen rpoB einer Bakterie der Gattung Acinetobacter, ausgewählt aus den folgenden 23 Spezies: A. calcoaceticus (genomische Spezies 1), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, **dadurch gekennzeichnet, dass** seine Sequenz eine Sequenz umfasst, ausgewählt aus den Sequenzen, beschrieben in den Sequenzen SEQ. ID. NO 9 bzw. 11 bis 32 und den Sequenzen, die mindestens 98 % Ähnlichkeit aufweisen sowie deren komplementären Sequenzen.

2. Vollständiges Gen rpoB einer Bakterie der Gattung Acinetobacter, ausgewählt aus den 23 Spezies nach Anspruch 1, **dadurch gekennzeichnet, dass** die Sequenz aus einer Sequenz besteht, ausgewählt aus den Sequenzen SEQ. ID. NO 9 und 11 bis 32, den inversen Sequenzen und komplementären Sequenzen und den Sequenzen, die mindestens 98 % Ähnlichkeit mit diesen Sequenzen aufweisen.

3. Fragment des Gens rpoB einer Bakterie der Gattung Acinetobacter, ausgewählt aus den folgenden 23 Spezies: A. calcoaceticus (genomische Spezies 1), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, **dadurch gekennzeichnet, dass** seine Sequenz eine Sequenz umfasst, ausgewählt aus den Sequenzen, beschrieben in den Sequenzen SEQ. ID. NO: 33 bzw. 35 bis 56 und den Sequenzen SEQ. ID NO: 77 bzw.79 bis 100 und den Sequenzen, die mindestens 98 % Ähnlichkeit aufweisen und deren komplementären Sequenzen.

4. Fragment des Gens rpoB einer Bakterie der Gattung Acinetobacter, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, **dadurch gekennzeichnet, dass** seine Sequenz aus einer Sequenz besteht, jeweils ausgewählt aus den Sequenzen SEQ. ID. NO: 33 bis 56 und jeweils den Sequenzen SEQ. ID. NO: 77 bis 100 und den Sequenzen, die mindestens 98 % Ähnlichkeit aufweisen und deren komplementären Sequenzen.

5. Nicht kodierendes Fragment, flankierend das Gen rpoB einer Bakterie der Gattung Acinetobacter, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, **dadurch gekennzeichnet, dass** seine Sequenz aus einer Sequenz besteht, jeweils ausgewählt aus den Sequenzen SEQ. ID. NO: 121 bis 144 und jeweils den Sequenzen SEQ. ID. NO: 165 bis 188 und den Sequenzen, die mindestens 98 % Ähnlichkeit aufweisen und deren komplementären Sequenzen.

6. Nichtkodierendes Genfragment, flankierend das Gen rpoB einer Bakterie der Gattung Acinetobacter nach Anspruch 5, **dadurch gekennzeichnet, dass** seine Sequenz aus einer Sequenz besteht, jeweils ausgewählt aus den Sequenzen SEQ. ID. NO : 121 bis 144 und jeweils den Sequenzen SEQ. ID. NO : 165 bis 188 und den Sequenzen, die mindestens 98 % Ähnlichkeit aufweisen und deren komplementären Sequenzen.

7. Oligonukleotid, **dadurch gekennzeichnet, dass** es eine spezifische Sequenz einer Bakterie Acinetobacter aufweist, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. *grimontii*, A. tandoii, A. tjernbergiae, A. towneri, A. parvus, **dadurch gekennzeichnet, dass** seine Sequenz eine Sequenz aus mindestens 18, stärker bevorzugt aus 18 bis 35 aufeinanderfolgenden Nukleotidmotiven umfasst, die in einer der Sequenzen enthalten sind, ausgewählt aus den Sequenzen, wie in den Sequenzen:
- jeweils SEQ. ID. NO: 33 bis 56,
- jeweils SEQ. ID. NO: 77 bis 100,
- jeweils SEQ. ID. NO: 121 bis 144,
- jeweils SEQ. ID. NO: 165 bis 188 beschrieben, und
- den Sequenzen, die mindestens 98 % Ähnlichkeit aufweisen und deren komplementären Sequenzen.

8. In-vitro-Verwendung als Speziessonde eines Genfragments nach einem der Ansprüche 3 bis 6 oder eines Oligonukleotids nach Anspruch 7.

9. Oligonukleotid, **dadurch gekennzeichnet, dass** es eine konservierte Sequenz einer Bakterie Acinetobacter aufweist, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, umfassend eine Sequenz mit mindestens 12, bevorzugt mindestens 18 aufeinanderfolgenden Nukleotidmotiven, die in einer der Sequenzen enthalten sind, ausgewählt aus den Sequenzen, wie in den folgenden Sequenzen SEQ. ID. NO: 1 bis 8 beschrieben, deren komplementären Sequenzen:
- SEQ. ID. NO: 1 : 5'-TAYCGYAAAGAYTTGAAAGAAG-3',
- SEQ. ID. NO: 2 : 5'-CMACACCYTTGTTMCCRTGA-3',
- SEQ. ID. NO: 3 : 5'-GTGATAARATGGCBGGTCGT-3',
- SEQ. ID. NO: 4 : 5'-CGBGCRTGCATYTTGTCRT-3',
- SEQ. ID. NO: 5 :5'-GAAGARCTTAAGAMDAARCTTG-3'
- SEQ. ID. NO: 6 : 5'-CGTTTCTTTTCGGTATATGAGT-3',
- SEQ. ID. NO: 7 : 5'- GTTCTTTAGGTATCAACATTGAA -3',
- SEQ.ID. NO: 8: 5'- GACGCAAGACCAATACGRAT -3',
worin:
- D für A, G oder T steht,
- Y für C oder T steht,
- B für C, G oder T steht,
- R für A oder G steht, und
- M für A oder C steht.

10. Oligonukleotidgemisch, **dadurch gekennzeichnet, dass** es ein äquimolares Gemisch aus Oligonukleodiden umfasst, wie sie in Anspruch 9 definiert wurden, mit unterschiedlichen Sequenzen, umfassend mindestens 12, bevorzugt mindestens 18 aufeinanderfolgende Nukleotidmotive, die in einer der Sequenzen SEQ. ID. NO: 1 bis 5 und 8 enthalten sind, oder den Oligonukleotiden mit komplementären Sequenzen.

11. Olignukleotidgemisch nach Anspruch 10, **dadurch gekennzeichnet, dass** es aus einem äquimolaren Gemisch aus 8 Oligonukleotiden mit unterschiedlichen Sequenzen besteht, bestehend aus der Sequenz SEQ. ID. NO: 1 oder Oligonukleotiden mit komplementären Sequenzen.

12. Olignukleotidgemisch nach Anspruch 10, **dadurch gekennzeichnet, dass** es aus einem äquimolaren Gemisch aus 16 Oligonukleotiden mit unterschiedlichen Sequenzen besteht, bestehend aus der Sequenz SEQ. ID. NO: 2 oder Oligonukleotiden mit komplementären Sequenzen.

13. Olignukleotidgemisch nach Anspruch 10, **dadurch gekennzeichnet, dass** es aus einem äquimolaren Gemisch aus 6 Oligonukleotiden mit unterschiedlichen Sequenzen besteht, bestehend aus der Sequenz SEQ. ID. NO: 3 oder Oligonukleotiden mit komplementären Sequenzen.

14. Olignukleotidgemisch nach Anspruch 10, **dadurch gekennzeichnet, dass** es aus einem äquimolaren Gemisch aus 24 Oligonukleotiden mit unterschiedlichen Sequenzen besteht, bestehend aus der Sequenz SEQ. ID. NO: 4 oder Oligonukleotiden mit komplementären Sequenzen.

15. Olignukleotidgemisch nach Anspruch 10, **dadurch gekennzeichnet, dass** es aus einem äquimolaren Gemisch aus 24 Oligonukleotiden mit unterschiedlichen Sequenzen besteht, bestehend aus der Sequenz SEQ. ID. NO: 5 oder Oligonukleotiden mit komplementären Sequenzen.

16. Oligonukleotid nach Anspruch 9, **dadurch gekennzeichnet, dass** seine Sequenz aus der Sequenz SEQ ID. NO: 6 oder einer komplementären Sequenz besteht.

17. Oligonukleotid nach Anspruch 9, **dadurch gekennzeichnet, dass** seine Sequenz aus der Sequenz SEQ ID. NO: 7 oder einer komplementären Sequenz besteht.

18. Olignukleotidgemisch nach Anspruch 10, **dadurch gekennzeichnet, dass** es aus einem äquimolaren Gemisch aus 2 Oligonukleotiden mit unterschiedlichen Sequenzen besteht, bestehend aus der Sequenz SEQ. ID. NO: 8 oder Oligonukleotiden mit komplementären Sequenzen.

19. Verwendung als Amplifikationsprimer und/oder Sequenzierungsreaktionsprimer eines Oligonukleotids oder eines Oligonukleotidgemischs gemäß einem der Ansprüche 9 bis 18 zum Nachweis durch Molekularidentifizierung einer Bakterie einer der genannten Spezies der Gattung Acinetobacter.

20. Verfahren zum Nachweis durch Molekularidentifizierung einer Bakterie einer der Spezies der Gattung Acinetobacter, **dadurch gekennzeichnet, dass**:
- das vollständige Gen rpoB dieser Bakterie nach Anspruch 1 oder 2 oder
- ein Genfragment nach einem der Ansprüche 3 bis 6 und/oder
- ein Oligonukleotid nach einem der Ansprüche 7, 9, 16 oder 17 oder ein Oligonukleotidgemisch nach einem der Ansprüche 10 bis 15 oder 18 verwendet wird.

21. Verfahren nach Anspruch 20, **dadurch gekennzeichnet, dass** versucht wird, spezifisch eine gegebene Spezies einer Bakterie Acinetobacter nachzuweisen, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, wobei in dem Verfahren:
1 - eine Probe, enthaltend oder befähigt zu enthalten Nukleinsäuren mindestens einer solchen Bakterie, mit mindestens einer Speziessonde in Kontakt gebracht wird, bestehend aus einem Oligonukleotid nach Anspruch 7 oder einem Genfragment nach einem der Ansprüche 3 bis 7, bevorzugt einem Genfragment, bestehend jeweils aus einer dieser Sequenzen, ausgewählt aus:
- jeweils SEQ. ID. NO: 33 bis 56,
- jeweils SEQ. ID. NO: 77 bis 100,
- jeweils SEQ. ID. NO: 121 bis 144,
- jeweils SEQ. ID. NO: 165 bis 188, und
- den Sequenzen, die mindestens 98 % Ähnlichkeit aufweisen und deren inversen Sequenzen und komplementären Sequenzen.
2 - die Bildung oder die Abwesenheit eines Hybridisierungskomplexes zwischen der Sonde und den Nukleinsäuren der Probe bestimmt wird, und auf diese Weise die Gegenwart der Acinetobacter-Spezies in der Probe bestimmt wird, wenn es eine Bildung eines Hybridisierungskomplexes gibt.

22. Verfahren nach Anspruch 20, **dadurch gekennzeichnet, dass** es die folgenden Schritte umfasst, worin:
1 - Amplifikationsprimer, umfassend die Oligonukleotidgemische nach einem der Ansprüche 10 bis 15 oder 18, mit einer Probe in Kontakt gebracht werden, enthaltend oder befähigt zu enthalten Nukleinsäuren mindestens einer solchen Bakterie der Gattung Acinetobacter, und eine Nukleinsäureamplifikation durch enzymatische Polymerisationsreaktion ausgeführt wird, umfassend:
- als 5'-Primer, mindestens ein Oligonukleotid oder Oligonukleotidgemisch nach einem der Ansprüche 10, 11, 13, 15 und 17, umfassend eine Sequenz, die in einer der Sequenzen SEQ. ID. NO: 1, 3, 5 und 7 enthalten ist, bevorzugt bestehend aus der vollständigen Sequenz SEQ. ID. NO: 1, 3, 5 und 7 oder den komplementären Sequenzen, und
- als 3'-Primer mindestens ein Oligonukleotid oder Oligonukleotidgemisch nach einem der Ansprüche 10, 12, 14, 16 und 18, umfassend Sequenzen, die in einer der Sequenzen SEQ. ID. NO: 2, 4 6 bzw. 8 enthalten sind, bevorzugt bestehend aus der vollständigen Sequenz SED ID NO: 2, 4, 6 und 8 bzw. einer komplementären Sequenz.
2 - und das Entstehen oder die Abwesenheit eines Amplifikationsprodukts bestimmt wird und auf diese Weise die Gegenwart oder Abwesenheit der Bakterie in der Probe bestimmt wird, wenn ein Amplifikationsprodukt entstanden bzw. nicht entstanden ist.

23. Verfahren nach Anspruch 22, **dadurch gekennzeichnet, dass** versucht wird, eine gegebene Spezies einer Bakterie Acinetobacter nachzuweisen, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, und in Schritt 2, die Gegenwart oder Abwesenheit einer gegebenen Spezies dieser Bakterie bestimmt wird, indem die folgenden Schritte durchgeführt werden, worin:
a) eine Sequenzierungsreaktion eines Genfragments, das mit diesen Primern amplifiziert wurde, ausgeführt wird, und
b) die Sequenz des amplifizierten Fragments verglichen wird, das mit der Sequenz eines Genfragments dieser Bakterie erhalten wurde, jeweils umfassend:
- die Sequenzen SEQ. ID. NO 33 bis 56, wenn diese 5'- und 3'-Primer Oligonukleotide mit Sequenzen sind, die in den Sequenzen SEQ.ID. NO: 1 bzw. 2 enthalten sind,
- diese Sequenzen SEQ. ID. NO: 77 bis 100, wenn die 5'- und 3'-Primer Oligonukleotide mit Sequenzen sind, die in den Sequenzen SEQ. ID. NO: 3 bzw. 4 enthalten sind, und
- diese Sequenzen SEQ. ID. NO: 121 bis 144, wenn die 5'- und 3'-Primer Oligonukleotide mit Sequenzen sind, die in den Sequenzen SEQ. ID. NO: 5 bzw. 6 enthalten sind, und
- diese Sequenzen SEQ. ID. NO: 165 bis 188, wenn die 5'- und 3'-Primer Oligonukleotide mit Sequenzen sind, die in den Sequenzen SEQ. ID. NO: 7 bzw. 8 enthalten sind.

24. Verfahren nach Anspruch 22, **dadurch gekennzeichnet, dass** versucht wird, eine gegebene Spezies einer Bakterie Acinetobacter nachzuweisen, ausgewählt aus den folgenden 24 Spezies: A. calcoaceticus (genomische Spezies 1), A. baumannii (genomische Spezies 2), genomische Spezies 3, A. haemolyticus (genomische Spezies 4), A. junii (genomische Spezies 5), genomische Spezies 6, A. johnsonii (genomische Spezies 7), A. lwoffii (genomische Spezies 8), genomische Spezies 9, genomische Spezies 10, genomische Spezies 11, A. radioresistens (genomische Spezies 12), genomische Spezies 13, genomische Spezies 16, A. schindleri, A. ursingii, A. baylyi, A. bouvetii, A. gerneri, A. grimontii, A. tandoii, A. tjernbergiae, A. towneri, A. parvus, und in Schritt 2, die Gegenwart oder Abwesenheit einer gegebenen Spezies einer genannten Bakterie bestimmt wird, indem die folgenden Schritte durchgeführt werden, worin:
a - eine Probe, enthaltend oder befähigt zu enthalten amplifizierte Nukleinsäuren mindestens einer solchen Bakterie, mit mindestens einer Speziessonde in Kontakt gebracht wird, bestehend aus einem Fragment des Gens rpoB nach einem der Ansprüche 3 bis 6, oder einem Oligonukleotid nach Anspruch 7, bevorzugt einem Fragment, bestehend jeweils aus einer dieser Sequenzen, ausgewählt aus:
- jeweils SEQ. ID. NO: 33 bis 56, wenn diese 5'- und 3'-Primer Oligonukleotide mit Sequenzen sind, die in den Sequenzen SEQ.ID. NO: 1 bzw. 2 enthalten sind,
- jeweils SEQ. ID. NO: 77 bis 100, wenn diese 5'- und 3'-Primer Oligonukleotide mit Sequenzen sind, die in den Sequenzen SEQ.ID. NO: 3 bzw. 4 enthalten sind und,
- jeweils SEQ. ID. NO: 121 bis 144, wenn diese 5'- und 3'-Primer Oligonukleotide mit Sequenzen sind, die in den Sequenzen SEQ.ID. NO: 5 bzw. 6 enthalten sind und,
- jeweils SEQ. ID. NO: 165 bis 188, wenn diese 5'- und 3'-Primer Oligonukleotide mit Sequenzen sind, die in den Sequenzen SEQ.ID. NO: 7 bzw. 8 enthalten sind, und
b - die Bildung oder die Abwesenheit eines Hybridisierungskomplexes zwischen der Sonde und den amplifizierten Nukleinsäuren der Probe bestimmt wird, und auf diese Weise die Gegenwart oder Abwesenheit der Acinetobacter-Spezies in der Probe bestimmt wird, wenn es eine Bildung eines Hybridisierungskomplexes gibt oder nicht gibt.

25. Verfahren nach einem der Ansprüche 22 bis 24, **dadurch gekennzeichnet, dass** die folgenden Schritte ausgeführt werden, umfassend:
1 - eine erste Amplifikation der Nukleinsäure der Probe mit einem 5'- und 3'-Primerpaar, ausgewählt aus den Oligonukleotidgemischen nach den Ansprüchen 11 und 12, umfassend Sequenzen, die in den Sequenzen SEQ. ID. NO: 1 bzw. SEQ. ID. NO: 2 enthalten sind, die bevorzugt aus diesen Sequenzen SEQ. ID. NO: 1 und 2 oder den komplementären Sequenzen bestehen, und
2 - eine erste Bestimmung des Entstehens oder der Abwesenheit eines Amplifikationsprodukts, umfassend Nukleinsäuren von mindestens einer genannten Bakterie, durch Hybridisierung oder gegebenenfalls Sequenzierung, und Vergleich des in Schritt 1 erhaltenen Amplifikationsprodukt mit den Fragmenten, die jeweils aus einer der genannten Sequenzen bestehen, ausgewählt aus jeweils SEQ. ID. NO: 33 bis 56, und
- wenn in diesem Schritt 2 die Gegenwart der Spezies A. grimontii oder A. junii bestimmt wird, ferner ausgeführt wird:
3a - eine zweite Amplifikationsreaktion mit 5'- und 3'-Primern, ausgewählt aus den Oligonukleotidgemischen nach Anspruch 10 oder den Ansprüchen 13 und 14, umfassend Sequenzen, die in den Sequenzen SEQ. ID. NO: 3 bzw. SEQ. ID. NO: 4 enthalten sind, die bevorzugt aus diesen Sequenzen SEQ. ID. NO: 3 und 4 oder den komplementären Sequenzen bestehen, und
4a - eine Bestimmung des Entstehens oder der Abwesenheit eines Amplifikationsprodukts, umfassend Nukleinsäuren von mindestens einer genannten Bakterie, durch Hybridisierung oder gegebenenfalls Sequenzierung, und Vergleich des in Schritt 3a erhaltenen Amplifikationsprodukts mit den Fragmenten, die jeweils aus einer der genannten Sequenzen bestehen, ausgewählt aus jeweils SEQ. ID. NO: 77 bis 100, oder
- wenn in diesem Schritt 2 die Gegenwart der Spezies A. baylyi oder A. genomische Spezies 11 bestimmt wird, ferner ausgeführt wird:
3b - eine zweite Amplifikationsreaktion mit 5'- und 3'-Primern, ausgewählt aus den Oligonukleotidgemischen nach den Ansprüchen 17 und 18, umfassend Sequenzen, die in den Sequenzen SEQ. ID. NO: 7 bzw. SEQ. ID. NO: 8 enthalten sind, die bevorzugt aus diesen Sequenzen SEQ. ID. NO: 7 und 8 oder den komplementären Sequenzen bestehen, und
4b - eine Bestimmung des Entstehens oder der Abwesenheit eines Amplifikationsprodukts, umfassend Nukleinsäuren von mindestens einer genannten Bakterie, durch Hybridisierung oder gegebenenfalls Sequenzierung und Vergleich des in Schritt 3b erhaltenen Amplifikationsprodukts mit den Fragmenten, die jeweils aus einer der genannten Sequenzen bestehen, ausgewählt aus jeweils SEQ. ID. NO: 165 bis 188.

26. Diagnosekit, der in einem Verfahren nach einem der Ansprüche 20 bis 25 nützlich ist, **dadurch gekennzeichnet, dass** er mindestens eines der Oligonukleotide nach einem der Ansprüche 7, 9, 16 oder 17 oder ein Oligonukleotidgemisch nach einem der Ansprüche 10 bis 15 oder 18 oder ein Genfragment nach einem der Ansprüche 3 bis 6 sowie bevorzugt Reagenzien umfasst, die in den Hybridisierungsreaktionen oder Amplifikationsreaktionen oder gegebenenfalls Sequenzierungsreaktionen nützlich sind.

## Claims

1. A complete *rpoB* gene of a bacterium of the genus *Acinetobacter* chosen from among the following 23 species: A. *calcoaceticus* (genomic species 1), genomic species 3, A. *haemolyticus* (genomic species 4), *A. junii* (genomic species 5), genomic species 6, *A. johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. baylyi, A. bouvetii, A. gerneri, A. grimontii, A. tandoii, A. tjernbergiae, A. towneri, A. parvus,* **characterized in that** its sequence comprises a sequence chosen from among the sequences such as described in sequences SEQ. ID. Nos. 9 and 11 to 32 respectively, and the sequences having at least 98% similarity, as well as their complementary sequences.

2. A complete *rpoB* gene of a bacterium of the genus *Acinetobacter* chosen from among said 23 species according to claim 1, **characterized in that** its sequence consists of a sequence chosen from among sequences SEQ. ID. Nos. 9 and 11 to 32, the reverse sequences and complementary sequences and sequences having at least 98% similarity with said sequences.

3. An *rpoB* gene fragment of a bacterium of the genus *Acinetobacter* chosen from among the following 23 species: A. *calcoaceticus* (genomic species 1), genomic species 3, A. *haemolyticus* (genomic species 4), *A. junii* (genomic species 5), genomic species 6, A. *johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. baylyi, A. bouvetii, A. gerneri, A. grimontii, A. tandoii, A. tjernbergiae, A. towneri, A. parvus,* **characterized in that** its sequence comprises a sequence chosen from among the sequences such as described in sequences SEQ. ID. Nos. 33 and 35 to 56 respectively, and sequences SEQ. ID. Nos. 77 and 79 to 100 respectively, and the sequences having at least 98% similarity and their complementary sequences.

4. An *rpoB* gene fragment of a bacterium of the genus *Acinetobacter* chosen from among the following 24 species: *A. calcoaceticus* (genomic species 1), *A. baumannii* (genomic species 2), genomic species 3, *A. haemolyticus* (genomic species 4), *A. junii* (genomic species 5), genomic species 6, *A. johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. bay*/*yi, A. bouvetii, A. gerneri, A. grimontii, A. tandoii, A. tjernbergiae, A. towneri, A. parvus,* **characterized in that** its sequences consists of a sequence chosen from among sequences SEQ. ID. Nos. 33 to 56 respectively and sequences SEQ. ID. Nos. 77 to 100 respectively, and sequences having at least 98% similarity, and their complementary sequences.

5. An intergenic fragment comprising a non-coding sequence flanking the *rpoB* gene of a bacterium of the genus *Acinetobacter* chosen from among the following 24 species: *A. calcoaceticus* (genomic species *1), A. baumannii* (genomic species 2), genomic species 3, *A. haemolyticus* (genomic species 4), *A. junii* (genomic species 5), genomic species 6, *A. johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. bay*/*yi, A. bouvetii, A. gerneri, A. grimontii, A. tandoii, A. tjernbergiae, A. towneri, A. parvu* **characterized in that** its sequence comprises a sequence chosen from among the sequences such as described in sequences SEQ. ID. Nos. 121 to 144 respectively, and sequences SEQ. ID. Nos. 165 to 188 respectively, and sequences having at least 98% similarity, and their complementary sequences.

6. An intergenic fragment comprising a non-coding sequence flanking the *rpoB* gene of a bacterium of the genus *Acinetobacter* according to claim 5, **characterized in that** its sequence consists of a sequence chosen from among sequences SEQ. ID. Nos. 121 to 144 respectively and sequences SEQ. ID. Nos. 165 to 188 respectively, and the sequences having at least 98% similarity, and their complementary sequences.

7. A fragment according to one of claims 3 to 6, **characterized in that** it consists of an oligonucleotide that has a sequence specific to an *Acinetobacter* bacterium chosen from among the following 24 species: *A. calcoaceticus* (genomic species 1), *A. baumannii* (genomic species 2), genomic species 3, *A. haemolyticus* (genomic species 4), *A. junii* (genomic species 5), genomic species 6, *A. johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. bay*/*yi, A. bouvetii, A. gerneri, A. grimontii, A. tandoii, A. tjernbergiae, A. towneri, A. parvus,* **characterized in that** its sequence consists of a sequence having at least 18, preferably 18 to 35 consecutive nucleotide patterns included in one of the sequences chosen from among the sequences such as described in sequences:
- SEQ. ID. Nos. 33 to 56 respectively;
- SEQ. ID. Nos. 77 to 100 respectively;
- SEQ. ID. Nos. 121 to 144 respectively;
- SEQ. ID. Nos. 165 to 188 respectively; and
- the sequences having at least 98% similarity and their complementary sequences.

8. *In vitro* use, as species primer, of a gene fragment according to any of claims 3 to 6 or an oligonucleotide according to claim 7.

9. An oligonucleotide **characterized in that** it has a conserved sequence of an *Acinetobacter* bacterium chosen from among the following 24 species: *A. calcoaceticus* (genomic species 1), *A. baumannii* (genomic species 2), genomic species 3, *A. haemolyticus* (genomic species 4), *A*. *junii* (genomic species 5), genomic species 6, *A. johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. A. A. bay*/*yi, A. bouvetii, A. gerneri, A. grimontii, A. tandoii, A. tjernbergiae, A. towneri, A. parvu,* comprising a sequence having at least 12, preferably at least 18 consecutive nucleotide patterns included in one of the sequences chosen from among the sequences such as described in the following sequences SEQ. ID. Nos. 1 to 8, and their complementary sequences:
- SEQ. ID. No. 1: 5'-TAYCGYAAAGAYTTGAAAGAAG-3';
- SEQ. ID. No. 2: 5'-CMACACCYTTGTTMCCRTGA-3';
- SEQ. ID. No. 3: 5'-GTGATAARATGGCBGGTCGT-3';
- SEQ. ID. No. 4: 5'-CGBGCRTGCATYTTGTCRT-3';
- SEQ. ID. No. 5:5'-GAAGARCTTAAGAMDAARCTTG-3';
- SEQ. ID. No. 6: 5'-CGTTTCTTTTCGGTATATGAGT-3';
- SEQ. ID. No. 7: 5'- GTTCTTTAGGTATCAACATTGAA -3'; and
- SEQ.ID. No. 8: 5'- GACGCAAGACCAATACGRAT -3',
in which:
- D represents A, G or T;
- Y represents C or T;
- B represents C, G or T;
- R represents A or G; and
- M represents A or C.

10. A mixture of oligonucleotides **characterized in that** it comprises an equimolar mixture of oligonucleotides, such as defined in claim 9, of different sequences comprising at least 12, preferably at least 18 consecutive nucleotide patterns included in one of sequences SEQ. ID. Nos. 1 to 5 and 8 or the oligonucleotides of complementary sequences.

11. A mixture of oligonucleotides according to claim 10, **characterized in that** it consists of an equimolar mixture of 8 oligonucleotides of different sequences consisting of sequence SEQ. ID. No. 1 or oligonucleotides of complementary sequences.

12. A mixture of oligonucleotides according to claim 10, **characterized in that** it consists of an equimolar mixture of 16 oligonucleotides of different sequences consisting of sequence SEQ. ID. No. 2 or oligonucleotides of complementary sequences.

13. A mixture of oligonucleotides according to claim 10, **characterized in that** it consists of an equimolar mixture of 6 oligonucleotides of different sequences consisting of sequence SEQ. ID. No. 3 or oligonucleotides of complementary sequences.

14. A mixture of oligonucleotides according to claim 10, **characterized in that** it consists of an equimolar mixture of 24 oligonucleotides of different sequences consisting of sequence SEQ. ID. No. 4 or oligonucleotides of complementary sequences.

15. A mixture of oligonucleotides according to claim 10, **characterized in that** it consists of an equimolar mixture of 24 oligonucleotides of different sequences consisting of sequence SEQ. ID. No. 5 or oligonucleotides of complementary sequences.

16. An oligonucleotide according to claim 9, **characterized in that** its sequence consists of sequence SEQ ID. No. 6 or a complementary sequence.

17. An oligonucleotide according to claim 9, **characterized in that** its sequence consists of sequence SEQ. ID. No. 7 or a complementary sequence.

18. A mixture of oligonucleotides according to claim 10, **characterized in that** it consists of an equimolar mixture of 2 oligonucleotides of different sequences consisting of sequence SEQ. ID. No. 8 or oligonucleotides of complementary sequences.

19. A use, as amplification and/or sequencing reaction primer, of an oligonucleotide or mixture of oligonucleotides according to any of claims 9 to 18, for the detection by molecular identification of a bacterium of any of said species of the genus *Acinetobacter.*

20. A molecular identification method to detect a bacterium of any of the species of the genus *Acinetobacter,* **characterized in that** the following are used:
- the complete *rpoB* gene of said bacterium according to claim 1 or claim 2; or
- a gene fragment according to any of claims 3 to 6; and/or
- an oligonucleotide according to any of claims 7, 9, 16 or 17 or a mixture of oligonucleotides according to any of claims 10 to 15 or 18.

21. A method according to claim 20, **characterized in that** it is sought to detect specifically a given species of an *Acinetobacter* bacterium chosen from among the following 24 species: *A. calcoaceticus* (genomic species 1), *A. baumannii* (genomic species 2), genomic species 3, *A*. *haemolyticus* (genomic species 4), *A. junii* (genomic species 5), genomic species 6, *A. johnsonii* (genomic species 7), *A. lwoffii* (genomic species 8), genomic species 9, genomic species 10, genomic species 11, *A. radioresistens* (genomic species 12), genomic species 13, genomic species 16, *A. schindleri, A. ursingii, A. bay*/*yi, A. bouvetii, A. gerneri, A. grimontii, A. tandoii, A. tjernbergiae, A. towneri, A. parvus,* a method in which:
1- a sample containing or likely to contain nucleic acids of at least one said bacterium is contacted with at least one species probe consisting of an oligonucleotide according to claim 7 or a gene fragment according to any of claims 3 to 7, preferably a gene fragment respectively consisting of one of said sequences chosen from among:
- SEQ. ID. Nos. 33 to 56 respectively;
- SEQ. ID. Nos. 77 to 100 respectively;
- SEQ. ID. Nos. 121 to 144 respectively;
- SEQ. ID. Nos. 165 to 188 respectively; and
- the sequences having at least 98% similarity and their reverse sequences and complementary sequences, and
2- the formation or absence of a hybridisation complex is determined between said probe and the nucleic acids of the sample, and the presence of said species of *Acinetobacter* in the sample is thereby determined if a hybridisation complex is formed.

22. A method according to claim 20, **characterized in that** it comprises the steps in which:
1- amplification primers comprising said mixtures of oligionucleotides according to any of claims 10 to 15 or 18 are contacted with a sample containing or likely to contain the nucleic acids of at least one said bacterium of the genus *Acinetobacter,* and amplification of the nucleic acids is conducted by enzymatic polymerisation reaction comprising:
- as 5*'* primer, at least one oligonucleotide or mixture of oligonucleotides according to any of claims 10, 11, 13, 15 and 17 comprising a sequence included in one of sequences SEQ. ID. Nos. 1, 3, 5, and 7, preferably consisting of said complete sequence SEQ. ID. Nos. 1, 3, 5, and 7 or the complementary sequences; and
- as 3*'* primer, at least one oligonucleotide or mixture of oligonucleotides according to any of claims 10, 12, 14, 16, and 18 comprising sequences included in one of sequences SEQ. ID. Nos. 2, 4 6, and 8 respectively, preferably consisting of said complete sequence SED ID Nos. 2, 4, 6, and 8 or respectively a complementary sequence, and
2- the onset or absence of an amplification product is determined, thereby determining the presence or absence of said bacterium in the sample depending on whether an amplification product has or has not appeared.

23. A method according to claim 22, **characterized in that** it is sought to detect a given species of an *Acinetobacter* bacterium chosen from among the following 24 species: *A. calcoaceticus* (genomic species 1*), A. baumannii* (genomic species 2), genomic species 3, A. *haemolyticus* (genomic species 4*), A. junii* (genomic species 5*),* genomic species 6, *A. johnsonii* (genomic species 7), *A. lwoffii* (genomic species 8), genomic species 9, genomic species 10*,* genomic species 11, *A. radioresistens* (genomic species 12), genomic species 13, genomic species 16, A. *schindleri, A. ursingii, A. bay*/*yi, A. bouvetii, A. gerneri, A. grimontii, A. tandoii, A. tjernbergiae, A. towneri, A. parvus,* and, at step 2, the presence or absence of the given species of said bacterium is determined by conducting the steps in which:
a) a sequencing reaction is performed of a gene fragment amplified with said primers; and
b) the sequence of said amplified fragment obtained is compared with the sequence of a gene fragment of said bacterium respectively comprising:
- said sequences SEQ. ID. Nos. 33 to 56, when said 5*'* and 3*'* primers are oligonucleotides of sequences included in sequences SEQ. ID. Nos. 1 and 2 respectively;
- said sequences SEQ. ID. Nos. 77 to 100, when said 5*'* and 3*'* primers are oligonucleotides of sequences included in sequences SEQ. ID. Nos. 3 and 4 respectively;
- said sequences SEQ. ID. Nos. 121 to 144, when said 5*'* and 3*'* primers are oligonucleotides of sequences included in sequences SEQ. ID. Nos. 5 and 6 respectively; and
- said sequences SEQ. ID. Nos. 165 to 188, when said 5*'* and 3*'* primers are oligonucleotides of sequences included in sequences SEQ. ID. Nos. 7 and 8 respectively.

24. A method according to claim 22, **characterized in that** it is sought to detect a given species of *Acinetobacter* bacterium chosen from among the following 24 species: *A. calcoaceticus* (genomic species 1*)*, *A*. *baumannii* (genomic species *2)*, genomic species *3, A. haemolyticus* (genomic species 4*), A. junii* (genomic species 5*),* genomic species *6, A. johnsonii* (genomic species *7)*, *A. lwoffii* (genomic species *8)*, genomic species *9,* genomic species 10, genomic species 11, *A. radioresistens* (genomic species 12), genomic species 13, *genomic species 16, A. schindleri, A. ursingii, A. bay*/*yi, A. bouvetii, A. gerneri, A. grimontii, A. tandoii, A. tjernbergiae, A. towneri, A. parvus,* and, at step 2, the presence or absence of the given species of said bacterium is determined by conducting the steps in which:
a- a sample containing or likely to contain amplified nucleic acids of at least one said bacterium is contacted with at least one species probe consisting of an *rpoB* gene fragment according to any of claims 3 to 6, or an oligonucleotide according to claim 7, preferably a fragment respectively consisting of one of said sequences chosen from among:
- SEQ. ID. Nos. 33 to 56 respectively, when said 5*'* and 3*'* primers are oligonucleotides of sequences included in sequences SEQ. ID. Nos. 1 and 2 respectively;
- SEQ. ID. Nos. 77 to 100 respectively, when said 5*'* and 3*'* primers are oligonucleotides of sequences included in sequences SEQ. ID. Nos. 3 and 4 respectively;
- SEQ. ID. Nos. 121 to 144 respectively, when said 5*'* and 3*'* primers are oligonucleotides of sequences included in sequences SEQ. ID. Nos. 5 and 6 respectively; and
- SEQ. ID. Nos. 165 to 188 respectively, when said 5*'* and 3*'* primers are oligonucleotides of sequences included in sequences SEQ. ID. Nos. 7 and 8 respectively, and
b- the formation or absence of a hybridisation complex is determined between said probe and the amplified nucleic acids of the sample, and the presence or absence of said species of *Acinetobacter* in the sample is thereby determined depending on whether a hybridisation complex is or is not formed.

25. A method according to any of claims 22 to 24, **characterized in that** the steps are conducted comprising:
1- a first amplification of the nucleic acid of said sample with a pair of 5' and 3' primers chosen from among said mixtures of oligonucleotides according to claims 11 and 12, comprising sequences respectively included in sequences SEQ. ID. No. 1 and SEQ. ID. No. 2, preferably consisting of said sequences SEQ. ID. Nos. 1 and 2, or the complementary sequences;
2- a first determination of the onset or absence of an amplification product containing nucleic acids of at least one said bacterium, by hybridisation or optionally sequencing and comparison of the amplicons obtained at step 1 with the fragments respectively consisting of one of said sequences chosen from among SEQ. ID. Nos. 33 to 56 respectively; and
- if, at this step 2, the presence of species *A. grimontii* or *A. junii* is determined, the following are also carried out:
3a - a second amplification reaction with 5' and 3' primers chosen from among said mixtures of oligonucleotides according to claim 10 or claims 13 and 14, comprising sequences respectively included in sequences SEQ. ID. No. 3 and SEQ. ID. No. 4, preferably consisting of said sequences SEQ. ID. Nos. 3 and 4, or the complementary sequences; and
4a- determination of the onset or absence of an amplification product containing nucleic acids of at least one said bacterium, by hybridisation or optionally sequencing and comparison of the amplicons obtained at step 3a with the fragments respectively consisting of one of said sequences chosen from among SEQ. ID. Nos. 77 to 100 respectively; or
- if, at this step 2, the presence of the species *A. baylii* or *A. genomic species 11* is determined*,* the following is also carried out:
3b- a second amplification reaction with 5' and 3' primers chosen from among said mixtures of oligonucleotides according to claims 17 and 18, comprising sequences respectively included in sequences SEQ. ID. No. 7 and SEQ. ID. No. 8, preferably consisting of sequences SEQ. ID. Nos. 7 and 8, or the complementary sequences; and
4b- determination of the onset or absence of an amplification product comprising nucleic acids of at least one said bacterium, by hybridisation or optionally sequencing, and comparison of the amplicons obtained at step 3b with the fragments respectively consisting of one of said sequences chosen from among SEQ. ID. Nos. 165 to 188 respectively.

26. A diagnosis kit which can be used in a method according to any of claims 20 to 25, **characterized in that** it comprises at least one said oligonucleotide according to any of claims 7, 9, 16 or 17 or a mixture of oligonucleotides according to any of claims 10 to 15 or 18 or a gene fragment according to any of claims 3 to 6, as well as, preferably, reagents which can be used in hybridisation reactions or amplification or sequencing reactions as applicable.


### SEQUENCE LISTING

<110> Université de la méditerranée (Aix-Marseille II) Centre National de la Recherche Scientifique (CNRS)
<120> Fragments d'acides nucléiques et méthode de détection spécifique par identification moléculaire de différentes espèces de bactéries du genre acinetobacter
<130> H52 437 cas 14 FR
<160> 208
<170> PatentIn version 3.1
<210> 1
   <211> 22
   <212> DNA
   <213> artificial sequence
<220>
   <223> Séquences conservées du gène rpoB des différentes espèces de bact éries du genre acinetobacter
<400> 1
   taycgyaaag ayttgaaaga ag 22
<210> 2
   <211> 20
   <212> DNA
   <213> artificial sequence
<220>
   <223> Séquences conservées du gène rpoB des différentes espèces de bact éries du genre acinetobacter
<400> 2
   cmacaccytt gttmccrtga 20
<210> 3
   <211> 20
   <212> DNA
   <213> artificial sequence
<220>
   <223> Séquences conservées du gène rpoB des différentes espèces de bact éries du genre acinetobacter
<400> 3
   gtgataarat ggcbggtcgt 20
<210> 4
   <211> 19
   <212> DNA
   <213> artificial sequence
<220>
   <223> Séquences conservées du gène rpoB des différentes espèces de bact éries du genre acinetobacter
<400> 4
   cgbgcrtgca tyttgtcrt 19
<210> 5
   <211> 22
   <212> DNA
   <213> artificial sequence
<220>
   <223> Séquences conservées encadrant le spacer rp/L-rpoB des différente s espèces de bactéries du genre acinetobacter
<400> 5
   gaagarctta agamdaarct tg 22
<210> 6
   <211> 22
   <212> DNA
   <213> artificial sequence
<220>
   <223> Séquences conservées encadrant le spacer rp/L-rpoB des différente s espèces de bactéries du genre acinetobacter
<400> 6
   cgtttctttt cggtatatga gt 22
<210> 7
   <211> 23
   <212> DNA
   <213> artificial sequence
<220>
   <223> séuqences conservées encadrant le spacer rpoB-rpoC des différente s espèces de bactéries du genre acinetobacter
<400> 7
   gttctttagg tatcaacatt gaa 23
<210> 8
   <211> 20
   <212> DNA
   <213> artificial sequence
<220>
   <223> séuqences conservées encadrant le spacer rpoB-rpoC des différente s espèces de bactéries du genre acinetobacter
<400> 8
   gacgcaagac caatacgrat 20
<210> 9
   <211> 4089
   <212> DNA
   <213> Acinetobacter calcoaceticus
<400> 9
   atggcatact catataccga aaagaaacgg atccgtaaga attttggtaa attgccccaa 60
   gtaatggaag caccgtactt actttcgatc caggtcgatt cgtatagaac gttcttgcaa 120
   ggtggcaaat ctccaaaaaa ccgcgaagat atcggtctcc aagccgcatt tcgttcagtt 180
   ttccctatag aaagttattc tggcaatgct gctttagaat ttgttgagta tagccttggt 240
   aagcctgagt ttgatgtgcg tgaatgtatt ttacgtggtt cgacttatgc ggcaccaatg 300
   cgcgtaaaaa ttcgtttgat tattaaagat cgcgaaacta aatctattaa agacgtacgt 360
   gaacaagaag tctacatggg tgaaatgcca ctcatgacag ataatggtac ctttgtgatc 420
   aatggtactg agcgtgtaat cgtatctcaa ttacaccgtt cacctggtgt gttctttgat    480
   cacgataaag gcaaaacaca ttcaagtggt aaagtacttt attctgcacg tattattcct    540
   taccgtggtt catggttaga ttttgaattt gatgcaaaag atttagttta tgtacgtatt    600
   gaccgtcgtc gtaaattatt agcaactgtt gtgttgcgcg cattgggtta taacaatgaa    660
   caaattttga atttgttcta tgaaaaagtg cctgtatatc ttgatatggg tagctatcaa    720
   attgacctcg ttccagatcg tctgcgtggc gaaatggcgc aatttgatat tgcagacaat    780
   gacggtaaaa tcattgttga gcaaggtaaa cgtattaatg cacgtcacgt acgtcaaatg    840
   gaagcttctg gtttagctaa gctatcagtt cctgatgagt acctgtatga gcgtatcact    900
   gccgaagata ttactttacg taatggtgat gtgattgctg caaatacatt gttaagccat    960
   gaagttatgg tgaaactggc agaaggcggt gttaaacagt tcaatatctt gtttacgaac   1020
   gatattgacc gtggttcatt tgttgctgac tcattacgtg ctgacacaac atctggtcgt   1080
   gaagaagcat tagttgaaat ctataaagta atgcgcccag gcgagccacc aacaaaagaa   1140
   gctgctgaaa acttatttaa taacttattc ttctcttctg aacgttatga cttatctcca   1200
   gtaggtcgta tgaaattcaa ccgtcgtttg ggtcgcccat acgaagttgg tactgaccag   1260
   aagtcacgtg aagtagaagg tattttatcg cacgaagata tcatcgatgt attacgtaca   1320
   ttagttgaaa tccgtaacgg taaaggtgaa gtcgatgata tcgatcactt gggtaaccgt   1380
   cgtgtacgtt ctgttggtga aatgactgaa aaccaattcc gtgttggttt ggttcgtgtt   1440
   gaacgtgctg ttaaagaacg tttaagccaa gcagaaacag ataatttatc tccgcaagat   1500
   ctcattaacg caaaaccagt tgcagctgcg atcaaagaat tctttggttc aagccaattg   1560
   tctcagttta tggatcaaaa caacccatta tctgagatta cacataagcg tcgtgtatca   1620
   gcgcttggac ctggtggttt aacacgtgaa cgtgcgggct ttgaagtacg tgacgtacat   1680
   caaactcact atggtcgtgt atgtccaatt gaaacgccgg aaggtccaaa cattggtttg   1740
   atcaactcgc tttctgttta tgcaaaagca aatgattttg gtttcttgga aacaccttac   1800
   cgtaaagtgg tagatggccg agtaactgat gctgttgaat acctttctgc tattgaagaa   1860
   gtaggtactg ttattgcaca ggccgattct gcgatagata aagatggtaa cttaacagaa   1920
   gattttgttt ctgttcgtca ccaaggtgac tttgtacgta tgccacctga aaaagtgacg   1980
   catatggatg tatctgcaca gcaggttgta tctgtagctg catcgcttat tccattcctt   2040
   gaacacgatg acgcgaaccg tgcattgatg ggttcaaaca tgcaacgtca ggcggttcct   2100
   actctacgtg cggataaacc gcttgtaggt acaggtatgg aagcgaacgt tgcacgtgac   2160
   tcgggcgtat gtgtgatcgc aaaccgtggt ggtgcgattg aatatgtaga tgcttctcgt   2220
   atcgttattc gtgtaaacga agatgaaatg attgcaggtg aggctggtgt agatatttac   2280
   aatctcatta aatatacacg ttctaaccaa aacacttgta tcaaccaaaa tattatcgtg   2340
   aatttgggcg acaaagttgc tcgcggtgac atcttggcag acggtccgtc aacagatatg   2400
   ggtgagcttg cgcttggtca aaacatgcgt gtagcgttca tgacgtggaa tggttacaac   2460
   tacgaagact cgattttatt atctgagcgt gtacttcaag aagaccgttt aacttctatt 2520
   catattcaag aattatcatg tgtagcgcgt gatactaagt taggtgcaga agaaattact 2580
   gccgatattc ctaacgtagg tgaagctgca ctttctaaac ttgatgaatc tggtatcgtt 2640
   tatattggtg cggaagttac tgctggtgac atccttgttg gtaaagtaac gcctaaaggt 2700
   gaaactcagt taactcctga agaaaaattg cttcgtgcaa tctttggtga gaaagcggct 2760
   gacgttaaag actcatcttt acgtgttcca tctggtacta aaggtacagt tatcgatgtt 2820
   caagtcttca cgcgtgatgg cttagagaaa gatgaccgtg cgatggcaat tgaaaaagca 2880
   caacttgacg cttaccgtaa agacttgaaa gaagaataca agatctttga agaagcagct 2940
   cgtgagcgtg taattcgttt gcttaacggc caagagtcta acggtggtgg ttcgactaaa 3000
   cgtggcgaca agctcgttga cggtatgttg tctggtttag agcttgttga cttacttgaa 3060
   atccaaccta cagatgaagc aattgctgaa cgtttatctc aaattcaagt gttcttgaaa 3120
   gagaagagcg cagaaattga tgagaagttt gcagagaaga aacgtaagct ttcgactggt 3180
   gatgagttaa caacaggtgt tctgaaagtt gttaaagttt acctagcagt taaacgtcgt 3240
   attcagcctg gtgataaaat ggctggtcgt cacggtaaca aaggtgttgt atctaacatt 3300
   ttacctgttg aagacatgcc acacgatgct aacggtgtgc cggtagatat cgtattgaac 3360
   ccattgggtg taccatctcg tatgaacgtg ggtcagattc ttgagactca cttgggtatg 3420
   gcagctaaag ggcttggtga taaaatcgaa aaaatgttga aagaacagcg cacagtttta 3480
   gaacttcgcg aattcttaga caagatttat aacaaagtcg gcggcgagca agaagatctt 3540
   gatagcttaa ctgatgctga agtcttagca ctttcaggca acttacgtgc tggtgtgcct 3600
   ttagctactc ctgtatttga tggtgctgaa gaaagtcaaa ttaaagactt acttgaatta 3660
   gctgatattt cgcgtactgg tcaaacagta ttgtttgatg gtcgtacggg tgaacagttt 3720
   gatcgtcctg taactgtagg ttacatgtat atgctcaaat tgaaccactt ggttgatgac 3780
   aaaatgcatg cgcgttcaac aggttcttac tcacttgtta ctcaacaacc gcttggtggt 3840
   aaagcacaat tcggtggtca gcgtttcggt gagatggagg tctgggcact cgaagcttac 3900
   ggcgcagcat atacacttca agagatgtta actgttaagt cggatgacgt tgaaggtcgt 3960
   acacgcatct ataagaatat tgtagatggt aaccattata tggatccggg tatgcctgaa 4020
   tcgttcaacg tattgaccaa agagatccgt tctttaggta tcaacattga actgaaaaat 4080
   ggtgactaa 4089
<210> 10
   <211> 4089
   <212> DNA
   <213> Acinetobacter baumannii
<400> 10
   atggcatact catataccga aaagaaacgg atccgtaaga attttggtaa attgccccaa      60
   gtaatggatg caccgtactt attatcgatt caggtcgatt cgtacagaac attcttgcaa     120
   gatggcaaat caccaaaaaa ccgcgaagat atcggtctcc aagccgcatt tcgttcagtt     180
   tttcctatcg aaagttattc tggcaatgct gctttagaat ttgttgagta tagccttggt     240
   aaacctgagt tcgatgtacg cgaatgtatt cttcgtggct cgacttatgc ggcaccaatg     300
   cgcgttaaaa ttcgtttgat tattaaagat cgcgaaacta aatcaattaa agacgtacgc     360
   gaacaagaag tctatatggg tgaaattccg ctcatgactg aaaatggtac ctttgttatc     420
   aacggtactg agcgtgtaat cgtatctcaa ttacaccgtt cgccaggcgt attctttgac     480
   catgataaag gtaagaccca ctcaagtggt aaagtgttgt attcagcacg tatcattcct     540
   taccgtggtt catggttaga cttcgaattt gatgcaaaag acttagttta cgtacgtatc     600
   gaccgtcgtc gtaaattact tgctacagtt gtgttacgtg cactaggtta taacaatgaa     660
   cagatcttga atttgttcta tgaaaaagta cctgtgtatc ttgacatggg tagctatcaa     720
   attgaccttg tacctgagcg tttacgtggt gaaatggctc aatttgatat tactgacaat     780
   gaaggtaaag tcattgttga gcaaggtaaa cgtattaatg ctcgtcacgt acgtcaaatg     840
   gaagctgcag gtttaactaa gctttcagtt cctgatgaat acttatatga gcgtatcact     900
   gctgaagata ttactttacg tgatggtgaa gtaattgctg caaatactct gttaagccat     960
   gaagtaatgg tgaagttggc agaaggcggt gttaagcaat ttaatatctt gttcactaac    1020
   gatattgacc gtggttcatt cgtagctgat acattacgtg ctgacttgac gcgtgatcgt    1080
   gaagaagcat tagtagaaat ctacaaagta atgcgtccag gcgagccacc aacaaaagaa    1140
   gctgctgaaa acttattcaa taacttgttc ttctcttctg aacgctatga cttatctcca    1200
   gtaggtcgta tgaagttcaa ccgtcgttta ggtcgtcctt acgaagttgg tactgatcag    1260
   aagtcacgcg aagttgaagg tattttatcg cacgaagata ttatcgatgt attacgtaca    1320
   ttggttgaaa tccgtaacgg taaaggtgaa gtcgacgata tcgaccactt aggtaaccgt    1380
   cgtgtacgtt ctgttggtga aatgacagag aaccaattcc gtgtaggttt agttcgtgtt    1440
   gagcgtgctg ttaaagagcg tttaagccaa gcagaaacag ataacttgtc tccacaagat    1500
   ttgattaatg caaaaccagt tgctgctgca atcaaagaat tctttggttc aagccagtta    1560
   tctcagttta tggaccaaaa caacccatta tctgagatta cacataaacg tcgtgtatct    1620
   gcgcttggtc ctggtggttt aacacgtgaa cgtgcaggct tcgaagtacg tgacgtacac    1680
   caaactcact atggtcgtgt ttgtccaatt gaaactcctg aaggtccaaa cattggtttg    1740
   atcaactcgc tttctgtata cgcaaaagcg aatgacttcg gtttcttgga aacaccatac    1800
   cgcaaagttg tagatggtcg tgtaactgat gatgttgaat atttatctgc aattgaagaa    1860
   gtaggtactg ttattgcaca ggccgactct gcagtagata aagatggcaa cttaacagaa    1920
   gaattcgttt ctgttcgtca tcaaggtgaa ttcgtacgta tgccgcctga aaaagtaacg    1980
   catatggacg tttctgcaca gcaggtagta tctgttgctg catcacttat tccattcctt    2040
   gaacacgatg acgcaaaccg tgcgctcatg ggttcaaaca tgcaacgtca ggcagttcct 2100
   actttacgtg cggataaacc gcttgtaggt acaggtatgg aagcgaacgt tgcacgtgac 2160
   tctggtgtgt gtgtaatcgc aaaccgtggc ggtgtaattg aatatgtaga tgcttctcgt 2220
   atcgttattc gtgtaaacga agatgaaatg gttgcaggtg aggcgggtgt agatatctat 2280
   aacctcatca aatatacgcg ttcaaaccaa aatacttgta ttaaccaaaa tgttatcgtg 2340
   aacttgggcg acaaagttgc tcgtggtgac atcttggcag acggtccgtc aacagacatg 2400
   ggtgaacttg cgcttggtca aaacatgcgt gtagcgttca tgacatggaa tggttacaac 2460
   tacgaagact cgatcttgtt atctgagcgt gtacttcaag aagaccgttt aacctctatt 2520
   cacattcaag aattgtcatg tgtagcacgt gatactaagt taggtgcaga agaaattact 2580
   gccgatattc ctaacgtagg tgaagctgcg ctttctaaac ttgatgaatc aggtatcgtt 2640
   tatatcggtg ctgaagttac tgctggtgac atcttagttg gtaaagtaac gcctaaaggt 2700
   gaaactcagt taactcctga agaaaaactg cttcgtgcaa tttttggtga gaaagcagct 2760
   gacgttaaag actcatcttt acgtgttcca tctggtacta aaggtacagt tattgacgtt 2820
   caagtcttca ctcgtgatgg cttagagaaa gatgaccgtg ctttagcaat tgaaaaagca 2880
   cagcttgatt cttaccgtaa agacttgaaa gaagaataca agatcttcga agaagcggct 2940
   cgtgagcgtg taattcgttt gcttaaaggc caagagtcta atggcggtgg ttcaactaaa 3000
   cgtggtgata aactttctga agatttatta tctggtttag agcttgttga tttacttgaa 3060
   attcaaccag cagatgaagc aatcgctgag cgtttaactc aaattcaagt gttcttgaaa 3120
   gagaagagcg cagaaatcga tgagaaattc gctgagaaga aacgtaagct tgccacaggt 3180
   gatgaattaa cgactggcgt attaaaagtt gttaaggttt acttagctgt taaacgtcgt 3240
   attcagcctg gtgataagat ggctggtcgt cacggtaaca agggtgttgt atctaacatc 3300
   ttacctgttg aagacatgcc acatgatgct aacggtgtgc cggtagatat cgtattgaac 3360
   ccgctgggtg taccatctcg tatgaacgtg ggtcagattc tagagactca cttgggtatg 3420
   gcggctaaag ggcttggtga caaaatcgaa aaaatgttga aagaacaacg tacagtttta 3480
   gaactgcgcg aattcttaga caagatttat aacaaagtcg gcggtgagca agaagatctt 3540
   gatagcttga ctgatgaaga aattctagcg cttgcaggta acttgcgtgc gggtgtgcct 3600
   ttagctactc ctgtatttga tggtgctgaa gaaagtcaaa ttaaagactt acttgaattg 3660
   gctgacattt cacgtacggg tcaaacagta ttgtttgacg gacgtacagg tgaacagttt 3720
   gaccgtccag taactgttgg ttacatgtac atgcttaaat tgaaccactt ggttgatgac 3780
   aagatgcatg cgcgttcaac tggttcttac tcacttgtta cacaacaacc gcttggtggt 3840
   aaagcacaat tcggtggtca gcgtttcggt gagatggaag tgtgggcact tgaagcatac 3900
   ggtgcagcat atacactcca agaaatgctt acagtgaagt cggatgatgt tgaaggccgt 3960
   actcgcatct ataagaatat tgtagatggt aaccattata tggatccggg tatgcctgaa 4020
   tcgttcaacg tattgaccaa agagatccgt tctttaggta tcaacattga actaaaaaat 4080
   ggtgactaa 4089
<210> 11
   <211> 4089
   <212> DNA
   <213> Acinetobacter sp.
<400> 11
   atggcatact catataccga aaagaaacgg atccgtaaga attttggtaa attgccccaa 60
   gtaatggatg caccgtactt actatcgatt caggtcgatt cgtacagaac attcttgcaa 120
   gatggcaaaa ctccaaaaaa ccgcgaagat atcggtctcc aagccgcatt tcgttcagtt 180
   tttcctatcg aaagttattc tggcaatgct gctttagaat ttgttgagta tagccttggt 240
   aaacctgagt tcgatgtacg cgaatgtatt cttcgtggtt cgacttatgc ggcaccaatg 300
   cgcgttaaaa ttcgtttgat tattaaagat cgcgaaacta aatcaattaa agacgtacga 360
   gaacaagaag tctatatggg tgaaattcca ctcatgactg aaaacggtac attcgtcatt 420
   aatggtactg agcgtgtaat cgtatctcaa ttacatcgtt caccaggcgt tttctttgat 480
   cacgataaag gtaaaaccca ctcaagtggt aaagtgttgt attcagcacg tatcattcct 540
   taccgtggtt catggttaga cttcgaattt gatgcaaaag acttagttta cgtacgtatt 600
   gaccgtcgtc gtaaattact tgctactgtt gtgttacgtg cactaggtta taacaacgaa 660
   caaatcttga atttgttcta tgaaaaagta cctgtgtatc ttgacatggg cagttaccag 720
   attgacctag tacctgaacg tttacgtggg gaaatggctc aatttgatat tgctgacaat 780
   gacggtaaag ttattgttga gcaaggtaaa cgtattaatg ctcgtcatgt acgtcaaatg 840
   gaagctgctg gtttaacaaa actttcagtt cctgatgaat acttatatga gcgtatcact 900
   gctgaaaata ttactttgcg tgatggtgaa gtaattgctg cgaatacttt gttaagccat 960
   gaagttatgg tgaagttggc agaaggtggt gttaaacagt tcaatatctt gtttacgaac 1020
   gatattgacc gtggttcgtt tgttgctgac acattacgtg cagatacaac agctggccgt 1080
   gaagaagcat tagttgaaat ttataaagta atgcgtccag gcgagccgcc aacaaaagaa 1140
   gctgctgaaa acttattcaa taacttattc ttctcttctg agcgttatga cttatctcca 1200
   gtaggtcgta tgaagttcaa ccgtcgtttg ggtcgtccat acgaagttgg tactgaccag 1260
   aagtcacgtg aagtagaagg tattttatcg cacgaagata tcattgatgt attacgtaca 1320
   ttagttgaaa tccgtaacgg taaaggtgaa gtcgacgata tcgatcactt gggtaaccgt 1380
   cgtgtacgtt ctgttggtga aatgactgaa aaccaattcc gtgttggttt ggtacgtgtt 1440
   gaacgtgctg ttaaagaacg tttaagccaa gcagaaacag ataacttgtc tccacaagat 1500
   ctaattaacg ctaaaccagt tgcagctgca atcaaagaat tctttggttc aagccaatta 1560
   tctcagttca tggaccaaaa caacccatta tctgagatta cgcataagcg tcgtgtatca 1620
   gcgcttgggc ctggtggttt aacacgtgaa cgtgcgggct ttgaagtacg tgacgtacat 1680
   caaactcact atggtcgtgt atgtccaatt gaaacgccgg aaggtccaaa cattggtttg 1740
   atcaactcgc tttctgtata tgcaaaagca aatgacttcg gtttcttgga aacaccttac 1800
   cgtaaagtag tagatggtcg agtaactgat gcagttgaat acctttctgc gattgaagaa 1860
   gtaggtactg ttattgcaca ggccgattct gcagtagata aagatggcaa cctaacagaa 1920
   gagtttgttt ctgttcgtca tcaaggtgaa ttcgtacgta tgccacctga aaaagtgacg 1980
   catatggacg tttctgctca gcaagttgta tctgttgctg catcacttat tccattcctt 2040
   gaacacgatg acgcgaaccg tgcgcttatg ggttcaaaca tgcaacgtca ggcagttcct 2100
   actctacgtg cggataaacc gcttgtaggt acaggtatgg aagcgaacgt tgcacgtgac 2160
   tcaggtgtgt gtgtgatcgc aaaccgtggt ggtgcaattg aatacgtaga tgcttctcgt 2220
   atcgttattc gtgtaaacga agatgaaatg atcgctggtg aggcgggtgt agatatctac 2280
   aacctcatta aatatacgcg ttctaaccag aacacttgta tcaaccaaaa tattatcgtg 2340
   aatttgggcg acaaagttgc tcgcggtgat atcttggcag acggtccgtc tacagatatg 2400
   ggtgagcttg cgcttggtca aaacatgcgc gttgcgttca tgacttggaa tggttacaac 2460
   tatgaagact cgatcttatt atctgagcgc gtacttcaag aagaccgttt aacttctatt 2520
   catattcaag aattatcttg tgtagcgcgt gatactaagt taggtgcaga agaaattact 2580
   gccgatattc ctaacgttgg tgaagctgca ctttctaaac ttgatgagtc aggtatcgtt 2640
   tatatcggtg ctgaagttac agcaggtgac atccttgttg gtaaagtaac gcctaaaggt 2700
   gaaactcagt taactcctga agaaaaattg cttcgtgcaa tctttggtga gaaagcagct 2760
   gacgttaaag attcatcttt acgtgttcca tctggtacaa aaggtacagt tatcgatgtt 2820
   caagtcttca ctcgtgatgg cttagagaaa gatgaccgtg cattggcgat cgaaaaagca 2880
   caacttgatg cttaccgtaa agacttgaaa gaagaataca agatctttga agaagcagct 2940
   cgtgagcgtg taattcgttt gcttaaaggc caagagtcta atggcggtgg ttcaactaaa 3000
   cgtggtgaca aactcgttga agaagtgtta tctggtttag agcttgttga tttacttgaa 3060
   attcaaccgg cagatgaagc aatcgctgag cgtttaactc aaattcaagt gttcttaaaa 3120
   gaaaagagcg cagaaattga tgagaaattc gctgagaaga aacgtaagct tgcaacaggt 3180
   gatgaattaa caactggcgt attgaaagtt gttaaagttt acttagctgt taaacgtcgt 3240
   attcagcctg gtgataagat ggctggtcgt cacggtaaca agggtgttgt atctaacatc 3300
   ttacctgttg aagacatgcc acacgatgct aacggtgtac cagtagatat cgtattgaac 3360
   ccgttgggcg taccatctcg tatgaacgtg ggtcagattc ttgagactca cttaggtatg 3420
   gcggctaaag ggcttggtga taaaatcgag aaaatgttga aagaacagcg tacagtttta 3480
   gaactgcgtg aattcttaga caagatttat aacaaagtcg gtggtgagca agaagatctt 3540
   gatagcttaa ctgatgctga agtcttggca ctttcaggca acttacgtgc tggtgtacct 3600
   ttggctactc ctgtattcga tggtgctgaa gaaagccaaa ttaaagactt gcttgagtta 3660
   gctggtatct ctcgtacagg tcaaacagta ttgtttgatg gccgtactgg tgaacagttt 3720
   gatcgtcctg taactgtggg ttacatgtac atgctcaaat tgaaccactt ggttgatgac 3780
   aagatgcatg cgcgttcaac tggttcttac tctcttgtta cacagcaacc gcttggtggt 3840
   aaagcacaat tcggtggtca gcgtttcggt gagatggagg tctgggcact cgaagcttac 3900
   ggcgcagcat atactctcca agagatgtta actgttaagt cggatgacgt tgaaggtcgt 3960
   acacgcatct ataagaatat tgtagatggt aaccattata tggacccggg tatgcctgaa 4020
   tcgttcaacg tattgaccaa agagatccgt tctttaggta tcaacattga actgaaaaat 4080
   ggtgactaa 4089
<210> 12
   <211> 4089
   <212> DNA
   <213> Acinetobacter haemolyticus
<400> 12
   atggcatact catataccga aaagaaacgg attcgtaaga attttggtaa attgccccac 60
   gtaatggaag caccgtactt actttcgatt caagtcgatt cgtaccgtac tttcttacaa 120
   ggtggcaaaa ctccaaaaaa tcgcgaagat atcggtctcc aagccgcatt tcgttcagtt 180
   tttcctattg aaagttattc tggcaatgct gctttagaat ttgttgagta tagtcttggt 240
   aaaccagagt ttgacgtacg tgaatgtatt ttacgtggtt caacttatgc ggcaccaatg 300
   cgcgtaaaaa ttcgtttgat cattaaagat cgcgaaacca aatcaattaa agacgttcgt 360
   gaacaagaag tttacatggg tgaaatgcca ctcatgaccg ataacggtac attcgttatt 420
   aacggtactg agcgtgtaat cgtatctcaa ttacaccgtt cgccaggcgt attctttgat 480
   catgacaagg gtaagaccca ctcaagcggt aaagtgttgt attcagcacg tattatccct 540
   taccgtggtt catggttaga tttcgaattt gatgctaaag atttagtatt cgtacgtatt 600
   gaccgtcgtc gtaaattatt ggcgactgtg attttacgtg ctttaaatta taccaatgag 660
   caaatcctga atttgttcta cgaaaaagta cctgtctatt tagatatggg cagctatcaa 720
   attgatctcg ttccagatcg cctacgtggt gagatggcgc aatttgatat cttagataac 780
   gatggcaaag cgattgttga acaaggcaaa cgtattaatg cacgtcacgt acgtcaaatg 840
   gaagcagcga acttagcgaa gctttcagta cctgatgaat acttgtatga gcgtattaca 900
   gctgaagata tcacattgaa agatggtgat gtaattcctg caaatacggt gcttagccat 960
   gaagtgatgg tgaaaatcgc tgaaggcggc gtgaaacagt ttaatattct gttcaccaac 1020
   gatatcgatc gtggtccttt cgttgcggat actttacgtg cagatacaac aacaggtcgt 1080
   gaagaagcac ttgttgaaat ctataaagtc atgcgtccag gtgagccgcc aacgaaagaa 1140
   gctgctgaaa acttatttaa taacttgttc ttctcttctg agcgttatga cctgtctcca 1200
   gtgggtcgta tgaagttcaa ccgtcgtttg ggtcgtcctt acgaagtggg tactgatcag 1260
   aagtctcgtg aagttgaagg tattttatca cacgacgata tcattgatgt acttcgtaca 1320
   ttggtggaaa tccgtaacgg taaaggtgaa gtcgatgata tcgatcactt aggtaaccgg 1380
   cgtgtacgtt ctgttggcga aatgacagaa aaccaattcc gtgttggttt agtgcgtgtt 1440
   gaacgtgctg ttaaagagcg tttaagccaa gcagaaactg ataacttgtc tccacaagat 1500
   ttaatcaacg cgaaaccagt tgctgctgca atcaaagagt tctttggttc aagccagtta 1560
   tctcagttca tggaccaaaa caacccattg tctgagatta cacacaaacg tcgtgtatct 1620
   gcacttggtc ctgggggttt gacacgtgaa cgcgcgggct tcgaagtacg tgacgtacat 1680
   caaactcact atggtcgtgt ttgtccgatt gaaacgcctg aaggtccaaa cattggtttg 1740
   atcaactcgc tttctgttta tgcaaaagca aatgacttcg gtttcttgga aacaccatac 1800
   cgtaaagttg tcgatggtcg tgtaactgat gatgttgaat atttatctgc aattgaagaa 1860
   gtagggactg tcattgcaca agccgactct gcagttgata aagatggtaa tttaactgaa 1920
   gaaatggttt ctgtgcgtca tcaaggtgaa ttcgtacgta tgtcgcctga acgcgtaacg 1980
   catatggacg tttctgcaca gcaggtagta tcagttgcgg catcattgat tccattcctt 2040
   gaacacgatg atgcgaaccg tgcattgatg ggttcgaaca tgcaacgtca ggctgttcct 2100
   acattacgtg ctgacaagcc gcttgttggt acaggtatgg aagcgaacgt agcgcgcgac 2160
   tcgggtgtgt gtgtgatcgc aaaccgtggt ggtgcgattg agtatgtaga tgcatctcgt 2220
   atcgttattc gtgtcaacga agatgaaatg attgcgggtg aagcaggtgt agatatctat 2280
   aacctgatca aatatacacg ttcaaaccag aacacatgta ttaaccagaa tgtcatcgtg 2340
   aacttgggcg acaaagttgc tcgtggtgat atcttggctg acggtccttc gactgacatg 2400
   ggtgaacttg cgctgggtca aaacatgcgc gtcgcgttca tgacatggaa cggttataac 2460
   tatgaagact cgattttact ttctgagcgt gttcttcaag aagatcgttt aacgtcgatt 2520
   cacattcaag aattgtcatg tgtagcacgt gatactaagt taggtgcaga agaaattact 2580
   gccgatattc ctaacgttgg tgaagcagca ctgtctaaat tggatgagtc aggtattgtt 2640
   tatatcggtg ctgaagtgac tgcgggtgat atcctagttg gtaaggtaac gcctaaaggt 2700
   gaaactcagt taacacctga agaaaaatta cttcgcgcaa tctttggtga aaaagcggca 2760
   gacgtaaaag attcatcttt gcgtgttccg tctggtacca aaggaacagt gattgacgtt 2820
   caagtcttca cacgtgatgg tttagaaaag gacgagcgtg cgcaagcaat tgagaaagct 2880
   caacttgatg cataccgtaa agacttgaaa gaagaataca aaatcttcga agaagcagca 2940
   cgtgaacgta ttgttcgctt gttgaaaggt caagagtcaa atggtggcgg tacaactaag 3000
   cgcggcgata aactctcaga agatgtattg tctggtttag agcttgttga tttacttgaa 3060
   atccaaccag ctgatgaagc gattgctgaa cgtttaacgc aaattcaagt gttcttgaaa 3120
   gagaagagca tcgaaatcga tgagaaattt gcagagaaga agcgtaagct ttctacaggt 3180
   gatgaattaa caacgggtgt attaaaagtt gttaaggttt accttgcggt taagcgtcgt 3240
   attcagcctg gtgataagat ggcgggtcgt cacggtaaca agggtgtggt atcaaatatc   3300
   ttacctgtag aagacatgcc gcatgatatt cacggtgtgc cagttgatat cgtattgaac   3360
   ccattgggtg taccatcacg tatgaacgtg ggtcagattc ttgaaactca cttgggtatg   3420
   gcggcaaaag gtctgggtga gcaaattgat aagatgctcc aacagcaacg tacgattgct   3480
   gaattgcgtg cgttcctcga caagatttac aacaaagttg gtggcgaaca agaagatctt   3540
   gatagcttaa ccgacgaaga agttttaaaa cttgcaggta acctgcgtgc aggtgtgcct   3600
   ttggcgacac cagtatttga tggtgctgaa gaaagtcaaa ttaaagagtt acttgagctt   3660
   gctgaattgc cacgtactgg tcaaaccgta ttgtttgatg gtcgtactgg tgaacagttt   3720
   gatcgtcctg taactgtcgg ttatatgtac atgctcaaat tgaaccactt ggttgatgac   3780
   aagatgcatg cgcgttcaac tggttcttac tcactagtga cacaacagcc gcttggtggt   3840
   aaagcacaat tcggtggtca gcgtttcggt gagatggaag tatgggcact tgaagcatac   3900
   ggtgcagcat atacgcttca agaaatgctt actgtgaagt cggatgacgt tgaaggtcgt   3960
   acacgcatct ataagaatat tgtagatggt aaccattata tggatccggg tatgcctgaa   4020
   tcgttcaacg tattgaccaa agagatccgt tctttaggta tcaacattga actgaaaaat   4080
   ggtgactaa                                                            4089
<210> 13
   <211> 4089
   <212> DNA
   <213> Acinetobacter junii
<400> 13
   atggcatact catataccga aaagaaacgg atccgtaaga attttggtaa attgccccac     60
   gtaatggaag caccgtactt actttcgatt caggtcgatt cgtatcgtac attcttacaa    120
   ggtggtaaaa ctccaaaaaa tcgcgaagat atcggtctcc aagccgcatt tcgttcagtt    180
   tttcctattg aaagttattc tggcaatgct gctttagaat ttgttgagta tagccttggt    240
   aaacccgagt ttgacgtgcg tgaatgtatt ttacgtggtt cgacttatgc ggcaccaatg    300
   cgtgtaaaaa ttcgtttgat cattaaagat cgcgaaacga aatcaatcaa agatgttcgt    360
   gaacaagaag tgtacatggg cgaaatgccg ctcatgaccg acaacggtac tttcgttatt    420
   aacggtactg aacgtgtaat cgtatctcaa ttacaccgtt caccaggcgt attctttgat    480
   catgataagg gtaaaacaca ctcaagcggt aaagtgttgt attcagcacg tatcattcct    540
   taccgtggtt catggttaga ttttgaattc gatgcaaaag atttagtttt cgtacgtatt    600
   gaccgtcgtc gtaaattgtt ggcgactgtg atcttacgtg ctttaaatta tagcaatgaa    660
   caaatcttga atttgttcta tgaaaaagta cctgtatatc ttgatatggg tagctatcaa    720
   attgacctcg ttccagatcg cttacgtggt gaaatggcgc aatttgatat cttggacaat    780
   gatggtaaag caatcgttga acaaggtaag cgtattaatg cacgtcatgt acgccaaatg 840
   gaagcagcta acttagctaa gctttctgta cctgatgaat atttatatga acgtattaca 900
   gctgaagata tcacactgaa aagtggtgat gtgattcctg caaataccgt acttagccat 960
   gaaattatgg tgaaattggc tgaaggtggt gttaaacaat ttaacatcct attcactaat 1020
   gacatcgatc gtggttcgtt cattgcagat acattacgtg cagatacaac aacaggtcgt 1080
   gaagaagcgc ttgttgaaat ctataaagta atgcgtccag gtgaaccacc gacaaaagaa 1140
   gcagcagaga acttatttaa taacttattc ttctcttctg aacgttatga cctttctcca 1200
   gtaggtcgta tgaagtttaa ccgtcgtttg ggtcgtcctt acgaagtggg tactgatcag 1260
   aagtcacgtg aagttgaagg tattttatcg catgacgata tcattgatgt acttcgtaca 1320
   ttggtggaga tccgcaatgg taaaggtgaa gtcgacgata tcgatcactt gggtaaccgt 1380
   cgcgtacgtt ctgttggtga aatgacagaa aaccaattcc gtgttggttt ggttcgtgtt 1440
   gaacgtgctg tgaaagagcg tttaagccaa gctgaaactg ataacttgtc tccacaagat 1500
   ttaatcaacg cgaaaccagt tgctgcggca atcaaagaat tctttggttc aagccagtta 1560
   tctcagttca tggaccaaaa caacccatta tctgagatta cacataaacg tcgtgtgtct 1620
   gcgcttggtc ctggtggttt gacacgtgaa cgcgcaggct tcgaagtgcg tgacgtacat 1680
   caaactcact atggtcgtgt ttgtccaatt gaaacacctg aaggtccaaa cattggtttg 1740
   atcaactcgc tttctgtcta tgcaaaagcg aatgacttcg gtttcttgga aacaccatac 1800
   cgtaaggttg tagacggtcg tgtgacagat gaagttgaat atttatctgc aattgaagaa 1860
   gtaggcactg tcattgcaca agccgactca gcagtggata aagatggcaa cttgactgaa 1920
   gaaatggttt ctgtacgtca tcaaggtgaa ttcgtacgta tgtcgcctga gcgcgtaaca 1980
   catatggacg tttctgcaca gcaggttgtt tctgttgcag cgtcattaat tccattcctt 2040
   gaacacgatg acgcaaaccg tgcattgatg ggttcgaaca tgcaacgtca ggctgttcct 2100
   acacttcgtg ctgacaaacc acttgtcggt acgggtatgg aagcaaacgt agcacgcgac 2160
   tcaggtgtat gtgtgatcgc gggtcgtggt ggtgtaattg aatatgttga tgcatctcgt 2220
   atcgttattc gtgtgaacga agatgaaatg attgcaggtg aagcaggtgt agatatttac 2280
   aacctgatca aatatacacg ttcaaaccaa aatacatgta ttaaccaaaa tgtcatcgta 2340
   aacttgggcg acaaagttgc tcgtggcgat attttggctg acggtccatc gactgacatg 2400
   ggtgaacttg cgctaggtca aaacatgcgc gtcgcgttca tgacatggaa cggttataac 2460
   tatgaagact cgatcttact ttctgagcgt gtgcttcaag aagaccgttt aacgtcgatt 2520
   catattcaag aattgtcatg tgtagcgcgt gatactaagt taggtgcaga agaaattact 2580
   gctgatattc ctaacgtcgg tgaagctgca ctgtctaaac ttgatgagtc aggtattgtt 2640
   tatatcggtg ctgaagttac tgcaggtgat attctggttg gtaaggtaac acctaaaggt 2700
   gaaactcagt taacacctga agaaaaacta cttcgtgcaa tctttggtga aaaagcggct 2760
   gacgtaaaag attcatcttt acgtgttccg tcaggcacta aaggtacagt gattgacgtt 2820
   caagtcttca cacgtgatgg tttagaaaaa gatgaacgtg cgcaagcaat tgagaaagct 2880
   cagcttgatg cataccgtaa agacttgaaa gaagaataca aaatcttcga agaagcagca 2940
   cgtgagcgta ttgttcgttt gttgaaaggt caagaatcta acggtggtgg ttcgactaaa 3000
   cgtggtgaga agctttcaga agatatgttg tctggtctag agttagttga tctacttgaa 3060
   atccaaccaa cagatgaagc aattgctgag cgtttaactc aaattcaagt gttcttgaaa 3120
   gaaaagagcc atgaaattga tgaaaaattt gctgagaaga aacgtaaact ttctacaggg 3180
   gatgagttaa caactggtgt attgaaagtt gttaaggttt acctagcagt taaacgtcga 3240
   atccaacctg gtgataagat ggcgggtcgt cacggtaaca agggtgttgt atcaaacatc 3300
   ttaccagttg aagatatgcc acatgatgcc aatggtgtgc cagttgatat cgtattgaac 3360
   ccactcggtg taccatcgcg tatgaacgtg ggtcagattc ttgaaactca cttaggtatg 3420
   gcagcaaaag gtttgggtga gcagattgat aaaatgctca aacaacaacg tacaattgcc 3480
   gagttacgtt cattccttga caagatttat aataaagtgg gtggtgagca agaacagctt 3540
   gacacactaa ctgacgaaga gatctttaag cttgcaggta atttacgtgc tggtgtgcct 3600
   ttggcaactc cagtatttga tggtgctgaa gagtcacaaa tcaaagagtt acttgagctt 3660
   gcagagttac cacgttctgg tcaacaaatc ttgtttgatg gacgtacagg tgaacagttt 3720
   gatcgtccag taactgtcgg ttacatgtac atgcttaagt tgaaccactt ggttgacgat 3780
   aagatgcatg cacgttcaac tggttcttac tcacttgtga cacaacaacc gcttggtggt 3840
   aaagcacaat tcggtggtca gcgtttcggt gagatggaag tatgggcact tgaagcatat 3900
   ggtgcagcat ataccctcca agaaatgctc actgtgaagt cggatgacgt cgaaggtcgt 3960
   acacgcatct ataagaacat tgtagatgga aaccattata tggatccggg tatgcctgaa 4020
   tcgttcaacg tattgaccaa agagatccgt tctttaggta tcaacattga actgaaaaat 4080
   ggtgactaa 4089
<210> 14
   <211> 4089
   <212> DNA
   <213> Acinetobacter sp. CIP-A165
<400> 14
   atggcatact catataccga aaagaaacgg atccgtaaga attttggtaa attgccccaa 60
   gtgatggaag caccgtactt actttcgatt caagtcgatt cgtaccgtac gtttttacaa 120
   ggtggcaaaa ctccaaagaa ccgcgaagat atcggtctcc aagccgcatt tcgttcagtt 180
   tttcctattg aaagttattc tggcaatgct gctttagaat ttgttgagta tagtcttggt 240
   aagcctgagt ttgacgtgcg tgaatgtatt ttacgtggtt cgacttatgc agcaccaatg 300
   cgcgtaaaaa ttcgtttgat cattaaagat cgtgaaacga aatcaattaa agacgttcgt 360
   gagcaagaag tgtacatggg tgaaatgcca ctcatgaccg ataacggtac tttcgttatc 420
   aatggtactg agcgtgtgat cgtatctcag ttacaccgtt cgccaggtgt gttctttgac 480
   catgacaagg gtaaaactca ctcaagtggt aaagtgttgt attcagcacg tattatccct 540
   taccgtggtt catggttaga ctttgaattt gacgcaaaag atcttgtgtt tgtacgtatt 600
   gaccgtcgtc gtaaattgtt ggcgactgtg attttacgtg ctttaaatta tagcaatgcg 660
   caaattttga atttgttcta cgaaaaagta cctgtatatc ttgatatggg tagctatcaa 720
   attgacctca ttccagatcg cttgcgtgga gaaatggcgc agtttgatat tgctgacaat 780
   gacggtaaag tgattgtaga gcaaggtaaa cgtattaatg cgcgtcacgt acgtcaaatg 840
   gaagcggcta acttagccaa gctttctgta cctgatgaat acttgtatga gcgtatcacc 900
   gctgaagata tcccattaaa agatggtgag gtgattgctg caaatacctt gttaagccat 960
   gaagtgatgg tgaagttagc tgaaggcggt gttaagcaat ttaacatcct atttactaac 1020
   gatatcgacc gtggttcttt tattgctgac accttacgtg cagatactac aacggggcgt 1080
   gaagaagcac tcgttgaaat ctacaaagta atgcgtccag gtgagccacc gacgaaagaa 1140
   gctgcagata atttatttaa taacttattc ttctcttctg agcgttatga cttgtcgcct 1200
   gttggtcgta tgaagtttaa ccgtcgcttg ggtcgtccat acgaagtcgg tactgatcaa 1260
   aagtctcgtg aagttgaagg tattttatcg cacgacgata tcattgatgt acttcgtaca 1320
   ttggttgaaa tccgtaacgg taaaggtgaa gtcgacgata tcgaccactt gggtaaccgt 1380
   cgcgtacgtt ctgttggtga aatgacagaa aaccaattcc gtgttggttt agtacgtgtt 1440
   gagcgtgctg ttaaagagcg tttaagccaa gctgaagcgg ataacctgtc tccacaggat 1500
   ttgattaacg caaaaccagt tgctgctgcg attaaagaat tctttggttc aagccaattg 1560
   tctcagttca tggatcaaaa caacccatta tctgaaatta cacacaaacg tcgtgtatca 1620
   gcgcttgggc ctggtggttt gacacgtgaa cgtgcgggct ttgaggttcg tgacgtacat 1680
   caaacgcatt atggtcgtgt ttgtccaatt gaaacgcctg aaggtccaaa cattggtttg 1740
   atcaactcgc tttctgttta tgcaaaaacg aacaactttg gtttcttaga aacaccttac 1800
   cgtaaagttg tagatggtcg tgttactgat gcggttgagt atttatctgc gattgaagaa 1860
   gtaggtactg ttattgcaca ggccgattca gcaattgata aagaaggtac tttgactgaa 1920
   gaaatggttt ctgtacgtca tcagggtgat ttcgttcgta tgtcacctga gcgtgtcact 1980
   catatggatg tatctgcaca gcaggttgtt tctgttgcag cgtcattgat tccgttcctt 2040
   gaacacgatg atgcgaaccg tgcattgatg ggttcaaaca tgcagcgtca ggcagttcca 2100
   acgttacgtg ctgacaagcc gcttgttggt acgggtatgg aagcaaacgt agcgcgtgac 2160
   tctggtgtgt gtgtgatcgc aaaccgtggt ggtgcgattg agtatgttga tgcctcacgt 2220
   attgttattc gtgtaaacga agatgaaatg attgctggtg aagcaggtgt agatatctac 2280
   aacctgatca aatatacccg ttcgaaccaa aacacctgta tcaaccaaaa tgttatcgta 2340
   aacatgggcg acaaagttgc gcgtggtgat atcttggctg atggtccatc gacggatatg 2400
   ggtgagcttg cgcttggtca aaacatgcgc gtcgcgttca tgacatggaa cggttataac   2460
   tacgaagact cgatcttact ttcagagcgt gtgcttcaag aagaccgttt aacttcgatt   2520
   catatccaag agttgtcatg tgtcgcacgt gataccaagt taggtgcaga agaaatcact   2580
   gccgatattc ctaacgttgg tgaagcagcg ctttctaagt tggatgagtc tggtattgtt   2640
   tatatcggtg ctgaagttac agcaggtgac atccttgttg gtaaagtaac acctaaaggt   2700
   gaaacacagt taacacctga agaaaaatta cttcgtgcta tctttggcga aaaagcagct   2760
   gatgtaaaag attcatcttt acgcgttcca tctggtacca aaggtacagt gatcgatgtt   2820
   caagtcttca cgcgtgatgg tttggaaaaa gatgatcgtg cacaagcgat tgaaaaagca   2880
   cagcttgatg cttaccgtaa agatttgaaa gaagaataca aaatctttga agaagcagca   2940
   cgtgaacgta ttgttcgctt gttgaaaggt aaagagtcta atggtggcgg tacaacgaag   3000
   cgcggcgata aacttgcaga agatatgttg tctggtttag agctggttga tttgttagaa   3060
   atccaaccaa cagatgaagc aatcgctgaa cgtttaactc aaattcaggt attcttgaaa   3120
   gagaagagta tcgagattga tgagaaattt gctgagaaga aacgcaaact ctctacaggt   3180
   gatgaattaa caacgggtgt attaaaagtt gttaaggttt accttgcagt gaaacgtcgt   3240
   atccaaccgg gtgataagat ggcgggtcgt cacgggaaca agggtgttgt ctctaacatc   3300
   ttacctgttg aagatatgcc acatgatgcc aatggtgtac ctgttgacat cgtattgaac   3360
   ccattgggtg taccgtcgcg tatgaacgtg ggtcagattc ttgaaaccca tttgggcttg   3420
   gcggcaaaag gtttgggtga gcagatcgat aagatgctgc aacaacaacg taccgttgct   3480
   gaacttcgtt tgttccttga taagatttac aacaaagttg gtggcgagca agaagatctt   3540
   gatagcttaa ctgatgaaga agtgttgaag cttgcaggta acttacgtgc aggtgttcct   3600
   ttggcaacac cagtgtttga tggtgctgaa gaaagccaaa ttaaagaatt acttgaactt   3660
   gctgaattgc cgcgttctgg tcaacagact ttgtttgatg gccgtacagg tgaacagttt   3720
   gaccgtcctg taactgttgg ttacatgtat atgctcaaat tgaaccactt ggttgatgac   3780
   aaaatgcacg cgcgttcaac tggttcttac tcgcttgtga cacagcagcc gcttggtggt   3840
   aaagcacaat tcggtggtca gcgttttggt gagatggaag tatgggcact tgaagcatat   3900
   ggtgcagcat ataccctcca agaaatgctc actgtgaagt cggatgacgt cgaaggtcgt   3960
   acacgcatct ataagaacat tgtagatgga aaccattata tggatccggg tatgcctgaa   4020
   tcgttcaacg tattgaccaa agagatccgt tctttaggta tcaacattga actgaaaaat   4080
   ggtgactaa                                                           4089
<210> 15
   <211> 4089
   <212> DNA
   <213> Acinetobacter johnsonii
<400> 15
   atggcatact catataccga aaagaaacgg atccgtaaga attttggtaa attgcctagt     60
   gttatggatg ctccgtactt gctcgcgatt caagtcgact cgtacagaac atttttacaa    120
   gatggcaaat caccaaaaaa ccgcgaagat atcggtctgc aagccgcgtt tcgttcagtt    180
   tttcctatag aaagttattc tggcaatgct gctttagaat ttgttgagta tagtcttggt    240
   aagcctgagt ttgatgtacg cgaatgtatt cttcgtggct caacttatgc agcaccaatg    300
   cgcgtaaaaa ttcgtttgat cttaaaagat cgcgaaacta aatcaattaa agacgtgcgc    360
   gagcaagaag tgtacatggg tgaaatgcca ctcatgaccg ataacggtac attcgttatt    420
   aatggtactg agcgtgtaat cgtatctcaa ttacaccgtt caccaggcgt gttctttgac    480
   cacgataaag gcaaaaccca ctctagtggt aaagtgcttt attctgcgcg tattattcct    540
   taccgtggtt catggttaga cttcgaattt gacgcaaaag accttgtatt tgtgcgtatt    600
   gaccgtcgtc gtaaattact cgcgactgtg gttcttcgtg ctttgggtta taacaatgcg    660
   aaaatcttag acttgttcta tgaaaaagtg cctgtatacc tagacatggg tagctaccag    720
   attgaccttg ttccagaacg cttacgtggc gaaatggcac aatttgacat cgtagataat    780
   gatggcaaaa ccattgttga gcaaggcaag cgtatcaacg cgcgtcatgt gcgtcaaatg    840
   gaagctgctg gcctagaaaa actttctgtg ccagatgagt acttgtacga gcgcattact    900
   gcagaagaca tcccacttaa agatggtgat gtgattgcag ctaatacctt gttaagccat    960
   gaagtgatgg tgaaattggc tgaagggggt gttaaacaat ttaacattct attcaccaat   1020
   gacatcgacc gtggttcatt cgttgcagat actttacgtg cagacaccac gacaggtcgt   1080
   gaagaagcat tggtagaaat ctacaaagta atgcgtccag gcgagccacc aacgaaagaa   1140
   gcggctgaaa atttattcaa taacttgttc ttctcttcag aacgttatga cctctctcca   1200
   gtgggtcgta tgaagttcaa ccgtcgttta ggtcgtcctt acgaagtggg tacggatcag   1260
   aagtctcgtg aagttgaagg tattttgtcg aacgaagata tcactgatgt attaaaaaca   1320
   ttggttgaaa tccgtaacgg taaaggtgaa gtcgacgata tcgatcactt gggtaaccgt   1380
   cgcgtgcgtt cagtaggcga aatgactgaa aaccaattcc gtgttggtct agttcgtgta   1440
   gaacgtgctg ttaaagaacg tttaagccaa gctgaaacag ataacttgtc tccgcaagat   1500
   ttgatcaatg cgaaaccagt ggctgctgca atcaaagaat tctttggttc aagccagtta   1560
   tctcagttca tggaccaaaa caacccattg tctgagatta cgcacaaacg tcgtgtatcg   1620
   gcgcttggtc ccggtggttt gacacgtgaa cgtgcgggct ttgaggtacg tgacgtacac   1680
   caaactcact acggtcgtgt atgtccaatt gaaacgccgg aaggtccaaa cattggtttg   1740
   atcaactcgc tttctgttta tgcgaaatgt aacaatttcg gtttcttaga aacaccatac   1800
   cgtaaagtgc ttgatggtcg tgtaacggat gaagttgagt atttatctgc aattgaagaa   1860
   gtaggtactg tgattgcaca ggccgattct ggcgtagata aagacggtaa cttaacagaa   1920
   gaatttgttt ctgtacgtca ccaaggtgat ttcgtacgta tgccgcctga aaaagtgacg   1980
   catatggacg tttctgcaca gcaggttgtt tctgttgctg catcactcat tccattcctt 2040
   gaacacgatg acgccaaccg tgcattgatg ggttcaaaca tgcagcgtca agctgtgcct 2100
   acattgattg ctgacaaacc gcttgtaggt acaggcatgg aagcaaatgt agcgcatgac 2160
   tctggtgtgt gtgtgattgc gggtcgtggt ggtcgtatcg aattcgtcga tgcttcacgt 2220
   gttgtgattc gtgtcaatga agatgaaatg gttgcaggcg aggcaggtgt agatatctat 2280
   aacctgatca aatatacacg ttcgaaccaa aacacttgta ttaaccaaaa agttcttgtg 2340
   aaacttggtg ataaagtggg tcgtggcgat gtattggctg atggtccatc aacagatggt 2400
   ggtgagcttg cgctaggtca aaacatgcgc gttgcgttca tgacgtggaa tggttacaac 2460
   tatgaagact cgatcttact ttcagagcgc gtacttcaag aagaccgttt aacctcgatt 2520
   cacattcaag aattatcatg tgttgcacgt gatacgaaat tgggtgcgga agagatcaca 2580
   gcggatatcc cgaatgtggg tgaagctgca ctgtctaagc ttgatgaatc aggtatcgta 2640
   tatatcggtg ctgaagtgac tgctggtgat atccttgtag gtaaagtaac gcctaaaggt 2700
   gaaacgcagt taacaccaga agaaaaattg cttcgtgcaa tcttcggtga aaaagcagct 2760
   gacgtaaaag actcatcttt acgtgttcca tcaggtacca aaggtacagt gattgacgtt 2820
   caagtgttta cacgtgacgg tcttgagaaa gacgaacgtg cgcaagcaat tgaaaaagct 2880
   cagcttgatt catatcgtaa agacttgaaa gaagaataca aaatcttcga agaagcagca 2940
   cgtgaacgta ttgttcgttt gttgacaggt caagagtcta acggtggtgg tacaaccaag 3000
   cgtggcgata agctttctgt agacgtattg tctggtttag agttggttga tttacttgaa 3060
   atccaaccga ctgatgaagc tattgcagag cgtttaactc aaattcaagt gttcttgaaa 3120
   gagaagagct ttgaaattga tgagaagttt gcagagaaaa aacgcaaact ttctacaggt 3180
   gatgaattaa caacaggtgt attgaaagtt gttaaggttt acttggctgt taaacgtcgc 3240
   atccaaccgg gtgataagat ggcgggtcgt cacggtaaca agggtgttgt ttctaacatc 3300
   ttgcctgttg aagacatgcc gcacgatgcc aatggtgttc cagtcgacat cgtattgaac 3360
   ccactgggtg taccgtcacg tatgaacgtg ggtcagattc tagagactca cttaggtatg 3420
   gcagcgaaag gtcttggcga agaaatcgac aagatgttaa aagcgcaacg tactgtactt 3480
   gagcttcgtg gattcttaga caagatttat aacaaagttg gtggcgagca agaagatctt 3540
   gatagcttaa ctgatgatga aattttggtg ctttcgggta acttgcgtgc gggtgttcct 3600
   cttgcaacgc cagtattcga tggtgctgaa gaatctcaaa ttaaagactt gttagagctt 3660
   gcgaacattt cacgtactgg tcaaacagta ttgtatgatg gtcgtacagg tgaacagttt 3720
   gaccgtcctg taactgtagg ttacatgtac atgttgaaac tgaaccactt ggtagacgac 3780
   aagatgcacg cacgttctac tggttcttac tcattagtaa ctcaacagcc gcttggtggt 3840
   aaagcacaat tcggtggtca gcgtttcggt gagatggaag tctgggcgct tgaagcatat 3900
   ggcgcagctt acacgcttca agaaatgctt actgttaagt cggatgacgt tgaaggtcgt 3960
   acccgtatct ataagaacat tgtagatggt aaccattata tggacccagg tatgcctgaa 4020
   tcgttcaacg tattgaccaa agagatccgt tctttaggta tcaacattga actgaaaaat 4080
   ggtgactaa 4089
<210> 16
   <211> 4089
   <212> DNA
   <213> Acinetobacter lwoffii
<400> 16
   atggcatact catataccga aaagaaacgg atccgtaaga attttggtaa attgcctagc 60
   gttatggatg ctccgtacct gctcgcgatt caagtcgact cgtacagaac gttcttacaa 120
   gatggcaaat caccaaaaaa ccgcgaagat atcggtctcc aagccgcatt tcgttcagtt 180
   tttcctatag aaagttattc tggcaatgct gctttagaat ttgttgagta tagtcttggt 240
   aagcctgagt ttgatgtacg cgaatgtatt cttcgtggct caacttatgc agcaccaatg 300
   cgtgtaaaaa ttcgtttgat cctgaaagat cgtgaaacga agtcaattaa agacgtacgt 360
   gaacaagaag tctatatggg cgaaatgcca ttgatgacgg ataacgggac ctttgtaatt 420
   aatggtaccg agcgtgtaat cgtatctcaa ttacaccgtt caccaggcgt attctttgac 480
   cacgataaag gtaagactca ctcaagtggt aaagtccttt attcagcgcg tatcattcct 540
   taccgtggtt catggttaga ttttgaattc gatgccaaag acctagtcta tgtacgtatt 600
   gaccgtcgtc gtaaattact tgcgactgtg gtgctgcgtg cgctgggtta tagcaacgaa 660
   aacattctca acatgttcta cgagaaagtc cctgtgtatc ttgacatggg tagctatcaa 720
   attgacttgg tgccggaacg tctgcgcggc gaaatggcac aatttgacat cttggataaa 780
   gatggcaagg caatcgttga acaaggtaaa cgtattaacg cgcgtcatgt acgtcaaatg 840
   gaagcttcag gtcttgaaaa acttgcagtg cctgatgagt atttgtatga gcgtatcacg 900
   gctgaagaca tcgcattaaa agatggcgac gtgattgctg caaataccgt attgagccat 960
   gaagtcatgg tgaaaattgc agaaggcggc gtgaagcagt ttaatgttct gttcaccaat 1020
   gatatcgacc gtggttcatt cgttgcagat tctctacgtg cagatactac gaccactcgt 1080
   gaagaagcat tagtagaaat ctacaaagtc atgcgtccgg gcgagccacc aaccaaagaa 1140
   gcagctgaga acctgttcaa taacctgttc ttctcttctg agcgttatga cttgtctcca 1200
   gtcggtcgta tgaagttcaa ccgtcgtttg ggtcgtcctt atgaagtggg tacagaccag 1260
   aagtcgcgtg aagttgaagg tattctctcg aacgaagata tcactgatgt attgaaaaca 1320
   ttagttgaaa tccgtaacgg taaaggtgaa gtcgacgata tcgatcactt gggtaaccgt 1380
   cgtgttcgtt ctgtgggcga aatgacagaa aaccaattcc gtgtcggtct ggttcgtgta 1440
   gaacgtgctg ttaaagaacg tttatctcaa gctgaaacag acaacctgtc tccgcaagat 1500
   ctaatcaatg cgaagcctgt tgctgctgca atcaaagaat tctttggttc aagccagttg 1560
   tctcagttca tggatcaaaa caacccgttg tctgaaatca cgcacaagcg tcgtgtatca 1620
   gcacttggtc ccggtggttt gacgcgtgaa cgtgcgggct ttgaagtacg tgacgtacat 1680
   caaacgcatt acggtcgtgt atgtccaatt gaaacgccgg aaggtccaaa cattggtttg 1740
   atcaactcgc tttctgtata tgctaaagcg aacaacttcg gtttcctgga aacgccttac 1800
   cgtaaagtag ttgatggccg tgtaaccatg gacattgaat acctgtctgc gattgaagaa 1860
   gtgggtactg tgattgcaca ggccgattct gcagtagatg ctgatggtaa tttattagaa 1920
   gaagttgtat ctgtacgtca ccaaggtgac ttcgtacgca tgccgccgga aaaagtaacg 1980
   catatggatg tatctgctca gcaggttgta tctgtggctg cgtcactgat tccgttcctt 2040
   gaacacgatg atgccaaccg tgcattgatg ggttcaaaca tgcaacgtca ggctgttcct 2100
   acgttaatcg ctgacaaacc actcgtaggt acgggtatgg aagcgaacgt agcacatgac 2160
   tctggtgtat gtgtgatcgc tcagcgtggt ggtcgtatcg agtttgttga tgcatctcgt 2220
   gtcgtgattc gtgtgaacga agaagagatg atcgcaggtg aggcaggtgt agatatctat 2280
   aacctgatca aatacacccg ttcaaaccaa aacacctgta tcaaccagaa agttctggtg 2340
   aacctgggcg ataaagtggg tcgtggtgac gtattagctg atggtccatc gactgatggc 2400
   ggtgagctag cgcttggtca aaacatgcgc gtcgcattca tgacctggaa cggttacaac 2460
   tacgaagact cgatcttact ttcagagcgt gtacttcaag aagaccgttt aacctcgatt 2520
   catatccagg aattgtcatg tgtcgcacgt gatactaagc ttggtgctga agaaatcacg 2580
   gccgatatcc cgaatgtggg tgaagcagcg ctttctaaac ttgatgaatc aggtattgtt 2640
   tacatcggtg ctgaagtaac agcaggtgat attcttgttg gtaaagtgac gcctaaaggt 2700
   gaaacgcagc taacgccgga agaaaaattg cttcgtgcaa tcttcggtga aaaagcagct 2760
   gacgttaaag attcatcttt acgcgttcca tctggtacca aaggtactgt gattgatgtt 2820
   caagtgttta cacgtgatgg tcttgaaaaa gacgaacgtg ctcaagcaat tgaaaaagct 2880
   cagcttgatg cttaccgtaa agacttgaaa gaagagttca agatctttga agaagcagca 2940
   cgtgaacgtg taattcgttt gttgaacggt caagagtcga atggtggcgg taccactaaa 3000
   cgtggcgaca aactgtctga agacgtgttg tctggtttag agcttgttga tcttcttgaa 3060
   attcaaccgg ttgatgaagc aatcgctgaa cgtctaacgc aaattcaagt gttcttgaaa 3120
   gagaagagct tcgaaattga cgagaaattt gctgagaaaa aacgcaaact ttctacaggc 3180
   gatgagctga ccactggcgt attgaaagta gttaaagttt atcttgcggt aaaacgtcgc 3240
   atccagccgg gtgataagat ggccggtcgt cacggtaaca agggtgttgt atcaaacatc 3300
   ttgccggtag aagacatgcc acacgatgcc aatggtgtac ctgttgatat cgtactgaac 3360
   ccgctgggcg taccatcgcg tatgaacgtg ggtcagattc ttgaaactca cttaggtatg 3420
   gctgccaaag gtcttggcga tcaaatcgac aagatgatga aagaacagcg tactgtactt 3480
   gagcttcgtg atttcctgga caagatttat aacaaagttg gtggcgagca agaagatctt 3540
   gacagcttga ctgatgaaga aatcttgaaa ctttctggta acttgcgtgc tggtgtgcct 3600
   ttggcaacac ctgtattcga tggtgctgaa gaaggtcaga tcaaagaatt gttacaactt 3660
   gcaggtctat caagtactgg tcagacagta ttatatgatg gtcgtactgg tgagcgtttc 3720
   gaccgtccag taactgtagg ttatatgtac atgctgaaac tgaaccactt agttgatgac 3780
   aagatgcatg cgcgttcaac tggttcttat tctctagtca cgcaacagcc gctgggtggt 3840
   aaagcacaat tcggtggtca gcgtttcggt gagatggaag tctgggcact agaagcttac 3900
   ggtgcagcat atacgctaca agaaatgctg actgtgaaat cggatgacgt tgaaggccgt 3960
   acccgtatct acaagaatat tgtagatggt aaccattata tggacccggg tatgcctgaa 4020
   tcgttcaacg tattgaccaa agagatccgt tctttaggta tcaacattga actgaaaaat 4080
   ggtgactaa 4089
<210> 17
   <211> 4089
   <212> DNA
   <213> Acinetobacter sp.
<400> 17
   atggcatact catataccga aaagaaacgg atccgtaaga attttggtaa attgcctagc 60
   gtcatggatg ctccgtactt gctcgcgatt caagtcgact cgtacagaac attcttacaa 120
   gatggcaaat caccaaaaaa ccgcgaagat atcggtctcc aagccgcatt tcgttcagtt 180
   tttcctattg aaagttattc tggcaatgct gctttagaat ttgttgagta tagtcttggt 240
   aagcctgagt ttgatgtacg cgaatgtatc cttcgtggct caacttatgc agcaccaatg 300
   cgtgtaaaaa ttcgtttgat cctgaaagat cgtgaaacga agtcaattaa agacgtacgt 360
   gaacaagaag tctatatggg cgaaatgcca ttgatgacgg ataacggtac ctttgtaatt 420
   aacggtaccg agcgtgtgat cgtgtcacaa ttacaccgtt caccaggcgt attctttgac 480
   cacgataaag gcaagactca ctcaagtggt aaagtccttt attcagcacg tatcattcct 540
   taccgtggtt catggttaga ctttgaattc gatgccaaag acctcgtcta tgtacgtatt 600
   gaccgtcgtc gtaaattgct tgcgactgtg gtactccgtg ccttgggtta tagcaacgaa 660
   aacattctcg acatgttcta cgagaaagta cctgtgtatc ttgacatggg tagctaccag 720
   attgacctgg tgcctgaacg tttgcgtggc gaaatggcac aatttgacat tctggacaag 780
   gatggcaagg caattgttga gcaaggtaaa cgtatcaatg cgcgtcatgt acgtcaaatg 840
   gaagcttcag gtcttgaaaa acttgcagtg ccagatgagt acctatatga gcgtatcact 900
   gctgaagaca tccagttaaa agatggcgat gtgattgcag ccaatactgt attaagccat 960
   gaaatcatgg tgaaaattgc agaaggcggc gtgaagcagt tcaatattct gttcaccaat 1020
   gatatcgacc gcggttcatt tgttgcagat tctctacgtg cagatacaac gagcaatcgt 1080
   gaagaagcat tggtagaaat ctacaaagtg atgcgtccgg gcgaaccacc aaccaaagaa 1140
   gctgctgaaa acttattcaa caacttgttc ttctcttctg aacgttatga cttgtctcca 1200
   gttggtcgta tgaagttcaa ccgtcgtttg ggtcgtcctt atgaagtggg tacagaccag 1260
   aagtcacgtg aggttgaagg cattctctcg aacgaagata tcactgatgt attaaaaaca 1320
   ttagttgaaa tccgtaacgg taaaggtgaa gtcgacgata tcgatcactt gggtaaccgt 1380
   cgtgttcgtt ctgtgggtga aatgacagaa aaccaattcc gtgtaggtct ggttcgtgta 1440
   gaacgtgctg ttaaagaacg tctatctcaa gctgaaactg acaacctgtc tccgcaagat 1500
   ttgatcaatg cgaaacctgt tgctgctgca atcaaagaat tctttggttc aagccagttg 1560
   tctcagttta tggatcaaaa caacccgtta tctgagatta cgcacaaacg tcgtgtttct 1620
   gcacttggtc ctggcggttt gacgcgtgaa cgtgcaggct ttgaagtacg tgacgtacat 1680
   caaactcact acggtcgtgt atgtccaatt gaaacgcctg aaggtccaaa cattggtttg 1740
   atcaactcgc tttctgttta tgcgaaatgt aacaactttg gttttctgga aaccccatac 1800
   cgtaaggttg ttgatggtcg tgtaacagat gaagttgaat acctgtctgc gattgaagaa 1860
   gtaggtactg tcattgcaca ggccgattct gcaatggata aagacggtaa cttaacagaa 1920
   gagtttgtat ctgttcgtca tcagggtgac ttcgtacgta ttcctcctga aaaagtaacg 1980
   catatggatg tatctgctca gcaggtcgta tctgtagcag cgtcactgat tccattccta 2040
   gaacacgatg acgccaaccg tgcgttaatg ggttcgaaca tgcaacgtca ggcagttccg 2100
   acgttgatcg ctgacaagcc gcttgttggt accggtatgg aagcgaacgt agcacatgac 2160
   tcaggtgtat gtgtgatcgc tcagcgtggt ggtcgtatcg agtttgttga tgcgtctcgt 2220
   gtggttattc gtgtgaatga agacgaaatg atcgcaggtg aagcaggtgt agatatctac 2280
   aacctgatca agtacacccg ttcgaaccag aacacttgta tcaaccagaa agttcttgtg 2340
   aacctgggcg ataaagtggg tcgtggtgat gtcctggctg atggtccatc gactgatggc 2400
   ggtgagctgg cactgggtca aaacatgcgc gttgcgttca tgacctggaa tggttacaac 2460
   tacgaagact cgatcttact ttctgagcgt gttcttcaag aagaccgttt aacgtctatt 2520
   catatccagg aattatcatg tgtcgcacgt gataccaaac tgggtgcgga agaaatcact 2580
   gctgatattc cgaacgtagg tgaagctgct ctgtctaaac tggacgagtc aggtatcgtt 2640
   tacatcggtg ctgaagtaac tgctggcgat atcctggttg gtaaagtaac ccctaaaggt 2700
   gaaacacaac ttactccgga agaaaaattg ctacgtgcaa tcttcggtga aaaagcagct 2760
   gacgtaaaag actcatcttt acgcgttcca tcaggtacta aaggtacagt cattgacgtt 2820
   caagtgttta cacgtgacgg tcttgaaaaa gacgaacgtg ctcaagcaat tgaaaaagcg 2880
   caattggatg cataccgtaa agacttgaaa gaagaattca aaatcttcga agaagctgca 2940
   cgtgaacgtg taatccgtct actgaatggc caagagtcga atggtggcgg tacaactaaa 3000
   cgtggcgaca aactgtctga agacgtgttg tctggtttag agcttgttga tcttcttgaa 3060
   attcaaccag ttgatgaagc aattgctgaa cgtttaactc aaattcaagt gttcttgaaa 3120
   gagaagagct tcgaaattga cgagaaattt gctgagaaaa aacgcaaact ttctacaggc 3180
   gatgaactga ccactggcgt tttaaaagtt gttaaggttt atcttgctgt aaaacgtcgc 3240
   atccaaccgg gtgataagat ggcgggtcgt cacggtaaca aaggtgttgt atcaaacatc 3300
   ttgccggtag aagacatgcc acatgatgcc aacggtgtac ctgttgatat cgtattgaac 3360
   ccgcttggcg taccatcacg tatgaacgtg ggtcagattc ttgaaactca cttgggtatg 3420
   gcggcgaaag gtcttggcga tcaaatcgac aagatgatga aagagcaacg tactgtactt 3480
   gagcttcgtg atttcctgga caagatttac aataaagttg gtggcgagca agaagatctt 3540
   gatagcttga ctgatgaaga aatcttgaaa ctttctggca acttgcgtgc tggtgtgcct 3600
   ttggctactc ctgtattcga tggtgctgaa gaaggtcaga tcaaagagtt gttacaactt 3660
   gcaggcctat ctagtactgg tcagaccgta ttatatgatg gtcgtactgg tgaacgtttc 3720
   gatcgtccgg taactgttgg ttatatgtac atgctgaaac tgaaccattt ggttgatgac 3780
   aagatgcatg cgcgttcaac tggctcttat tctctggtaa cgcaacagcc gctgggtggt 3840
   aaagcacaat tcggtggtca gcgtttcggt gagatggaag tctgggcact agaagcttac 3900
   ggtgcagcat atacgctaca agaaatgctg actgtgaaat cggatgacgt tgaaggccgt 3960
   acccgtatct acaagaacat tgtggatggc aaccattata tggacccggg catgcctgaa 4020
   tcgttcaacg tattgaccaa agagatccgt tctttaggta tcaacattga actgaaaaat 4080
   ggtgactaa 4089
<210> 18
   <211> 4089
   <212> DNA
   <213> Acinetobacter sp.
<400> 18
   atggcatact catataccga aaagaaacgg atccgtaaga attttggtaa attgccccaa 60
   gtcatgcatg ctccgtactt gctcgcgatt caagtcgact cgtacagaac attcttgcaa 120
   gatggcaaaa ctccaaaaaa tcgcgaagat atcggtctcc aagctgcatt tcgttcagtt 180
   tttcctattg aaagttattc gggcaatgct gctttagaat tcgttgagta tagtcttggt 240
   aaaccagagt ttgatgttcg cgagtgtatt cttcgcggtt caacctttgc ggcaccaatg 300
   cgcgttaaaa ttcgtttgat catcaaagat cgtgaaacga aatctatcaa agacgtacgt 360
   gaacaagaag tgtacatggg tgaaatgcca ctcatgactg agaatggtac ttttgtaatc 420
   aatggtactg agcgtgtaat cgtatctcaa ttacaccgtt ctccgggcgt cttctttgat 480
   cacgataagg gtaaaactca ttcaagtggt aaagtgcttt attcagcacg tatcattcct 540
   taccgtgggt catggttgga ttttgaattt gatgctaaag acttagtatt tgttcgtatt 600
   gaccgtcgtc gtaaattgtt ggcgactgtt attctacgtg cattgaacta tagcaatgaa 660
   caaattctca atatgttcta tgagaaagta cctgtatatc ttgatatggg tagctatcag 720
   attgaccttg ttccagaacg ccttcgcggt gaaatggcgc agttcgatat tgcagacaat 780
   gacggtaaag tgattgttga acaaggtaaa cgtatcaatg cacgtcatgt gcgtcaaatg 840
   gaagctgcgg gtttaactaa actttctgtt cctgatgaat acttgtatga gcgtattaca 900
   gctgaagata ttactttacg tgatggtgaa gtgattgctg ctaataccat tttaagccat 960
   gaagtattgg ttaaaatcgc tgaaggtggt gttaaacaat ttaacatctt gttcaccaat 1020
   gatatcgatc gtggttcgtt tgttgcagat acactacgtg cagatacgac aacaggtcgt 1080
   gaagaagcac ttgtagaaat ctacaaagtc atgcgtccag gtgagccacc aacaaaagaa 1140
   gcggctgaaa acttattcaa taacttattc ttctctacag agcgttatga cttgtctcct 1200
   gtaggtcgta tgaagtttaa tcgtcgtttg ggtcgtcctt atgaagtcgg tacagatcag 1260
   aagtctcgtg aagtagaagg tattctttct aacgatgaca tcattgatgt attgaaaaca 1320
   ttggtagaaa tccgtaacgg taaaggtgaa gtcgacgata tcgatcactt aggtaaccgt 1380
   cgtgtacgtt ctgttggtga aatgacagaa aaccaattcc gtgttggtct agttcgtgtt 1440
   gaacgtgctg taaaagagcg tttaaaccaa gctgaaacag ataacttgtc tccacaagat 1500
   ttgatcaatg cgaaaccagt tgcagctgca atcaaagaat tctttggttc aagccaattg 1560
   tctcagttta tggatcaaaa caatccattg tctgaaatta cgcacaaacg tcgtgtttca 1620
   gcgcttggtc ctggtggttt gacacgtgaa cgcgcaggct ttgaggtacg tgacgtacat 1680
   caaactcact atggtcgtgt gtgtccaatt gagacacctg aaggtccaaa cattggtttg 1740
   atcaactcac tttctgtata tgcaaaagcc aacaacttcg gtttcttgga aacaccatac 1800
   cgtaaagtgg ttgatggtcg tgtaacggat gatgttgagt atttatctgc gattgaagaa 1860
   gtaggcactg taattgcaca agccgattct ggtgtagata aagacggtca cttgactgaa 1920
   gaattcgttt ctgtacgtca tcaaggtgaa ttcgttcgta tgcctcctga aaaagtgacg 1980
   catatggatg tatctgctca gcaggttgtt tctgttgctg catcacttat tccattcctt 2040
   gaacacgatg atgcgaaccg tgcattaatg ggttcaaaca tgcaacgtca ggctgttcct 2100
   acattgcttg ctgacaagcc acttgttggt acaggtatgg aagcaaacgt agcgcatgac 2160
   tctggcgtgt gtgtaatcgc aaaacgtggc ggacgtattg agttcgttga tgcttctcgt 2220
   gttgttattc gtgtgaacga agatgaaatg atcgcgggtg aagcaggtgt agacatctac 2280
   aacttgatca aatacacacg ttcaaaccaa aacacttgta tcaaccaaaa agttcttgtg 2340
   agcttgggcg ataaagtagg tcgtggtgac gttcttgctg atggtccatc gactgacggt 2400
   ggtgaattag cgcttggtca aaacatgcgt gtcgcgttca tgacgtggaa cggttataac 2460
   tacgaagact cgatcttatt atctgagcgt gtacttcaag aagaccgttt gacttcgatt 2520
   cacatccaag agttgtcatg tgtagcgcgt gataccaagt taggtgcaga agaaattact 2580
   gcagatattc ctaacgtggg tgaagcagct ttatctaaac ttgatgaatc aggtattgtt 2640
   tatatcggtg ctgaagtttc agcaggtgac atccttgttg gtaaggtaac gcctaaaggt 2700
   gaaacacaat taacacctga agaaaaatta cttcgtgcaa ttttcggtga aaaagctgcg 2760
   gatgtaaaag actcttcttt acgtgtttct tcaagcgtaa aaggtacagt gattgacgtt 2820
   caagtgttta cacgtgacgg tatcgagaaa gatgagcgtg ctcaagcaat tgaaaaagcg 2880
   caacttgatg cttaccgtaa agacttgaaa gaagagttca aaatcttcga agaagctgct 2940
   cgtgaacgta ttgtgcgttt attgaaaggt caagagtcaa atggtggtgg tacaacgaaa 3000
   cgtggtgaca agctaactga agacgtattg tctaacttag agcttgttga tctgttagaa 3060
   gttcaaccag cagacgaagg tattgctgag cgtttaacgc agattcaagt gttcttgaaa 3120
   gagaagagcc acgagatcga tgagaagttt gctgagaaaa aacgtaaact ttcaacgggt 3180
   gatgaactga caactggtgt gttgaaagtt gttaaagttt atcttgctgt taaacgtcgt 3240
   atccagcctg gtgataagat ggcgggtcgt cacggtaaca agggtgttgt atcaaacatc 3300
   ttaccagttg aagatatgcc gcatgacatc catggtgttc cagtggatgt ggtacttaac 3360
   ccactcggtg taccatcacg tatgaacgtg ggtcagattc ttgaaactca cttgggtatg 3420
   gcagcgaaag gtcttggcga taagatcgac aagatgatga aagagcaacg tactgttctt 3480
   gaacttcgtg aattcttaga caagatttat aacaaagttg gtggcgagca agaagatctt 3540
   gatagcttga ctgatgaaga aatcttggtg ttatcaggta acttgcgtaa aggtgttcct 3600
   ttagctacac cagtatttga tggtgcagaa gaaggacaaa tcaaagagtt acttgaactt 3660
   ggtggtatct cacgtacagg tcaaacagta ttgtatgatg gacgtacagg tgagcgtttt 3720
   gaccgcccag taacagtagg ttatatgtac atgctcaagt tgaaccactt ggttgacgac 3780
   aagatgcatg cacgttctac tggttcttac tcactggtga ctcaacaacc gcttggtggt 3840
   aaagcacaat tcggtggtca gcgtttcggt gagatggaag tttgggcact tgaggcttat 3900
   ggtgctgctt atacacttca agaaatgtta actgtgaaat cggatgacgt tgaaggtcgt 3960
   actcgcatct ataagaacat tgtagatggt aaccattata tggatccggg tatgcctgaa 4020
   tcgttcaacg tattgaccaa agagatccgt tctttaggta tcaacattga actgaaaaat 4080
   ggtgactaa 4089
<210> 19
   <211> 4089
   <212> DNA
   <213> Acinetobacter sp.
<400> 19
   atggcatact catataccga aaagaaacgg atccgtaaga attttggtaa attgccccaa 60
   gtcatgcatg ctccgtacct gctctcgatt caagtcgact cgtacagaac attcttgcaa 120
   gacggcaaaa caccaaaaaa tcgcgaagat atcggtctcc aagctgcatt tcgttcagtt 180
   tttcctattg aaagttattc gggcaatgct gctttagaat tcgttgagta tagtcttggt 240
   aaaccagagt ttgatgttcg cgaatgtatt cttcgtggct caacctttgc ggcaccaatg 300
   cgcgttaaaa ttcgtttgat catcaaagat cgtgaaacga aatctattaa agacgtacgt 360
   gaacaagaag tgtacatggg tgaaatgcca ctcatgactg agaatggtac ctttgtcatc 420
   aatggtactg agcgtgtaat cgtatctcaa ttacaccgtt caccaggcgt attctttgac 480
   catgataaag gtaaaacgca ttcaagcggt aaagtgcttt attcagcacg tattattcct 540
   tatcgtggtt catggttaga ttttgagttt gatgctaaag atttagtctt tgtacgtatt 600
   gaccgtcgtc gtaaattgct tgcgactgtt gtgttgcgtg cattgagcta tagcaatgaa 660
   caaattctga atatgttcta cgaaaaagta cctgtatatc ttgatatggg tagctatcag 720
   attgaccttg tgcctgaacg tcttcgtggt gaaatggctc aatttgatat cgtggacaat 780
   gatggtaaag ccattgttga acaaggtaaa cgtattaatg ctcgccatgt acgtcaaatg 840
   gaagctgctg gtttaactaa acttccagtt ccagatgaat atttgtatga gcgtattact 900
   gctgaagata tcgtacttaa agacggtgaa gtaattactg ctaacactgt attaagtcat 960
   gagattttgg tcagaattgc tgaaggtggt attaaacaat ttaatatcct gttcaccaat 1020
   gacatcgatc gtggttcttt tgttgctgac accttacgtg cagatacaac atctggtcgt 1080
   gaagaagcac ttgtagaaat ctacaaagtg atgcgtccag gtgagccacc aacgaaagaa 1140
   gcggctgaaa acttattcaa taacttattc ttctctacag agcgttatga tttatcgcct 1200
   gtgggtcgta tgaagtttaa ccgtcgtttg ggtcgtcctt acgaagtagg tacagatcag 1260
   aagtctcgtg aagtagaagg tattctttct aacgatgaca tcattgatgt actgaaaaca 1320
   ctggtagaaa ttcgtaacgg taaaggtgaa gtcgacgata tcgatcactt gggtaaccgt 1380
   cgcgtacgtt ctgttggtga aatgacagaa aaccaattcc gtgttggttt agttcgtgtt 1440
   gaacgtgctg ttaaagagcg tttaaaccaa gctgaaacag ataacttgtc tccacaagat 1500
   ttgatcaatg cgaaaccagt tgctgctgca atcaaagaat tctttggttc aagccaattg 1560
   tcacagttta tggatcaaaa caacccattg tcagaaatta cacacaaacg tcgtgtatct 1620
   gcgcttgggc ctggtggttt gacacgtgaa cgtgcgggct ttgaagtacg tgacgtacat 1680
   caaactcact atggtcgtgt atgtccaatt gaaacacctg aaggaccaaa cattggtttg 1740
   atcaactcgc tttctgttta tgcaaaagcg aacaacttcg gtttcttgga aacaccatac 1800
   cgtcgcgttg ttgatggtcg tgtaacagat gatgttgaat atttatctgc aattgaagaa 1860
   gtaggtactg ttattgcaca ggccgattct gcattggata aagatggaca tttaacagaa 1920
   gacttcgttt cagtacgtca ccaaggtgac ttcgttcgta tgccacctga aaaagtgacg 1980
   catatggatg tatctgctca acaggttgta tctgtcgctg catcacttat tccattcctt 2040
   gaacacgatg atgccaaccg tgcattgatg ggttcaaaca tgcaacgtca ggctgttcct 2100
   acattgcttg ctgataaacc acttgtgggt accggcatgg aagcaaacgt agcgcacgac 2160
   tctggtgtat gtgtgatcgc gaaacgtggc ggacgcattg agtttgtaga tgcatcacgt 2220
   gtggttattc gtgtcaacga agatgaaatg atcgcgggtg aagcaggtgt agatatctac 2280
   aacttgatca aatacacgcg ttcaaaccaa aacacatgta ttaaccaaaa agtgcttgtg 2340
   agcatgggcg ataaagtcgg ccgtggtgac gttcttgctg atggtccatc aactgatggt 2400
   ggtgaattag cattgggtca gaacatgcgt gtcgcgttca tgacttggaa cggttataac 2460
   tacgaagact cgattttatt atctgaacgt gttcttcaag aagatcgttt aacttcaatt 2520
   catattcaag aattatcatg tgttgcgcgt gatacgaagt taggtgcgga agaaatcact 2580
   gccgatattc ctaacgtagg tgaagcagcg ttatctaaac ttgatgaatc aggtattgtt 2640
   tatatcggtg ctgaagttgc agcgggtgat attcttgttg gtaaagtgac gcctaaaggt 2700
   gaaacacaat taacccctga agaaaaatta cttcgtgcaa tctttggtga gaaagcggca 2760
   gacgttaaag attcatcttt acgtgtttct tcaagcgtta aaggtacagt catcgacgtt 2820
   caagtgttta cacgtgacgg tatcgagaaa gatgaacgtg ctcaagcgat tgagaaagcg 2880
   cagcttgatg cttaccgtaa agacttgaaa gaagaattca aaatcttcga agaagcagct 2940
   cgtgaacgta ttatccgttt gttaaaaggc caagagtcga atggcggcgg tactactaag 3000
   cgcggtgata agctatctga agatgtattg tctggtttag agcttgttga tcttttagaa 3060
   gttcaaccaa cagatgaagg catcgctgaa cgcttaactc aaattcaagt gttcttgaaa 3120
   gagaagagct acgagattga tgagaaattt gctgagaaaa aacgcaaact ttctacaggt 3180
   gatgagctta caacaggtgt cttgaaagtt gttaaagttt atttagctgt aaaacgtcgt 3240
   atccagcctg gtgataagat ggcgggtcgt cacggtaaca aaggtgttgt atcaaacatc 3300
   ttgcctgttg aagacatgcc gcatgacatc catggtgttc cagttgatgt cgtacttaac 3360
   ccattgggtg taccatcacg tatgaacgtg ggtcagattc ttgaaactca cttaggtatg 3420
   gctgcaaaag gtcttggcga taagatcgac aagatgatga aagagcaacg taccgttctt 3480
   gagcttcgtg atttcttaga caagatttat aacaaagttg gtggcgagca agaagatctt 3540
   gatagcttaa ctgatgaaga aatcttggtg ttatcaggta acttgcgtaa aggtgttcct 3600
   ttagctacgc cagtatttga tggtgcagaa gaaagtcaga tcaaagagtt acttgagctt 3660
   ggtggtatct cacgtacagg tcaaacagta ttgtatgacg gacgtacagg tgagcgtttt 3720
   gaccgcccag taactgttgg ttatatgtac atgctcaagt tgaaccattt ggttgatgac 3780
   aagatgcatg cacgttctac tggttcttat tcacttgtaa ctcaacaacc gcttggtggt 3840
   aaagcacaat tcggtggtca gcgtttcggt gagatggaag tctgggcact agaagcttat 3900
   ggtgctgctt atacacttca agaaatgctt actgtgaagt cggatgacgt tgaaggtcgt 3960
   actcgcatct ataagaacat cgtagatggt aaccattata tggatccggg tatgcctgaa 4020
   tcgtttaacg tattgaccaa agagatccgt tctttaggta tcaacattga actgaaaaat 4080
   ggtgactaa 4089
<210> 20
   <211> 4089
   <212> DNA
   <213> Acinetobacter radioresistens
<400> 20
   atggcatact catataccga aaagaaacgg atccgtaaga attttggtaa attgccccaa 60
   gtcatggaag caccgtacct gttgtctatt caagtcgact cgtaccgtac tttcctgcaa 120
   gacggcaaaa ctccaaaaaa ccgcgaagat atcggtctcc aagccgcatt tcgttcagtt 180
   tttcctatag aaagttattc tggcaatgct gctttagaat ttgttgagta tagccttggt 240
   aaacccgagt ttgatgtccg cgaatgtatt cttcgtggct caacctatgc ggcaccaatg 300
   cgcgtaaaga ttcgtctgat cattaaagat cgcgaaacga aatcaatcaa agatgttcgt 360
   gaacaagaag tctatatggg tgaaattcca ctcatgactg aaaatggtac ctttgtcatc 420
   aatggtaccg agcgtgtaat cgtatctcag ttacatcgtt caccgggtgt attctttgac 480
   catgacaaag gtaaaaccca ctcaagcggt aaagtgttgt attcagcacg tattattcct 540
   taccgtggtt catggcttga ttttgagttt gatgccaaag acctggtata tgtacgtatc 600
   gaccgtcgtc gtaaattact tgctactgta gttatgcgtg cactgggcta taacaatgaa 660
   cagattctgg atctgttcta tgagaaagtc cctgtgtatc tggatatggg cagttatcag 720
   attgatctcg tgcctgaacg cctgcgtggt gaaatggcgc agttcgatat tgctgacaat 780
   gatggcaaag tgattgttga gcagggtaaa cgtatcaatg cacgtcatgt acgtcaaatg 840
   gaagcttctg gcctgactaa actgccagtt cctgatgagt acctgtacga acggattact 900
   gctgaagata ttagcctgaa atcaggcgat gtaattgctg cgaatacctt gctgagccat 960
   gaaatcatgg tgaagattgc tgaaggtggc gtgaagcagt ttaatatctt gtataccaat 1020
   gatattgacc gtggttcatt cgtggcagat accttacgtg cagatacaac aagtggccgt 1080
   gaagaagccc tggttgagat ctataaggta atgcgtccag gcgagccgcc aacaaaagaa 1140
   gctgctgaga acctgttcaa taacctgttc ttctcttctg agcgttatga cctgtcacct 1200
   gttggtcgta tgaagttcaa ccgtcgttta ggtcgcccat acgaagtagg aactgaccag 1260
   aagtcacgtg aagttgaagg tattctctcg aatgaagata tcatcgatgt attacgtact 1320
   ttagttgaaa ttcgtaacgg taagggtgag gtcgatgata ttgaccattt gggtaaccgt 1380
   cgtgtccgtt ctgtaggtga aatgactgag aaccagttcc gtgtaggtct ggtccgtgtt 1440
   gaacgtgcag ttaaagagcg tctgtcacag gcagaaactg acaacctgtc tccacaggac 1500
   ctgatcaatg ctaaaccggt agctgctgca atcaaagaat tctttggttc aagccagttg 1560
   tcccagttta tggatcagaa taacccgcta tctgaaatta cacataagcg tcgtgtatcg 1620
   gcacttggac caggcggttt gacccgtgag cgcgcaggct ttgaggtgcg tgacgtacac 1680
   cagacccact atggtcgtgt gtgtccgatt gaaacccctg aaggaccaaa catcggtctg 1740
   attaactcgc tgtctgtata cgcaaaaacc aatgaatttg gtttcttgga aacgccttac 1800
   cgtaaagtgg tagatggccg tgtaacagat gaagttgaat atctgtctgc aattgaagaa 1860
   gtaggaactg tgattgctca ggccgactct gcactcgata aagatggtta tttaaccgaa 1920
   gagctggttt ctgtacgtta ccagggcgaa tttgtgcgta tggctccaga gcgtattacg 1980
   catatggatg tttctgcaca gcaggtagtt tctgtagcgg catctctgat tccattcctt 2040
   gagcacgatg atgccaaccg tgcattgatg ggttcaaaca tgcagcgtca ggctgtaccg 2100
   acactgattg cggacaaacc gctagtaggg acaggtatgg aagcgaatgt tgcacgtgac 2160
   tcaggtgtat gtgtaattgc aaaacgtggc ggtaccattg aattcgtaga tgcgtcacgt 2220
   gtagttattc gtgttaacga agacgagatg attgcgggtg aagcgggtgt agatatctat 2280
   aacctgatca aatacacacg ttcaaaccag aatacctgta ttaaccagaa agttctggtg 2340
   aatctgggtg ataaagttgg tcgtggtgat gtactggctg atggcccgtc tacggatggt 2400
   ggtgaattag cacttggtca gaacatgcgt gttgcattca tgacttggaa tggttacaac 2460
   tacgaagact caatcctgct ttctgaacgt gtacttcagg aagaccgttt aacttctatt 2520
   cacattcagg aattgtcatg tgtagcacgt gataccaaac tgggtgcaga agaaattact 2580
   gcggatattc caaacgtagg tgaagctgcg ctttctaaac tggatgaatc cggtattgtt 2640
   tatatcggtg cagaagtaac ggctggcgat attctggttg gtaaagtgac acctaaaggt 2700
   gaaactcagc tcacacctga agaaaaactg cttcgcgcta tcttcggtga gaaagcagct 2760
   gatgtaaaag attcatcttt acgtgttcca tcaggtacca aaggtacggt aattgacgtc 2820
   caggtgttta cacgtgatgg tctggaaaaa gatgaccgtg cactggcgat tgagaaagca 2880
   caacttgacg cttatcgtaa agatttgaaa gaagaatata aaatctttga agaagcggct 2940
   cgtgaacgta ttgtacgttt gctgaaagat caggtgtcta acggcggtgg aaatactaaa 3000
   cgtggtgaga aactgtctga agaattgcta tctggccttg aactgattga tctgctcgaa 3060
   atccagccaa gcgatgaagc gattgctgaa cgtttaaccc agatccaggt gttcttgaaa 3120
   gagaaaagca ccgagattga cgagaagttt gccgagaaga aacgcaagct ttctacgggt 3180
   gatgagctga ctcatggcgt attgaaagtt gtgaaggttt atctagcagt taaacgtcgt 3240
   atccagccgg gtgataaaat ggcgggtcgt cacgggaaca agggtgtggt atcacaaatc 3300
   ctgcctgtag aagacatgcc acatgatgcc aatggtgttc cggttgatgt ggtattaaac 3360
   ccgctaggtg taccatcacg tatgaacgtg ggacagattc tggaaacaca tttgggtctt 3420
   gctgcaaaag gtttaggtga gcagatcgac aagatgctta aacagcagcg tgctattgtt 3480
   gaactgcgtg attttcttga taagatttac aataaagtcg gtggtgagca agaacagctt 3540
   gatacactga ctgatgatga aatcttgaaa cttgcaggaa acctcagcaa gggtgtgcca 3600
   ctggcaactc cagtatttga tggtgccgaa gaaggccaga tcaaagagtt acttgaactt 3660
   gcagaactgc cacgttctgg ccagcagatc ctgtttgatg gacgtacagg cgaacagttt 3720
   gaccgtccgg taactgtagg ttacatgtat atgcttaaac tcaaccactt ggtggatgac 3780
   aagatgcatg cgcgttctac cggttcttac tctcttgtaa cgcaacagcc gcttggtggt 3840
   aaagcacaat tcggtggtca gcgtttcggt gagatggaag tctgggcact tgaagcttac 3900
   ggtgcagcat atactcttca agagatgctg accgtgaagt cggatgacgt tgaaggccgt 3960
   acccgcatct ataagaatat tgtagatgga aaccattata tggatccggg catgcctgaa 4020
   tcgttcaacg tattgaccaa agagatccgt tctttaggta tcaacattga actgaaaaat 4080
   ggtgactaa 4089
<210> 21
   <211> 4089
   <212> DNA
   <213> Acinetobacter sp.
<400> 21
   atggcatact catataccga aaagaaacgg atccgtaaga attttggtaa attgccccac 60
   gtaatggaag caccgtactt actttcgatt caggtcgatt cgtaccgtac attcttacaa 120
   ggcggtaaaa ctccaaaaaa tcgcgaagat atcggtctcc aagccgcatt tcgttcagtt 180
   tttcctattg aaagttattc tggcaatgct gctttagaat ttgttgagta tagtcttggt 240
   aagcctgagt ttgacgtacg tgaatgtatt ttacgtggtt caacttatgc agcaccaatg 300
   cgcgtaaaaa ttcgtttgat cattaaagat cgtgaaacga aatcaattaa agacgttcgt 360
   gaacaagaag tttacatggg tgaaatgcca ctcatgaccg ataacggtac tttcgttatc 420
   aacggtacag agcgtgtaat cgtatctcaa ttacaccgtt caccaggcgt attctttgac 480
   catgataagg gtaaaaccca ctcaagcggt aaagtgttgt attcagcacg tattattcct 540
   taccgtggtt catggttaga ctttgaattt gatgctaaag atttagtctt tgtacgtatt 600
   gaccgtcgtc gtaaattatt ggcaacggtg gttttacgtg ccttaaatta cagcaatgaa 660
   cagatcttga atttgttcta cgaaaaagtg cctgtatatc ttgatatggg tagctatcag 720
   attgaccttg ttccagatcg cttacgtggt gaaatggcgc aatttgatat cttggacaat 780
   gatggtaaag cgatcgttga acaaggtaag cgtattaatg cacgtcacgt acgtcaaatg 840
   gaagctgcta acttagcgaa gctttctgta cctgatgaat atttatatga gcgtattaca 900
   gctgaagaca ttccattgaa aaatggcgat gtgattgctg cgaatacagt gcttagccat 960
   gaaatcttgg tgaaattggc tgaaggtggt gttaaacaat ttaacatcct gttcaccaat 1020
   gacatcgacc gtggttcttt cgttgcagat acattacgtg cagatacgac aacaggccgt 1080
   gaagaagcgc ttgttgaaat ctataaagta atgcgtccag gcgagccacc aacaaaagaa 1140
   gctgctgaaa acttattcaa caacttgttc ttctcttctg agcgttatga cctttctcca 1200
   gtgggtcgta tgaagttcaa ccgtcgtttg ggtcgtcctt acgaagtggg tacagatcag 1260
   aagtcgcgtg aagttgaagg tattttatcg cacgaagata ttattgatgt actgcgtaca 1320
   ttggttgaaa tccgtaacgg taaaggtgaa gtcgatgata tcgatcactt gggtaaccgt 1380
   cgtgtacgtt ctgttggtga aatgacagaa aaccaattcc gtgttggttt agtccgtgtt 1440
   gaacgtgctg ttaaagagcg tttaagccaa gcagaaacag ataacttgtc tccgcaagat 1500
   ttgatcaatg caaaaccagt tgctgctgca atcaaagaat tctttggttc aagccagttg 1560
   tctcagttca tggatcaaaa caacccattg tctgagatta cacataaacg tcgtgtatct 1620
   gcgcttggtc ctggtggttt gacacgtgaa cgtgcgggct ttgaagtacg tgacgtacat 1680
   caaactcact atggtcgtgt ttgtccaatt gaaacgcctg aaggtccaaa cattggtttg 1740
   atcaactcgc tttctgttta tgcaaaagca aacgatttcg gtttcttgga aacaccttac 1800
   cgtaaagttg ttgatggccg tgtaactgat gatgttgaat atttatctgc aattgaagaa 1860
   gtagggactg tcattgcaca ggccgattct gctgttgata aagatggtca cctgactgaa 1920
   gaaatggttt ctgtacgtca tcaaggtgaa ttcgtacgta tgtcgcctga gcgcgtaaca 1980
   catatggacg tttctgcaca gcaggttgtt tctgttgcag catcattgat tccattcctt 2040
   gaacacgatg atgcgaaccg tgcattgatg ggttcgaaca tgcaacgtca ggctgttcct 2100
   accttacgtg ctgacaagcc gcttgttggt acgggtatgg aagcaaacgt agcacgtgac 2160
   tctggcgtgt gtgtgattgc tgaccgtggt ggtgcgattg aatatgtaga tgcatctcgt 2220
   atcgtgattc gtgtaaacga agatgaaatg atcgcgggtg aagcgggtgt agatatctac 2280
   aacctgatca aatacacacg ttcaaaccaa aatacatgta tcaaccaaaa cgttatcgta 2340
   aacttgggtg acaaagttgc tcgtggcgat atcttggctg atggtccatc gactgatatg 2400
   ggtgaacttg cgcttggtca aaacatgcgc gtcgcgttca tgacatggaa cggttataac 2460
   tacgaagact cgatcttact ttctgagcgt gtacttcaag aagaccgttt aacctcgatt 2520
   cacattcaag aattgtcatg tgtagcgcgt gatactaagt taggtgcaga agaaattact 2580
   gccgatattc ctaacgttgg tgaggctgca ctgtctaagt tggatgagtc tggtattgtt 2640
   tatatcggtg ctgaagtaac tgctggtgac atccttgttg gtaaggtaac gcctaaaggt 2700
   gaaactcagt taacacctga agaaaaacta cttcgcgcaa tctttggtga aaaagctgct 2760
   gatgttaaag actcttcttt acgtgttcca tctggcacta aaggtactgt gattgacgtt 2820
   caagtcttca cacgtgatgg tttggaaaaa gatgaacgtg ctcaagcaat tgagaaagct 2880
   cagcttgatg cataccgtaa agacttgaaa gaagagtaca aaatcttcga agaagcagca 2940
   cgtgaacgta ttgttcgttt gttgaaaggt caagagtcta acggtggcgg ttcaactaaa 3000
   cgcggtgata aacttgctga agacgtattg tctggtttag agcttgttga tttacttgaa 3060
   atccaaccga ctgatgaggc aattgcagag cgtctaactc aaattcaagt gttcttgaaa 3120
   gagaagagct atgaaattga tgagaagttt gcagagaaga agcgtaaact ttctacaggt 3180
   gatgaattaa ccactggcgt attgaaagtt gttaaggttt accttgcggt taaacgtcgt 3240
   atccagcctg gtgataaaat ggcgggtcgt cacggtaaca aaggtgttgt gtcaaacatc 3300
   ttgcctgttg aagacatgcc acacgatgcg aatggtgtac cagtcgatat cgtattgaac 3360
   ccattgggtg taccgtcacg tatgaacgtg ggtcagattc ttgagactca cttaggtatg 3420
   gcggccaaag gtcttggcga taaacttgaa aaaatgttga aagaacaacg tacagtgtta 3480
   gaactacgtg acttcttaga caagatttat aacaaggtcg gtggtgagca agaagagctt 3540
   gatagcttaa ctgatgccga aatcttggcg ctttcaggta acttacgtgc tggtgttcca 3600
   ttagcaacac ctgtatttga tggtgctgaa gaaagccaga tcaaagactt acttgaatta 3660
   gcagacatct cacgtacagg tcaaacggta ttgtttgacg gacgtacagg tgagcagttt 3720
   gatcgtcctg taactgtagg ttacatgtac atgctcaaat tgaaccactt ggttgatgac 3780
   aagatgcatg cgcgttcaac aggttcttac tcacttgtta cacaacagcc gcttggtggt 3840
   aaagcacaat tcggtggtca gcgtttcggt gagatggaag tatgggcact tgaagcatac 3900
   ggtgcagcat atacactcca agaaatgctc actgtgaagt cggatgacgt cgaaggtcgt 3960
   acacgcatct ataagaatat tgtagatggt aaccattata tggatccggg tatgcctgaa 4020
   tcgttcaacg tattgaccaa agagatccgt tctttaggta tcaacattga actgaaaaat 4080
   ggtgactaa 4089
<210> 22
   <211> 4089
   <212> DNA
   <213> Acinetobacter sp.
<400> 22
   atggcatact catataccga aaagaaacgg atccgtaaga attttggtaa attgccccac 60
   gtaatggaag caccgtactt actttcgatt caggtcgatt cgtaccgtac attcttacaa 120
   ggcggtaaaa caccaaaaaa tcgcgaagat atcggtctcc aagccgcatt tcgttcagtt 180
   tttcctattg aaagttattc tggcaatgct gctttagaat ttgttgagta tagccttggt 240
   aagcctgagt ttgacgtacg tgaatgtatt ttacgtggtt caacttatgc ggcaccaatg 300
   cgcgtaaaaa ttcgtttgat catcaaagat cgtgaaacga aatcaattaa agacgttcgt 360
   gaacaagaag tttacatggg tgaaatgcca ctcatgaccg ataacggtac attcgttatt 420
   aacggtactg agcgtgtaat cgtatctcaa ttacaccgtt caccaggcgt attctttgac 480
   catgacaagg gtaaaaccca ctcaagcggt aaagtgttgt attcagcacg tattattcct 540
   taccgtggtt catggttaga ctttgaattt gatgccaaag atttagtctt tgtacgtatt 600
   gaccgtcgtc gtaagttgct tgcaacggtg attttacgtg ccttgaatta tagcaatgaa 660
   caaatcttga atttgttcta tgaaaaagta cctgtatatc ttgatatggg tagctatcaa 720
   attgacctgg ttccagatcg cttacgtggc gagatggcgc aatttgatat cttggataac 780
   gatggcaaaa caatcgttga acaaggtaaa cgtattaatg cacgtcacgt acgtcaaatg 840
   gaagcggcta acttagccaa gctttctgta cctgatgagt acttgtatga gcgtattacg 900
   gctgaagaca ttaccttgaa aagtggtgat gtgattcctg ccaataccgt acttagccat 960
   gaaattatgg tgaagttggc tgaaggtggt gtgaagcagt ttaacatcct gttcaccaat 1020
   gacatcgatc gtggttcttt cgttgcagat accttgcgcg cagatacaac gacaggccgc 1080
   gaagaagcgc ttgtcgaaat ctataaagtg atgcgtccag gtgagccacc aacgaaagaa   1140
   gctgctgaaa acttattcaa taacttgttc ttctcttctg agcgttatga cctgtctcca   1200
   gtcggtcgta tgaagttcaa ccgtcgtttg ggtcgtcctt acgaagtggg tactgatcag   1260
   aagtcacgtg aagttgaagg tattttatcg cacgacgata tcatcgatgt attgcgtaca   1320
   ttggttgaga ttcgtaacgg taaaggtgaa gtcgatgata tcgatcactt gggtaaccgt   1380
   cgtgtacgtt ctgtaggcga aatgacagaa aaccaattcc gtgttggttt agtccgtgtt   1440
   gaacgtgctg ttaaagaacg tttaagccaa gcagaaacag ataacttgtc tccacaagat   1500
   ttgatcaatg caaaaccagt tgctgctgca atcaaagagt tctttggctc gagccagttg   1560
   tctcagttca tggaccaaaa caacccattg tctgagatta cacacaaacg tcgcgtatct   1620
   gcgcttggtc ctggtggttt aacacgtgaa cgcgcgggct tcgaagtacg tgacgtacat   1680
   caaactcact atggtcgtgt ttgtccaatt gaaacacctg aaggtccaaa cattggtttg   1740
   atcaattcgc tttctgtcta tgctaaagcg aatgacttcg gtttcttgga aacaccttac   1800
   cgtaaagttg ttgatggccg tgtgactgat gaagttgaat acttatctgc aattgaagaa   1860
   gtaggtactg tcattgcaca ggccgattct gcagttgata aagatggtat gttgactgaa   1920
   gagatggttt ctgtacgtca tcaaggtgac ttcgttcgta tgtcgcctga gcgcgtaacc   1980
   catatggacg tttctgcgca acaggtcgta tctgtcgcag cgtcattgat tccattcctt   2040
   gaacacgatg atgcgaaccg tgcattgatg ggttcaaaca tgcaacgtca ggctgttcct   2100
   accttacgtg ctgacaagcc gcttgttggt acaggtatgg aagcaaacgt agcacgcgac   2160
   tctggtgtgt gtgtgatcgc aaaccgtggt ggtgcgattg aatatgttga tgcatctcgt   2220
   atcgttattc gtgtcaacga agatgaaatg attgcgggtg aagcaggtgt agatatctat   2280
   aacctgatca aatatacacg ttcaaaccag aatacatgta ttaaccagaa tgtcatcgtg   2340
   aacttgggcg acaaagttgc tcgtggtgat atcttggctg acggtccatc gacagatatg   2400
   ggtgaacttg cgctgggtca aaacatgcgc gtcgcgttca tgacctggaa tggttataac   2460
   tatgaagact cgatcttact ttctgagcgt gtacttcaag aagaccgttt aacctcgatt   2520
   cacattcagg aattgtcatg tgtagcacgt gataccaagt taggcgcaga agaaattact   2580
   gccgatattc ctaacgtcgg tgaagctgca ctgtctaaac tggatgagtc tggtattgtt   2640
   tacatcggtg ctgaagtaac tgcgggtgac atccttgttg gtaaggtaac gcctaaaggt   2700
   gaaactcagt tgacacctga agaaaaatta ctgcgcgcaa tctttggtga aaaagcggct   2760
   gacgtaaaag attcatcttt acgtgttccg tctggtacta aaggtacggt tatcgacgtt   2820
   caagtcttca cacgtgatgg cttggagaaa gatgaacgtg cacaagcaat tgaaaaagct   2880
   cagcttgatg cataccgtaa agacttgaaa gaagaataca aaatcttcga agaagcagca   2940
   cgtgaacgta ttgttcgttt gttgaaaggt caagaatcta acggtggcgg ttcaaccaaa   3000
   cgcggcgata aactttcaga agatgtattg tctggcttag agcttgttga tttacttgaa   3060
   atccagccaa atgatgaagc aattgctgaa cgtttaactc agattcaagt gttcttgaaa   3120
   gagaagagct acgagattga cgagaagttt gctgagaaga agcgtaaact ttctacaggt 3180
   gatgaattaa caacgggcgt attgaaagtt gttaaggttt accttgcggt gaaacgtcgt 3240
   atccagcctg gtgataagat ggcgggtcgt cacggtaaca agggtgttgt atcaaacatc 3300
   ttaccggttg aagacatgcc acatgatgcg aatggcgtac cagtcgacat cgtattgaac 3360
   ccactgggtg taccgtcacg tatgaacgtg ggtcagattc ttgagactca cttgggtatg 3420
   gcagcgaaag gtcttggcga taaaatcgaa aaaatgttga aagaacaacg tacagtgatt 3480
   gaactgcgtg aattcttaga caagatttat aacaaggttg gcggtgagca ggaagagctt 3540
   gatagcttga ctgatgcaga aatcttggcg ctttcaggta acttacgtgc tggtgttcca 3600
   ttggcaacac ctgtatttga tggtgctgaa gaaagccaga tcaaagacct acttgaactt 3660
   gctgatatct cacgtactgg tcaaacggta ttgtttgacg gacgtacagg tgaacagttt 3720
   gaccgtcctg taactgtagg ttacatgtac atgctcaaat tgaaccactt ggttgatgac 3780
   aagatgcatg cgcgttcaac gggttcttac tcacttgtga ctcaacagcc gcttggtggt 3840
   aaagcacaat tcggtggtca gcgtttcggt gagatggaag tatgggcact tgaagcatac 3900
   ggtgctgcat atacgctcca agaaatgctt actgtgaagt cggatgacgt cgaaggccgt 3960
   acacgcatct acaagaacat tgtagatggt aaccattata tggatccggg tatgcctgaa 4020
   tcgttcaacg tattgaccaa agagatccgt tctttaggta tcaacattga actgaaaaat 4080
   ggtgactaa 4089
<210> 23
   <211> 4089
   <212> DNA
   <213> acinetobacter schindleri
<400> 23
   atggcatact catataccga aaagaaacgg atccgtaaga attttggtaa attgcctagc 60
   gttatggatg ctccgtactt gctttcgatt caagtcgact cgtacagaac attcttacaa 120
   gatggcaaat caccaaaaaa ccgcgaagat atcggtctcc aagccgcatt tcgttcagtt 180
   tttcctattg aaagttattc tggcaatgct gctttagaat ttgttgagta tagtcttggt 240
   aagcctgagt ttgatgtacg cgaatgtatt cttcgtggct caacttatgc agcaccaatg 300
   cgtgtgaaga ttcgtttgat cctgaaagat cgtgaaacga aatcgatcaa agacgtacgt 360
   gaacaagaag tctatatggg cgaaatgcca ttgatgacgg ataacggtac tttcgtaatt 420
   aacggtaccg agcgtgtaat cgtatctcaa ttacaccgtt caccaggcgt attctttgac 480
   cacgataaag gtaaaactca ctcaagtggt aaagtccttt attctgcgcg tatcattcct 540
   taccgtggtt catggttaga ctttgaattc gatgccaaag acctagtcta tgtacgtatt 600
   gaccgtcgtc gtaaattgct tgcgactgtg gtacttcgtg cattgggtta taacaacgaa 660
   caaattctga acatgttcta tgagaaagtg cctgtatatc ttgatatggg tagctatcag 720
   attgaccttg ttccagaacg cctgcgtggt gaaatggcac aatttgatat tgctgacaaa 780
   gacggcaaga tcattgttga gcaaggtaaa cgtattaacg cacgtcacgt acgtcaaatg 840
   gaagcttcag gtcttgaaaa acttgcagtg cctgatgaat acctgtacga acgtatcact 900
   gctgaagaca tccagttaaa agatggcgat gtgatcgcgg ccaatactgt attaagccat 960
   gaaatcatgg tgaagattgc agaaggcggc gtgaagcaat tcaacttact gttcacaaat 1020
   gatatcgacc gtggttcatt catcgcggat acattacgtg ccgatacgac gacaggtcgt 1080
   gaagaagcac tagtagaaat ctacaaagta atgcgtccag gtgagccacc aacaaaagaa 1140
   gctgctgaaa acctgttcaa caacctgttc ttctcttctg agcgttatga cttgtcgcca 1200
   gtcggccgta tgaagttcaa ccgtcgtttg ggtcgtcctt acgaagtggg tacagaccag 1260
   aagtcacgtg aagttgaagg tatcctctcg aacgacgata tcactgatgt attgaaaaca 1320
   ttagttgaaa ttcgtaacgg taagggtgaa gtcgatgata tcgaccactt gggtaaccgt 1380
   cgtgttcgtt cagttggtga aatgactgaa aaccaattcc gtgtcggtct ggttcgtgta 1440
   gagcgtgcgg ttaaagaacg tctatcacag gctgaaactg ataacctgtc tccgcaagat 1500
   ttaatcaatg cgaagccagt ggctgctgca atcaaggaat tctttggttc aagccagtta 1560
   tctcagttca tggaccaaaa caacccattg tctgaaatca cccacaagcg tcgtgtatca 1620
   gcgcttgggc ctggtggttt gacgcgtgaa cgtgctggct tcgaagtacg tgacgtacat 1680
   caaactcact acggtcgtgt ttgtccaatt gaaacgcctg aaggtccaaa cattggtttg 1740
   atcaactcgc tttctgtata tgcaaaatgt aacaactttg gtttcttaga aacgccttac 1800
   cgtaaagttg ttgatggtcg tgtaacggat gaagttgaat acctgtctgc aatcgaagaa 1860
   gtaggtactg ttatcgcaca ggccgactct gcaatcgata aagatggcaa cctgactgaa 1920
   gaattcgtat ctgtacgtca ccaaggtgaa ttcgtacgta ttccaccaga aaaagtgacg 1980
   catatggatg tatctgctca gcaggtggta tctgtcgctg catcactgat tccgttcctt 2040
   gaacacgatg atgcgaaccg tgcattgatg ggttcaaaca tgcaacgtca ggcagttcct 2100
   acgttaatcg ctgacaagcc actggttggt accggtatgg aagcgaacgt agcacatgac 2160
   tctggtgtat gtgtgatcgc gaaacgtggt ggccgtattg aatatgtaga cgcttctcgt 2220
   gtcgtgattc gtgtcaacga agacgaaatg gtggcgggcg aagcaggtgt agatatctac 2280
   aacctgatca aatacacacg ttctaaccag aacacttgta tcaaccagaa agttcttgtg 2340
   aacatgggcg ataaagtggg tcgcggtgac gttcttgctg atggtccatc gactgatggc 2400
   ggtgaactgg cactgggtca aaacatgcgc gtagcattca tgacctggaa cggttacaac 2460
   tatgaagact cgatcttgtt atctgagcgt gtacttcagg aagaccgttt aacgtctatc 2520
   cacatccagg aactttcatg tgttgcacgt gatactaaac tgggtgctga agaaattact 2580
   gccgatatcc cgaacgtggg tgaagctgca ctttctaaac tggatgagtc tggtatcgtt 2640
   tatatcggtg ctgaagtgac tgctggtgac atcctggtag gtaaagtaac acctaaaggt 2700
   gaaactcagt taacacctga agaaaaactg cttcgcgcaa ttttcggtga aaaagcagct 2760
   gacgtaaagg actcttcttt acgtgttcca tcgggtacta aaggtaccgt gattgacgtt 2820
   caagtgttta cacgtgatgg tcttgaaaaa gacgaacgtg ctcaagcaat tgaaaaagct 2880
   cagcttgatg cttaccgtaa agacttgaaa gaagaataca aaatcttcga agaagcagca 2940
   cgtgaacgta ttattcgtct gttgaaaggt caggaatcta acggtggtgg cacaactaaa 3000
   cgcggtgaca aactgtctga agatgttctg tctggtttag agcttgttga tctgttagac 3060
   atccaaccag tagatgaagc aattgctgag cgtttaactc aaattcaagt gttcttgaaa 3120
   gagaagagcc ttgaaattga tgaaaagttt gctgagaaga aacgcaaatt atctacaggc 3180
   gatgaactta caactggcgt actgaaagtt gttaaggttt atctagcggt taaacgtcgt 3240
   atccagcctg gtgataagat ggcgggtcgt cacggtaaca agggtgttgt atctaacatc 3300
   ttgccggtag aagacatgcc acacgatgcc aacggtgtac ctgttgatat cgttcttaac 3360
   ccgctaggtg taccgtcacg tatgaacgtg ggtcagattc ttgagactca cttgggtatg 3420
   gccgccaaag gtcttggcga caaaatcgac aagatgcttc aagagcaacg tacggtgctt 3480
   gagcttcgtg aattcttaga caagatttac aacaaagttg gtggtgagca agaagatctt 3540
   gatagcctga ctgatgatga aatcttggca ttgtctggta acttgcgtaa aggtgttcct 3600
   ttggcaactc cagtattcga cggtgctgaa gaatcgcaaa tcaaagaatt gttagagctt 3660
   ggtggcattt cacgtactgg tcaaacagta ttgtatgatg gccgtactgg tgaacgtttc 3720
   gaccgtccgg taactgtagg ttacatgtac atgctgaaac tgaaccactt ggttgacgac 3780
   aagatgcatg cgcgttctac tggttcttac tctctagtaa cgcaacagcc gcttggtggt 3840
   aaagcacaat tcggtggtca gcgtttcggt gagatggaag tatgggcact ggaagcatac 3900
   ggtgcagcat atacactcca ggaaatgctt actgtgaaat cggatgacgt tgaaggtcgt 3960
   acccgtatct ataagaatat tgtagatggt aaccattaca tggacccagg tatgcctgaa 4020
   tcgttcaacg tattgaccaa agagatccgt tctttaggta tcaacattga actgaaaaat 4080
   ggtgactaa 4089
<210> 24
   <211> 4089
   <212> DNA
   <213> acinetobacter ursingii
<400> 24
   atggcatact catataccga aaagaaacgg atccgtaaga attttggtaa attgcctagc 60
   gtcatggacg ctccgtactt gctttcgatt caggtcgatt cgtacagaac gtttttacaa 120
   gatggcaaaa caccgaaaaa ccgcgaagat atcggtctcc aagccgcatt tcgttcagtt 180
   tttcctatag aaagttattc tggcaatgct gctttagaat ttgttgagta tagtcttggt 240
   aaaccagagt ttgatgtacg cgagtgtatt cttcgcggat caacttatgc ggcaccaatg 300
   cgcgtaaaaa ttcgtttgat cattaaagat cgtgaaacga aatcaattaa agatgttcgc 360
   gaacaagaag tgtatatggg tgaaatgcca ctcatgactg agaacggaac atttgtgatt 420
   aacggtactg agcgtgtcat cgtatctcag ttacaccgtt caccaggtgt gttcttcgat 480
   catgataaag gtaaaactca ttcaagcggt aaagtgctgt attcagcacg tattattcct 540
   taccgtggtt catggttaga ctttgaattt gatgccaaag atttggttta cgtgcgtatt 600
   gaccgtcgtc gtaagctatt ggctacggtt attctacgtg cactgggtta taacaatgag 660
   caaatccttg atttgttcta tgaaaaagta cctgtgtatc tggacatggg cagctaccaa 720
   attgatcttg tgcctgaacg tttacgcggt gaaatggcgc agtttgatat tactgataat 780
   gacggtaaag tcattgtaga acaaggcaaa cgtattaatg cgcgtcatgt tcgtcaaatg 840
   gaagcgtctg gtctaagcaa gctttcagtg cctgatgagt atctgtatga gcgtatcact 900
   gctgaagata ttacactaaa agatggtgat atcattcctg ccaacacttt gctcagccat 960
   gaagtcatgg tcaagctagc tgaaggcggc gtaaagcagt ttaatatcct gtataccaac 1020
   gatatcgatc atggtccgtt tattgcggat actttacgtg ctgataccac atcaggtcgt 1080
   gaagaagcac tggttgaaat ctacaaggta atgcgtccag gtgagccacc aacgaaagaa 1140
   gcggctgaaa acttgtttaa taacttgttc ttctcttctg aacgttatga tttgtcacct 1200
   gttggtcgta tgaagtttaa ccgtcgtttg ggtcgtcctt acgaagttgg tacagatcaa 1260
   aaatcacgtg aagttgaagg tatcctctct aatgaggata tcactgatgt attacgtaca 1320
   ttggttgaga ttcgtaacgg taaaggtgaa gtcgacgata tcgatcactt gggtaaccgt 1380
   cgtgtccgtt cagttggtga aatgacagag aaccagttcc gtgtgggtct ggtgcgtgtt 1440
   gaacgtgctg ttaaagagcg tttaagccaa gctgaaaccg ataacttgtc tccacaagac 1500
   ttgatcaatg cgaaacctgt tgctgccgcg atcaaagaat tctttggttc aagccaattg 1560
   tctcagttca tggatcaaaa caacccattg tctgaaatta cacacaaacg tcgtgtatca 1620
   gcgcttgggc caggtggttt gacacgtgaa cgtgcaggct ttgaggtacg tgacgtacat 1680
   caaactcact atggtcgtgt gtgtccgatt gaaacgcctg aaggtccaaa cattggtttg 1740
   atcaactcgc tatctgttta tgcaaaagcc aacgatttcg gtttcttgga aacgccttat 1800
   cgtaaggtag tcgatggtcg tgtaacggac gatgttgaat atctttctgc gattgaagaa 1860
   gtggggactg tgattgcaca ggccgactct ggtgttgatg cagagggtaa cctcgttgaa 1920
   gaaatggttt cagtccgtca tcagggcgaa tttgtacgta tgcctcctga aaaagtgacg 1980
   catatggatg tttctgcgca gcaagttgtt tctgtagcag catcattgat tccattccta 2040
   gaacacgatg atgcgaaccg tgccttaatg ggttcgaaca tgcaacgtca ggcagtgcca 2100
   accttacttg cggataaacc gcttgtgggt acgggtatgg aagcgaccgt tgcacgtgac 2160
   tcaggcgtat gtgtaattgc aaaacgtggt ggtgtgattg agtttgttga tgcatcacgt 2220
   gtggtcattc gtgttcatga aaatgaaatg atcgcgggtg aagcaggtgt tgatatttat 2280
   aacctgatca aatacacccg ttctaaccaa aatacttgta ttaaccagaa agttcttgtg 2340
   aatctgggcg ataaagttgg tcgtggtgat gtattggctg atggtccttc taccgatggt 2400
   ggtgagttgg cattgggtca aaacatgcgc gtcgcgttca tgacgtggaa tggttataac 2460
   tacgaagact caatcttgtt atctgagcga gttttgcagg aagatcgttt aacttcaatt 2520
   cacattcagg aattatcatg tgttgcacgt gatactaaat tgggtgcaga agaaattact 2580
   gccgatattc cgaatgtagg tgaagctgct ctatccaagc tagatgaatc tggtattgtt 2640
   tatatcggtg cggaagtgac tgcgggtgat attctggtag gtaaagttac gcctaaaggt 2700
   gaaactcaac ttacgccaga agaaaaattg cttcgtgcga tcttcggtga aaaagcggct 2760
   gatgtgaaag attcatcttt acgtgttccg tctggtacga aaggtactgt catcgatgta 2820
   caagtcttca cacgtgatgg tcttgagaaa gatgatcgtg cacttgcgat tgaaaaagcg 2880
   caattggatt cgtaccgtaa agatttgaaa gaagaataca aaatctttga agaagcagca 2940
   cgtgaacgta ttgtgcgctt attaaaaggt caggattcta acggtggtgg cacgactaaa 3000
   cgtggtgaca aactgactga agatttattg tctggtcttg agctggtcga tttacttgaa 3060
   attcaaccaa gtgatgaagg catcgctgag cgtttaagtc aaattcaagt attcttgaaa 3120
   gagaagagtg ctgaaattga tgagaaattt gctgagaaaa aacgcaaatt agcgacgggt 3180
   gatgagttaa cgacgggtgt cttgaaagtc gttaaagtct accttgcagt taaacgtcgt 3240
   atccagccag gtgataaaat ggcgggtcgt cacgggaaca aaggtgttgt ttctaacatc 3300
   ttgcctgtag aagacatgcc acatgatgcc aatggtgtac ctgtcgatat cgtcttgaac 3360
   ccattaggtg taccatcgcg tatgaacgtg ggtcagattc tggagacaca tctaggattg 3420
   gcagccaaag gtctgggtga acaaatcgat aagatgttgc aacaacagcg taccattgcc 3480
   gaacttcgta tcttccttga taagatttac aacaaggtcg gtggtgagca agaagatcta 3540
   aacagtctga ctgatgatga agtcttggta ttggctggca acttgcgtaa aggtgtacca 3600
   ctagcaactc ctgtatttga tggtgctgaa gaaagtcaaa ttaaagagtt acttgagttg 3660
   gctgaattgc cacgtactgg tcaacagatt ttgtttgatg gacgtacagg tgaacagttt 3720
   gaccgtcctg taaccgtagg ttatatgtac atgttgaaac tgaaccactt ggtggatgac 3780
   aagatgcatg cgcgttcaac aggttcatat tctctggtaa cgcaacaacc attgggtggt 3840
   aaagctcaat tcggtggtca gcgtttcggt gagatggaag tctgggcact ggaagcttat   3900
   ggcgcagcat atacccttca ggaaatgctc actgtgaagt cggatgacgt tgaaggtcgt   3960
   acacgcatct ataagaacat tgtagatggt aaccattata tggatccggg tatgcctgaa 4020
   tcattcaacg tattgaccaa agagatccgt tctttaggta tcaacattga actgaaaaat 4080
   ggtgactaa 4089
<210> 25
   <211> 4089
   <212> DNA
   <213> acinetobacter baylyi
<400> 25
   atggcatact catataccga aaagaaacgg atccgtaaga attttggtaa attgccccaa     60
   gtcatgcatg ctccgtacct gctctcgatt caagtcgact cgtacagaac attcttgcaa    120
   gacggcaaaa caccaaaaaa tcgcgaagat atcggtctcc aagctgcatt tcgttcagtt    180
   tttcctattg aaagttattc gggcaatgct gctttagaat tcgttgagta tagtcttggt    240
   aaaccagagt ttgatgttcg cgaatgtatt cttcgtggct caacctttgc ggcaccaatg    300
   cgcgttaaaa ttcgtttgat catcaaagat cgtgaaacga aatctattaa agacgtacgt    360
   gaacaagaag tgtacatggg tgaaatgcca ctcatgactg agaatggtac ctttgtgatc    420
   aatggtactg agcgtgtaat cgtatctcaa ttacaccgtt caccaggcgt attctttgac    480
   catgataaag gtaaaacgca ttcaagcggt aaagtgcttt attcagcacg tatcattcct    540
   taccgtggtt catggttaga ttttgagttt gatgctaaag atttagtctt tgtacgtatt    600
   gaccgtcgtc gtaaattgct tgcgactgtt gtgttacgtg cattgagcta tagcaatgaa    660
   caaattctga atatgttcta cgaaaaagta cctgtatatc ttgatatggg tagctatcag    720
   attgaccttg tgcctgaacg tcttcgtggt gaaatggctc aatttgatat cgtggacaat    780
   gatggtaaag ccattgttga acaaggtaaa cgtattaatg ctcgccatgt acgtcaaatg    840
   gaagctgctg gtttaactaa acttccagtt ccagatgaat atttgtatga gcgtattact    900
   gctgaagata tcgtacttaa agacggtgaa gtaattactg ctaacactgt attaagtcat    960
   gagattttgg tcagaattgc tgaaggtggt attaaacaat ttaatatcct gttcaccaat   1020
   gacatcgatc gtggttcttt tgttgctgac accttacgtg cagatacaac atctggtcgt   1080
   gaagaagcgc ttgtagaaat ctacaaagtg atgcgtccag gtgagccacc aacgaaagaa   1140
   gcggctgaaa acttattcaa taacttattc ttctctacag agcgctatga tttatcgcct   1200
   gtgggtcgta tgaagtttaa ccgtcgtttg ggtcgccctt acgaagtagg tacagatcag   1260
   aagtctcgtg aagtagaagg tattctttct aacgatgaca tcatcgatgt actgaaaaca   1320
   ctggtagaaa ttcgtaacgg taaaggtgaa gtcgatgata tcgatcactt gggtaaccgt   1380
   cgcgtacgtt ctgttggtga aatgacagaa aaccaattcc gtgttggttt agttcgtgtt   1440
   gaacgtgctg ttaaagagcg tttaaaccaa gctgaaacag ataacttgtc tccacaagat   1500
   ttgatcaatg cgaaaccagt tgctgctgca atcaaagaat tctttggttc aagccaattg   1560
   tcacagttta tggatcaaaa caacccattg tcagaaatta cacacaaacg tcgtgtatca   1620
   gcattgggac caggtggttt gacacgtgaa cgtgcaggct ttgaagtgcg tgacgtacat   1680
   caaactcact atggtcgtgt atgtccaatt gaaactcctg aaggaccaaa cattggtttg   1740
   atcaactcgc tttctgttta tgcaaaagcg aacaacttcg gtttcttgga aacaccatac   1800
   cgtcgcgttg ttgatggtcg tgtaacagat gatgttgaat atttatctgc aattgaagaa   1860
   gtaggtactg ttattgcaca ggccgattct gcattggata aagatggaca tttaacagaa 1920
   gacttcgttt cagtacgtca ccaaggtgac ttcgttcgta tgccacctga aaaagtgacg 1980
   catatggatg tatctgctca acaggttgta tctgtcgctg catcacttat tccattcctt 2040
   gaacacgatg atgccaaccg tgcattgatg ggttcaaaca tgcaacgtca ggctgttcct 2100
   acattgcttg ctgataaacc acttgtgggt accggcatgg aagcaaacgt agcgcacgac 2160
   tctggtgtat gtgtgatcgc gaaacgtggc ggacgcattg agtttgtaga tgcatcacgt 2220
   gtggttattc gtgtcaacga agatgaaatg atcgcgggtg aagcaggtgt agatatctac 2280
   aacttgatca aatacacgcg ttcaaaccaa aacacatgta ttaaccaaaa agtgcttgtg 2340
   agcatgggcg ataaagtcgg ccgtggtgac gttcttgctg atggtccatc aactgatggt 2400
   ggtgaattag cattgggtca gaacatgcgt gtcgcgttca tgacttggaa cggttataac 2460
   tacgaagact cgattttatt atctgaacgt gttcttcaag aagatcgttt aacgtcaatt 2520
   catattcaag aattatcatg tgttgcacgc gatacgaagt taggtgcgga agaaatcact 2580
   gccgatattc ctaacgtagg tgaagcagcg ttatctaaac ttgatgaatc aggtattgtt 2640
   tatatcggtg ctgaagttgc agcgggtgat attcttgttg gtaaagtgac acctaaaggt 2700
   gaaacacaat taacccctga agaaaaatta cttcgtgcaa tctttggtga gaaagcagca 2760
   gacgttaaag attcatcttt acgtgtttct tcaagcgtta aaggtacagt catcgacgtt 2820
   caagtgttta cacgtgacgg tatcgagaaa gatgaacgtg ctcaagcgat tgagaaagcg 2880
   caacttgatg cttatcgtaa agacttgaaa gaagaattca aaatcttcga agaagctgct 2940
   cgtgaacgta ttatccgttt gttaaaaggc caagagtcaa atggcggcgg tacgaccaag 3000
   cgcggtgata agctatctga agatgtattg tctggtttag agcttgttga tcttttagaa 3060
   gttcaaccaa cagacgaagg catcgctgaa cgcttaactc aaattcaagt gttcttgaaa 3120
   gagaagagct acgagattga tgagaaattt gctgagaaaa aacgcaaact ttctacaggt 3180
   gatgagctta caacaggtgt attgaaagtt gttaaagttt acttagctgt aaaacgtcgt 3240
   atccagcctg gtgataaaat ggcgggtcgt cacggtaaca aaggtgttgt atcaaacatc 3300
   ttgcctgttg aagacatgcc gcatgatatc catggtgttc cagttgatgt cgtacttaac 3360
   ccattgggtg taccatcacg tatgaacgtg ggtcagattc ttgaaactca cttaggtatg 3420
   gctgcaaaag gtcttggcga taagatcgac aagatgatga aagagcaacg taccgttctt 3480
   gagcttcgtg atttcttaga caagatttat aacaaagttg gtggcgagca agaagatctt 3540
   gatagcttaa ctgatgaaga aatcttggtg ttatcaggta acttgcgtaa aggtgttcct 3600
   ttagctacgc cagtatttga tggtgcagaa gaaagtcaga tcaaagagtt acttgagctt 3660
   ggtggtatct cacgtacagg tcaaacagta ttgtatgacg gacgtacagg tgagcgtttt 3720
   gaccgcccag taactgttgg ttatatgtac atgctcaagt tgaaccattt ggttgatgac 3780
   aagatgcatg cacgttctac tggttcttat tcacttgtaa ctcaacaacc gcttggtggt 3840
   aaagcacaat tcggtggtca gcgtttcggt gagatggaag tctgggcact agaagcttat 3900
   ggtgctgctt atacacttca agaaatgctt actgtgaagt cggatgacgt tgaaggtcgt 3960
   actcgcatct ataagaacat cgtagatggt aaccattata tggatccggg tatgcctgaa 4020
   tcgtttaacg tattgaccaa agagatccgt tctttaggta tcaacattga actgaaaaat 4080
   ggtgactaa 4089
<210> 26
   <211> 4089
   <212> DNA
   <213> acinetobacter bouvetii
<400> 26
   atggcatact catataccga aaagaaacgg atccgtaaga attttggtaa attgcccaag 60
   gtaatggatg ttccgtactt gctcgcgatt caagtcgact cgtacagaac tttcttgcaa 120
   gatggcaaaa ctccaaaaaa ccgcgaagat atcggtctcc aagccgcatt tcgttcagtt 180
   tttcctatag aaagttattc tggcaatgct gctttagaat ttgttgagta tagtcttggt 240
   aagcctgagt ttgatgtgcg tgagtgtatt ctccgcggct caacttatgc agcgccaatg 300
   cgtgtaaaaa ttcgtctgat cttaaaagac cgcgaaacta aatcaatcaa agacgtgcgc 360
   gagcaagaag tctacatggg cgaaatgccg ctcatgactg ataacggtac cttcgttatt 420
   aacggtactg agcgtgtcat cgtatctcaa ttgcaccgtt cgccgggcgt gttctttgac 480
   cacgataagg gcaaaaccca ttcaagcggt aaagtgcttt attcagcgcg gattattcct 540
   taccgcggtt catggttaga ctttgaattt gatgcgaaag acctggtctt tgtacgtatt 600
   gaccgccgcc gcaaattgct tgcgactgtg gtgcttcgtg ccttaggtta caacaactct 660
   caaattcttg atttgttcta tgaaaaagtg cctgtctacc tagacatggg cagctatcag 720
   attgatcttg tgcctgaacg cctgcgcggt gaaatggccc aatttgatat tgctgataaa 780
   gacggcaaag tcattgtaga gcaaggcaaa cgtatcaacg cgcgtcacgt gcgtcagatg 840
   gaagcagcag ggcttgagaa gctttctgtt ccagatgaat acttgtatga acgcattatt 900
   gctgaagacg tggctttacg cggcggcgat gtgattgcgg caaataccgt gcttagccat 960
   gaaattatgg tgaaattggc tgaaggcggt gtgaagcaat tcaacatcct gttcaccaac 1020
   gatattgacc gcggttcatt catcgcggac tctttgcgtg cagatacaac gtccagccgt 1080
   gaagaagcgc ttgtagaaat ctacaaagta atgcgtccag gcgagccgcc gacgaaagaa 1140
   gcggctgaaa acttattcaa caacctgttc ttctcttctg aacgttatga cctgtctcct 1200
   gtaggtcgca tgaagttcaa ccgccgtttg ggccgccctt acgaagtggg tacagatcag 1260
   aagtcacgtg aagttgaagg cattctttca aacgaagaca ttacagatgt tcttcaaaca 1320
   ctgattgaaa tccgcaacgg taaaggtgaa gtcgatgata tcgatcactt gggtaaccgc 1380
   cgtgtgcgtt ctgtcggtga aatgacagaa aaccaattcc gtgtaggcct agttcgtgta 1440
   gagcgtgctg ttaaagaacg tctatctcaa gctgaaactg acaacttgtc tccgcaagat 1500
   ttaatcaatg cgaagccagt tgctgctgca atcaaagaat tctttggttc aagccaattg 1560
   tctcagttca tggaccaaaa caacccattg tctgaaatta cacacaaacg tcgtgtatca 1620
   gcgcttgggc ctggtggttt gactcgtgaa cgcgcgggct tcgaagtacg tgacgtacat 1680
   caaactcact acggtcgtgt gtgcccaatt gagactcctg aaggtccaaa cattggtttg 1740
   atcaactcac tttcagttta cgctaaatgt aacaacttcg gtttcttgga aactccatac 1800
   cgtaaagttg ttgatggccg tgtaacggat gacgttgaat acttatctgc aattgaagaa 1860
   gtaggtactg ttattgcaca ggccgattct ggcgtcgata aagacggtaa cttgcaagaa 1920
   gagtttgttt ctgtacgtca tcaaggtgaa ttcgtacgta tgcctcctga aaaagtgacg 1980
   catatggacg tgtctgcaca acaggttgta tctgtagcag catcattgat tccgttcctt 2040
   gaacacgatg acgccaaccg tgcattgatg ggttcgaaca tgcagcgtca agctgtgcct 2100
   acgttaattg ctgacaagcc gcttgtaggt acaggcatgg aagcaaatgt agcgcatgac 2160
   tctggtgtgt gtgtaattgc gaaccgcggc ggccgcattg aatttgttga cgcatcacgt 2220
   gtcgtgatcc gtgtgaatga agacgaaatg gttgctggtg aagcaggcgt agatatctac 2280
   aacctgatca aatacacccg ttcgaaccaa aacacctgta tcaaccagaa agttcttgta 2340
   aaactgggtg ataaagttgg ccgcggtgac gtattggctg atggtccatc gacggatggc 2400
   ggtgagctgg cgctgggtca aaacatgcgc gtagcgttca tgacttggaa cggttacaac 2460
   tacgaagact cgatcttatt atctgagcgc gtacttcaag aagaccgttt gacgtctatt 2520
   cacatccaag aattatcatg cgtcgcgcgt gatactaaac tgggtgctga agaaatcact 2580
   gcggatattc caaacgtggg tgaagctgca ctgtctaagc ttgatgaatc aggtattgtt 2640
   tacatcggtg ctgaagtcac tgctggcgat atcttggttg gtaaagtgac gcctaaaggt 2700
   gaaactcagc tgactcctga agaaaaactg cttcgcgcaa tcttcggtga aaaagcggct 2760
   gacgtaaaag attcatcttt acgtgtatct tcatctgtga aaggtacggt tattgacgtt 2820
   caagtgttta cacgtgatgg tcttgaaaaa gacgaacgtg ctcaagcaat tgaaaaagcg 2880
   cagttagatg cataccgcaa agacttgaaa gaagaataca aaatcttcga agaagctgcg 2940
   cgtgaacgta ttgtgcgctt gctaaaaggt caagaatcga atggcggcgg cacaactaaa 3000
   cgcggtgaca aactttctga agaagtattg tctggtttag agcttgctga tctgcttgaa 3060
   attcagccta cagacgaagg cattgctgag cgcttaactc aaattcaagt gttcttgaaa 3120
   gagaagagca ctgaaattga tgagaaattt gctgagaaaa aacgcaaact ttctacaggc 3180
   gatgagctta caactggtgt attgaaagtt gttaaagttt acttagctgt aaaacgccgc 3240
   atccagccgg gtgataagat ggcgggtcgt cacggtaaca aaggtgttgt atctaacatc 3300
   ttgcctgtag aagacatgcc gcacgatgcc aacggtgttc ctgtagacgt ggtgcttaac 3360
   ccgctgggtg taccgtcacg tatgaacgtg ggtcagattc ttgagactca cttgggcatg 3420
   gcagcgaaag gtcttggcga caaaatcgac aagatgatga aagagcagcg cactgttctt 3480
   gaacttcgtg aattcttaga caagatttac aacaaagttg gcggcgagca agaagatctt 3540
   gacagcttaa ctgatgatga aatcttggcg ctttcaggca acctgcgtgc aggtgttcct 3600
   ttggcaacgc ctgtatttga cggtgctgaa gaatcacaaa ttaaagaatt gctagagctt 3660
   ggcggcattt cacgtactgg tcaaacagta ttgtatgatg gccgtactgg tgagcgtttc 3720
   gaccgtcctg taactgtagg ttacatgtac atgctgaaac tgaaccactt ggtagacgac 3780
   aaaatgcatg cgcgttctac tggttcttat tctcttgtta cgcagcagcc attgggcggt 3840
   aaagcgcagt tcggtggtca gcgtttcggt gagatggaag tctgggcact ggaagcatat 3900
   ggcgcagcgt acacgctcca agaaatgctt acagttaagt cggatgacgt tgaaggccgt 3960
   acccgcatct ataagaacat tgtagatggc aaccattata tggatccggg catgcctgaa 4020
   tcgttcaacg tattgaccaa agagatccgt tctttaggta tcaacattga actgaaaaat 4080
   ggtgactaa 4089
<210> 27
   <211> 4089
   <212> DNA
   <213> acinetobacter gerneri
<400> 27
   atggcatact catataccga aaagaaacgg atccgtaaga attttggtaa attgcccgaa 60
   gtaatggaag ctccgtactt gctcgcgatt caagtcgact cgtacagaac cttccttcaa 120
   gatggcaaaa caccaaaaaa ccgcgaagat atcggtctcc aagccgcatt tcgttcagtt 180
   tttccaattg aaagttattc tggcaatgct gctttagaat tcgttgagta tagccttggt 240
   aaaccagagt ttgatgttcg tgagtgtatt cttcgcggct cgacttttgc ggcaccaatg 300
   cgcgttaaaa ttcgtttgat catcaaagat cgtgaaacga aatcgatcaa agacgttcgt 360
   gaacaagaag tttacatggg tgaaatgcca ctcatgactg agaacggtac ctttgttatt 420
   aacggtactg aacgtgtaat cgtttctcaa ttacaccgtt ctccaggtgt gttctttgac 480
   cacgataaag gtaagactca ttcaagtggt aaagtgcttt attctgcacg tatcattcct 540
   taccgtggtt catggttgga ctttgagttt gatgcgaaag atttagttta tgttcgtatc 600
   gaccgtcgtc gtaaattatt agcaactgct attcttcgtg ctttagatta tacaaatgaa 660
   caaatcttag aaatgttcta tgagaaagtt cctgtatatc tagatatggg tagctaccaa 720
   attgaccttg tgcctgaacg tctgcgtggc gaaatggctc aattcgacat tactgatgct 780
   gatggcaaag tgattgttga acaaggtaaa cgtattaatg cgcgtcatgt tcgtcaaatg 840
   gaagcttctg gtttaactaa gctttctgtt cctgatgaat atttatatga gcgtattaca 900
   gctgaagata tcacactgaa agatggtgaa gtgattcaag caaacactgt gcttggtcat 960
   gacattatgg tgaaattggc tgaaggcggt attaaacaat ttaatatcct attcaccaac 1020
   gatatcgacc gtggttcatt tatcgcagat acattacgta cagatactac aacaggccgt 1080
   gaagaagcgc ttgttgaaat ctataaagta atgcgtccag gcgagccacc aacaaaagaa 1140
   gctgctgaaa acttattcaa caacttgttc ttctcttctg aacgttatga cttatctcct 1200
   gtaggtcgta tgaagttcaa ccgtcgtttg ggtcgtcctt acgaagtggg tactgatcag 1260
   aagtctcgtg aagttgaagg tattctttct aacgaagaca ttatcgacgt attaaaaact 1320
   ctcgttgaaa tccgtaacgg taaaggcgaa gtcgacgata tcgaccattt aggtaaccgt 1380
   cgtgtacgtt cagttggtga aatgactgaa aaccaattcc gtgttggttt agttcgtgtt 1440
   gaacgtgctg ttaaagaacg tttaagccaa gctgaaacag ataacttgtc tccacaagat 1500
   ttaatcaatg cgaaaccagt tgctgctgca atcaaagaat tctttggttc aagccaattg 1560
   tctcagttca tggaccaaaa caacccattg tctgaaatca cacacaaacg tcgtgtatca 1620
   gctctcggac ctggtggttt gactcgtgaa cgtgcgggct ttgaggtacg tgacgtacat 1680
   caaactcact atggtcgtgt atgtcctatt gaaacgcctg aaggtccaaa cattggtttg 1740
   atcaactcgc tttctgttta tgcaaaatgt aataacttcg gtttcttgga aactccatac 1800
   cgtaaagttg ttgatggtcg tgtaactgaa gatgttgagt atttatctgc tattgaagaa 1860
   gtaggtactg ttattgcaca ggccgattct agtgtagatg gcgataataa cttaactgaa 1920
   gaattcgttt ctgtacgtca tcaaggtgaa ttcgttcgta tgcctcctga aaaagtgacg 1980
   catatggatg tatcagctca acaggttgta tctgtagctg catcattgat tccgttcctt 2040
   gaacacgatg atgccaaccg tgcattgatg ggttcaaaca tgcaacgtca ggctgttcct 2100
   acattgcttg ctgacaaacc acttgttggt acaggtatgg aagcaaacgt agcgcgtgac 2160
   tcaggtgtat gtgtgatcgc gaaacgtggc ggtatgatcg aatttgttga cgcttcacgt 2220
   gttgtgattc gtgttaacga agatgaaatg attgctggtg aagctggtgt agatatctac 2280
   aacctcatca aatatacccg ttcgaaccaa aatacttgta tcaaccaaaa agttctcgtg 2340
   agcttgggtg ataaagtagg tcgcggtgat gtattggctg atggtccatc tacagacggt 2400
   ggtgaacttg ctcttggtca gaacatgcgc gtagcgttca tgacttggaa cggttataac 2460
   tacgaagact cgattttatt atctgaacgt gttcttcaag aagatcgttt gacttctatt 2520
   cacattcaag aattgtcatg tgtagctcgt gatactaagt taggtgcaga agaaatcact 2580
   gcagatattc ctaacgttgg tgaagctgcg ctgtctaaac ttgatgagtc aggtatcgtt 2640
   tatatcggtg ctgaagtaac tgctggtgac attcttgtag gtaaagtaac gcctaaaggt 2700
   gaaactcagt tgactcctga agaaaaactt cttcgtgcga tcttcggtga aaaagctgct 2760
   gacgttaaag attcatcttt acgcgttcca tctggtacta aaggtacagt gattgacgtt 2820
   caagtcttta ctcgtgatgg tattgaaaaa gatgaacgtg ctcaagcaat tgagaaagct 2880
   cagcttgatg cttaccgtaa agatttgaaa gaagaatata aaatctttga agaagctgct 2940
   cgtgaacgta ttgttcgctt gttgaaaggt caagaatcaa atggtggtgg ttcaactaaa 3000
   cgtggtgaca aactttctga agaattgtta tctggtttag agctagttga tcttcttgaa 3060
   attcaaccaa gtgatgaagg tattgctgaa cgtttaactc aaattcaagt gttcttgaaa 3120
   gaaaagagcc atgaaattga tgagaaattt gctgagaaaa aacgcaaact ttctacaggt 3180
   gatgagctta caactggtgt attgaaagtt gttaaagtgt atttggctgt taaacgtcgt 3240
   atccaaccgg gtgataaaat ggcgggtcgt cacgggaaca aaggtgttgt atcaaacatc 3300
   cttcctgtag aagatatgcc gcatgacatc aacggtgttc ctgttgacgt agtacttaac 3360
   ccactgggtg taccgtcacg tatgaacgtg ggtcagattc ttgaaacaca tttaggttta 3420
   gctgccaaag gtcttggtga gcaaatcgat aagatgctca aagagcaacg tacgattgct 3480
   gaacttcgtg tgttcttgga caagatttat aacaaagttg gtggcgagca agaagatctt 3540
   gatagcttaa ctgatgaaga aatccttgtt ctttcaggta atttacgtaa aggtgttcct 3600
   ttagcaactc cagtatttga tggtgctgaa gaaggtcaaa ttaaagagct tcttgaactt 3660
   gctgaacttc cacgttctgg tcaaacagta ttgtatgacg gacgtacagg tgagcgtttt 3720
   gaccgtcctg taaccgttgg ttatatgtac atgttgaaac ttaaccactt ggttgatgac 3780
   aagatgcatg cacgttctac tggttcttac tcattagtga cacaacaacc gcttggtggt 3840
   aaagcacaat tcggtggtca gcgtttcggt gagatggaag tctgggcact tgaagcatac 3900
   ggtgcggctt acacactaca agaaatgctt acagttaagt cggatgacgt tgaaggtcgt 3960
   acacgtgtct acaaaaacat tgtagatggc aaccattata tggatccggg tatgcctgaa 4020
   tcgttcaacg tattgaccaa agagatccgt tctttaggta tcaacattga actgaaaaat 4080
   ggtgactaa 4089
<210> 28
   <211> 4089
   <212> DNA
   <213> acinetobacter grimontii
<400> 28
   atggcatact catataccga aaagaaacgg atccgtaaga attttggtaa attgccccac 60
   gtaatggaag caccgtactt actttcgatt caggtcgatt cgtatcgtac attcttacaa 120
   ggtggtaaaa ctccaaaaaa tcgcgaagat atcggtctcc aagccgcatt tcgttcagtt 180
   tttcctattg aaagttattc tggcaatgct gctttagaat ttgttgagta tagccttggt 240
   aaacccgagt ttgacgtgcg tgaatgtatt ttacgtggtt cgacttatgc ggcaccaatg 300
   cgtgtaaaaa ttcgtttgat cattaaagat cgcgaaacga aatcaatcaa agatgttcgt 360
   gaacaagaag tgtacatggg cgaaatgccg ctcatgaccg acaacggtac tttcgttatt 420
   aacggtactg aacgtgtaat cgtatctcaa ttacaccgtt caccaggcgt attctttgat 480
   catgataagg gtaaaacaca ctcaagcggt aaagtgttgt attcagcacg tatcattcct 540
   taccgtggtt catggttaga ttttgaattc gatgcaaaag atttagtttt cgtacgtatt 600
   gaccgtcgtc gtaaattgtt ggcgactgtg atcttacgtg ctttaaatta tagcaatgaa 660
   caaatcttga atttgttcta tgaaaaagta cctgtatatc ttgatatggg tagctatcaa 720
   attgacctcg ttccagatcg cttacgtggt gaaatggcgc aatttgatat cttggacaac 780
   gatggtaaag caatcgttga gcaaggtaag cgtattaatg cacgtcatgt acgccaaatg 840
   gaagcagcta acttagctaa gctttctgta cctgatgaat atttatatga gcgtattaca 900
   gctgaagaca tcacacttaa gaatggtgat gtgattcctg caaataccgt acttagccat 960
   gaaattatgg tgaaattggc tgaaggtggt gttaaacaat ttaacatcct attcactaat 1020
   gacatcgatc gtggttcgtt cattgcagat acattacgtg cagatacaac aacaggtcgt 1080
   gaagaagcgc ttgttgaaat ctataaagta atgcgtccag gtgaaccacc gacaaaagaa 1140
   gcagcagaga acttatttaa taacttattc ttctcttctg aacgttatga cctttctcca 1200
   gtaggtcgta tgaagtttaa ccgtcgtttg ggtcgtcctt acgaagtggg tactgatcag 1260
   aagtcacgtg aagttgaagg tattttatcg catgacgata tcattgatgt acttcgtaca 1320
   ttagttgaga tccgcaatgg taaaggtgaa gtcgacgata tcgatcactt gggtaaccgt 1380
   cgcgtacgtt ctgttggtga aatgacagaa aaccaattcc gtgttggttt ggttcgtgtt 1440
   gaacgtgctg tgaaagagcg tttaagccaa gctgaaactg ataacttgtc tccacaagat 1500
   ttaatcaacg cgaaaccagt tgctgcggca atcaaagaat tctttggttc aagccagtta 1560
   tctcagttca tggaccaaaa caacccatta tctgagatta cacataaacg tcgtgtgtct 1620
   gcgcttggtc ctggtggttt gacacgtgaa cgcgcaggct tcgaagtgcg tgacgtacat 1680
   caaactcact atggtcgtgt ttgtccaatt gaaacacctg aaggtccaaa cattggtttg 1740
   atcaactcgc tttctgtcta tgctaaagcg aatgacttcg gtttcttgga aacaccatac 1800
   cgtaaagttg tagacggtcg tgttacagat gaagttgaat atttatctgc aattgaagaa 1860
   gtaggcaccg tcattgcaca agccgactca gcagtggata aagatggcaa cttgactgaa 1920
   gaaatggttt ctgtacgtca tcaaggtgaa ttcgtacgta tgtcgcctga gcgcgtaaca 1980
   catatggacg tttctgcaca gcaggttgtt tctgttgcag cgtcattaat tccattcctt 2040
   gaacacgatg acgcaaaccg tgcattgatg ggttcgaaca tgcaacgtca ggctgttcct 2100
   acacttcgtg ctgacaaacc acttgtcggt acgggtatgg aagcaaacgt agcacgcgac 2160
   tcaggtgtat gtgtgatcgc gggtcgtggt ggtgtaattg aatatgttga tgcatctcgt 2220
   atcgttattc gtgttaacga agatgaaatg attgcaggtg aagcaggtgt agatatttac 2280
   aacctgatca aatatacacg ttcaaaccaa aatacatgta ttaaccaaaa tgtcatcgta 2340
   aacttgggcg acaaagttgc tcgtggcgat attttggctg acggtccatc gactgacatg 2400
   ggtgaacttg cgctaggtca aaacatgcgc gtcgcgttca tgacatggaa cggttataac 2460
   tatgaagact caatcttact ttctgagcgt gtgcttcaag aagaccgttt aacgtcgatt 2520
   catattcaag aattgtcatg tgtagcgcgt gatactaagt taggtgcaga agaaattact 2580
   gctgatattc ctaacgtcgg tgaagctgca ctgtctaaac ttgatgagtc aggtattgtt 2640
   tatatcggtg ctgaagttac tgcaggtgat attcttgttg gtaaggtaac acctaaaggt 2700
   gaaactcagt taacacctga agaaaaacta cttcgtgcaa tctttggtga aaaagcggct 2760
   gacgtaaaag attcatcttt acgtgttccg tcaggcacta aaggtacagt gattgacgtt 2820
   caagtcttca cacgtgatgg tttagaaaaa gatgaacgtg cgcaagcaat tgagaaagct 2880
   cagcttgatg cataccgtaa agacttgaaa gaagaataca aaatcttcga agaagcagca 2940
   cgtgagcgta ttgttcgttt gttgaaaggt caagaatcta acggtggtgg ttcgactaaa 3000
   cgtggtgaga agctttcaga agatatgttg tctggtctag agttagttga tctacttgaa 3060
   atccaaccaa cagatgaagc aattgctgag cgtttaactc aaattcaagt gttcttgaaa 3120
   gaaaagagcc atgaaattga tgaaaagttt gctgagaaga aacgtaaact ttctacaggt 3180
   gatgagttaa caactggtgt attgaaagtt gttaaggttt acctagcagt taaacgtcgt 3240
   atccaacctg gtgataagat ggcgggtcgt cacggtaaca agggtgttgt atcaaacatc 3300
   ttaccagttg aagacatgcc acatgatgcc aatggtgtgc cagttgatat cgtattgaac 3360
   ccactcggtg taccatcgcg tatgaacgtg ggtcagattc ttgaaactca cttaggtatg 3420
   gcagcaaaag gtttgggtga gcagattgat aaaatgctca aacaacaacg tacaattgcc 3480
   gagttacgtt cattccttga caagatttat aataaagtgg gtggtgagca agaacagctt 3540
   gacacactga ctgatgaaga gatcttgaaa ctttcaggta atttacgtgc tggtgtgcct 3600
   ttggcaactc cagtattcga tggtgctgaa gagtcacaaa tcaaagagtt acttgaactt 3660
   gcagagttac cacgttctgg tcaacagatc ttgtttgatg gacgtacagg tgagcagttt 3720
   gatcgtccag taactgtcgg ttacatgtat atgcttaagt tgaaccactt ggttgacgac 3780
   aagatgcatg cacgttcaac tggttcttac tcacttgtga cacaacaacc gcttggtggt 3840
   aaagcacaat tcggtggtca gcgtttcggt gagatggaag tatgggcact tgaagcatat 3900
   ggtgcagcat ataccctcca agaaatgctc actgtgaagt cggatgacgt cgaaggtcgt 3960
   acacgcatct ataagaacat tgtagatgga aaccattata tggatccggg tatgcctgaa 4020
   tcgttcaacg tattgaccaa agagatccgt tctttaggta tcaacattga actgaaaaat 4080
   ggtgactaa 4089
<210> 29
   <211> 4089
   <212> DNA
   <213> Acinetobacter tandoii
<400> 29
   atggcatact catataccga aaagaaacgg atccgtaaga attttggtaa attgcctagt 60
   gttatggatg ctccgtactt gctcgcgatt caagtcgact cgtacagaac gttcttgcaa 120
   gatggcaaat caccaaaaaa ccgcgaagat atcggtctcc aagccgcatt tcgttcagtt 180
   tttcctatag aaagttattc tggcaatgct gctctagaat ttgttgagta tagccttggt 240
   aagccggagt ttgatgttcg cgaatgtatt cttcgtggct caacttatgc ggcaccaatg 300
   cgtgtaaaaa ttcgtttgat cattaaagat cgtgaaacga aatcaatcaa agacgtgcgt 360
   gagcaagaag tgtacatggg tgaaatgcca ctcatgacgg ataacgggac attcgttatt 420
   aatggtactg agcgtgtaat cgtatctcag ttacaccgtt cgccaggcgt gttctttgat 480
   catgataagg gtaaaactca ctcaagcggt aaagtgcttt attcggcacg tattattcct 540
   taccgtggtt catggttaga ctttgaattt gatgcaaaag atttagtcta tgtccgtatt 600
   gaccgtcgtc gtaaattgct tgcgactgtg gttttacgtg cattggatta cagcaacgaa 660
   caaattctag atttattcta cgagaaagta cctgtctatt tagacatggg tagctaccaa 720
   attgaccttg taccagaacg cctgcgtggc gaaatggctc aattcgacat taccgataat 780
   gacggtaaag tgattgttga acaaggtaag cgtattaacg cgcgtcatgt acgtcaaatg 840
   gaagcttcag gcttaacgaa gctttctgtt ccagacgaat atttatatga gcgtattact 900
   gcccaagata tcactttacg tgatggcgaa gtgattcctg cgaatacctt gctaagccat 960
   gaagtgatgg tgaagctggc tgaaggtggc gtgaagcagt ttaatattct gtttacgaat 1020
   gacatcgatc gtggttcgtt cattgcggat tctctacgtg cagatacaac aacaggtcgt 1080
   gaagaagcgc ttgttgaaat ctacaaagta atgcgtccag gtgagccacc aacaaaagaa 1140
   gcagctgaaa acttatttaa taacttattc ttctcttctg agcgttacga tttatcgcca 1200
   gttggtcgta tgaagtttaa ccgtcgtttg ggtcgtcctt acgaagtcgg tactgatcag 1260
   aagtcacgtg aagttgaagg tatcctttcg aacagcgata tcactgatgt attaaaaaca 1320
   ttggttgaaa ttcgtaacgg taaaggcgaa gtggatgata ttgaccactt gggtaaccgt 1380
   cgtgttcgtt ctgttggtga aatgacagaa aaccaattcc gtgttggttt ggttcgtgtt 1440
   gagcgtgctg taaaagagcg tttaagccaa gcagaagcag ataacttgtc tccgcaagat 1500
   ttaatcaatg cgaaaccagt tgcagcggca atcaaagaat tctttggttc aagccagtta 1560
   tctcagttca tggatcaaaa caacccattg tctgagatta cacacaaacg tcgtgtatct 1620
   gcacttggtc ctggtggttt aacacgtgaa cgtgcgggct ttgaagtacg tgacgtacat 1680
   caaactcact atggtcgtgt atgtccgatt gaaacgcctg aaggtccaaa cattggtttg 1740
   atcaactcgc tatctgttta tgcaaaatgt aataacttcg gtttcttgga aactccatac 1800
   cgtaaagttg ttgatggacg tgtaacggat gaagttgaat acttatctgc gattgaagaa 1860
   gtaggcactg tcattgcaca agccgactct gcagtcgatg cagacaatca cttgacggaa 1920
   gagtttgttt ctgtacgtca ccaaggtgat ttcgtgcgta tgccacctga aaaagtgacg 1980
   catatggatg tatctgctca acaggttgta tctgttgcag catcattgat tccgttcctt 2040
   gaacacgatg atgcgaaccg tgcattaatg ggttcgaaca tgcaacgtca ggctgttccg 2100
   acattattgg cggataaacc gcttgttggt acagggatgg aagcaaatgt agcacgtgat 2160
   tctggtgtgt gtgtcattgc aaaccgtggt ggtgcgattg aatacgtaga cgcttctcgt 2220
   atcgtgattc gtgtaaacga agaagagatg gtagcgggtg aagcgggtgt agatatctac 2280
   aacctgatca aatatacacg ttctaaccaa aatacatgta tcaaccaaaa cgtcgtagtt 2340
   cgcatgggtg acaaagttgc tcgtggtgat gtattggctg atggtccatc gactgatggt 2400
   ggtgaacttg cgcttggtca aaacatgcgc gtcgcgttca tgacttggaa cggttataac 2460
   tatgaagact cgatcttact ttctgagcgt gttcttcaag aagaccgttt aacttcgatc 2520
   catatccaag agttatcgtg tgttgcacgt gatactaaat tgggtgctga agaaattact 2580
   gcggatatcc cgaacgtggg tgaagctgca ctttctaagc ttgatgaatc aggtattgtt 2640
   tatatcggtg ctgaagttgc agcgggcgac atccttgttg gtaaagtaac gcctaaaggt 2700
   gaaacgcaat taacaccaga agaaaaactg cttcgtgcaa tttttggtga aaaagcagct 2760
   gacgttaaag attcttcttt acgtgttcca tctggtacga aaggtacagt aattgacgtt 2820
   caagtgttta cacgtgatgg tcttgaaaaa gacgaacgtg cgcaagcaat tgaaaaagcg 2880
   caattagatg cttaccgtaa agacttgaaa gaagaatata aaatcttcga agaagcagca 2940
   cgtgaacgta ttgttcgctt gttgagcggt caagaatcga atggcggtgg cggcacgaag 3000
   cgtggtgaca aactttcaga agatatgttg tctggcttag agttggttga tttacttgaa 3060
   atccagccaa gtgatgaagc gattgctgaa cgtttaaccc aaattcaagt gttcttgaaa 3120
   gagaagagct ttgaaattga cgagaaattt gctgagaaaa aacgcaaact ttctacaggt 3180
   gatgaactaa caactggcgt attgaaagtt gtgaaggttt accttgcagt taaacgtcgt 3240
   atccagcctg gtgataagat ggcgggtcgt cacggtaaca aaggtgttgt gtctaacatc 3300
   ctaccagtcg aagacatgcc gcatgatgcc aatggtgttc cagtcgatat cgtattgaac 3360
   ccgttgggtg taccgtcacg tatgaacgtg gggcagattc ttgaaactca cttgggtatg 3420
   gctgcgaaag gtttgggtga gcaaattgat aagatgctca aacaacagcg tgaaattgct 3480
   gaactacgtg ttttcctaga caaaatctac aacaaagtgg gcggtcagca agaagattta 3540
   gacagcttaa cagatgatga aatcttggtg ttggcaggta acttacgtgc aggtgtacct 3600
   ttagcaactc ctgtatttga tggtgctgaa gaaagccaaa tcaaagagtt actagagctg 3660
   gctgaaattc cacgttcggg tcaaaccgta ttgtatgatg gacgtacagg tgaacgtttc 3720
   gaccgtcctg taactgtagg ctatatgtac atgcttaagt tgaaccactt ggttgacgac 3780
   aagatgcatg cccgttctac aggttcttac tcattagtaa cgcaacaacc attgggtggt 3840
   aaagctcagt tcggtggtca gcgtttcggt gagatggagg tctgggcact tgaggcttat 3900
   ggcgcagctt atacacttca agaaatgctc actgtgaagt cggatgacgt tgaaggtcgt 3960
   actcgtatct ataagaatat tgtagatggt aaccattata tggacccagg tatgcctgaa 4020
   tcgttcaacg tattgaccaa agagatccgt tctttaggta tcaacattga actgaaaaat 4080
   ggtgactaa 4089
<210> 30
   <211> 4089
   <212> DNA
   <213> acinetobacter tjernbergiae
<400> 30
   atggcatact catataccga aaagaaacgg atccgtaaga attttggtaa attgccccat     60
   gtaatggaag caccgtactt actttcgatt caggtcgatt cgtatcgtac attcttacaa    120
   ggcggtaaaa ctccaaaaaa tcgcgaagat atcggtctcc aagccgcatt tcgttcagtt    180
   tttcctattg aaagttattc tggcaatgct gctttagaat ttgttgagta tagtcttggt    240
   aagcccgagt ttgacgtacg tgaatgtatt ttacgtggtt caacttatgc agcaccaatg    300
   cgcgtaaaaa ttcgtttgat tattaaagat cgtgaaacga aatcaattaa agatgttcgt    360
   gaacaagaag tctacatggg tgaaatgcca ctcatgaccg ataacggtac ttttgttatc    420
   aatggtactg agcgtgtgat tgtgtctcag ttacaccgtt cgccaggtgt attctttgat    480
   catgataaag gtaagaccca ttcaagtggt aaagtgttgt attcggcacg tattattcct    540
   taccgtggtt catggttaga ttttgaattt gatgctaaag atcttgttta tgtgcgtatt    600
   gaccgtcgtc gtaaactatt agcaactgta attctgcgcg ctttaggcta tgcaaatgca    660
   caaatcttga atttattcta tgaaaaagtg cctgtatatc ttgatatggg tagttatcag    720
   attgatcttg tgccagatcg attacgcggt gaaatggcgc agtttgatat tgccgataat    780
   gacggtaaag tcattgttga acaaggtaaa cgtattaatg tacgtcatgt acgtcaaatg    840
   gaagcggcta atttagccaa actttctgta cctgatgagt atttatatga acgtattacc    900
   gctgaagata ttaccttaaa agatggtgat gtgattcctg caaatacgct gcttagtcat    960
   gaagtcatgg tgaagttagc tgaaggtggc gttaaacagt tcaatatctt gtttactaat   1020
   gatattgatc gtggctcttt tattgctgat agtttacgtg cagacacaac ttcagggcgt   1080
   gaagaagcat tagtagaaat ttacaaagta atgcgtccag gtgaaccacc aacaaaagaa   1140
   gctgctgaaa acttatttaa taatttattc ttctcttctg aacgttatga cctttctcca   1200
   gtaggtcgca tgaagtttaa ccgtcgtttg ggtcgtcctt acgaagtggg tacagatcag   1260
   aaatcgcgtg aagttgaagg tattttatcg cacgacgata tcatcgatgt actgcgtaca   1320
   ttggttgaaa ttcgtaacgg taaaggtgaa gtcgatgata tcgaccactt aggtaaccgt   1380
   cgtgtacgtt ctgttggtga aatgacagaa aaccaattcc gtgttggttt agttcgtgtt   1440
   gaacgtgctg ttaaagagcg tttaagccaa gcagagacag ataatttgtc tccacaagat   1500
   ttaatcaatg cgaaaccagt tgctgctgca atcaaagaat tctttggttc aagccaattg   1560
   tctcagttca tggatcaaaa caatccattg tctgaaatta cacataaacg tcgtgtatct   1620
   gcgcttggcc ctggtggttt gacacgtgag cgtgcgggct tcgaagtccg tgacgtacat   1680
   caaactcact atggtcgtgt ttgtccaatt gaaacgcctg aaggtccaaa cattggtttg   1740
   atcaactcgc tttctgtcta tgcaaaagcg aatgactttg gtttcttgga aacaccatac   1800
   cgtaaagtcg ttgatggtcg tgtgactgat gaggttgaat atttatctgc aattgaagaa 1860
   gttgggactg tgattgcaca ggccgattct ggtgtagata aagatggtaa cttaacagaa 1920
   gaaatggctt ctgtacgtca tcagggcgaa tttgtacgta tgccacctga aaaagtgacg 1980
   catatggacg tatctgctca gcaggttgtt tctgttgctg catcgcttat tccgtttctt 2040
   gaacacgatg atgcgaaccg tgcattgatg ggttcaaaca tgcaacgtca ggctgttccg 2100
   acattgcgtg ctgacaagcc gcttgttggt acgggtatgg aagcaaacgt agcacgtgat 2160
   tctggtgtgt gtgtgatcgc agaccgtggt ggtgcgattg aatatgttga tgcatctcgt 2220
   atcgtgattc gtgtaaacga agatgaaatg attgcgggtg aagcgggtgt agatatctat 2280
   aacctgatca aatatacacg ttcaaaccaa aatacctgta tcaaccaaaa cgttatcgta 2340
   aacttgggtg acaaagttgc tcgtggcgat atcttggctg atggtccatc gactgatatg 2400
   ggtgaacttg cgcttggtca aaacatgcgc gtcgcgttca tgacatggaa cggttataac 2460
   tacgaagact cgatcttact ttctgagcgt gtacttcaag aagaccgttt aacgtcgatt 2520
   catatccaag aattgtcatg tgtagcgcgt gatactaagt taggtgcaga agaaattact 2580
   gccgatattc ctaacgtggg tgaagctgca ctgtctaagt tggatgagtc tggtattgtt 2640
   tatatcggtg ctgaagtgac tgcgggtgac atccttgttg gtaaggtaac gcctaaaggt 2700
   gaaactcagt taacacctga agaaaaacta cttcgcgcga tctttggtga aaaagctgct 2760
   gacgttaaag actcttcttt acgcgttcca tctggtacca aaggtactgt gattgacgtt 2820
   caagtcttta cgcgtgatgg tttggaaaaa gatgaacgtg ctcaagccat tgagaaagct 2880
   cagcttgatg cataccgtaa agatttgaaa gaagaataca aaatcttcga agaagcagca 2940
   cgtgaacgta ttgttcgttt gttgacaggt caagagtcta acggtggtgg ctcaactaag 3000
   cgtggtgata aactttctgc agatgtcttg tctggtttag agctggttga tttacttgaa 3060
   attcaaccga ctgatgaagc aattgcagag cgtttaactc agattcaagt gttcttgaaa 3120
   gagaagagct acgaaattga cgagaagttt gcagagaaga aacgtaaact ttctacaggt 3180
   gatgaattaa caacgggtgt attgaaagtt gttaaggttt acctcgctgt taaacgtcgt 3240
   atccagcctg gtgataagat ggcgggtcgt catggtaaca aaggtgttgt atctaacatc 3300
   ttacctgttg aagacatgcc tcatgatgcg aatggtgtgc cagtcgatat cgtattgaac 3360
   ccattgggtg taccgtcacg tatgaacgtg ggtcagattc ttgagactca cttaggtatg 3420
   gcggcgaaag gtcttggcga taaaatcgaa aaaatgttga aagaacagcg tacagtgatt 3480
   gaactgcgtg aattcttaga caagatttat aacaaggtcg gtggtgagca agaagagctt 3540
   gacagcttaa ctgatgcgga agtcttggca ctttcaggca acttacgtgc tggtgttcca 3600
   ttggcaacgc ctgtatttga cggtgctgaa gaaagtcaga ttaaagactt acttgaattg 3660
   gcagacatct cacgtacggg tcaaacggta ttgtttgacg gacgtacagg tgaacagttt 3720
   gatcgtcctg taactgtagg ttacatgtac atgctcaaat tgaaccactt ggttgatgac 3780
   aagatgcatg cgcgttcaac gggttcttac tcacttgtga ctcaacagcc gcttggtggt 3840
   aaagcacaat tcggtggtca gcgtttcggt gagatggaag tatgggcact tgaagcatac 3900
   ggtgctgcat acacgctcca agaaatgctt acagtgaagt cggatgacgt cgaaggtcgt 3960
   acacgcatct ataagaacat tgtagatggt aaccattata tggatccggg tatgcctgaa 4020
   tcgttcaacg tattgaccaa agagatccgt tctttaggta tcaacattga actgaaaaat 4080
   ggtgactaa 4089
<210> 31
   <211> 4089
   <212> DNA
   <213> acinetobacter towneri
<400> 31
   atggcatact catataccga aaagaaacgg atccgtaaga attttggtaa attgccccaa 60
   gtaatggatg caccgtactt gctttcgatt caagtcgact cgtaccgtac tttccttcaa 120
   gatggcaaaa ctccaaaaag ccgcgaagat atcggtctcc aagccgcatt tcgttcagtt 180
   tttcctatag aaagttattc tggcaatgct gctttagaat ttgttgagta tagtcttggt 240
   aagcctgagt ttgatgtacg tgagtgtatt cttcgcggct caacttatgc ggcaccaatg 300
   cgcgtaaaaa ttcgtttgat cattaaagat cgtgaaacga aatcaatcaa agatgtgcgt 360
   gagcaagaag tctacatggg cgaaatgcca ttgatgacgg ataacggtac ctttgtgatt 420
   aacggtactg agcgtgtaat tgtttctcaa ttacaccgtt caccaggcgt gttctttgac 480
   cacgataaag gtaagaccca ctcgagcggt aaagtgttgt attcagcacg cattattcct 540
   taccgtggct catggttaga ctttgaattc gatgccaaag acctcgttta tgtgcgtatt 600
   gaccgtcgtc gtaaattgct tgcgactgta gtgttgcgtg cattgggcta tagcaatgaa 660
   caaatcctag acttgttctt tgagaaagta cctgtctatt tagacatggg tagctaccaa 720
   attgaccttg tcccagaacg cttacgtggc gaaatggcac aatttgacat cactgatact 780
   gacggcaaag tgattgttga gcaaggtaaa cgcattaatg cgcgtcacgt acgtcaaatg 840
   gaagcagctg gtcttgagaa gctttcagtt cctgatgagt acttgtacga acgtattact 900
   gcagaagaca tccagctaaa agatggcgat gtgattgctg caaacacctt gttaagccat 960
   gaaatcatgg tgaaacttgc agaaggtggt gttaaacaat ttaacatcct attcaccaat 1020
   gacatcgacc gtggttcgtt cattgcggac accttacgtg cagatacaac tgttggtcgt 1080
   gaagatgctt tggtagaaat ctacaaagtg atgcgtccag gtgagccacc gacgaaagaa 1140
   gccgctgaaa atctgttcaa taacttgttc ttctcttctg agcgttatga tttatcgcca 1200
   gtaggtcgta tgaagttcaa ccgtcgtttg ggtcgtcctt acgaagtggg tacagatcag 1260
   aaatcacgtg aagttgaagg tattttgtct aacgaagaca ttaccgatgt acttaaaaca 1320
   ttgatcgaaa tccgtaacgg taaaggtgaa gtcgacgata tcgaccactt gggtaaccgt 1380
   cgcgtacgtt cagtgggcga gatgactgaa aaccaattcc gtgttggttt ggtacgtgtt 1440
   gagcgtgctg taaaagagcg tttgtcacaa gctgaaacag acaacctgtc tccgcaagat 1500
   ttaatcaatg cgaagccagt ggctgctgca atcaaagaat tctttggttc aagccaattg 1560
   tctcagttca tggaccaaaa caacccgttg tctgaaatta cccacaagcg tcgcgtatct 1620
   gcgcttggtc caggtggttt gacacgtgaa cgtgcaggct ttgaggtacg tgacgtacac 1680
   caaactcact atggtcgtgt gtgtccaatt gaaacgcctg aaggtccaaa cattggtttg 1740
   atcaactcgc tttctgttta tgcaaaatgt aataattttg gcttcttgga aacgccatac 1800
   cgtaaagtgg ttgatggtcg tgtaaccgat gaggttgagt atttatctgc aattgaagaa 1860
   gtgggcacga tcattgcgca agcggactca agcgtagacc aagatggcaa tttaacagaa 1920
   gaattcgtat ctgtacgtca ccaaggtgac ttcgtgcgta tgccgccaga gcgcgtgacg 1980
   cacatggacg tttctgcaca gcaggttgta tctgtagcag catcactgat tccattcctt 2040
   gagcacgatg acgccaaccg tgccttgatg ggatcgaaca tgcaacgtca ggctgttccg 2100
   acgcttatcg ctgataaacc acttgtgggt acagggatgg aagcgaacgt agcacatgac 2160
   tcaggtgtat gtgtgattgc aaaccgtggt ggtcgtattg aatatgtcga tgcttcacgt 2220
   gtagtcattc gtgtgaacga agaagaaatg atcgcgggtg aagcaggtgt agatatctac 2280
   aacctgatca aatacacacg ttcaaaccaa aacacttgta ttaaccaaaa agttttggtg 2340
   aagatgggtg ataaggtagg tcgtggtgat gtattggctg acggtccatc tacagatggc 2400
   ggtgagcttg cactcggtca gaacatgcgt gtcgcgttca tgacttggaa tggctataac 2460
   tacgaagact cgatcttgct ttctgagcgt gtacttcaag aagaccgttt aacttcgatt 2520
   cacattcaag aattgtcatg tgttgcacgt gataccaaat tgggtgcgga agaaatcaca 2580
   gctgatattc caaacgtggg cgaagctgca ctgtctaaac tagacgagtc tggtatcgtt 2640
   tacattggtg ctgaagttac tgcaggcgac atcctggtag gtaaagtaac gccgaagggt 2700
   gaaactcagt taacccctga agaaaaattg cttcgtgcaa tcttcggtga aaaagcagca 2760
   gatgtgaaag attcatcatt gcgcgttcca tcaggtacta aaggtacagt gattgacgtt 2820
   caagtgttta cccgcgatgg tctagaaaaa gacgaacgtg cgcaagcaat tgaaaaagca 2880
   cagttagatg catatcgcaa agacttgaaa gaagaataca aaatctttga agaagctgca 2940
   cgtgaacgta ttatccgctt attgaaaggc caagagtcta acggtggcgg tacaaccaaa 3000
   cgtggcgaca agctttctga agatatgttg tcaggcttgg ctttagtgga tttacttgaa 3060
   attcaaccaa gtgatgaagc gattgctgaa cgcttaacgc aaattcaaac cttcttgaaa 3120
   gagaagagct ttgaaattga tgagaaattt gctgagaaga aacgtaaact ttctacaggc 3180
   gatgagctca ctacaggcgt gttgaaagtt gttaaggtgt acttggctgt taaacgtcgc 3240
   atccaaccgg gtgataagat ggcgggtcgt cacggtaaca aaggtgttgt atctaacatc 3300
   ttgccagtag aagacatgcc gcacgatgct aacggtgtac ctgttgacat cgtattgaac 3360
   ccactaggtg taccatctcg tatgaacgtg ggtcagattc ttgaaacaca cttgggtatg 3420
   gcagccaaag gtttgggtga gcaaattgac aagatgctca aacaacaacg tgagattgct 3480
   gaactacgtg cgttcctaga caagatttat aacaaagtgg gtggcgagca agaagatctt 3540
   gacagcttaa ctgatgatga aatcttggta ttggcgggta acttgcgtgc aggtgttcca 3600
   ttggctactc ctgtatttga tggtgctgaa gaaggtcaaa tcaaagaatt gcttgaactg 3660
   gctgaattac cacgttcagg tcagaccgta ttgtatgatg gacgtacagg cgagaaattc 3720
   gaccgtcctg tgactgtagg ttatatgtac atgctcaaac ttaaccactt ggtggatgac 3780
   aagatgcacg cacgttctac aggttcttac tcacttgtaa cgcaacagcc gttaggtggt 3840
   aaggcacaat tcggtggtca gcgtttcggt gagatggaag tctgggcact tgaagcatac 3900
   ggtgctgctt atacgctgca agaaatgctc actgtgaagt cggatgacgt tgaaggtcgt 3960
   acccgcatct ataagaacat tgtagatggc aaccattata tggatccggg catgcctgaa 4020
   tcatttaacg tattgaccaa agagatccgt tctttaggta tcaacattga actgaaaaat 4080
   ggtgactaa 4089
<210> 32
   <211> 4089
   <212> DNA
   <213> acinetobacter parvus
<400> 32
   atggcatact catataccga aaagaaacgg atccgtaaga attttggtaa attgccccac 60
   gtaatggaag caccgtactt actgtcgatt caggtcgatt cgtaccgtac attcttacaa 120
   ggcggtaaaa ctccaaaaaa tcgcgaagat atcggtctcc aagccgcatt tcgttcagtt 180
   tttcctattg aaagttattc tggcaatgct gctttagaat ttgttgagta tagtcttggt 240
   aagcctgagt ttgacgtacg tgaatgtatt ttacgtggtt caacttatgc agcaccaatg 300
   cgcgtaaaaa ttcgtttgat cattaaagat cgtgaaacga aatcaattaa agacgtgcgt 360
   gaacaagaag tttacatggg tgaaatgcca ctcatgaccg ataacggtac tttcgttatc 420
   aacggtactg agcgtgtcat cgtatctcaa ttacaccgtt caccaggtgt attctttgac 480
   catgacaagg gtaaaaccca ctcaagtggt aaagtgttgt attcagcacg tattattcct 540
   taccgtggtt catggttaga ttttgaattt gatgctaaag atttagtatt cgtacgtatt 600
   gaccgtcgtc gtaagttact ggcaacggtg attctacgtg ccttgaatta cagcaatgaa 660
   caaatcttga atttgttcta tgaaaaagta cctgtatatc ttgatatggg tagctatcaa 720
   attgatcttg ttccagatcg cttacgtggt gaaatggcac aatttgatat cttagacaat 780
   gatggtaaag cgatcgttga acaaggtaaa cgtattaacg cacgtcacgt acgtcaaatg 840
   gaagctgcga acttagccaa gctttctgta cctgatgaat atttatatga gcgtattaca 900
   gctgaagata tcacactgaa aagtggtgat gtgattcctg cgaataccgt tcttagccat 960
   gacatcatgg tgaagatcgc tgaaaatggt gtgaagcagt ttaacatcct attcacgaat 1020
   gatattgatc gtggttcgtt tgttgcggat acattacgtg cagatacaac aacaggtcgt 1080
   gaagaagcac ttgttgaaat ctataaagtc atgcgtccag gcgagccgcc gacgaaagaa 1140
   gctgctgaaa acctattcaa taatttgttc ttctcttctg agcgttatga cctttctcca 1200
   gtgggtcgta tgaagttcaa ccgtcgtttg gggcgtccat acgaagtcgg tactgaccag 1260
   aagtcacgtg aagttgaagg tattctgtca cacgacgata ttattgatgt attacgtaca 1320
   ttagtagaaa tccgtaacgg taaaggtgaa gtcgacgata tcgatcactt aggtaaccgt 1380
   cgtgtacgtt ctgtgggtga aatgacagaa aaccaattcc gtgttggtct agtccgtgtt 1440
   gaacgtgctg tgaaagagcg tttaagccaa gcagaaactg ataacttgtc tccgcaagat 1500
   ttaattaacg ccaaaccagt tgctgcggca atcaaagaat tctttggttc aagccaattg 1560
   tctcagttca tggaccaaaa taacccattg tctgagatta cgcacaaacg tcgtgtatct 1620
   gcgcttggtc ctggtggttt gacgcgtgaa cgtgcaggct ttgaagtacg tgacgtacat 1680
   caaactcact atggtcgtgt ttgtccaatt gaaacacctg aaggtccaaa cattggtttg 1740
   atcaactcgc tttctgtcta tgctaaagcg aatgacttcg gtttcttgga aacaccatac 1800
   cgtaaagttg ttgatggtcg tgtgactgat gaagttgaat acttatctgc aattgaagaa 1860
   gtaggcactg tcattgcaca agccgactca gcagtggata aagatggcaa cttgactgaa 1920
   gaaatggttt ctgtccgtca tcaaggtgaa tttgtgcgta tgtcgcctga gcgcgtcaca 1980
   catatggatg tttctgcaca gcaggttgtt tctgttgcag catcattgat tccattcctt 2040
   gaacacgatg atgcgaaccg tgccttgatg ggttcgaaca tgcaacgtca ggctgttcct 2100
   acattgcgtg ctgacaagcc gcttgttggt acaggtatgg aagcaaacgt agcacgtgac 2160
   tcaggtgtgt gtgtaatcgc caaccgcggt ggtgcgattg aatatgttga tgcatctcgt 2220
   atcgtgattc gtgtcaacga agatgaaatg attgcgggtg aagcaggtgt agatatctat 2280
   aacctgatca aatatacacg atcaaaccaa aatacctgta ttaaccagaa tgtcatcgtg 2340
   aacctgggcg acaaagttgc tcgtggtgat attttggctg acggtccatc gactgacatg 2400
   ggtgaacttg cgctgggtca aaacatgcgc gtcgcgttca tgacatggaa cggttataac 2460
   tatgaagact cgatcttact ttctgagcgt gtgcttcaag aagatcgttt aacgtcgatt 2520
   cacattcagg aattgtcatg tgtcgcacgt gatactaagt tgggctcgga agaaattacc 2580
   gctgatattc caaacgttgg cgaagcagct ttatctaagt tggatgagtc aggtattgtg 2640
   tatatcggtg cggaagtgac tgcgggtgac atccttgttg gtaaagtcac gcctaaaggt 2700
   gaaactcagt taacacctga agaaaaatta cttcgtgcaa tctttggtga aaaagcggct 2760
   gacgtaaaag actcgtcttt acgtgttcca tcgggtacta aaggtacagt gattgacgtt 2820
   caagtcttca cacgtgatgg tttggaaaaa gatgaacgtg cgcaggcaat tgagaaagct 2880
   cagcttgatg cataccgtaa agacttgaaa gaagaataca aaatcttcga agaagcagca 2940
   cgtgaacgta ttgttcgttt gttgaaaggt caagaatcta atggtggtgg ttcaaccaaa 3000
   cgcggtgata aactttctgc agaggtattg tctggtttag agttggttga tttacttgaa 3060
   atccagccaa atgatgaagc aattgctgag cgtttaactc aaattcaagt gttcttgaaa 3120
   gagaagagtt acgagattga cgagaagttt gctgagaaga agcgtaaact ttctacaggt 3180
   gatgaattaa caactggcgt attgaaagtt gttaaggttt acctggcagt taaacgtcgt 3240
   atccagcctg gtgataagat ggcgggtcgt cacggtaaca aaggtgttgt atcaaacatc 3300
   ttgccagttg aagacatgcc acatgatgcg aatggtgtgc cagtcgacat cgtattgaac 3360
   ccacttggtg taccgtcacg tatgaacgtg ggtcagattc ttgagactca cttgggtatg 3420
   gcggcgaaag gtcttggcga taagatcgaa aaaatgttga aagaacaacg tacagtgatt 3480
   gaactgcgtg aattcttaga caagatttat aacaaggttg gtggtgagca ggaagagctt 3540
   gatagcttga ctgacgcaga aatcttggcg ctttcaggta acttacgtgc tggtgttcca 3600
   ttagcgactc ctgtatttga tggtgctgaa gaaagccaga tcaaagactt acttgaattg 3660
   gcagacattt ctcgtacagg tcaaacagta ttgtttgatg gtcgtacagg tgaacagttt 3720
   gaccgtcctg taactgtagg ttacatgtac atgctgaaac tgaaccactt ggttgatgac 3780
   aagatgcatg cgcgttcaac aggttcttac tcacttgtaa ctcaacaacc gcttggtggt 3840
   aaagcacaat tcggtggtca gcgtttcggt gagatggaag tctgggcact tgaagcatac 3900
   ggtgctgcat atacgctcca agaaatgctt acagtgaagt cggatgacgt cgaaggtcgt 3960
   acacgcatct ataagaacat tgtagatggg aaccattaca tggatccggg tatgcctgaa 4020
   tcgttcaacg tattgaccaa agagatccgt tctttaggta tcaacattga actgaaaaat 4080
   ggtgactaa 4089
<210> 33
   <211> 350
   <212> DNA
   <213> Acinetobacter calcoaceticus
<400> 33
   agacttgaaa gaagaataca agatctttga agaagcagct cgtgagcgtg taattcgttt 60
   gcttaacggc caagagtcta acggtggtgg ttcgactaaa cgtggcgaca agctcgttga 120
   cggtatgttg tctggtttag agcttgttga cttacttgaa atccaaccta cagatgaagc 180
   aattgctgaa cgtttatctc aaattcaagt gttcttgaaa gagaagagcg cagaaattga 240
   tgagaagttt gcagagaaga aacgtaagct ttcgactggt gatgagttaa caacaggtgt 300
   tctgaaagtt gttaaagttt acctagcagt taaacgtcgt attcagcctg 350
<210> 34
   <211> 350
   <212> DNA
   <213> Acinetobacter baumannii
<400> 34
   agacttgaaa gaagaataca agatcttcga agaagcggct cgtgagcgtg taattcgttt    60
   gcttaaaggc caagagtcta atggcggtgg ttcaactaaa cgtggtgata aactttctga   120
   agatttatta tctggtttag agcttgttga tttacttgaa attcaaccag cagatgaagc   180
   aatcgctgag cgtttaactc aaattcaagt gttcttgaaa gagaagagcg cagaaatcga   240
   tgagaaattc gctgagaaga aacgtaagct tgccacaggt gatgaattaa cgactggcgt   300
   attaaaagtt gttaaggttt acttagctgt taaacgtcgt attcagcctg              350
<210> 35
   <211> 350
   <212> DNA
   <213> Acinetobacter sp.
<400> 35
   agacttgaaa gaagaataca agatctttga agaagcagct cgtgagcgtg taattcgttt    60
   gcttaaaggc caagagtcta atggcggtgg ttcaactaaa cgtggtgaca aactcgttga   120
   agaagtgtta tctggtttag agcttgttga tttacttgaa attcaaccgg cagatgaagc   180
   aatcgctgag cgtttaactc aaattcaagt gttcttaaaa gaaaagagcg cagaaattga   240
   tgagaaattc gctgagaaga aacgtaagct tgcaacaggt gatgaattaa caactggcgt   300
   attgaaagtt gttaaagttt acttagctgt taaacgtcgt attcagcctg              350
<210> 36
   <211> 350
   <212> DNA
   <213> Acinetobacter haemolyticus
<400> 36
   agacttgaaa gaagaataca aaatcttcga agaagcagca cgtgaacgta ttgttcgctt    60
   gttgaaaggt caagagtcaa atggtggcgg tacaactaag cgcggcgata aactctcaga   120
   agatgtattg tctggtttag agcttgttga tttacttgaa atccaaccag ctgatgaagc   180
   gattgctgaa cgtttaacgc aaattcaagt gttcttgaaa gagaagagca tcgaaatcga   240
   tgagaaattt gcagagaaga agcgtaagct ttctacaggt gatgaattaa caacgggtgt   300
   attaaaagtt gttaaggttt accttgcggt taagcgtcgt attcagcctg              350
<210> 37
   <211> 350
   <212> DNA
   <213> Acinetobacter junii
<400> 37
   agacttgaaa gaagaataca aaatcttcga agaagcagca cgtgagcgta ttgttcgttt 60
   gttgaaaggt caagaatcta acggtggtgg ttcgactaaa cgtggtgaga agctttcaga 120
   agatatgttg tctggtctag agttagttga tctacttgaa atccaaccaa cagatgaagc 180
   aattgctgag cgtttaactc aaattcaagt gttcttgaaa gaaaagagcc atgaaattga    240
   tgaaaaattt gctgagaaga aacgtaaact ttctacaggg gatgagttaa caactggtgt    300
   attgaaagtt gttaaggttt acctagcagt taaacgtcga atccaacctg 350
<210> 38
   <211> 350
   <212> DNA
   <213> Acinetobacter sp.
<400> 38
   agatttgaaa gaagaataca aaatctttga agaagcagca cgtgaacgta ttgttcgctt 60
   gttgaaaggt aaagagtcta atggtggcgg tacaacgaag cgcggcgata aacttgcaga 120
   agatatgttg tctggtttag agctggttga tttgttagaa atccaaccaa cagatgaagc 180
   aatcgctgaa cgtttaactc aaattcaggt attcttgaaa gagaagagta tcgagattga 240
   tgagaaattt gctgagaaga aacgcaaact ctctacaggt gatgaattaa caacgggtgt 300
   attaaaagtt gttaaggttt accttgcagt gaaacgtcgt atccaaccgg 350
<210> 39
   <211> 350
   <212> DNA
   <213> Acinetobacter johnsonii
<400> 39
   agacttgaaa gaagaataca aaatcttcga agaagcagca cgtgaacgta ttgttcgttt 60
   gttgacaggt caagagtcta acggtggtgg tacaaccaag cgtggcgata agctttctgt 120
   agacgtattg tctggtttag agttggttga tttacttgaa atccaaccga ctgatgaagc 180
   tattgcagag cgtttaactc aaattcaagt gttcttgaaa gagaagagct ttgaaattga 240
   tgagaagttt gcagagaaaa aacgcaaact ttctacaggt gatgaattaa caacaggtgt 300
   attgaaagtt gttaaggttt acttggctgt taaacgtcgc atccaaccgg 350
<210> 40
   <211> 350
   <212> DNA
   <213> Acinetobacter lwoffii
<400> 40
   agacttgaaa gaagagttca agatctttga agaagcagca cgtgaacgtg taattcgttt     60
   gttgaacggt caagagtcga atggtggcgg taccactaaa cgtggcgaca aactgtctga    120
   agacgtgttg tctggtttag agcttgttga tcttcttgaa attcaaccgg ttgatgaagc    180
   aatcgctgaa cgtctaacgc aaattcaagt gttcttgaaa gagaagagct tcgaaattga    240
   cgagaaattt gctgagaaaa aacgcaaact ttctacaggc gatgagctga ccactggcgt    300
   attgaaagta gttaaagttt atcttgcggt aaaacgtcgc atccagccgg               350
<210> 41
   <211> 350
   <212> DNA
   <213> Acinetobacter sp.
<400> 41
   agacttgaaa gaagaattca aaatcttcga agaagctgca cgtgaacgtg taatccgtct     60
   actgaatggc caagagtcga atggtggcgg tacaactaaa cgtggcgaca aactgtctga    120
   agacgtgttg tctggtttag agcttgttga tcttcttgaa attcaaccag ttgatgaagc    180
   aattgctgaa cgtttaactc aaattcaagt gttcttgaaa gagaagagct tcgaaattga    240
   cgagaaattt gctgagaaaa aacgcaaact ttctacaggc gatgaactga ccactggcgt    300
   tttaaaagtt gttaaggttt atcttgctgt aaaacgtcgc atccaaccgg               350
<210> 42
   <211> 350
   <212> DNA
   <213> Acinetobacter sp.
<400> 42
   agacttgaaa gaagagttca aaatcttcga agaagctgct cgtgaacgta ttgtgcgttt     60
   attgaaaggt caagagtcaa atggtggtgg tacaacgaaa cgtggtgaca agctaactga    120
   agacgtattg tctaacttag agcttgttga tctgttagaa gttcaaccag cagacgaagg    180
   tattgctgag cgtttaacgc agattcaagt gttcttgaaa gagaagagcc acgagatcga    240
   tgagaagttt gctgagaaaa aacgtaaact ttcaacgggt gatgaactga caactggtgt    300
   gttgaaagtt gttaaagttt atcttgctgt taaacgtcgt atccagcctg               350
<210> 43
   <211> 350
   <212> DNA
   <213> Acinetobacter sp.
<400> 43
   agacttgaaa gaagaattca aaatcttcga agaagcagct cgtgaacgta ttatccgttt 60
   gttaaaaggc caagagtcga atggcggcgg tactactaag cgcggtgata agctatctga 120
   agatgtattg tctggtttag agcttgttga tcttttagaa gttcaaccaa cagatgaagg 180
   catcgctgaa cgcttaactc aaattcaagt gttcttgaaa gagaagagct acgagattga 240
   tgagaaattt gctgagaaaa aacgcaaact ttctacaggt gatgagctta caacaggtgt 300
   cttgaaagtt gttaaagttt atttagctgt aaaacgtcgt atccagcctg 350
<210> 44
   <211> 350
   <212> DNA
   <213> Acinetobacter radioresistens
<400> 44
   agatttgaaa gaagaatata aaatctttga agaagcggct cgtgaacgta ttgtacgttt 60
   gctgaaagat caggtgtcta acggcggtgg aaatactaaa cgtggtgaga aactgtctga 120
   agaattgcta tctggccttg aactgattga tctgctcgaa atccagccaa gcgatgaagc 180
   gattgctgaa cgtttaaccc agatccaggt gttcttgaaa gagaaaagca ccgagattga 240
   cgagaagttt gccgagaaga aacgcaagct ttctacgggt gatgagctga ctcatggcgt 300
   attgaaagtt gtgaaggttt atctagcagt taaacgtcgt atccagccgg 350
<210> 45
   <211> 350
   <212> DNA
   <213> Acinetobacter sp.
<400> 45
   agacttgaaa gaagagtaca aaatcttcga agaagcagca cgtgaacgta ttgttcgttt 60
   gttgaaaggt caagagtcta acggtggcgg ttcaactaaa cgcggtgata aacttgctga 120
   agacgtattg tctggtttag agcttgttga tttacttgaa atccaaccga ctgatgaggc 180
   aattgcagag cgtctaactc aaattcaagt gttcttgaaa gagaagagct atgaaattga 240
   tgagaagttt gcagagaaga agcgtaaact ttctacaggt gatgaattaa ccactggcgt 300
   attgaaagtt gttaaggttt accttgcggt taaacgtcgt atccagcctg 350
<210> 46
   <211> 350
   <212> DNA
   <213> Acinetobacter sp.
<400> 46
   agacttgaaa gaagaataca aaatcttcga agaagcagca cgtgaacgta ttgttcgttt 60
   gttgaaaggt caagaatcta acggtggcgg ttcaaccaaa cgcggcgata aactttcaga 120
   agatgtattg tctggcttag agcttgttga tttacttgaa atccagccaa atgatgaagc 180
   aattgctgaa cgtttaactc agattcaagt gttcttgaaa gagaagagct acgagattga 240
   cgagaagttt gctgagaaga agcgtaaact ttctacaggt gatgaattaa caacgggcgt 300
   attgaaagtt gttaaggttt accttgcggt gaaacgtcgt atccagcctg 350
<210> 47
   <211> 350
   <212> DNA
   <213> acinetobacter schindleri
<400> 47
   agacttgaaa gaagaataca aaatcttcga agaagcagca cgtgaacgta ttattcgtct 60
   gttgaaaggt caggaatcta acggtggtgg cacaactaaa cgcggtgaca aactgtctga 120
   agatgttctg tctggtttag agcttgttga tctgttagac atccaaccag tagatgaagc 180
   aattgctgag cgtttaactc aaattcaagt gttcttgaaa gagaagagcc ttgaaattga 240
   tgaaaagttt gctgagaaga aacgcaaatt atctacaggc gatgaactta caactggcgt 300
   actgaaagtt gttaaggttt atctagcggt taaacgtcgt atccagcctg 350
<210> 48
   <211> 350
   <212> DNA
   <213> acinetobacter ursingii
<400> 48
   agatttgaaa gaagaataca aaatctttga agaagcagca cgtgaacgta ttgtgcgctt 60
   attaaaaggt caggattcta acggtggtgg cacgactaaa cgtggtgaca aactgactga 120
   agatttattg tctggtcttg agctggtcga tttacttgaa attcaaccaa gtgatgaagg 180
   catcgctgag cgtttaagtc aaattcaagt attcttgaaa gagaagagtg ctgaaattga 240
   tgagaaattt gctgagaaaa aacgcaaatt agcgacgggt gatgagttaa cgacgggtgt 300
   cttgaaagtc gttaaagtct accttgcagt taaacgtcgt atccagccag 350
<210> 49
   <211> 350
   <212> DNA
   <213> acinetobacter baylyi
<400> 49
   agacttgaaa gaagaattca aaatcttcga agaagctgct cgtgaacgta ttatccgttt      60
   gttaaaaggc caagagtcaa atggcggcgg tacgaccaag cgcggtgata agctatctga     120
   agatgtattg tctggtttag agcttgttga tcttttagaa gttcaaccaa cagacgaagg     180
   catcgctgaa cgcttaactc aaattcaagt gttcttgaaa gagaagagct acgagattga     240
   tgagaaattt gctgagaaaa aacgcaaact ttctacaggt gatgagctta caacaggtgt     300
   attgaaagtt gttaaagttt acttagctgt aaaacgtcgt atccagcctg                350
<210> 50
   <211> 350
   <212> DNA
   <213> acinetobacter bouvetii
<400> 50
   agacttgaaa gaagaataca aaatcttcga agaagctgcg cgtgaacgta ttgtgcgctt      60
   gctaaaaggt caagaatcga atggcggcgg cacaactaaa cgcggtgaca aactttctga     120
   agaagtattg tctggtttag agcttgctga tctgcttgaa attcagccta cagacgaagg     180
   cattgctgag cgcttaactc aaattcaagt gttcttgaaa gagaagagca ctgaaattga     240
   tgagaaattt gctgagaaaa aacgcaaact ttctacaggc gatgagctta caactggtgt     300
   attgaaagtt gttaaagttt acttagctgt aaaacgccgc atccagccgg                350
<210> 51
   <211> 350
   <212> DNA
   <213> acinetobacter gerneri
<400> 51
   agatttgaaa gaagaatata aaatctttga agaagctgct cgtgaacgta ttgttcgctt      60
   gttgaaaggt caagaatcaa atggtggtgg ttcaactaaa cgtggtgaca aactttctga     120
   agaattgtta tctggtttag agctagttga tcttcttgaa attcaaccaa gtgatgaagg     180
   tattgctgaa cgtttaactc aaattcaagt gttcttgaaa gaaaagagcc atgaaattga     240
   tgagaaattt gctgagaaaa aacgcaaact ttctacaggt gatgagctta caactggtgt     300
   attgaaagtt gttaaagtgt atttggctgt taaacgtcgt atccaaccgg                350
<210> 52
   <211> 350
   <212> DNA
   <213> acinetobacter grimontii
<400> 52
   agacttgaaa gaagaataca aaatcttcga agaagcagca cgtgagcgta ttgttcgttt     60
   gttgaaaggt caagaatcta acggtggtgg ttcgactaaa cgtggtgaga agctttcaga    120
   agatatgttg tctggtctag agttagttga tctacttgaa atccaaccaa cagatgaagc    180
   aattgctgag cgtttaactc aaattcaagt gttcttgaaa gaaaagagcc atgaaattga    240
   tgaaaagttt gctgagaaga aacgtaaact ttctacaggt gatgagttaa caactggtgt    300
   attgaaagtt gttaaggttt acctagcagt taaacgtcgt atccaacctg               350
<210> 53
   <211> 350
   <212> DNA
   <213> acinetobacter tandoii
<400> 53
   agacttgaaa gaagaatata aaatcttcga agaagcagca cgtgaacgta ttgttcgctt     60
   gttgagcggt caagaatcga atggcggtgg cggcacgaag cgtggtgaca aactttcaga    120
   agatatgttg tctggcttag agttggttga tttacttgaa atccagccaa gtgatgaagc    180
   gattgctgaa cgtttaaccc aaattcaagt gttcttgaaa gagaagagct ttgaaattga    240
   cgagaaattt gctgagaaaa aacgcaaact ttctacaggt gatgaactaa caactggcgt    300
   attgaaagtt gtgaaggttt accttgcagt taaacgtcgt atccagcctg               350
<210> 54
   <211> 350
   <212> DNA
   <213> acinetobacter tjernbergiae
<400> 54
   agatttgaaa gaagaataca aaatcttcga agaagcagca cgtgaacgta ttgttcgttt     60
   gttgacaggt caagagtcta acggtggtgg ctcaactaag cgtggtgata aactttctgc    120
   agatgtcttg tctggtttag agctggttga tttacttgaa attcaaccga ctgatgaagc    180
   aattgcagag cgtttaactc agattcaagt gttcttgaaa gagaagagct acgaaattga    240
   cgagaagttt gcagagaaga aacgtaaact ttctacaggt gatgaattaa caacgggtgt    300
   attgaaagtt gttaaggttt acctcgctgt taaacgtcgt atccagcctg               350
<210> 55
   <211> 350
   <212> DNA
   <213> acinetobacter towneri
<400> 55
   agacttgaaa gaagaataca aaatctttga agaagctgca cgtgaacgta ttatccgctt     60
   attgaaaggc caagagtcta acggtggcgg tacaaccaaa cgtggcgaca agctttctga    120
   agatatgttg tcaggcttgg ctttagtgga tttacttgaa attcaaccaa gtgatgaagc    180
   gattgctgaa cgcttaacgc aaattcaaac cttcttgaaa gagaagagct ttgaaattga    240
   tgagaaattt gctgagaaga aacgtaaact ttctacaggc gatgagctca ctacaggcgt    300
   gttgaaagtt gttaaggtgt acttggctgt taaacgtcgc atccaaccgg               350
<210> 56
   <211> 350
   <212> DNA
   <213> acinetobacter parvus
<400> 56
   agacttgaaa gaagaataca aaatcttcga agaagcagca cgtgaacgta ttgttcgttt     60
   gttgaaaggt caagaatcta atggtggtgg ttcaaccaaa cgcggtgata aactttctgc    120
   agaggtattg tctggtttag agttggttga tttacttgaa atccagccaa atgatgaagc    180
   aattgctgag cgtttaactc aaattcaagt gttcttgaaa gagaagagtt acgagattga    240
   cgagaagttt gctgagaaga agcgtaaact ttctacaggt gatgaattaa caactggcgt    300
   attgaaagtt gttaaggttt acctggcagt taaacgtcgt atccagcctg               350
<210> 57
   <211> 350
   <212> DNA
   <213> Acinetobacter baumannii
<400> 57
   agatttgaaa gaagaataca agatcttcga agaagcggct cgtgagcgtg taattcgttt     60
   gcttaaaggc caagagtcta atggcggtgg ttcaactaaa cgtggtgata aactttctga    120
   agatttatta tctggtttag agcttgttga cttacttgaa attcaaccag cagatgaagc    180
   aatcgctgag cgtttaactc aaattcaagt gttcttgaaa gagaagagcg cagaaatcga    240
   tgagaaattc gctgagaaga aacgtaagct tgcaacaggt gatgaattaa caactggtgt    300
   attaaaagtt gttaaagttt acttagctgt taaacgtcgt attcagcctg               350
<210> 58
   <211> 350
   <212> DNA
   <213> Acinetobacter baumannii
<400> 58
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaggttt acttagctgt taaacgtcgt attcagcctg 350
<210> 59
   <211> 350
   <212> DNA
   <213> Acinetobacter baumannii
<400> 59
   agacttgaaa gaagaataca agatcttcga agaagcggct cgtgagcgtg taatccgttt 60
   acttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaagttt acttagctgt taaacgtcgt attcagcctg 350
<210> 60
   <211> 350
   <212> DNA
   <213> Acinetobacter baumannii
<400> 60
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaagttt acttagctgt taaacgtcgt attcagcctg 350
<210> 61
   <211> 350
   <212> DNA
   <213> Acinetobacter baumannii
<400> 61
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaggttt acttagctgt taaacgtcgt attcagcctg 350
<210> 62
   <211> 350
   <212> DNA
   <213> Acinetobacter baumannii
<400> 62
   agacttgaaa gaagaataca agatcttcga agaagcggct cgtgagcgtg taatccgttt 60
   acttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaagttt acttagctgt taaacgtcgt attcagcctg 350
<210> 63
   <211> 350
   <212> DNA
   <213> Acinetobacter baumannii
<400> 63
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga tttacttgaa attcaaccag cagatgaagc 180
   aatcgctgag cgtttaactc aaattcaagt gttcttgaaa gagaagagcg cagacatcga 240
   tgagaaattc gctgagaaga aacgtaagct tgcaacaggt gatgaattaa cgactggcgt 300
   attaaaagtt gttaaggttt acttagctgt taaacgtcgt attcagcctg 350
<210> 64
   <211> 350
   <212> DNA
   <213> Acinetobacter baumannii
<400> 64
   agacttgaaa gaagaataca agatcttcga agaagcggct cgtgagcgtg taattcgttt      60
   gcttaaaggc caagagtcta atggcggtgg ttcaactaaa cgtggtgata aactttctga     120
   agatttatta tctggtttag agcttgttga cttacttgaa attcaaccag cagatgaagc     180
   aatcgctgag cgtttaactc aaattcaagt gttcttgaaa gagaagagcg cagaaatcga     240
   tgagaaattc gctgagaaga aacgtaagct tgcaacaggt gatgaattaa caactggcgt     300
   attaaaagtt gttaaagttt acttagctgt taaacgtcgt attcagcctg                350
<210> 65
   <211> 350
   <212> DNA
   <213> Acinetobacter baumannii
<400> 65
   agacttgaaa gaagaataca agatcttcga agaagcggct cgtgagcgtg taatccgttt      60
   acttaaaggc caagagtcta atggcggtgg ttcaactaaa cgtggtgata aactttctga     120
   agatttatta tctggtttag agcttgttga cttacttgaa attcaaccag cagatgaagc     180
   aatcgctgag cgtttaactc aaattcaagt gttcttgaaa gagaagagcg cagaaatcga     240
   tgagaaattc gctgagaaga aacgtaagct tgcaacaggt gatgaattaa caactggcgt     300
   attaaaagtt gttaaagttt acttagctgt taaacgtcgt attcagcctg                350
<210> 66
   <211> 350
   <212> DNA
   <213> Acinetobacter baumannii
<400> 66
   agacttgaaa gaagaataca agatcttcga agaagcggct cgtgagcgtg taattcgttt      60
   gcttaaaggc caagagtcta atggcggtgg ttcaactaaa cgtggtgata aactttctga     120
   agatttatta tctggtttag agcttgttga cttacttgaa attcaaccag cagatgaagc     180
   aatcgctgag cgtttaactc aaattcaagt gttcttgaaa gagaagagcg cagaaatcga     240
   tgagaaattc gctgagaaga aacgtaagct tgcaacaggt gatgaattaa caactggtgt     300
   attaaaagtt gttaaagttt acttagctgt taaacgtcgt attcagcctg                350
<210> 67
   <211> 350
   <212> DNA
   <213> Acinetobacter baumannii
<400> 67
   agacttgaaa gaagaataca agatcttcga agaagcggct cgtgagcgtg taattcgttt     60
   gcttaaaggc caagagtcta atggcggtgg ttcaactaaa cgtggtgata aactttctga    120
   agatttatta tctggtttag agcttgttga cttacttgaa attcaaccag cagatgaagc    180
   aatcgctgag cgtttaactc aaattcaagt gttcttgaaa gagaagagcg cagaaatcga    240
   tgagaaattc gctgagaaga aacgtaagct tgcaacaggt gatgaattaa caactggtgt    300
   attaaaagtt gttaaagttt acttagctgt taaacgtcgt attcagcctg              350
<210> 68
   <211> 350
   <212> DNA
   <213> Acinetobacter baumannii
<400> 68
   agacttgaaa gaagaataca agatcttcga agaagcggct cgtgagcgtg taattcgttt     60
   gcttaaaggc caagagtcta atggcggtgg ttcaactaaa cgtggtgata aactttctga    120
   agatttatta tctggtttag agcttgttga cttacttgaa attcaaccag cagatgaagc    180
   aatcgctgag cgtttaactc aaattcaagt gttcttgaaa gagaagagcg cagaaatcga    240
   tgagaaattc gctgagaaga aacgtaagct tgcaacaggt gatgaattaa caactggcgt    300
   attgaaagtt gttaaagttt acttagctgt taaacgtcgt attcagcctg              350
<210> 69
   <211> 350
   <212> DNA
   <213> Acinetobacter baumannii
<400> 69
   agacttgaaa gaagaataca agatcttcga agaagcggct cgtgagcgtg taattcgttt     60
   gcttaaaggc caagagtcta atggcggtgg ttcaactaaa cgtggtgata aactttctga    120
   agatttatta tctggtttag agcttgttga tttacttgaa attcaaccag cagatgaagc    180
   aatcgctgag cgtttaactc aaattcaagt gttcttgaaa gagaagagcg cagaaatcga    240
   tgagaaattc gctgagaaga aacgtaagct tgcaacaggt gatgaattaa cgactggcgt    300
   attaaaagtt gttaaggttt acttagctgt taaacgtcgt attcagcctg              350
<210> 70
   <211> 350
   <212> DNA
   <213> Acinetobacter baumannii
<400> 70
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggtgt 300
   attgaaagtt gttaaagttt acttagctgt taaacgtcgt attcagcctg 350
<210> 71
   <211> 350
   <212> DNA
   <213> Acinetobacter baumannii
<400> 71
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga tttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgccacaggt gatgaattaa cgactggcgt 300
   attaaaagtt gttaaggttt acttagctgt taaacgtcgt attcagcctg 350
<210> 72
   <211> 350
   <212> DNA
   <213> Acinetobacter baumannii
<400> 72
   agacttgaaa gaagaataca agatcttcga agaagcggct cgtgagcgtg taatccgttt 60
   acttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaagttt acttagctgt taaacgtcgt attcagcctg 350
<210> 73
   <211> 350
   <212> DNA
   <213> Acinetobacter baumannii
<400> 73
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggtgt 300
   attgaaagtt gttaaagttt acttagctgt taaacgtcgt attcagcctg 350
<210> 74
   <211> 350
   <212> DNA
   <213> Acinetobacter baumannii
<400> 74
   agacttgaaa gaagaataca agatcttcga agaagcggct cgtgagcgtg taattcgttt 60
   gcttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttaaaa gagaagagcg cagaaatcga 240
   tgagaaattc gctgagaaga aacgtaagct tgcaacaggt gatgaattaa caactggcgt 300
   attgaaagtt gttaaagttt acttagctgt taaacgtcgt attcagcctg 350
<210> 75
   <211> 350
   <212> DNA
   <213> Acinetobacter baumannii
<400> 75
   agacttgaaa gaagagtata aaatcttcga agaagcggct cgtgaacgtg taattcgttt 60
   acttaaaggt caagagtcta atggcggtgg ttcaactaaa cgtggcgata aactttctga 120
   agatttattg tctggcttag agcttgttga tttacttgaa attcaaccga cagatgaagc 180
   aatcgctgag cgtttaactc agattcaagt gttcttgaaa gagaagagcg cagaaattga 240
   tgagaaattc gctgagaaga aacgtaagct tgcaacaggt gatgagttaa caactggcgt 300
   attgaaagtt gttaaagttt acttagctgt taaacgtcgt attcagcctg 350
<210> 76
   <211> 350
   <212> DNA
   <213> Acinetobacter baumannii
<400> 76
   agacttgaaa gaagaataca agatcttcga agaagcggct cgtgagcgtg taatccgttt 60
   acttaaaggc caagagtcta atggcggtgg ttcaactaaa cgtggtgata aactttctga 120
   agatttatta tctggtttag agcttgttga cttacttgaa attcaaccag cagatgaagc 180
   aatcgctgag cgtttaactc aaattcaagt gttcttgaaa gagaagagcg cagaaatcga 240
   tgagaaattc gctgagaaga aacgtaagct tgcaacaggt gatgaattaa caactggcgt 300
   attaaaagtt gttaaagttt acttagctgt taaacgtcgt attcagcctg 350
<210> 77
   <211> 450
   <212> DNA
   <213> Acinetobacter calcoaceticus
<400> 77
   gtgataaaat ggctggtcgt cacggtaaca aaggtgttgt atctaacatt ttacctgttg 60
   aagacatgcc acacgatgct aacggtgtgc cggtagatat cgtattgaac ccattgggtg 120
   taccatctcg tatgaacgtg ggtcagattc ttgagactca cttgggtatg gcagctaaag 180
   ggcttggtga taaaatcgaa aaaatgttga aagaacagcg cacagtttta gaacttcgcg 240
   aattcttaga caagatttat aacaaagtcg gcggcgagca agaagatctt gatagcttaa 300
   ctgatgctga agtcttagca ctttcaggca acttacgtgc tggtgtgcct ttagctactc 360
   ctgtatttga tggtgctgaa gaaagtcaaa ttaaagactt acttgaatta gctgatattt 420
   cgcgtactgg tcaaacagta ttgtttgatg 450
<210> 78
   <211> 450
   <212> DNA
   <213> Acinetobacter baumannii
<400> 78
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgacg 450
<210> 79
   <211> 450
   <212> DNA
   <213> Acinetobacter sp.
<400> 79
   gtgataagat ggctggtcgt cacggtaaca agggtgttgt atctaacatc ttacctgttg    60
   aagacatgcc acacgatgct aacggtgtac cagtagatat cgtattgaac ccgttgggcg   120
   taccatctcg tatgaacgtg ggtcagattc ttgagactca cttaggtatg gcggctaaag   180
   ggcttggtga taaaatcgag aaaatgttga aagaacagcg tacagtttta gaactgcgtg   240
   aattcttaga caagatttat aacaaagtcg gtggtgagca agaagatctt gatagcttaa   300
   ctgatgctga agtcttggca ctttcaggca acttacgtgc tggtgtacct ttggctactc   360
   ctgtattcga tggtgctgaa gaaagccaaa ttaaagactt gcttgagtta gctggtatct   420
   ctcgtacagg tcaaacagta ttgtttgatg                                    450
<210> 80
   <211> 450
   <212> DNA
   <213> Acinetobacter haemolyticus
<400> 80
   gtgataagat ggcgggtcgt cacggtaaca agggtgtggt atcaaatatc ttacctgtag    60
   aagacatgcc gcatgatatt cacggtgtgc cagttgatat cgtattgaac ccattgggtg   120
   taccatcacg tatgaacgtg ggtcagattc ttgaaactca cttgggtatg gcggcaaaag   180
   gtctgggtga gcaaattgat aagatgctcc aacagcaacg tacgattgct gaattgcgtg   240
   cgttcctcga caagatttac aacaaagttg gtggcgaaca agaagatctt gatagcttaa   300
   ccgacgaaga agttttaaaa cttgcaggta acctgcgtgc aggtgtgcct ttggcgacac   360
   cagtatttga tggtgctgaa gaaagtcaaa ttaaagagtt acttgagctt gctgaattgc   420
   cacgtactgg tcaaaccgta ttgtttgatg                                    450
<210> 81
   <211> 450
   <212> DNA
   <213> Acinetobacter junii
<400> 81
   gtgataagat ggcgggtcgt cacggtaaca agggtgttgt atcaaacatc ttaccagttg    60
   aagatatgcc acatgatgcc aatggtgtgc cagttgatat cgtattgaac ccactcggtg   120
   taccatcgcg tatgaacgtg ggtcagattc ttgaaactca cttaggtatg gcagcaaaag   180
   gtttgggtga gcagattgat aaaatgctca aacaacaacg tacaattgcc gagttacgtt   240
   cattccttga caagatttat aataaagtgg gtggtgagca agaacagctt gacacactaa   300
   ctgacgaaga gatctttaag cttgcaggta atttacgtgc tggtgtgcct ttggcaactc   360
   cagtatttga tggtgctgaa gagtcacaaa tcaaagagtt acttgagctt gcagagttac   420
   cacgttctgg tcaacaaatc ttgtttgatg                                    450
<210> 82
   <211> 450
   <212> DNA
   <213> Acinetobacter sp.
<400> 82
   gtgataagat ggcgggtcgt cacgggaaca agggtgttgt ctctaacatc ttacctgttg      60
   aagatatgcc acatgatgcc aatggtgtac ctgttgacat cgtattgaac ccattgggtg     120
   taccgtcgcg tatgaacgtg ggtcagattc ttgaaaccca tttgggcttg gcggcaaaag     180
   gtttgggtga gcagatcgat aagatgctgc aacaacaacg taccgttgct gaacttcgtt     240
   tgttccttga taagatttac aacaaagttg gtggcgagca agaagatctt gatagcttaa     300
   ctgatgaaga agtgttgaag cttgcaggta acttacgtgc aggtgttcct ttggcaacac     360
   cagtgtttga tggtgctgaa gaaagccaaa ttaaagaatt acttgaactt gctgaattgc     420
   cgcgttctgg tcaacagact ttgtttgatg                                      450
<210> 83
   <211> 450
   <212> DNA
   <213> Acinetobacter johnsonii
<400> 83
   gtgataagat ggcgggtcgt cacggtaaca agggtgttgt ttctaacatc ttgcctgttg      60
   aagacatgcc gcacgatgcc aatggtgttc cagtcgacat cgtattgaac ccactgggtg     120
   taccgtcacg tatgaacgtg ggtcagattc tagagactca cttaggtatg gcagcgaaag     180
   gtcttggcga agaaatcgac aagatgttaa aagcgcaacg tactgtactt gagcttcgtg     240
   gattcttaga caagatttat aacaaagttg gtggcgagca agaagatctt gatagcttaa     300
   ctgatgatga aattttggtg ctttcgggta acttgcgtgc gggtgttcct cttgcaacgc     360
   cagtattcga tggtgctgaa gaatctcaaa ttaaagactt gttagagctt gcgaacattt     420
   cacgtactgg tcaaacagta ttgtatgatg                                      450
<210> 84
   <211> 450
   <212> DNA
   <213> Acinetobacter lwoffii
<400> 84
   gtgataagat ggccggtcgt cacggtaaca agggtgttgt atcaaacatc ttgccggtag 60
   aagacatgcc acacgatgcc aatggtgtac ctgttgatat cgtactgaac ccgctgggcg 120
   taccatcgcg tatgaacgtg ggtcagattc ttgaaactca cttaggtatg gctgccaaag 180
   gtcttggcga tcaaatcgac aagatgatga aagaacagcg tactgtactt gagcttcgtg 240
   atttcctgga caagatttat aacaaagttg gtggcgagca agaagatctt gacagcttga 300
   ctgatgaaga aatcttgaaa ctttctggta acttgcgtgc tggtgtgcct ttggcaacac 360
   ctgtattcga tggtgctgaa gaaggtcaga tcaaagaatt gttacaactt gcaggtctat 420
   caagtactgg tcagacagta ttatatgatg 450
<210> 85
   <211> 450
   <212> DNA
   <213> Acinetobacter sp.
<400> 85
   gtgataagat ggcgggtcgt cacggtaaca aaggtgttgt atcaaacatc ttgccggtag 60
   aagacatgcc acatgatgcc aacggtgtac ctgttgatat cgtattgaac ccgcttggcg 120
   taccatcacg tatgaacgtg ggtcagattc ttgaaactca cttgggtatg gcggcgaaag 180
   gtcttggcga tcaaatcgac aagatgatga aagagcaacg tactgtactt gagcttcgtg 240
   atttcctgga caagatttac aataaagttg gtggcgagca agaagatctt gatagcttga 300
   ctgatgaaga aatcttgaaa ctttctggca acttgcgtgc tggtgtgcct ttggctactc 360
   ctgtattcga tggtgctgaa gaaggtcaga tcaaagagtt gttacaactt gcaggcctat 420
   ctagtactgg tcagaccgta ttatatgatg 450
<210> 86
   <211> 450
   <212> DNA
   <213> Acinetobacter sp.
<400> 86
   gtgataagat ggcgggtcgt cacggtaaca agggtgttgt atcaaacatc ttaccagttg 60
   aagatatgcc gcatgacatc catggtgttc cagtggatgt ggtacttaac ccactcggtg 120
   taccatcacg tatgaacgtg ggtcagattc ttgaaactca cttgggtatg gcagcgaaag 180
   gtcttggcga taagatcgac aagatgatga aagagcaacg tactgttctt gaacttcgtg 240
   aattcttaga caagatttat aacaaagttg gtggcgagca agaagatctt gatagcttga 300
   ctgatgaaga aatcttggtg ttatcaggta acttgcgtaa aggtgttcct ttagctacac 360
   cagtatttga tggtgcagaa gaaggacaaa tcaaagagtt acttgaactt ggtggtatct 420
   cacgtacagg tcaaacagta ttgtatgatg 450
<210> 87
   <211> 450
   <212> DNA
   <213> Acinetobacter sp.
<400> 87
   gtgataagat ggcgggtcgt cacggtaaca aaggtgttgt atcaaacatc ttgcctgttg    60
   aagacatgcc gcatgacatc catggtgttc cagttgatgt cgtacttaac ccattgggtg   120
   taccatcacg tatgaacgtg ggtcagattc ttgaaactca cttaggtatg gctgcaaaag   180
   gtcttggcga taagatcgac aagatgatga aagagcaacg taccgttctt gagcttcgtg   240
   atttcttaga caagatttat aacaaagttg gtggcgagca agaagatctt gatagcttaa   300
   ctgatgaaga aatcttggtg ttatcaggta acttgcgtaa aggtgttcct ttagctacgc   360
   cagtatttga tggtgcagaa gaaagtcaga tcaaagagtt acttgagctt ggtggtatct   420
   cacgtacagg tcaaacagta ttgtatgacg                                    450
<210> 88
   <211> 450
   <212> DNA
   <213> Acinetobacter radioresistens
<400> 88
   gtgataaaat ggcgggtcgt cacgggaaca agggtgtggt atcacaaatc ctgcctgtag    60
   aagacatgcc acatgatgcc aatggtgttc cggttgatgt ggtattaaac ccgctaggtg   120
   taccatcacg tatgaacgtg ggacagattc tggaaacaca tttgggtctt gctgcaaaag   180
   gtttaggtga gcagatcgac aagatgctta aacagcagcg tgctattgtt gaactgcgtg   240
   attttcttga taagatttac aataaagtcg gtggtgagca agaacagctt gatacactga   300
   ctgatgatga aatcttgaaa cttgcaggaa acctcagcaa gggtgtgcca ctggcaactc   360
   cagtatttga tggtgccgaa gaaggccaga tcaaagagtt acttgaactt gcagaactgc   420
   cacgttctgg ccagcagatc ctgtttgatg                                    450
<210> 89
   <211> 450
   <212> DNA
   <213> Acinetobacter sp.
<400> 89
   gtgataaaat ggcgggtcgt cacggtaaca aaggtgttgt gtcaaacatc ttgcctgttg 60
   aagacatgcc acacgatgcg aatggtgtac cagtcgatat cgtattgaac ccattgggtg 120
   taccgtcacg tatgaacgtg ggtcagattc ttgagactca cttaggtatg gcggccaaag 180
   gtcttggcga taaacttgaa aaaatgttga aagaacaacg tacagtgtta gaactacgtg 240
   acttcttaga caagatttat aacaaggtcg gtggtgagca agaagagctt gatagcttaa 300
   ctgatgccga aatcttggcg ctttcaggta acttacgtgc tggtgttcca ttagcaacac 360
   ctgtatttga tggtgctgaa gaaagccaga tcaaagactt acttgaatta gcagacatct 420
   cacgtacagg tcaaacggta ttgtttgacg 450
<210> 90
   <211> 450
   <212> DNA
   <213> Acinetobacter sp.
<400> 90
   gtgataagat ggcgggtcgt cacggtaaca agggtgttgt atcaaacatc ttaccggttg 60
   aagacatgcc acatgatgcg aatggcgtac cagtcgacat cgtattgaac ccactgggtg 120
   taccgtcacg tatgaacgtg ggtcagattc ttgagactca cttgggtatg gcagcgaaag 180
   gtcttggcga taaaatcgaa aaaatgttga aagaacaacg tacagtgatt gaactgcgtg 240
   aattcttaga caagatttat aacaaggttg gcggtgagca ggaagagctt gatagcttga 300
   ctgatgcaga aatcttggcg ctttcaggta acttacgtgc tggtgttcca ttggcaacac 360
   ctgtatttga tggtgctgaa gaaagccaga tcaaagacct acttgaactt gctgatatct 420
   cacgtactgg tcaaacggta ttgtttgacg 450
<210> 91
   <211> 450
   <212> DNA
   <213> acinetobacter schindleri
<400> 91
   gtgataagat ggcgggtcgt cacggtaaca agggtgttgt atctaacatc ttgccggtag 60
   aagacatgcc acacgatgcc aacggtgtac ctgttgatat cgttcttaac ccgctaggtg 120
   taccgtcacg tatgaacgtg ggtcagattc ttgagactca cttgggtatg gccgccaaag 180
   gtcttggcga caaaatcgac aagatgcttc aagagcaacg tacggtgctt gagcttcgtg 240
   aattcttaga caagatttac aacaaagttg gtggtgagca agaagatctt gatagcctga 300
   ctgatgatga aatcttggca ttgtctggta acttgcgtaa aggtgttcct ttggcaactc 360
   cagtattcga cggtgctgaa gaatcgcaaa tcaaagaatt gttagagctt ggtggcattt 420
   cacgtactgg tcaaacagta ttgtatgatg 450
<210> 92
   <211> 450
   <212> DNA
   <213> acinetobacter ursingii
<400> 92
   gtgataaaat ggcgggtcgt cacgggaaca aaggtgttgt ttctaacatc ttgcctgtag     60
   aagacatgcc acatgatgcc aatggtgtac ctgtcgatat cgtcttgaac ccattaggtg    120
   taccatcgcg tatgaacgtg ggtcagattc tggagacaca tctaggattg gcagccaaag    180
   gtctgggtga acaaatcgat aagatgttgc aacaacagcg taccattgcc gaacttcgta    240
   tcttccttga taagatttac aacaaggtcg gtggtgagca agaagatcta aacagtctga    300
   ctgatgatga agtcttggta ttggctggca acttgcgtaa aggtgtacca ctagcaactc    360
   ctgtatttga tggtgctgaa gaaagtcaaa ttaaagagtt acttgagttg gctgaattgc    420
   cacgtactgg tcaacagatt ttgtttgatg                                     450
<210> 93
   <211> 450
   <212> DNA
   <213> acinetobacter baylyi
<400> 93
   gtgataaaat ggcgggtcgt cacggtaaca aaggtgttgt atcaaacatc ttgcctgttg     60
   aagacatgcc gcatgatatc catggtgttc cagttgatgt cgtacttaac ccattgggtg    120
   taccatcacg tatgaacgtg ggtcagattc ttgaaactca cttaggtatg gctgcaaaag    180
   gtcttggcga taagatcgac aagatgatga aagagcaacg taccgttctt gagcttcgtg    240
   atttcttaga caagatttat aacaaagttg gtggcgagca agaagatctt gatagcttaa    300
   ctgatgaaga aatcttggtg ttatcaggta acttgcgtaa aggtgttcct ttagctacgc    360
   cagtatttga tggtgcagaa gaaagtcaga tcaaagagtt acttgagctt ggtggtatct    420
   cacgtacagg tcaaacagta ttgtatgacg                                     450
<210> 94
   <211> 450
   <212> DNA
   <213> acinetobacter bouvetii
<400> 94
   gtgataagat ggcgggtcgt cacggtaaca aaggtgttgt atctaacatc ttgcctgtag     60
   aagacatgcc gcacgatgcc aacggtgttc ctgtagacgt ggtgcttaac ccgctgggtg    120
   taccgtcacg tatgaacgtg ggtcagattc ttgagactca cttgggcatg gcagcgaaag    180
   gtcttggcga caaaatcgac aagatgatga aagagcagcg cactgttctt gaacttcgtg    240
   aattcttaga caagatttac aacaaagttg gcggcgagca agaagatctt gacagcttaa    300
   ctgatgatga aatcttggcg ctttcaggca acctgcgtgc aggtgttcct ttggcaacgc    360
   ctgtatttga cggtgctgaa gaatcacaaa ttaaagaatt gctagagctt ggcggcattt    420
   cacgtactgg tcaaacagta ttgtatgatg                                     450
<210> 95
   <211> 450
   <212> DNA
   <213> acinetobacter gerneri
<400> 95
   gtgataaaat ggcgggtcgt cacgggaaca aaggtgttgt atcaaacatc cttcctgtag     60
   aagatatgcc gcatgacatc aacggtgttc ctgttgacgt agtacttaac ccactgggtg    120
   taccgtcacg tatgaacgtg ggtcagattc ttgaaacaca tttaggttta gctgccaaag    180
   gtcttggtga gcaaatcgat aagatgctca aagagcaacg tacgattgct gaacttcgtg    240
   tgttcttgga caagatttat aacaaagttg gtggcgagca agaagatctt gatagcttaa    300
   ctgatgaaga aatccttgtt ctttcaggta atttacgtaa aggtgttcct ttagcaactc    360
   cagtatttga tggtgctgaa gaaggtcaaa ttaaagagct tcttgaactt gctgaacttc    420
   cacgttctgg tcaaacagta ttgtatgacg                                     450
<210> 96
   <211> 450
   <212> DNA
   <213> acinetobacter grimontii
<400> 96
   gtgataagat ggcgggtcgt cacggtaaca agggtgttgt atcaaacatc ttaccagttg     60
   aagacatgcc acatgatgcc aatggtgtgc cagttgatat cgtattgaac ccactcggtg    120
   taccatcgcg tatgaacgtg ggtcagattc ttgaaactca cttaggtatg gcagcaaaag    180
   gtttgggtga gcagattgat aaaatgctca aacaacaacg tacaattgcc gagttacgtt    240
   cattccttga caagatttat aataaagtgg gtggtgagca agaacagctt gacacactga    300
   ctgatgaaga gatcttgaaa ctttcaggta atttacgtgc tggtgtgcct ttggcaactc    360
   cagtattcga tggtgctgaa gagtcacaaa tcaaagagtt acttgaactt gcagagttac    420
   cacgttctgg tcaacagatc ttgtttgatg                                     450
<210> 97
   <211> 450
   <212> DNA
   <213> acinetobacter tandoii
<400> 97
   gtgataagat ggcgggtcgt cacggtaaca aaggtgttgt gtctaacatc ctaccagtcg     60
   aagacatgcc gcatgatgcc aatggtgttc cagtcgatat cgtattgaac ccgttgggtg    120
   taccgtcacg tatgaacgtg gggcagattc ttgaaactca cttgggtatg gctgcgaaag    180
   gtttgggtga gcaaattgat aagatgctca aacaacagcg tgaaattgct gaactacgtg    240
   ttttcctaga caaaatctac aacaaagtgg gcggtcagca agaagattta gacagcttaa    300
   cagatgatga aatcttggtg ttggcaggta acttacgtgc aggtgtacct ttagcaactc    360
   ctgtatttga tggtgctgaa gaaagccaaa tcaaagagtt actagagctg gctgaaattc    420
   cacgttcggg tcaaaccgta ttgtatgatg                                      450
<210> 98
   <211> 450
   <212> DNA
   <213> acinetobacter tjernbergiae
<400> 98
   gtgataagat ggcgggtcgt catggtaaca aaggtgttgt atctaacatc ttacctgttg     60
   aagacatgcc tcatgatgcg aatggtgtgc cagtcgatat cgtattgaac ccattgggtg    120
   taccgtcacg tatgaacgtg ggtcagattc ttgagactca cttaggtatg gcggcgaaag    180
   gtcttggcga taaaatcgaa aaaatgttga aagaacagcg tacagtgatt gaactgcgtg    240
   aattcttaga caagatttat aacaaggtcg gtggtgagca agaagagctt gacagcttaa    300
   ctgatgcgga agtcttggca ctttcaggca acttacgtgc tggtgttcca ttggcaacgc    360
   ctgtatttga cggtgctgaa gaaagtcaga ttaaagactt acttgaattg gcagacatct    420
   cacgtacggg tcaaacggta ttgtttgacg                                      450
<210> 99
   <211> 450
   <212> DNA
   <213> acinetobacter towneri
<400> 99
   gtgataagat ggcgggtcgt cacggtaaca aaggtgttgt atctaacatc ttgccagtag 60
   aagacatgcc gcacgatgct aacggtgtac ctgttgacat cgtattgaac ccactaggtg 120
   taccatctcg tatgaacgtg ggtcagattc ttgaaacaca cttgggtatg gcagccaaag 180
   gtttgggtga gcaaattgac aagatgctca aacaacaacg tgagattgct gaactacgtg 240
   cgttcctaga caagatttat aacaaagtgg gtggcgagca agaagatctt gacagcttaa 300
   ctgatgatga aatcttggta ttggcgggta acttgcgtgc aggtgttcca ttggctactc 360
   ctgtatttga tggtgctgaa gaaggtcaaa tcaaagaatt gcttgaactg gctgaattac 420
   cacgttcagg tcagaccgta ttgtatgatg 450
<210> 100
   <211> 450
   <212> DNA
   <213> acinetobacter parvus
<400> 100
   gtgataagat ggcgggtcgt cacggtaaca aaggtgttgt atcaaacatc ttgccagttg 60
   aagacatgcc acatgatgcg aatggtgtgc cagtcgacat cgtattgaac ccacttggtg 120
   taccgtcacg tatgaacgtg ggtcagattc ttgagactca cttgggtatg gcggcgaaag 180
   gtcttggcga taagatcgaa aaaatgttga aagaacaacg tacagtgatt gaactgcgtg 240
   aattcttaga caagatttat aacaaggttg gtggtgagca ggaagagctt gatagcttga 300
   ctgacgcaga aatcttggcg ctttcaggta acttacgtgc tggtgttcca ttagcgactc 360
   ctgtatttga tggtgctgaa gaaagccaga tcaaagactt acttgaattg gcagacattt 420
   ctcgtacagg tcaaacagta ttgtttgatg 450
<210> 101
   <211> 450
   <212> DNA
   <213> Acinetobacter baumannii
<400> 101
   gtgataagat ggctggtcgt cacggtaaca aaggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgatg 450
<210> 102
   <211> 450
   <212> DNA
   <213> Acinetobacter baumannii
<400> 102
   gtgataagat ggctggtcgt cacggtaaca aaggtgttgt atctaacatc ttacctgttg     60
   aagacatgcc acatgatgct aacggtgtgc cagtagatat cgtattgaac ccgctgggtg    120
   taccatctcg tatgaacgtg ggtcagattc tagagactca cttgggtatg gcggctaaag    180
   ggcttggtga caaaatcgaa aaaatgttga aagaacaacg tacagtttta gaactgcgcg    240
   aattcttaga caagatttat aacaaagtcg gtggtgagca agaagatctt gatagcttga    300
   ctgatgaaga aattctagcg cttgcaggta acttgcgtgc gggtgtgcct ttagctactc    360
   ctgtatttga tggtgctgaa gaaagtcaaa ttaaagactt acttgaattg gctgacattt    420
   cacgtacggg tcaaacagta ttgtttgacg                                     450
<210> 103
   <211> 450
   <212> DNA
   <213> Acinetobacter baumannii
<400> 103
   gtgataagat ggctggtcgt cacggtaaca agggtgttgt atctaacatc ttacctgttg     60
   aagacatgcc acatgatgct aacggtgtgc cggtagatat cgtattgaac ccgctgggtg    120
   taccatctcg tatgaacgtg ggtcagattc tagagactca cttgggtatg gcggctaaag    180
   ggcttggtga caaaatcgaa aaaatgttga aagaacaacg tacagtttta gaactgcgcg    240
   aattcttaga caagatttat aacaaagtcg gcggtgagca agaagatctt gatagcttga    300
   ctgatgaaga aattctagcg cttgcaggta acttgcgtgc gggtgtgcct ttagctactc    360
   ctgtatttga tggtgctgaa gaaagtcaaa ttaaagactt acttgaattg gctgacattt    420
   cacgtactgg tcaaacagta ttgtttgacg                                     450
<210> 104
   <211> 450
   <212> DNA
   <213> Acinetobacter baumannii
<400> 104
   gtgataagat ggctggtcgt cacggtaaca agggtgttgt atctaacatc ttacctgttg 60
   aagacatgcc acatgatgct aacggtgtgc cggtagatat cgtattgaac ccgctaggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgacg 450
<210> 105
   <211> 450
   <212> DNA
   <213> Acinetobacter baumannii
<400> 105
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgacg 450
<210> 106
   <211> 450
   <212> DNA
   <213> Acinetobacter baumannii
<400> 106
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtactgg tcaaacagta ttgtttgacg 450
<210> 107
   <211> 450
   <212> DNA
   <213> Acinetobacter baumannii
<400> 107
   gtgataagat ggctggtcgt cacggtaaca agggtgttgt atctaacatc ttacctgttg     60
   aagacatgcc acatgatgct aacggtgtgc cggtagatat cgtattgaac ccgctgggtg    120
   taccatctcg tatgaacgtg ggtcagattc tagagactca cttgggtatg gcggctaaag    180
   ggcttggtga caaaatcgaa aaaatgttga aagaacaacg tacagtttta gaactgcgcg    240
   aattcttaga caagatttat aacaaagtcg gcggtgagca agaagatctt gatagcttga    300
   ctgatgaaga aattctagcg cttgcaggta acttgcgtgc gggtgtgcct ttagctactc    360
   ctgtatttga tggtgctgaa gaaagtcaaa ttaaagactt acttgaattg gctgacattt    420
   cacgtacggg tcaaacagta ttgtttgacg                                     450
<210> 108
   <211> 450
   <212> DNA
   <213> Acinetobacter baumannii
<400> 108
   gtgataagat ggctggtcgt cacggtaaca agggtgttgt atctaacatc ttacctgttg     60
   aagacatgcc acatgatgct aacggtgtgc cggtagatat cgtattgaac ccgctaggtg    120
   taccatctcg tatgaacgtg ggtcagattc tagagactca cttgggtatg gcggctaaag    180
   ggcttggtga caaaatcgaa aaaatgttga aagaacaacg tacagtctta gaactgcgcg    240
   aattcttaga caagatttat aacaaagtcg gcggtgagca agaagatctt gatagcttga    300
   ctgatgaaga aattctagcg cttgcaggta acttgcgtgc gggtgtgcct ttagctactc    360
   ctgtatttga tggtgctgaa gaaagtcaaa ttaaagactt acttgaattg gctgacattt    420
   cacgtacggg tcaaacagta ttgtttgatg                                     450
<210> 109
   <211> 450
   <212> DNA
   <213> Acinetobacter baumannii
<400> 109
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcgggta acttgcgtgc gggtgtgcct ttagctactc 360
   ctgtatttga tggtgctgaa gaaagtcaaa ttaaagactt acttgaattg gctgacattt 420
   cacgtactgg tcaaacagta ttgtttgacg 450
<210> 110
   <211> 450
   <212> DNA
   <213> Acinetobacter baumannii
<400> 110
   gtgataagat ggctggtcgt cacggtaaca aaggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtag gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgatg 450
<210> 111
   <211> 450
   <212> DNA
   <213> Acinetobacter baumannii
<400> 111
   gtgataagat ggctggtcgt cacggtaaca aaggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtag gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgatg 450
<210> 112
   <211> 450
   <212> DNA
   <213> Acinetobacter baumannii
<400> 112
   gtgataagat ggctggtcgt cacggtaaca aaggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gtggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtactgg tcaaacagta ttgtttgacg 450
<210> 113
   <211> 450
   <212> DNA
   <213> Acinetobacter baumannii
<400> 113
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgatg 450
<210> 114
   <211> 450
   <212> DNA
   <213> Acinetobacter baumannii
<400> 114
   gtgataagat ggctggtcgt cacggtaaca aaggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagattt acttgaattg gctgacattt 420
   cacgtacggg tcaaacagta ttgtttgatg 450
<210> 115
   <211> 450
   <212> DNA
   <213> Acinetobacter baumannii
<400> 115
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtacggg tcaaacagta ttgtttgacg 450
<210> 116
   <211> 450
   <212> DNA
   <213> Acinetobacter baumannii
<400> 116
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtactgg tcaaacagta ttgtttgacg 450
<210> 117
   <211> 450
   <212> DNA
   <213> Acinetobacter baumannii
<400> 117
   gtgataagat ggctggtcgt cacggtaaca aaggtgttgt atctaacatc ttacctgttg     60
   aagacatgcc acatgatgct aacggtgtgc cggtagatat cgtattgaac ccgctgggtg    120
   taccatctcg tatgaacgtg ggtcagattc tagagactca cttgggtatg gcggctaaag    180
   ggcttggtga caaaatcgaa aaaatgttga aagaacaacg tacagtttta gaactgcgcg    240
   aattcttaga caagatttat aacaaagtcg gcggtgagca agaagatctt gatagcttga    300
   ctgatgaaga aattctagcg cttgcaggta acttgcgtgc gggtgtgcct ttagctactc    360
   ctgtatttga tggtgctgaa gaaagtcaaa ttaaagactt acttgaattg gctgacattt    420
   cacgtacggg tcaaacagta ttgtttgacg                                     450
<210> 118
   <211> 450
   <212> DNA
   <213> Acinetobacter baumannii
<400> 118
   gtgataagat ggctggtcgt cacggtaaca aaggtgttgt atctaacatc ttacctgttg     60
   aagacatgcc acatgatgct aacggtgtgc cggtagatat cgtattgaac ccgctgggtg    120
   taccatctcg tatgaacgtg ggtcagattc tagagactca cttgggtatg gcggctaaag    180
   ggcttggtga caaaatcgaa aaaatgttga aagaacaacg tacagtttta gaactgcgcg    240
   aattcttaga caagatttat aacaaagtcg gcggtgagca agaagatctt gatagcttga    300
   ctgatgaaga aattctagcg cttgcaggta acttgcgtgc gggtgtgcct ttagctactc    360
   ctgtatttga tggtgctgaa gaaagtcaaa ttaaagactt acttgaattg gctgacattt    420
   cacgtacggg tcaaacagta ttgtttgatg                                     450
<210> 119
   <211> 450
   <212> DNA
   <213> Acinetobacter baumannii
<400> 119
   gtgataagat ggctggtcgt catggtaaca aaggtgttgt ttctaacatc ttacctgttg 60
   aagacatgcc acacgatgcg aacggtgtac ctgtagatat cgtattgaac ccgttgggcg 120
   taccatctcg tatgaacgtg ggtcagattc tcgaaactca cttgggtatg gcggctaaag 180
   ggcttggtga caaaatcgaa aaaatgttga aagaacagcg tacagtttta gaactacgtg 240
   aattcttaga caagatttat aacaaagtcg gtggcgagca agaagatctt gatagcttga 300
   ctgatgatga aatcctagca ctttcaggca acttgcgtgc tggtgttcct ttggctactc 360
   ctgtatttga tggtgctgaa gaaagtcaaa ttaaagattt acttgagttg gctgacattt 420
   cacgtacagg tcaaacagta ttgtttgatg 450
<210> 120
   <211> 450
   <212> DNA
   <213> Acinetobacter baumannii
<400> 120
   gtgataagat ggctggtcgt cacggtaaca agggtgttgt atctaacatc ttacctgttg 60
   aagacatgcc acatgatgct aacggtgtgc cggtagatat cgtattgaac ccgctgggtg 120
   taccatctcg tatgaacgtg ggtcagattc tagagactca cttgggtatg gcggctaaag 180
   ggcttggtga caaaatcgaa aaaatgttga aagaacaacg tacagtttta gaactgcgcg 240
   aattcttaga caagatttat aacaaagtcg gcggtgagca agaagatctt gatagcttga 300
   ctgatgaaga aattctagcg cttgcaggta acttgcgtgc gggtgtgcct ttagctactc 360
   ctgtatttga tggtgctgaa gaaagtcaaa ttaaagactt acttgaattg gctgacattt 420
   cacgtactgg tcaaacagta ttgtttgacg 450
<210> 121
   <211> 305
   <212> DNA
   <213> Acinetobacter calcoaceticus
<400> 121
   ttgtaaaagg agtcggtttt taaatcggct ccataaaatg gctgatggct cttgggtcat 60
   cagccttttt gcgttacaat aatcggctcg atttttgttt gttctgtaca ataattgtcg 120
   tgttttagaa taatcagagt aatttcaaat gtttaccaat atttttcttt ttaaaaaata 180
   ttgttaagcg ttttaatacc actaaaaatt gcagcatttg taaacagtgg tggccatatc 240
   ggcctgtgta attccttctg agcccgttct gcaggcgggc ttggtttact ttccgaggac 300
   tccag 305
<210> 122
   <211> 305
   <212> DNA
   <213> Acinetobacter baumannii
<400> 122
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 123
   <211> 304
   <212> DNA
   <213> Acinetobacter sp.
<400> 123
   ttgtaaaagg agtcggtttt taaatcggct ccataaaatg gctgatggct cttgggtcat 60
   cagccttttt gcgttacaat aatcggctcg atttttgtta gttttgtttg tgttttagga 120
   aaattcaaat aaaacaagta atttcaaatg tttaccaata tttttctttt ttaaaaatat 180
   tgttaagcgt ttttatacca ctaaaaattg cagcatttgt aaacagtggt ggccatatcg 240
   gcctgcgtaa ttccttctga gcccgttctg caggcgggct tggtttactt tccgaggact 300
   ccag 304
<210> 124
   <211> 308
   <212> DNA
   <213> Acinetobacter haemolyticus
<400> 124
   tgctgtaggg agtcgatttt ttgaaaatcg gctccaaaaa atggctgatg gctcttgggt 60
   catcagcctt tttgcgttac aataatcggc tcgttttttg ttagttttgc gtttttgacg 120
   catataatta gaacaaacgg ctaaatatca aacgcttacc aatatttttt tgcttataaa 180
   aatattgtta agcgttttaa taccactaaa aattgcagca tttgtaaaca gtggtggcca 240
   tatcggcttg cgtaattcct tctaagcccg ttctgcaggc ggcttagttt actttccgag 300
   gactccag 308
<210> 125
   <211> 308
   <212> DNA
   <213> Acinetobacter junii
<400> 125
   ttctgtaggg agtcgatttt tttataatcg gctccaaaaa atggctgatg gctcttgggt 60
   catcagcctt tttgcgttac aataatcggc tcgttttttg ttagttttgc atattttgtg 120
   cagattttta tagaaaacaa ataaaatcaa atgcttacca atattttttt gcttataaaa 180
   atattgttaa gcgttttgat accactaaaa attgcagcat ttgtaaacag tggtggccat 240
   atcggcctgc gtaattcctt ctaggcccgt tctgcaggcg ggcttagttt actttccgag 300
   gactccag 308
<210> 126
   <211> 308
   <212> DNA
   <213> Acinetobacter sp.
<400> 126
   tactgtaggg agtcggtttt tttgataatc ggctccaaaa aatggctgat ggctcttggg 60
   tcatcagcct ttttgcgtta caataatcgg ctcgtttttt gttagtttcg catataatat 120
   gcgggttttt gcaataaaaa gactaatatc aaacgcttaa caatgttttt tgcttataaa 180
   aatattgtta agcgttttaa taccactaaa aattgcagca tttgtaaaca gtggtggcca 240
   tatcggcctg cgtaatcctt ctaggcccgt tctgcaggcg ggcttagttt acttcccgag 300
   gactccag 308
<210> 127
   <211> 301
   <212> DNA
   <213> Acinetobacter johnsonii
<400> 127
   tgttaccagg gggggaatat taatttcacc ccaaaaattg gctgatggct cttgggtcat 60
   cagccttttt gcgttacaat aatcggctcg atttttgttt gcttggtgga aaatccatca 120
   aattaaaata aagaaaagaa tcaaacgttt accaatattt ttcaaattta aaaatattgg 180
   taagcgtttc aataccactg aaaattgcag catttgtaaa aagtggtggc catatcggcc 240
   tgcgtaattc cttctgagcc cgttctgcag gcgggctcgg tttactttcc gaggactcca 300
   g 301
<210> 128
   <211> 308
   <212> DNA
   <213> Acinetobacter lwoffii
<400> 128
   tagcttaagg gagccggttt ttttataatc agctccaaaa attggctgat ggctcttggg 60
   tcatcagcct ttttgcgtta caataatcgg ctcgattttt gtttgcatga tataaaaatc 120
   atacaaatta aaaataaaca taatgaacaa acgtttacca atatttttca gtaaaaaaat 180
   attgataagc gttttaatac cactaaaaat tgcagcattt gtaaagagtg gtggccatat 240
   cggccagcgt aattccttct gagcccgttc tgcaggcggg cttggtttac actttccgag 300
   gactccag 308
<210> 129
   <211> 306
   <212> DNA
   <213> Acinetobacter sp.
<400> 129
   tttcttaagg gagccggttt tttataatcg gctccaaaaa ttggctgatg gctcttgggt 60
   catcagcctt tttgcgttac aataatcggc tcgatttttg tttgcatgat ataaaaatca 120
   tacaaattga aaataaacat aatgaacaaa cgtttaccaa tatttttcat taaaaaatat 180
   tgataagcgt tttaatacca ctaaaaattg cagcatttgt aaagagtggt ggccatatcg 240
   gccagcgtaa ttccttctga gcccgttctg caggcgggct tggtttacac tttccgagga 300
   ctccag 306
<210> 130
   <211> 307
   <212> DNA
   <213> Acinetobacter sp.
<400> 130
   tcttaagggg agtcgatttt tttataatcg gctccacaaa ttggctgatg gctcttgggt 60
   catcagcctt tttgcgttac aataatcggc tcgattttag attgattgat ataaaaatca 120
   tcaattttta aaataaataa acataatcaa acgcttacca atatttttca gttttaaaaa 180
   tattgttaag cgttttgtac cactgaaatt tgcagcattt gtataaaagt ggtggtcata 240
   tcgacctgcg taattccttc taagttcgtt ctgcaggcgg gcttagttta ctttccgagg 300
   actccag 307
<210> 131
   <211> 304
   <212> DNA
   <213> Acinetobacter sp.
<400> 131
   tttcgctagg ggtcgaattt taatttgacc ccaaaaattg gctgatggct cttgggtcat    60
   cagccttttt gcgttacaat aatcggctcg attttagatt gattggtaga aaaaccatca   120
   attttaaaaa taaataaaca caatcaaacg cttaccaata tttttccatt ttaaaaatat   180
   tgctaagcgt tttgtaccac tgaaatttgc agcatttgta taaaagtggt ggtcatatcg   240
   acctgcgtaa ttccttctaa gttcgttctg caggcggact tagtttactt tccgaggact   300
   ccag                                                                304
<210> 132
   <211> 304
   <212> DNA
   <213> Acinetobacter radioresistens
<400> 132
   ttctttaagg agtcgatttt ttaatcggct ccataaaatg gctgatggct cttgggtcat    60
   cagccttttt gcgttacaat aatcggctcg atttttgtta gtttgctgaa tatatcggtt   120
   aatttaataa taaatcaaat aaattcaaac gcttaccaat attttttgat ttgaaaatat   180
   tgataagcgt tttcatacca ctcaagattg cagcttttgt aaaaagtggt ggccatatcg   240
   gcctgcgtaa ttccttctga gcccgttctg caggcgggct tggtttactt tccgaggact   300
   ccag                                                                304
<210> 133
   <211> 309
   <212> DNA
   <213> Acinetobacter sp.
<400> 133
   tgctgtaggg agtcgatttt ttgaaaatcg gctccaaaaa atggctgatg gctcttgggt    60
   catcagcctt tttgcgttac aataatcggc tcgctttttg ttagttttac gtttagcgtg   120
   cgtattattt gaacaaaata catgaaatca aacgcttacc aatatttttt tgcttataaa   180
   aatattgtta agcgttttaa taccactaaa aattgcagca tttgtaaaca gtggtggcca   240
   tatcggcctg cgtaattcct tctagacccg ttctgcaggc gggtttagtt tactttccga   300
   ggactccag                                                           309
<210> 134
   <211> 309
   <212> DNA
   <213> Acinetobacter sp.
<400> 134
   ttctgtaggg agtcgatttt ttcaaaatcg gctccaaaaa atggctgatg gctcttgggt    60
   catcagcctt tttgcgttac aataatcggc tcgttttttg ttagttttgc atataatacg 120
   cgtgttattt gagcaaattt agcaaaatca aacgcttacc aatatttttt tgcttataaa 180
   aatattgtta agcgttttaa taccactaaa aattgcagca tttgtaaaca gtggtggcca 240
   tatcggcctg cgtaattcct tctaagcccg ttctgcaggc gggcttagtt tactttccga 300
   ggactccag 309
<210> 135
   <211> 310
   <212> DNA
   <213> acinetobacter schindleri
<400> 135
   tttcttaaag gagtcggttt ttttataatc ggctccaaaa attggctgat ggctcttggg 60
   tcatcagcct ttttgcgtta caataatcgg ctcgattttt gtttgcatga tagaaattca 120
   tacaaattta aaaaacaaac ataattgatc aaatgcttac caatattttt gcagtaaaaa 180
   atattgttaa gcgttttaat accactaaaa attgcagcat ttgtaaagag tggtggccat 240
   atcggccagc gtaattcctt ctgagcccgt tctgcaggcg ggcttagttt acactttccg 300
   aggactccag 310
<210> 136
   <211> 308
   <212> DNA
   <213> acinetobacter ursingii
<400> 136
   tgcatcaagg ggtcgatttt ttgataatcg gccccataaa atggctgatg gctcttgggt 60
   catcagcctt tttgcgttac aataatcggc tcgatttttg tttgtttgat ataaaaacgc 120
   tcgatttaaa gaacaaatga aataaattca aacgcttacc aatatttttc tggtttaaaa 180
   atattgttaa gcgttttaat accacttaaa aattgcagca tttgtaaaag tggtggccat 240
   atcggcctgc gtcattccat ccaagctcgt tctgcaggcg agcctggttt actttccgag 300
   gactccag 308
<210> 137
   <211> 304
   <212> DNA
   <213> acinetobacter baylyi
<400> 137
   tttcgctagg ggtcgaattt taatttgacc ccaaaaattg gctgatggct cttgggtcat 60
   cagccttttt gcgttacaat aatcggctcg attttagatt gattggtaga aaaaccatca 120
   attttaaaaa taaataaaca caatcaaacg cttagcaata tttttccatt ttaaaaatat 180
   tgctaagcgt tttgtaccac tgaaatttgc agcatttgta taaaagtggt ggtcatatcg 240
   acctgcgtaa ttccttctaa gttcgttctg caggcggact tagtttactt tccgaggact 300
   ccag 304
<210> 138
   <211> 305
   <212> DNA
   <213> acinetobacter bouvetii
<400> 138
   tcttaagagg agccgatttt tttataatcg gctccagaaa atggctgatg gctcttgggt 60
   catcagcctt tttgcgttac aataatcggc tcgatttttg tttgcatgat ataaaaatca 120
   tacaaattag aaatacagat aaatgtcaaa cgcttaccaa tatttttccg tttaaaaaat 180
   attgttaagc gttttaatac cactataaat tgcagcattt gtcaaagtgg tggccatatc 240
   ggcctgcgta attccttcta aacccgttct gcaggcgggt ttagtttact ttccgaggac 300
   tccag 305
<210> 139
   <211> 309
   <212> DNA
   <213> acinetobacter gerneri
<400> 139
   tcgctttatg gagtcgattt tttgtaatcg gctccaaaaa acggctgatg gctcttgggt 60
   catcagcctt tttgcgttac aataatcggc tcgattttta gattgatcga tagaataatc 120
   atcaatttta aaaataaata tacaaattca aacgcttacc aatatttttc cattttaaaa 180
   atattgttaa gcgttttata ccactgaaaa ttgcagcatt tgtataaagg tggtggtcat 240
   atcgacctgc gtaattcctt ctaagttcgt tctgcaggcg ggcttggtat tactttccga 300
   ggactccag 309
<210> 140
   <211> 308
   <212> DNA
   <213> acinetobacter grimontii
<400> 140
   ttctgtaggg agtcgatttt tttataatcg gctccaaaaa atggctgatg gctcttgggt 60
   catcagcctt tttgcgttac aataatcggc tcgttttttg ttagttttgc atattttgtg 120
   cagattttta tagaaaacaa ataaaatcaa atgcttacca atattttttt gcttataaaa 180
   atattgttaa gcgttttgat accactaaaa attgcagcat ttgtaaacag tggtggccat 240
   atcggcctgc gtaattcctt ctaggcccgt tctgcaggcg ggcttagttt actttccgag 300
   gactccag 308
<210> 141
   <211> 306
   <212> DNA
   <213> acinetobacter tandoii
<400> 141
   tcttaagagg agtcgatttt ttataatcgg ctccacaaaa tggctgatgg ctcttgggtc 60
   atcagccttt ttgcgttaca ataatcggct cgactccaat ttgactgatt taaaaatcat 120
   caaatttaaa caataaagac aaactttaaa cgcttaccaa tatttttcta ttcaaaaaat 180
   attgttaagc gttttaatac cactaaaaat tgcagcattt gtaaacagtg gtggccatat 240
   cggcctgcgt aattccttct aaacccgttc tgcaggcggg tttagtttac tttccgagga 300
   ctccag 306
<210> 142
   <211> 307
   <212> DNA
   <213> acinetobacter tjernbergiae
<400> 142
   tgtgtaggga gtcgattttt ttataatcgg ctccaaaaaa tggctgatgg ctcttgggtc 60
   atcagccttt ttgcgttaca ataatcggct cgttttttgt tagttttacg tttgagtgcg 120
   tgtttttcaa acaaataaca gaaaatcaaa cgcttaccaa tatttttttg cctataaaaa 180
   tattgttaag cgttttaata ccactaaaaa ttgcagcatt tgtaaacagt ggtggccata 240
   tcggcctgcg taattccttc taagcccgtt ctgcaggcgg gtttagttta ctttccgagg 300
   actccag 307
<210> 143
   <211> 307
   <212> DNA
   <213> acinetobacter towneri
<400> 143
   tcttaagggg agtcgatttt tttataatcg actccaaaaa atggctgatg gctcttgggt 60
   catcagcctt tttgcgttac aattatcggc tcgatttttg tttgaatgat gggaaaacca 120
   tcgaaaacaa tataaatcaa taaaatcaaa cgcttaccaa tatttttcca catttaaaaa 180
   tattgttaag cgttttaata ccactaaaaa ttgcagcatt tgtaaagagt ggtggccata 240
   tcggcctgcg taattccttc taaacccgtt ctgcaggcgg gtttagttta ctttccgagg 300
   actccag 307
<210> 144
   <211> 308
   <212> DNA
   <213> acinetobacter parvus
<400> 144
   tgctgtaggg agtcgatttt ttcaaaatcg gctccaaaaa atggctgatg gctcttgggt 60
   catcagcctt tttgcgttac aataatcggc tcgttttttg atagttttac gttcattgtg 120
   cgtattattt aaacaaaatt tatgaaatca aacgcttacc aatatttttt tgcttataaa 180
   aatattgtta agcgttttaa taccactaaa attgcagcat ttgtaaacag tggtggccat 240
   atcggcctgc gtaattcttt ctagacccgt tctgcaggcg ggtttagttt actttccgag 300
   gactccag 308
<210> 145
   <211> 305
   <212> DNA
   <213> Acinetobacter baumannii
<400> 145
   ttgtaaaagg agtcggtttt taaatcggct ccataaaatg gctgatggct cttgggtcat 60
   cagccatttt gcgttacaat aatcggctcg atttttgtta gttttgtttg gtttttagat 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 146
   <211> 305
   <212> DNA
   <213> Acinetobacter baumannii
<400> 146
   ttgtaaaagg agtcggtttt taaatcggct ccataaaatg gctgatggct cttgggtcat      60
   cagccttttt gcgttacaat aatcggctcg atttttgtta gttttgtttg gtttttagag     120
   aaagcctaaa taaaataaat aaattcaaat gcttaccaat atttttcatt tttaaaaata     180
   ttgttaagcg tttttatacc actaaaaatt gcagcatttg taaacagtgg tggccatatc     240
   ggcctgcgta attccttctg agcccgttct gcaggcgggc ttggtttact ttccgaggac     300
   tccag                                                                 305
<210> 147
   <211> 305
   <212> DNA
   <213> Acinetobacter baumannii
<400> 147
   ttgtaaaagg agtcggtttt taaatcggct ccataaaatg gctgatggct cttgggtcat      60
   cagccttttt gcgttacaat aatcggctcg atttttgtta gttttgtttg gtttttagag     120
   aaagcctaaa taaaataaat aaattcaaat gcttaccaat atttttcatt tttaaaaata     180
   ttgttaagcg tttttatacc actaaaaatt gcagcatttg taaacagtgg tggccatatc     240
   ggcctgcgta attccttctg agcccgttct gcaggcgggc ttggtttact ttccgaggac     300
   tccag                                                                 305
<210> 148
   <211> 305
   <212> DNA
   <213> Acinetobacter baumannii
<400> 148
   ttgtaaaagg agtcggtttt taaatcggct ccataaaatg gctgatggct cttgggtcat      60
   cagccttttt gcgttacaat aatcggctcg atttttgtta gttttgtttg gtttttagag     120
   aaagcctaaa taaaataaat aaattcaaat gcttaccaat atttttcatt tttaaaaata     180
   ttgttaagcg tttttatacc actaaaaatt gcagcatttg taaacagtgg tggccatatc     240
   ggcctgcgta attccttctg agcccgttct gcaggcgggc ttggtttact ttccgaggac     300
   tccag                                                                 305
<210> 149
   <211> 305
   <212> DNA
   <213> Acinetobacter baumannii
<400> 149
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 150
   <211> 305
   <212> DNA
   <213> Acinetobacter baumannii
<400> 150
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 151
   <211> 305
   <212> DNA
   <213> Acinetobacter baumannii
<400> 151
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 152
   <211> 305
   <212> DNA
   <213> Acinetobacter baumannii
<400> 152
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 153
   <211> 305
   <212> DNA
   <213> Acinetobacter baumannii
<400> 153
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 154
   <211> 305
   <212> DNA
   <213> Acinetobacter baumannii
<400> 154
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 155
   <211> 305
   <212> DNA
   <213> Acinetobacter baumannii
<400> 155
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 156
   <211> 305
   <212> DNA
   <213> Acinetobacter baumannii
<400> 156
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 157
   <211> 305
   <212> DNA
   <213> Acinetobacter baumannii
<400> 157
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 158
   <211> 305
   <212> DNA
   <213> Acinetobacter baumannii
<400> 158
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 159
   <211> 305
   <212> DNA
   <213> Acinetobacter baumannii
<400> 159
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 160
   <211> 305
   <212> DNA
   <213> Acinetobacter baumannii
<400> 160
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agctcgttct gcaggcgggc ttggtttact ttccgaggac 300
   tccag 305
<210> 161
   <211> 305
   <212> DNA
   <213> Acinetobacter baumannii
<400> 161
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 162
   <211> 305
   <212> DNA
   <213> Acinetobacter baumannii
<400> 162
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 163
   <211> 304
   <212> DNA
   <213> Acinetobacter baumannii
<400> 163
   ttgtaaaagg agtcggtttt taaatcggct ccataaaatg gctgatggct cttgggtcat 60
   cagccttttt gcgttacaat aatcggctcg atttttgtta gttttgtttg atttttagaa 120
   aaatctaaat aaaacaagaa atttcaaatg tttaccaata tttttctttt ttaaaaatat 180
   tgttaagcgt ttttatacca ctaaaaattg cagcatttgt aaacagtggt ggccatatcg 240
   gcctgcgtaa ttccttctga gcccgttctg caggcgggct tagtttactt tccgaggact 300
   ccag 304
<210> 164
   <211> 305
   <212> DNA
   <213> Acinetobacter baumannii
<400> 164
   ttgtaaaagg agtcggtttt taaatcggct ccataaaatg gctgatggct cttgggtcat 60
   cagccttttt gcgttacaat aatcggctcg atttttgtta gttttgtttg gtttttagag 120
   aaagcctaaa taaaataaat aaattcaaat gcttaccaat atttttcatt tttaaaaata 180
   ttgttaagcg tttttatacc actaaaaatt gcagcatttg taaacagtgg tggccatatc 240
   ggcctgcgta attccttctg agcccgttct gcaggcgggc ttggtttact ttccgaggac 300
   tccag 305
<210> 165
   <211> 86
   <212> DNA
   <213> Acinetobacter calcoaceticus
<400> 165
   gtcgtaagac tcacgatttt ttaggaaaaa acaatatttg tgacccagtc gggtaagcgt 60
   tgcttcccga cacacggaga aaaaaa 86
<210> 166
   <211> 86
   <212> DNA
   <213> Acinetobacter baumannii
<400> 166
   gtcgtaagac tcacgatttt ttaggtaaaa acaatatttg tgacccagtc gggtaagcga 60
   ggcttcccga cacacggaga aaaaaa 86
<210> 167
   <211> 86
   <212> DNA
   <213> Acinetobacter sp.
<400> 167
   gtcgtaagac tcacgatttt ttaggtaaaa acaatatttg tgacccagtc gggtaagcat 60
   tgcttcccga cacacggaga aaaaaa 86
<210> 168
   <211> 172
   <212> DNA
   <213> Acinetobacter haemolyticus
<400> 168
   ttagccatta atcccccctc agcccccttt atgaaagggg gagttcccac tcgaaagagt 60
   aggggcttcc ctcctcagaa aaaggaggga ctgagggagg attattcagt taaaaaacaa 120
   tatttgtgac ccagttgagt gagtgtagct cctcgacaca cggagaaaaa aa 172
<210> 169
   <211> 149
   <212> DNA
   <213> Acinetobacter junii
<400> 169
   ttagccatta attctcctag tttccactcg aaaagggtgg gggcttccct cctgagaaaa    60
   ggagaggttg agggagggtt gttcagttaa aaaacaatat ttgtgaccca gtcaggtaag   120
   cctagcttcc tgacacacgg agaaaaaaa                                     149
<210> 170
   <211> 170
   <212> DNA
   <213> Acinetobacter sp.
<400> 170
   ttagccatta atcccccgag cccccttgac gaaaggggga gttcccactt gaaagggtag    60
   gagcttccct ccttagaaaa aagagggact gagggaggat tattcagtta aaaaacaata   120
   tttgtgaccc agtcgggcga gcttagctcc ctgacacacg gagaaaaaaa             170
<210> 171
   <211> 141
   <212> DNA
   <213> Acinetobacter johnsonii
<400> 171
   gtttctccat tgaatctccc caacctctct tgttcaaaga gaggcttcct cgaaaggtaa    60
   gcggggagat ttctcagtta aaaaacaata tttgtgaccc agttgtgtag tgaaagtctc   120
   cacaacacac ggagaaaaaa a                                             141
<210> 172
   <211> 177
   <212> DNA
   <213> Acinetobacter lwoffii
<400> 172
   ttagttccat tgaatctccc ttgcgaagca gtgcttctca cctctttaag aaagagggga    60
   aagtccctct ttataaaggg ggatttaggg ggattcttgg gattcccaat tcagttaaaa   120
   acaatatttg tgacccagtc gagaagtgaa aatctcctcg acacacggag aaaaaaa      177
<210> 173
   <211> 150
   <212> DNA
   <213> Acinetobacter sp.
<400> 173
   ttagttccat ttaatccctc caacccctct ttgataaaga gggcttcccc cttaacaaag 60
   ggggactgag ggggattaaa attcagttaa aaaacaatat ttgtgaccca gttgttgagt 120
   gaaaatctcc acaacacacg gagaaaaaaa 150
<210> 174
   <211> 144
   <212> DNA
   <213> Acinetobacter sp.
<400> 174
   atcccccttg atcccccttt gttaaagggg ggatttccct tctttcttaa agagggggca 60
   ggggagattc caaatctcag tgaaaaaaca atatttgtga cccagccgta gagaaaatgc 120
   ctcttcggca cacggagaaa aaaa 144
<210> 175
   <211> 154
   <212> DNA
   <213> Acinetobacter sp.
<400> 175
   acttcaccat tgaatctccc taaatccctc tttatgaaag agggactttc ccccttagaa 60
   aaagggggat caagggggat taaaattcag tgaaaaaaca atatttgtga cccagtcgta 120
   gcctgaaaag cgcttcgaca cacggagaaa aaaa 154
<210> 176
   <211> 89
   <212> DNA
   <213> Acinetobacter radioresistens
<400> 176
   ggtctaggac ccttcacctt ttcaggtaaa aacaatattt gtgacccagc cgggcgggca 60
   atgtgccccc cggtacacgg agaaaaaaa 89
<210> 177
   <211> 154
   <212> DNA
   <213> Acinetobacter sp.
<400> 177
   aaaatctccc ccaacccctc tttaagaaag aggggagaaa gtcctccttt gtaaaggggg 60
   atttaggggg gattatagat tcccaaattt cagtaaaaac aatatttgtg acccagtcgg 120
   gtgagcttag cttcccgaca cacggagaaa aaaa 154
<210> 178
   <211> 153
   <212> DNA
   <213> Acinetobacter sp.
<400> 178
   aaaatctccc ccaacccctc tttgagaaag aggggagaaa gtcccccttt ataaaggggg 60
   atttaggggg attagatatt cccaaatttc agtaaaaaca atatttgtga cccagtcggg 120
   tgagcttagc tcctcgacac acggagaaaa aaa 153
<210> 179
   <211> 159
   <212> DNA
   <213> acinetobacter schindleri
<400> 179
   gaaatctccc ctgacccctc tttgagaaag aggggagaag tccccccctt ttttaaaggg 60
   ggatttaggg ggattcttaa aattcccaaa tttcagtaaa aaacaatatt tgtgacccag 120
   ttgttgagtg aaaatctcca caacacacgg agaaaaaaa 159
<210> 180
   <211> 136
   <212> DNA
   <213> acinetobacter ursingii
<400> 180
   atccccctcg attccccttt gttaaagggg gcccctcttt tcaaagaggg gttagaggag 60
   attttcaaat ttcagtaaaa acaatatttg tgacccagtc ggatgagcct agctctctga 120
   cacacggaga aaaaaa 136
<210> 181
   <211> 88
   <212> DNA
   <213> acinetobacter baylyi
<400> 181
   gcgttaagct gactcagatt tttcaggaaa aacagtattt gtgacccagc cgagtgagcg 60
   aatgctcctc ggtatacgga gaaataaa 88
<210> 182
   <211> 156
   <212> DNA
   <213> acinetobacter bouvetii
<400> 182
   gaaatctccc ccgacccctc tttgaaaaag aggggagaaa atcccccttt ataaaggggg      60
   atttaggggg attttcaaga ttccagaaat tcagtaaaaa caatatttgt gacccagttg 120
   tgaagtgaaa atctccacaa cacacggaga aaaaaa 156
<210> 183
   <211> 170
   <212> DNA
   <213> acinetobacter gerneri
<400> 183
   ttaaccatta atccccccaa ccccctttga aaagggggag gtccagcact taaatagttg 60
   caacctccct cctttattaa aggagggatt gagggaggat tctcagttaa aaaacaatat 120
   ttgtgaccca gtcgagaagt gaaaatctcc tcgacgcacg gagaaaaaaa 170
<210> 184
   <211> 150
   <212> DNA
   <213> acinetobacter grimontii
<400> 184
   ttagccatta attctcctag tttccactcg aaaagggtgg gggcttccct cctgagaaaa 60
   ggaggggttg agggagggtt gttcagttaa aaaaacaata tttgtgaccc agtcaggtaa 120
   gcctagcttc ctgacacacg gagaaaaaaa 150
<210> 185
   <211> 156
   <212> DNA
   <213> acinetobacter tandoii
<400> 185
   gaaatctccc ccgatccctc tttaagaaag aggggagaag tcccccttag taaaggggga      60
   tttaggggga ttttttgaga ttccctattc agtgaaaaaa caatatttgt gacccagtcg 120
   agaagtgaaa atctcctcga cacacggaga aaaaaa 156
<210> 186
   <211> 143
   <212> DNA
   <213> acinetobacter tjernbergiae
<400> 186
   atccccctcg atcccccttt gttaaagggg gaagttcccc tctttttcaa agaggggtta 60
   ggggagattt cataatttca gtaaaaaaca atatttgtga cccagtcggg tgagcgtagc 120
   tccccgacac acggagaaaa aaa 143
<210> 187
   <211> 157
   <212> DNA
   <213> acinetobacter towneri
<400> 187
   tcatctccat taaatctccc tagatccctc tttataaaag agggactttt ccccctttga 60
   aaaaggggga ttaaggggga ttaaaactca gttaaaaaac aatatttgtg acccagttgg 120
   gatgtgaaaa cagcctccca gcacacggag aaaaaaa 157
<210> 188
   <211> 143
   <212> DNA
   <213> acinetobacter parvus
<400> 188
   atccccctcg ttcccccttt aaaaaagggg ggaagttccc ctctttgaaa agaggggcta      60
   ggcggagatt ccataatttc agtaaaaaca atatttgtga cccagtcgga agagcgtagc 120
   tccccgacac acggagaaaa aaa 143
<210> 189
   <211> 86
   <212> DNA
   <213> Acinetobacter baumannii
<400> 189
   gtcgtaagac tcacgatttt ttaggtaaaa acaatatttg tgacccagtc gggtaagcga 60
   ggcttcccga cacacggaga aaaaaa 86
<210> 190
   <211> 86
   <212> DNA
   <213> Acinetobacter baumannii
<400> 190
   gtcgtaagac tcacgatttt ttaggtaaaa acaatatttg tgacccagtc gggtaagcga 60
   gccttcccga cacacggaga aaaaaa 86
<210> 191
   <211> 86
   <212> DNA
   <213> Acinetobacter baumannii
<400> 191
   gtcgtaagac tcacgatttt ttaggtaaaa acaatatttg tgacccagtc gggtaagcga 60
   ggcttcccga cacacggaga aaaaaa 86
<210> 192
   <211> 86
   <212> DNA
   <213> Acinetobacter baumannii
<400> 192
   gtcgtaagac tcacgatttt ttaggtaaaa acaatatttg tgacccagtc gggtaagcga 60
   ggcttcccga cacacggaga aaaaaa 86
<210> 193
   <211> 86
   <212> DNA
   <213> Acinetobacter baumannii
<400> 193
   gtcgtaagac tcacgatttt ttaggtaaaa acaatatttg tgacccagtc gggtaagcga 60
   ggcttcccga cacacggaga aaaaaa 86
<210> 194
   <211> 86
   <212> DNA
   <213> Acinetobacter baumannii
<400> 194
   gtcgtaagac tcacgatttt ttaggtaaaa acaatatttg tgacccagtc gggtaagcga 60
   ggcttcccga cacacggaga aaaaaa 86
<210> 195
   <211> 86
   <212> DNA
   <213> Acinetobacter baumannii
<400> 195
   gtcgtaagac tcacgatttt ttaggtaaaa acaatatttg tgacccagtc gggtaagcga 60
   ggcttcccga cacacggaga aaaaaa 86
<210> 196
   <211> 86
   <212> DNA
   <213> Acinetobacter baumannii
<400> 196
   gtcgtaagac tcacgatttt ttaggtaaaa acaatatttg tgacccagtc gggtaagcga 60
   ggcttcccga cacacggaga aaaaaa 86
<210> 197
   <211> 86
   <212> DNA
   <213> Acinetobacter baumannii
<400> 197
   gtcgtaagac tcacgatttt ttaggtaaaa acaatatttg tgacccagtc gggtaagcga 60
   ggcttcccga cacacggaga aaaaaa 86
<210> 198
   <211> 86
   <212> DNA
   <213> Acinetobacter baumannii
<400> 198
   gtcgtaagac tcacgatttt ttaggtaaaa acaatatttg tgacccagtc gggtaagcga 60
   ggcttcccga cacacggaga aaaaaa 86
<210> 199
   <211> 86
   <212> DNA
   <213> Acinetobacter baumannii
<400> 199
   gtcgtaagac tcacgatttt ttaggtaaaa acaatatttg tgacccagtc gggtaagcga 60
   ggcttcccga cacacggaga aaaaaa 86
<210> 200
   <211> 86
   <212> DNA
   <213> Acinetobacter baumannii
<400> 200
   gtcgtaagac tcacgatttt ttaggtaaaa acaatatttg tgacccagtc gggtaagcga 60
   ggcttcccga cacacggaga aaaaaa 86
<210> 201
   <211> 86
   <212> DNA
   <213> Acinetobacter baumannii
<400> 201
   gtcgtaagac tcacgatttt ttaggtaaaa acaatatttg tgacccagtc gggtaagcga 60
   ggcttcccga cacacggaga aaaaaa 86
<210> 202
   <211> 86
   <212> DNA
   <213> Acinetobacter baumannii
<400> 202
   gtcgtaagac tcacgatttt ttaggtaaaa acaatatttg tgacccagtc gggtaagcga 60
   ggcttcccga cacacggaga aaaaaa 86
<210> 203
   <211> 86
   <212> DNA
   <213> Acinetobacter baumannii
<400> 203
   gtcgtaagac tcacgatttt ttaggtaaaa acaatatttg tgacccagtc gggtaagcga 60
   ggcttcccga cacacggaga aaaaaa 86
<210> 204
   <211> 86
   <212> DNA
   <213> Acinetobacter baumannii
<400> 204
   gtcgtaagac tcacgatttt ttaggtaaaa acaatatttg tgacccagtc gggtaagcga 60
   ggcttcccga cacacggaga aaaaaa 86
<210> 205
   <211> 86
   <212> DNA
   <213> Acinetobacter baumannii
<400> 205
   gtcgtaagac tcacgatttt ttaggtaaaa acaatatttg tgacccagtc gggtaagcga 60
   ggcttcccga cacacggaga aaaaaa 86
<210> 206
   <211> 86
   <212> DNA
   <213> Acinetobacter baumannii
<400> 206
   gtcgtaagac tcacgatttt ttaggtaaaa acaatatttg tgacccagtc gggtaagcga 60
   ggcttcccga cacacggaga aaaaaa 86
<210> 207
   <211> 86
   <212> DNA
   <213> Acinetobacter baumannii
<400> 207
   gtcgtaagac tcacgatttt ttaggaaaaa acaatatttg tgacccagtc gggtaagcga    60
   ggcttcccga cacacggaga aaaaaa                                          86
<210> 208
   <211> 86
   <212> DNA
   <213> Acinetobacter baumannii
<400> 208
   gtcgtaagac tcacgatttt ttaggtaaaa acaatatttg tgacccagtc gggtaagcga    60
   ggcttcccga cacacggaga aaaaaa                                          86